# EUROPEAN PATENT SPECIFICATION

(11) **EP 1 203 770 B1**
(45) Date of publication and mention of the grant of the patent: **09.03.2005**
(21) Application number: 02002073.1
(22) Date of filing: 09.11.1993
(51) Int. Cl.: C07D 309/38, C07D 309/32, A61K 31/365, A61P 31/18

(54) **Pyran-2-ones and 5,6-dihydropyran-2-ones useful for treating hyperplasia and other diseases**
Pyran-2-onen und 5,6-dihydropyran-2-onen brauchbar für die Behandlung von Hyperplasie und andere Krankheiten
Pyrannes 2-ones et 5,6-dihydropyrannes 2-ones utiles pour traiter l'hyperplasie et autres affections

(30) Priority: 13.11.1992 US 975343; 13.07.1993 US 90876; 01.10.1993 US 130641
(43) Date of publication of application: 08.05.2002
(62) Divisional of application: 94900546.6
(73) Proprietor: PHARMACIA & UPJOHN COMPANY, Kalamazoo, Michigan 49001 (US)
(72) Inventor: Thaisrivongs, Suvit, Portage, MI 49002 (US); Yang, Chih-Ping, Neihu, Taipei (TW); Strohbach, Joseph Walter, Mendon, MI 49072 (US); Turner, Stephen Ronald, Kalamazoo, MI 49001 (US); Romero, Donna Lee, Kalamazoo, MI 49008 (US); Skaletzki, Louis L., Kalamazoo, MI 49008 (US); Aristoff, Paul Adrian, Kalamazoo, MI 49002 (US); Gammill, Ronald B., Kalamazoo, MI 49002 (US); Johnson, Paul D., Portage, MI 49002 (US); Skulnick, Harvey Irving, Kalamazoo, MI 49009 (US); Piper, Richard C., Kalamazoo, MI 49001 (US); Tommasi, Ruben A., Kalamazoo, MI 49001 (US); Zhang, Qingwei, Kalamazoo, MI 49007 (US)
(74) Representative: Perry, Robert Edward

(56) References cited:
- FR-A- 1 276 654
- KL. REHSE: "ANTICOAGULANTE 3-ARALKYL-4-HYDROXY-2-PYRONE." ARCH.PHARM., vol. 316, no. 12, 1983, pages 988-94, XP001064366 WEINHWEIM

## Description

### FIELD OF THE INVENTION

The present invention relates to pyran-2-ones and 5,6-dihydropyran-2-ones useful for treating hyperplasia and other diseases.

### BACKGROUND OF THE INVENTION

It is well known in the art that certain undesirable physiological manifestations, such as acne vulgaris, seborrhea, female hirsutism, male pattern baldness and benign prostatic hypertrophy, are the result of hyperandrogenic stimulation caused by an excessive accumulation of testosterone or related active hormones in the target tissue; see US-A-4377584, col. 1, lines 18-24. Additionally, the reduction of androgen levels has been shown to have a therapeutic effect on prostate cancer; see, e.g. US-A-5017568, col. 2, lines 4-6.

It is also known in the art that the principal mediator of androgenic activity in some target organs is 5α-dihydrotestosterone (and related 5α-reduced androgens), and that it is formed locally in the target organ by the action of steroid-5α-reductase. Inhibitors of steroid-5α-reductase prevent or reduce symptoms of hyperandrogenic stimulation. See, e.g. US-A-4377584, col. 1, lines 38-45.

Examples of compounds that are antiandrogens, by virtue of their ability to inhibit testosterone-5α-reductase, are disclosed in US-A-4377584; US-A-4760071; and US-A-5017568.

Finasteride [also named N-(1,1-dimethylethyl)-3-oxo-4-aza-5α-androst-1-ene, 17β-carboxamide] (PROSCAR®) is a 5α-reductase inhibitor which is used to reduce the prostate size in men. It has recently been approved by the FDA for the treatment of benign prostatic hyperplasia.

US-A-4377584 and US-A-4760071 disclose that 4-aza-17β-substituted-5α-androstan-3-ones, specifically the compound 17β-(N-t-butylcarbamoyl)-4-aza-5α-androst-1-en-3-one, and their A-homo analogs, are active as testosterone 5α-reductase inhibitors, and thus useful topically for treatment for acne, seborrhea and female hirsutism, and systematically in treatment of benign prostatic hypertrophy. See also Journal of Andrology, 10:259-262 (1989); J. Steroid. Biochem. Molec. Biol., Vol. 44, No. 2, pp. 121-131 (1993).

US-A-5017568 discloses substituted acrylate analogues of steroidal synthetic compounds having 5α-reductase inhibitor activity, and which are therefore useful in treating diseases such as acne vulgaris, seborrhea, female hirsutism, prostate diseases such as benign prostatic hypertrophy and prostatic adenocarcinoma, and male pattern baldness.

A selective, non-steroidal inhibitor of human steroid 5α-reductase type 1 is LY 191704 [8-chloro-4-methyl-1,2,3,4,4α,5,6,10b-octahydro-benzo[*f*]quinolin-3(2H)-one]; Proc. Natl. Acad. Sci. USA, Vol. 90:5277-5281 (1993).

FR-A-1276654 discloses 4-hydroxy-2-pyrones and their use as antifungal agents and anticoagulants.

### SUMMARY OF THE INVENTION

According to the present invention, a compound of formula I as defined in claim 1 is used to prepare a medicament for preventing or treating benign prostatic hypertrophy or hyperplasia, prostatic cancer, alopecia, hirsutism, acne vulgaris or seborrhea, in a mammal in need of such prevent or treatment. Preferred compounds for use in the invention are defined in claims 2 to 5.

### DESCRIPTION OF THE INVENTION

The compounds of the present invention are named according to the IUPAC or CAS nomenclature system.

The carbon atom content of various hydrocarbon-containing moieties is indicated by a prefix designating the minimum and maximum number of carbon atoms in the moiety, i.e. the prefix Cᵢ-Cⱼ indicates a moiety of the integer "i" to the integer "j" carbon atoms, inclusive. Thus, for example, C₁-C₃ alkyl refers to alkyl of one to three carbon atoms, inclusive, or ethyl, ethyl, propyl and isopropyl.

Alkyl of one to nine carbon atoms means methyl, ethyl, propyl, butyl, pentyl, hexyl, heptyl, octyl, nonyl and all isomeric forms thereof, straight and branched.

Alkenyl of up to five carbon atoms means ethenyl, propenyl, butenyl, pentenyl and all isomeric forms thereof.

Examples of "halo" or "halogen" are fluoro, chloro and bromo.

By "amino acid residue,, is meant the residue of a naturally occurring amino acid, such as: Alanine, Arginine, Asparagine, Aspartic acid, Cysteine, Glutamine, Glutamic Acid, Glycine, Histidine, Isoleucine, Leucine, Lysine, Methionine, Phenylalanine, Proline, Serine, Threonine, Tryptophan, Tyrosine, Valine, Aspartic acid or Asparagine, and synthetic derivatives thereof. These amino acid residues may be in the L- or D- configuration and are readily known and available to those skilled in the art. These amino acid residues (or their N-terminal protected forms) are connected to a free amino group via their C-terminus.

Compounds of formula I are prepared as described in the Preparations and Examples below, or are prepared by methods analogous thereto, which are readily known and available to one of ordinary skill in the art of organic synthesis.

### CHART A

The preparation of 6-aryl-4-hydroxy-2-pyrone (such as A-4:X is CH) and 3-alkylated-6-aryl-4-hydroxy-2-pyrone (such as A-5: X is CH, R is ethyl) are shown in Chart A. Deprotonation of ethyl acetoacetate of formula A-1, which is commercially available, with potassium hydride and n-butyl lithium in tetrahydrofuran, followed by addition of ethyl benzoate, which is the compound of formula A-2 (wherein X is CH) affords ethyl 5-phenyl-3,5-dioxopentanoate of formula A-3 (wherein X is CH). Heating the compound of formula A-3 at 120°C under reduced pressure (1 mm Hg, neat) affords 4-hydroxy-6-phenyl-2-pyrone, the compound of formula A-4 (wherein X is CH). Alkylation of the compound of formula A-4 by heating with a number of the corresponding substituted benzyl bromides or treatment of the compound of formula A-4 with the corresponding substituted benzyl alcohols in the presence of boron trifluoride-diethyl ether results in the desired product of formula A-5 (wherein X is CH and R is ethyl), which is the compound: 3-(.alpha.-ethylbenzyl)-4-hydroxy-6-phenyl-2H-pyran-2-one.

### CHART B

The preparation of the compound of formula B-5 (wherein n is 1) which is the compound: 6-benzyl-3-(.alpha.-ethylbenzyl)-4-hydroxy-2H-pyran-2-one, and the compound of formula B-5 (wherein n is 2), which is the compound: 3-(.alpha.-ethylbenzyl)-6-phenethyl-4-hydroxy-2H-pyran-2-one, are shown in Chart B. The dianion of formula B-1 is generated by the same conditions as described in Chart A. Either ethyl phenylacetate of formula B-2 (wherein n is 1) or ethyl dihydrocinnamate of formula B-2 (wherein n is 2) is then added to give ethyl 6-phenyl-3,5-dioxohexanoate of formula B-3 (wherein n is 1) or ethyl 7-phenyl-3,5-dioxoheptanoate of formula B-3 (wherein n is 2), respectively. Formation of the pyrone ring in the compound of formula B-4 (wherein n is 1 or 2) is accomplished by heating the compound of formula B-3 (wherein n is 1 or 2, respectively) under reduced pressure. Heating of the compound of formula B-4 (wherein n is 1 or 2) with (±)-1-bromo-1-phenylpropane or treatment with (±)1-phenylpropanol in the presence of boron trifluoride in dioxane gives the desired products of formula B-5 (wherein n is 1 or 2, respectively).

### CHART C

The desired product of formula C-4, which is the compound: 4-hydroxy-6-phenethyl-3-(.alpha.-propyl-p-bromobenzyl)-2H-pyran-2-one, is obtained by heating the pyrone of formula C-3 (prepared in Chart B as the compound of formula B-4 (wherein n is 2)) with the compound of formula C-2 (wherein R is bromide). The requisite compound of formula C-2 (wherein R is bromide) is obtained by a two-step sequence starting from 4-bromobenzaldehyde, the compound of formula C-1. Treatment of the compound of formula C-1 with propylmagnesium bromide affords 1-(4'-bromophenyl)-1-butanol, the compound of formula C-2 (wherein R is OH). The resulting alcohol, the compound of formula C-2 (wherein R is OH), is then treated with 48% hydrobromic acid to give the desired bromide of formula C-2 (wherein R is bromide).

### CHART D

*O*-allylation of 4-hydroxy-6-methyl-2-pyrone, the compound of formula D-1, with cinnamyl bromide is achieved to yield the compound of formula D-2. Claisen rearrangement of the resulting pyrone of formula D-2 is carried out in refluxing toluene to give the vinyl analogue of the formula D-3. Claisen product of formula D-3 is subjected to catalytic hydrogenation to afford the compound of formula D-4. Treatment of the compound of formula D-4 with two equivalents of lithium diisopropylamide in tetrahydrofuran followed by the addition of electrophile, such as benzyl bromide, gives the product of formula D-5, which is the compound: 3-(.alpha.-ethylbenzyl)-6-phenethyl-4-hydroxy-2H-pyran-2-one. This is the preferred process for preparing this compound.

### CHART E

A variety of analogues may be prepared by employing similar conditions which are used in the preparation of the compound of formula D-5 of Chart D. Under these conditions, 4-bromobenzyl bromide, 2-fluorobenzyl bromide, or allyl bromide are reacted with the dianion of formula E-1 (prepared in Chart D as the compound of formula D-4) rapidly to yield the compound of formula E-2 (wherein R is 4-bromobenzyl, 2-fluorobenzyl or 3-propylene), which are the compounds: 6-(p-bromophenethyl)-3-(.alpha.-ethylbenzyl)-4-hydroxy-2H-pyran-2-one; 3-(.alpha.-ethylbenzyl)-6-(o-fluorophenethyl)-4-hydroxy-2H-pyran-2-one; and 3-(.alpha.-ethylbenzyl)-4-hydroxy-6-(3-butenyl)-2H-pyran-2-one, respectively. However, reacting iodoethane and phenylethyl bromide with the compound of formula E-1 would require stirring the reaction mixture at room temperature for a few hours to yield the compound of formuala E-2 (wherein R is ethyl or phenylethyl), which are the compounds: 3-(.alpha.-ethylbenzyl)-4-hydroxy-6-propyl-2H-pyran-2-one and 3-(.alpha.-ethylbenzyl)-4-hydroxy-6-(3-phenylpropyl)-2H-pyran-2-one, respectively.

### CHART F

Treatment of the compound of formula F-1 (prepared as the compound of formula D-4 in Chart D) with 2 eq. of lithium diisopropylamide and tetrahydrofuran at -40°C, followed by the addition of phenyl isocyanate yields the compound of formula F-2, which is the compound: 3-(.alpha.-ethylbenzyl)-4-hydroxy-6-[[(phenylamino)carbonyl]methyl]-2H-pyran-2-one.

### CHART G

The preparation of the compound of formula G-2, which is the compound: 4-hydroxy-6-phenethyl-3-(.alpha.-vinylbenzyl)-2H-pyran-2-one, is achieved by the direct alkylation of the compound of formula G-1 (prepared as the compound of formula D-3 in Chart D) with benzyl bromide.

### CHART H

Commercially available 6-methyl-4-hydroxy-2H-pyran-2-one of formula H-1 is reacted with commercially available α-ethylbenzyl alcohol of formula H-2 with acid catalyst to give the compound of formula H-3.

Treatment of the compound of formula H-3 (may also be prepared as the compound of formula D-4 in Chart D) with three equivalents of lithium diisopropylamide in tetrahydrofuran, followed by the sequential addition of benzyl bromide and ethyl iodide gives the compound of formula H-4 (wherein R₁ is benzyl and R₂ is ethyl) which is the compound: 3-(.alpha.-ethylbenzyl)-6-(.alpha.ethylphenethyl)-4-hydroxy-2H-pyran-2-one, Under similar conditions, the compound of formula H-4 (wherein R₁ is ethyl and R₂ is ethyl) which is the compound: 3-(.alpha.-ethylbenzyl)-1-ethylpropyl-4-hydroxy-2H-pyran-2-one is obtained by employing two equivalents of ethyl iodide as electrophile.

### CHART I

Alkylation of the anion of methylacetoacetate of formula I-1, which is commercially available, after treatment with sodium hydride, with α-ethylbenzyl bromide of formula I-2, which is commercially available, gives methyl 2-(α-ethylbenzyl)-acetoacetate of formula I-3. The corresponding dianion, which is generated from sequential treatment with sodium hydride and *n*-butyllithium, adds to propiophenone of formula I-4, which is commercially available, to give methyl 2-(α-ethylbenzyl)-5-hydroxy-3-oxo-5-phenyl-heptanoate of formula I-5. The methyl ester is then hydrolyzed with sodium hydroxide, and upon acidification with hydrochloric acid, the desired material, 6-ethyl-3-(α-ethylbenzyl)-6-phenyl-tetrahydropyran-2,4-dione of formula I-6, is isolated.

### CHART J

Alkylation of the anion of methylacetoacetate of formula J-1 (also used in Chart I as the compound of formula I-1), which is commercially available, after treatment with sodium hydride, with 1-bromo-3-phenylpropane formula J-2, which is commercially available, gives methyl 2-(3-phenylpropyl)-acetoacetate of formula J-3. The corresponding dianion, which is generated from sequential treatment with sodium hydride and *n*-butyllithium, adds to 3-pentanone of formula J-4, which is commercially available, to give methyl 2-(3-phenylpropyl)-5-ethyl-5-hydroxy-3-oxo-heptanoate of formula J-5. The methyl ester is then hydrolyzed with sodium hydroxide, and upon acidification with hydrochloric acid, the desired material, 6,6-diethyl-3-(3-phenylpropyl)-tetrahydropyran-2,4-dione of formula J-6, is isolated.

### CHART K

Chart K offers an alternative highly effective way to introduce C-3 substituents on the 4-hydroxy-α-pyrone and 5,6-dihydro-4-hydroxy-2*H*-pyran-2-one nucleus. Allylic carbonates of formula K-2 (a, b and c) and nucleophile 5,6-dihydro-4-hydroxy-2-pyrones of formula K-1 (a, b and c) are treated with palladium acetate and triphenylphosphine in toluene at 50°C to yield compounds of formula K-3 (a, b and c). Representative examples of this reaction follow in Chart K. Also, it is possible to employ allylic carbonates containing substitution in the phenyl ring (Chart K, eq. a,b). In cases where diastereomeric products are possible (Chart K, eq. a,b), chromatographically inseparable mixtures are formed. The final products are obtained via desilylation and reduction. In some cases, it is possible to isolate one diastereomer of the product via successive recystallation. The mother liquors. contain a mixture enriched in the other diastereomer, but attempts to purify them via further recrystallizations may fail. Subsequent chemical manipulations on the substituents at the 6-position of the 5,6-dihydropyran-2-one ring are performed by those skilled in the art.

### CHART L

Chart L describes a generic procedure for the synthesis of C-3 α-branched substituted 4-hydroxy-2-pyrones or 4-hydroxy-5,6-dihydropyran-2-ones. The key reaction involves a palladium catalyzed allylic alkylation of the cyclic β-ketoester nucleophile of formula L-1 (wherein R₁₁ and R₃ are defined above) utilizing a silyl substituted allylic carbonate of formula L-2. Subsequent desilylation and reduction of the compound of formula L-3 (wherein R₁₁ and R₃ are defined above) affords the desired final products, such as compounds of the formula L-4 (wherein R₁₁ is phenyl and R₃ is propyl or cyclohexyl; and R₈ is hydrogen or hydroxy) which are the compounds: 4-hydroxy-6-propyl-6-phenyl-3-(1-phenylpropyl)-2H-pyran-2-one; 4-hydroxy-6-phenyl-6-propyl-3-[1-(2-hydroxyphenyl)-propyl]-2H-pyran-2-one; and 4-hydroxy-6-cyclohexyl-6-phenyl-3-[1-(3-hydroxyphenyl)propyl]-2H-pyran-2-one. This method is especially useful to synthesize 6,6-disubstituted-5,6-dihydro-2H-pyran-2-ones, such as those in Charts K and L.

### CHART M

The dianion of commercially available 4-hydroxy-6-methyl-2-pyrone of formula M-1 is generated by deprotonation with two equivalents of lithium diisopropylamide in tetrahydrofuran and hexamethylphosphoramide. Alkylation with benzyl bromide gives the compound of formula M-2. It is then treated with two equivalents of lithium diisopropylamide in tetrahydrofuran and hexamethylphosphoramide, followed by ethyl iodide to give the compound of formula M-3. Reaction between the compound of formula M-3 and the compound of formula O-5, prepared as described in Chart O, in benzene with *p*-toluenesulfonic acid catalyst in the presence of molecular sieves affords the compound of formula M-4, which is 3-(α-Cyclopropyl-*meta*-(*tert*-butyloxycarbonylamino)benzyl)-6-(α-ethyl-phenethyl)-4-hydroxy-2H-pyran-2-one.

Hydrogenolysis of the compound of formula M-4 in methanol with hydrogen and palladium on charcoal gives the free amine of formula M-5. Condensation of the compound of formula M-5 with *tert*-butyloxycarbonyl-β-alanine in dichloromethane using diisopropylcarbodiimide gives the compound of formula M-6, which is N-(3-Cyclopropyl-[6-(1-ethyl-phenethyl)-4-hydroxy-2-oxo-5,6-dihydro-2H-pyran-3-yl]-methyl)phenyl)-3-(*tert*-butyloxycarbonylamino)-propionamide. The amine M-5 is reacted with alkyl sulfonyl chloride or aryl sulfonyl chloride in the presence of pyridine to give compound of formula M-7 wherein R₁ is aryl, e.g., phenyl, 3-(α-Cyclopropyl-*meta*-(phenylsulfonylamino)benzyl)-6-(α-ethylphenethyl)-4-hydroxy-2H-pyran-2-one.

### CHART N

Treatment of commercially available 4-hydroxy-6-methyl-2-pyrone of formula N-1 with three equivalents of lithium diisopropylamide in tetrahydrofuran and hexamethylphosphoramide is followed by bromomethylcyclopropane to afford the compound of formula N-2. Reaction between the compound of formula N-2 and the compound of formula O-5, prepared as described in Chart O, in benzene with *p*-toluenesulfonic acid catalyst in the presence of molecular sieves affords the compound of formula N-3, which is 3*-*(*α-*Cyclopropyl*-meta-*(*tert*-butyloxycarbonylamino)benzyl)-6-(α-cyclopropylmethyl-cyclopropylethyl)-4-hydroxy-2H-pyran-2-one.

Hydrogenolysis of the compound of formula N-3 in methanol with hydrogen and palladium on charcoal gives the free amine of formula N-4. Condensation of the compound of formula N-4 with 3-(1-indolyl)-propionic acid in dichloromethane and dimethylforamide using diisopropylcarbodiimide gives the compound of formula N-5, which is N-(3-(Cyclopropyl-[6-(2-cyclopropyl-1-cyclopropylmethyl-ethyl)4-hydroxy-2-oxo-5,6-dihydro-2H-pyran-3-yl]-methyl)-phenyl)-3-indol-1-yl-propionamide.

### CHART O

Nitration of commercially available cyclopropyl phenyl ketone of formula O-1 with fuming nitric acid affords the compound of formula O-2. Reduction of the compound of formula O-2 in methanol with hydrogen catalyzed by platinum on carbon gives the amine of formula O-3. The compound of formula O-3 is treated with benzylchloroformate and diisopropylethylamine in dichloromethane to give the compound of formula O-4. Reduction of the compound of formula O-4 with sodium borohydride in tetrahydrofuran and ethanol gives the compound of formula O-5.

### CHART P

Reaction between 5-bromo-4-hydroxy-6-methyl-pyran-2-one of formula P-1 (preparation of this material is described in *Syn. Comm.* 1984. *14,* 521) and commercially available 1-phenyl-1-propanol in benzene catalyzed by *p*-toluenesulfonic acid gives the compound of formula P-2. Treatment of the compound of formula P-2 with lithium diisopropyl amide and commercially available (bromomethyl)cyclopropane affords the compound of formula P-3, which is 5-Bromo-6-(2-cyclopropyl-cyclopropylmethyl-ethyl)-4-hydroxy-3-(1-phenyl-propyl)-pyran-2-one.

### CHART Q

Treatment of 3-(cyclopropyl-phenyl-methyl)-4-hydroxy-6-methyl-pyran-2-one of formula Q-1 of Example 20 with lithium diisopropylamide, followed by 2-(2-methoxy-ethoxy)-ethyl tosylate, gives the compound of formula Q-2. Treatment of the compound of formula Q-2 with lithium diisopropylamide, followed by ethyl iodide, gives the compound of formula Q-3, which is 3-(Cyclopropyl-phenyl-methyl)-4-hydroxy-5-(2-(2-methoxy-ethoxy)-ethyl)-6-propyl-pyran-2-one.

### CHART R

Reaction of commercially available 1,3-diphenylacetone of formula R-1 with the anion of *tert-*butyl P,P-dimethylphosphonoacetate gives the compound of formula R-2, which is hydrogenolyzed in ethyl acetate with 50 psi of hydrogen and platinum on carbon to give the compound of formula R-3. Treatment of the compound of formula R-3 with lithium diisopropylamide, followed by diketene, affords the compound of formula R-4, which is treated with trifluoroacetic acid and followed by acetic anhydride to give the compound of formula R-5. Treatment of the compound of formula R-5 with lithium diisopropylamide, followed by (bromomethyl)cyclopropane, gives the compound of formula R-6, which is 3-(1-Benzyl-2-phenyl-ethylyl)-6-(2-cyclopropyl-1-cyclopropylmethyl-ethyl)-4-hydroxy-pyran-2-one.

### CHART S

The compound of formula S-1 is prepared by the method of Y.S. Shabarov, N.A. Donskaya and R.Y. Lovina, Zh. Obshch. Khim., 33:3434 (1963) (CA60:1624c). The compound of formula S-3 is prepared from compounds S-1 and S-2 (M-2) by the procedure described in Example 1 below. The final compound of formula S-4, which is 4-Hydroxy-3-(1-phenylcyclobutyl)-6-[1-(phenylmethyl)propyl]-2H-pyran-2-one, is prepared by an analogous procedure to Example 6.

### CHART T

The compound of formula T-5, which is 4-Hydroxy-3-(1-phenyl-2-propenyl)-1-oxaspiro[5.7]tridec-3-en-2-one, is prepared from cyclooctanone (compound T-1) by procedures described in Preparations 16A, 16B and 16C below. The compound of formula T-6, which is 4-Hydroxy-3-(1-phenylpropyl)-1-oxaspiro[5.7]tridec-3-en-2-one, is prepared from compound T-5 by procedure described in Preparation 16D below.

### CHART U

The unsaturated amide U-1 (Chemistry Letters (1981, 913-16) is treated with ethyl magnesium bromide in diethyl ether at -40°C to yield U-2. Acid hydrolysis of U-2 affords the intermediate U-3. In a manner similar to the above, treatment of U-4 with phenyl magnesium bromide in diethyl ether affords U-5 which upon treatment with acid affords intermediate U-6. Compounds of the formula U-3 and U-6 wherein phenyl is substituted with, e.g., halogen, trifluoromethyl, -NHBOC, -NHCBZ, NHSO₂Ph, or N-(1,1,4,4-tetramethyl-1,4-bisdisilethylene) (e.g., see Formula BBB-5 in Chart BBB), or wherein phenyl is replaced with optionally substituted heterocycles, e.g., furan and thiophene, are prepared following the above procedure.

### CHART V

The unsaturated amide V-1 (Hruby, et. al., J. Org. Chem. (1993) 58, 766) is treated with phenyl magnesium bromide in the presence of a copper catalyst in tetrahydrofuran to yield V-2. Hydrolysis of V-2 yields V-3 (same as U-6). In a similar fashion, V-4 is converted to V-5 and finally to V-6 (same as U-3). Compounds of the formula V-3 and V-6 wherein phenyl is substituted with, e.g., halogen, trifluoromethyl, -NHBOC or -NHCBZ, or wherein phenyl is replaced with optionally substituted heterocycles, e.g., furan and thiophene, are prepared following the above procedure. Also beginning with compounds of the formula V-7 and V-8, compounds of the formula V-3 and V-6 wherein the ethyl group is replaced with a cyclopropyl are prepared by the procedure of this chart.

### CHART W

[4S,5R]-(+)-4-methyl-5-phenyl-2-oxazolidinone, which is commercially available, is dissolved in THF and cooled to -78°C. n-Butyl lithium is added over 5 minutes and stirring continued for one hour. Butyryl chloride is added in a single portion to yield W-1 after aqueous work up. W-1 is treated with lithium diisopropyl amide (LDA) and reacted with W-2, which is commercially available, (or with other benzyl halides substituted with, e.g., halogen, alkoxy, - CN, nitro or trifluoromethyl, to achieve the substituted compounds corresponding to the following) to yield W-3 as a single diastereomer. Treatment of W-3 with lithium hydroxide and hydrogen peroxide in a THF/water mixture affords W-4 [R] which is then reacted with methyl lithium in ethyl ether to yield methyl ketone W-5. Carboxylation of W-5 yields the β-ketoacid W-6 (see Hogeveen, Menge Tetrahedron Letters (1986) 2767) which is treated with acetic anhydride and acid in the presence of acetone to afford the 1,3-dioxin-4-one derivative W-7.

W-4 is also treated with oxalyl chloride to yield W-8. W-8 is reacted with the lithium enolate derived from t-butylacetate (W-9; enolate formed in THF with lithium diisopropylamide at -78°C), to yield the β-ketoester W-10. Treatment of W-10 with H₂SO₄/Acetic anhydride/acetone then yields W-7 (Kaneko, Sato, Sakaki, Abe J. Heterocyclic Chem. (1990) 27:25).

### CHART X

[4R,5S]-(-)-4-methyl-5-phenyl-2-oxazolidinone, which is commercially available, is dissolved in THF and cooled to -78°C. n-Butyl lithium is added over 5 minutes and stirring continued for one hour. Butyryl chloride is added in a single portion to yield X-1 after aqueous work up. X-1 is treated with lithium diisopropyl amide (LDA) and reacted with X-2, which is commercially available, (or with other benzyl halides substituted with, e.g., halogen, alkoxy, - CN, nitro or trifluoromethyl, to achieve the substituted compounds corresponding to the following) to yield X-3 as a single diastereomer. Treatment of X-3 with lithium hydroxide and hydrogen perioxide in a THF/water mixture affords X-4 [S] which is then reacted with methyl lithium in ethyl ether to yield methyl ketone X-5. Carboxylation of X-5 yields the β-ketoacid X-6 (see Hogeveen, Menge Tetrahedron Letters (1986) 2767) which is treated with acetic anhydride and acid in the presence of acetone to afford the 1,3-dioxin-4-one derivative X-7.

X-4 is also treated with oxalyl chloride to yield X-8. X-8 is reacted with the lithium enolate of tert-butylacetate (X-9) as in the above example to yield X-10. Treatment of X-10 with H₂SO₄/Acetic anhydride/acetone then yields X-7.(Kaneko, Sato, Sakaki, Abe J. Heterocyclic Chem. (1990) 27:25).

### CHART Y

Treatment of Y-1, which is commercially available, with oxalyl chloride in methylene chloride affords the acid chloride Y-2. Treatment of a mixture of Y-2 and 2,2,6-trimethyl-4H-1,3-dioxin-4-one (Y-3), which is commercially available, with triethyl amine (TEA) in toluene at an elevated temperature affords the pyrone Y-4. Treatment of Y-4 with Na₂CO₃ in methanol affords the hydroxy pyrone Y-5 (3-α-methylbenzyl-4-hydroxy-6-methyl-2H-pyran-2-one) and the ester Y-6.

### CHART Z

Treatment of Z-1 (which is the same as U-3) with oxalyl chloride in methylene chloride affords the acid chloride Z-2. Treatment of a mixture of Z-2 and commercially available 2,2,6-trimethyl-4H-1,3-dioxin-4-one (Z-3) (which is the same as Y-3) with triethyl amine (TEA) in toluene at an elevated temperature affords the pyrone Z-4. Treatment of Z-4 with Na₂CO₃ in methanol affords the hydroxy pyrone Z-5 (3[R]-α-ethylbenzyl-4-hydroxy-6-methyl-2H-pyran-2-one) and the ester Z-6.

### CHART AA

Treatment of AA-1 (which is the same as U-3) with oxalyl chloride in methylene chloride affords the acid chloride AA-2. Treatment of a mixture of (S)-3-phenylvaleryl chloride (AA-2) (which is the same as Z-2) and (R)-2,2-dimethyl-6-(α-ethylphenethyl)-4H-1,3-dioxin-4-one (AA-3) (which is the same as W-7) with triethyl amine (TEA) in toluene at elevated temperatures affords the pyrone AA-4. Basic hydrolysis of AA-4 in methanol affords the final hydroxy pyrone AA-5 (3-([R]-α-ethylbenzyl)-4-hydroxy-6-([R]-α-ethylphenethyl)-2H-pyran-2-one) and the ester AA-6 (which is the same as Z-6).

### CHART BB

Treatment of BB-1 (which is the same as U-3) with oxalyl chloride in methylene chloride affords the acid chloride BB-2. Treatment of a mixture of (S)-3-phenylvaleryl chloride (BB-2) (which is the same as Z-2) and (S)-2,2-dimethyl-6-(α-ethylphenethyl)-4H-1,3-dioxin-4-one (BB-3) (which is the same as X-7) with triethyl amine (TEA) in toluene at elevated temperatures affords the pyrone BB-4. Basic hydrolysis of BB-4 in methanol affords the final hydroxy pyrone BB-5 (3-([R]-α-ethylbenzyl)-4-hydroxy-6-([S]-α-ethylphenethyl)-2H-pyran-2-one) and the ester BB-6 (which is the same as Z-6).

### CHART CC

Treatment of CC-1 (which is the same as U-6) with oxalyl chloride in methylene chloride affords the acid chloride CC-2. Treatment of a mixture of (R)-3-phenylvaleryl chloride (CC-2) and (R)-2,2-dimethyl-6-(α-ethylphenethyl)-4H-1,3-dioxin-4-one (CC-3) (which is the same as W-7) with a trialkylamine, e.g., triethyl amine (TEA) in a hydrocarbon solvent, e.g., toluene, at elevated temperatures (e.g. 100°C) affords the pyrone CC-4. Basic hydrolysis of CC-4, using, e.g., sodium carbonate, in a water/alcohol (e.g., methanol) mixture (about 1:9), followed by treatment with an acid; e.g., 1 N hydrochloric acid, affords the final hydroxy pyrone CC-5 (3-([S]-α-ethylbenzyl)-4-hydroxy-6([R]-α-ethylphenethyl)-2H-pyran-2-one; and the ester CC-6.

Also removal of methyl amine hydrochloride by filtration followed by addition of a molar equivalent of sodium hydroxide (methanol) to the toluene solution of CC-4 yields the sodium salt of CC-5 as a precipitate. Collection of that precipitate is conducted by standard procedures.

### CHART DD

Treatment of DD-1 (which is the same as U-6) with oxalyl chloride in methylene chloride affords the acid chloride DD-2. Treatment of a mixture of (R)-3-phenylvaleryl chloride (DD-2) (which is the same as CC-2) and (S)-2,2-dimethyl-6-(α-ethylphenethyl)-4H-1,3-dioxin-4-one (DD-3) (which is the same as X-7) with triethyl amine (TEA) in toluene at elevated temperatures affords the pyrone DD-4. Basic hydrolysis of DD-4 in methanol affords the final hydroxy pyrone DD-5 (3-([S]-α-ethylbenzly)-4-hydroxy-6-([S]-α-ethylphenethyl)-2H-pyran-2-one) and the ester DD-6 (which is the same as CC-6).

The synthesis of all four diastereomers of 3-(α-ethylbenzyl)-6-(α-ethylpehnethyl)-4-hydroxy-2H-pyran-2-one are preferably obtained by following the procedures of Charts AA, BB, CC and DD.

### CHART EE

(R)-3-Phenylvaleric acid (EE-1) (which is the same as U-3) is converted to the corresponding methyl ester with thionyl chloride in methanol to yield EE-2 (which is the same as Z-6). EE-2 is treated with a strong base such as lithium diisopropylamide followed by trimethylsilyl chloride to give EE-3. A mixture of EE-3 and EE-4 (W-7) is heated in an organic solvent such as toluene followed by treating the reaction mixture with acid to yield the final product EE-5 (which is the same as AA-5) (3-([R]-α-ethylbenzyl)-4-hydroxy-6-([R]-α-ethylphenethyl)-2H-pyran-2-one).

### CHART FF

A mixture of FF-1 (which is the same as EE-3 and derived from (S)-3-phenylvaleric acid (U-3), and FF-2 (which is the same as X-7 in Chart X) is heated in an organic solvent such as toluene followed by treating the reaction mixture with acid to yield FF-3 (which is the same as BB-5) (3-([R]-α-methylbenzyl)-4-hydroxy-6-([S]-α-ethylphenethyl)-2H-pyran-2-one).

### CHART GG

(R)-3-Phenylvaleric acid (GG-1) (which is the same as U-6) is converted to the corresponding methyl ester with thionyl chloride in methanol to yield GG-2 (which is the same as CC-6). GG-2 is treated with a strong base such as lithium diisopropyl amide followed by trimethylsilyl chloride to give GG-3. A mixture of GG-3 and GG-4 (W-7) is heated in an organic solvent such as toluene followed by treating the reaction mixture with acid to yield the final product (GG-5) (which is the same as CC-5) (3-([S]-α-methylbenzyl)-4-hydroxy-6-([R]-α-ethylphenethyl)-2H-pyran-2-one).

### CHART HH

A mixture of HH-1 (which is the same as GG-3 in Chart GG) and HH-2 (X-7) is heated in an organic solvent such as toluene followed by treating the reaction mixture with acid to yield the final product HH-3 (which is the same as DD-5) (3-([S]-α-methylbenzyl)-4-hydroxy-6-([S]-α-ethylphenethyl)-2H-pyron-4-one).

### CHART II

Furfuryl alcohol (II-1), which is commercially available, is treated with chloromethyl methyl ether in the presence of diisopropylethylamine in an organic solvent to yield the protected alcohol II-2. II-2 is treated with n-butyl lithium at low temperature in an ether solvent for several hours followed by the addition of cyclopropyl carboxaldehyde, which is commercially available, to give alcohol II-3. A mixture of alcohol II-3 and pyrone II-4, which is commercially available, is treated with a catalytic amount of trifluoroacetic acid in methylene chloride to yield II-5. II-5 is treated with 22 equivalents of LDA followed by ethyl iodide to yield II-6. Treatment of II-6 with 2.2 equivalents of LDA followed by benzyl bromide yields the final product II-7, which is treated with mild acid to give the final product II-8 (3-(α-cyclopropyl((5-hydroxymethyl)furfur-2-yl))-4-hydroxy-6-(α-ethylphenethyl)-2H-pyron-4-one). In the procedure above, other alkyl aldehydes are used in place of cyclopropyl carboxaldehyde to achieve alkyl compounds corresponding to II-3. Also, other alkyl or cycloalkyl halides are used in place of ethyl iodide to achieve compounds corresponding to II-6, and other benzyl halides substituted with, e.g., halogen, trifluoromethyl, -CN, nitro or alkoxy, am used in place of benzyl bromide to achieve substituted compounds corresponding to II-7.

### CHART JJ

Treatment of II-8 with methanesulphonyl chloride in the presence of a base such as pyridine gives the sulfonate JJ-1. Treatment of JJ-1 with sodium methoxide in methanol yields the final product JJ-2. Treatment of JJ-1 with sodium azide in an organic solvent affords the final product, azide JJ-3, which is reduced with hydrogen (Pd/carbon) to give the final product JJ-4. JJ-4 is acylated with acetyl chloride in the presence of triethyl amine in a chlorocarbon or ether solvent, or in pyridine without additional base, to give the final product JJ-5 or reacted with an alkyl or arylsulphonyl halide to give the final product, sulphonate JJ-6. JJ-3 is reacted with methyl propionate in an organic solvent at elevated temperatures to yield the final product JJ-7. Other alkyl alcohols are used in place of methanol, which is used in preparing JJ-2 from JJ-1, to give the corresponding alkyl analogs of JJ-2. Other acid chlorides are used in place of acetyl chloride, which is used in preparing JJ-5 from JJ-4. to give the corresponding analogs of JJ-5. Other acetylenes, substituted with alkyl and aryl groups, are used in place of methyl propionate, which is used in preparing JJ-7 from JJ-3, to achieve the corresponding analogs of JJ-7. Aryl or heterocycle substituted sulphonyl halides are used in preparing the corresponding analogs of JJ-6.

### CHART KK

KK-1 (JJ-4) is reacted with 1,1-bis(methylthio)-2-nitroethylene (KK-2), which is commercially available, in an organic solvent to give the final product KK-3. Reaction of KK-3 with an equivalent of a primary or secondary amine gives the final product KK-4. In a similar fashion, KK-1 (JJ-4) is reacted with dimethyl N-cyanothioiminocarbonate (KK-5, which is commercially available, to give the final product KK-6 which is reacted with a primary or secondary amine to give the final product KK-7. Other amines, such as primary and secondary amines, are used in place of isopropyl amine to prepare the corresponding analogs of KK-4 and KK-7.

### CHART LL

LL-1, which is commercially available, is added to a saturated solution of NaHCO₃. To that suspension is added benzyl chloroformate to yield LL-2. LL-2 is dissolved in methylene chloride and cooled to 0°C. Cyclopropyl carboxylic acid chloride, which is prepared from commercially available cyclopropane carboxylic acid using oxalyl chloride, is added followed by an excess of AlCl₃. The reaction is poured into ice water to yield LL-3. Reduction of LL-3 with sodium borohydride in a mixture of THF and ethanol gives LL-4. Treatment of a solution of LL-4 and pyrone LL-5 (which is the same as M-3) in methylene chloride with trifluoroacetic acid gives final product LL-6. Hydrogenation of LL-6 gives the final product LL-7 (3-(α-cyclopropyl((5-aminomethyl)furfur-2-yl))-4-hydroxy-6-(α-ethylphenethyl-2H-pyran-2-one)(which is the same as JJ-4). Other alkyl acid chlorides are used in place of cyclopropyl carboxylic acid chloride, which is used in preparing LL-3 from LL-2, to achieve the following corresponding analogs.

### CHART MM

MM-1, which is commercially available, is added to a saturated solution of NaHCO₃. To that suspension is added benzyl chloroformate to yield MM-2. MM-2 is dissolved in methylene chloride and cooled to 0°C. Cyclopropyl carboxylic acid chloride, prepared from commercially available cyclopropane carboxylic acid using oxalyl chloride, is added followed by an excess of AlCl₃. The reaction is poured into ice water to yield MM-3. Reduction of MM-3 with sodium borohydride in a mixture of THF and ethanol gives MM-4. Treatment of a solution of MM-4 and pyrone MM-5 (which is the same as M-3) in methylene chloride with trifluoroacetic acid gives the final product MM-6. Hydrogenation of MM-6 gives the final product MM-7 (3-(α-cyclopropyl((5-aminomethyl)thiophen-2-ylmethyl))-4-hydroxy-6-(α-ethylphenethyl)-2H-pyran-2-one). Treatment of MM-7 with phenylsulphonylchloride gives the final product MM-8. Other alkyl acid chlorides and substituted heterocycles are used in place of cyclopropyl carboxylic acid to achieve analogs corresponding to M-3. Other substituted arylsulfonyl halides are used in place of phenylsulfonyl chloride to achieve analogs corresponding to MM-8.

### CHART NN

A mixture of NN-1, which is commercially available, and cyclopropylcarboxcylic acid chloride, which is prepared from commercially available cyclopropane carboxylic acid, is treated with a Lewis acid such as AlCl₃ in methylene chloride at 0°C to yield NN-2. Treatment of NN-2 with sodium borohydride in an alcohol solvent gives NN-3. Treatment of a mixture of NN-3 and NN-4 (which is the same as M-3) in methylene chloride or toluene with an anhydrous acid such as trifluoroacetic acid or p-toluenesulfonic acid gives the final product NN-5. Treatment of NN-5 with azasulphene (NN-6). S. K. Gupta, Synthesis, p. 39 (1977), then affords the final product NN-7. In a similar manner, treatment of NN-5 with chlorosulfonic acid followed by addition of aniline also yields NN-7 in a two-step protocol. Aniline substituted by one or more halogen, alkoxy, trifluoromethyl, alkyl, nitro and -CN, are also used in this process to yield substituted analogs of NN-7.

### CHART OO

Thiophen-2-yl methanol (OO-1), which is commercially available, is treated with chloromethyl methyl ether in the presence of diisopropylethylamine in an organic solvent to yield the protected alcohol OO-2. OO-2 is treated with n-butyl lithium at low temperature in an ether solvent for several hours followed by the addition of cyclopropyl carboxaldehyde to give alcohols OO-3 and OO3a. A mixture of alcohol OO-3 and pyrone OO-4 (which is the same as D-1) is treated with a catalytic amount of trifluoroacetic acid in methylene chloride to yield OO-5. OO-5 is treated with 2.2 equivalents of LDA followed by ethyl iodide to yield OO-6. Treatment of OO-6 with 2.2 equivalents of LDA followed by benzyl bromide yields the final product OO-7 which is treated with mild acid to give the final product OO-8 (3-(α-cyclopropyl((5-hydroxymethyl)thiophen-2-yl)-4-hydroxy-6-(α-ethylphenethyl)-2H-pyron-4-one). In the procedure above, other alkyl aldehydes are used in place of cyclopropyl carboxaldehyde to achieve alkyl compounds corresponding to OO-3. Also, other alkyl or cycloalkyl halides are used in place of ethyl iodide to achieve compounds corresponding to OO-6. and other benzyl halides substituted with, e.g., halogen, trifluoromethyl, -CN, nitro or alkoxy, are used in place of benzyl bromide to achieve substituted compounds corresponding to OO-7.

### CHART PP

Treatment of OO-8 with methanesulphonyl chloride in the presence of a base such as triethylamine gives the sulfonate PP-1. Treatment of PP-1 with sodium methoxide in methanol yields the final product PP-2. Treatment of PP-1 with sodium azide in an organic solvent affords the final product, azide PP-3, which is reduced with hydrogen (Pd/carbon) to give the final product PP-4. PP-4 is acylated with acetyl chloride in the presence of triethyl amine in a chlorocarbon or ether solvent, or in pyridine without additional base, to give the final product PP-5 or reacted with an alkyl or arylsulphonyl halide to give the final product, sulphonate PP-6 (N-(5-(1-cyclopropyl)-1-(4-hydroxy-6-(a-ethylphenethyl)-2H-pyron-4-one-3-yl))methyl)thiophen-2-yl phenyl sulphonamide). PP-3 is reacted with methyl propiolate in an organic solvent at elevated temperatures to yield the final product PP-7. Other alkyl alcohols are used in place of methanol, which is used in preparing PP-2 from PP-1, to give the corresponding alkyl analogs of PP-2. Other acid chlorides are used in place of acetyl chloride, which is used in preparing PP-5 from PP-4, to give the corresponding analogs of PP-5. Other acetylenes, substituted with alkyl and aryl groups, are used in place of methyl propionate, which is used in preparing PP-7 from PP-3, to achieve the corresponding analogs of PP-7. Aryl or heterocycle substituted sulphonyl halides are used in preparing the corresponding analogs of PP-6.

### CHART QQ

A mixture of QQ-1 (OO-3a from Chart OO) and QQ-2 (which is the same as D-1) in methylene chloride is treated with a catalytic amount of trifluoroacetic acid which yields QQ-3. QQ-3 is treated with 3.3 equivalents of lithium diisopropylamide (LDA) in an ether solvent below room temperature followed by the addition of ethyl iodide to yield the final product QQ-4. Treatment of QQ-4 with 3.3 equivalents of LDA in an ether solvent followed by the addition of benzyl bromide yields the final product QQ-5 (which is the same as 00-8).

### CHART RR

RR-1 (PP-4) is reacted with 1,1-bis(methylthio)-2-nitroethylene (RR-2), which is commercially available, in an organic solvent to give the final product RR-3. Reaction of RR-3 with an equivalent of a primary or secondary amine gives the final product RR-4. In a similar fashion. RR-1 is reacted with dimethyl N-cyanothioiminocarbonate (RR-5), which is commercially available, to give the final product RR-6 with is reacted with a primary or secondary amine to give the final product RR-7. Other amines, such as primary and secondary amines, are used in place of isopropyl amine to prepare the corresponding analogs of RR-4 and RR-7.

### CHART SS

A mixture of SS-1, which is commercially available, and cyclopropylcarboxcylic acid chloride, which is prepared from commercially available cyclopropane carboxylic acid, is treated with a Lewis acid such as AlCl₃ in methylene chloride at 0°C to yield SS-2. Treatment of SS-2 with sodium borohydride in an alcohol solvent gives SS-3. Treatment of a mixture of SS-3 and SS-4 (which is the same as M-3) in methylene chloride or toluene with an anhydrous acid, such as trifluoroacetic acid or p-toluenesulfonic acid, gives the final product SS-5. Treatment of SS-5 with azasulphene SS-6, S. K. Gupta, Synthesis, p. 39 (1977), then affords the final product SS-7. In a similar manner, treatment of SS-5 with chlorosulfonic acid followed by addition of aniline also yields SS-7 in a two-step protocol. Aniline substituted by one or more halogen, alkoxy, trifluoromethyl, alkyl, nitro and -CN, is also used in this process to yield substituted analogs of SS-7.

### CHART TT

Pyrone TT-1 (which is the same as Q-1) is treated with two equivalents of lithium diisopropylamide in an ether solvent. An aryl aldehyde is added at low temperature and the reaction quenched by adding a saturated solution of NH₄Cl. This affords the final product TT-2. Additionally, TT-1 is treated with 2.2 equivalents of lithium diisopropylamide (LDA) in an ether solvent followed by the addition of ethyl iodide to give TT-3. To that solution is added another equivalent of LDA followed by benzaldehyde to yield the final product TT-4.

### CHART UU

Carboxylic acids UU-1 and UU-7 are prepared by methodolgy described in J. Amer. Chem. Soc. (1981) 103:2127, and Tetrahedron Letters (1986) 27:897. The β-hydroxyl group in both UU-1 and UU-7 is protected by reacting the hydroxy acid with t-butyldimethylsilyl chloride in DMF in the presence of imidazole (or 2,6-lutidine, ethyldiisopropylamine) followed by treatment with aqueous base to yield the free carboxylic acid. The carboxylic acid is treated with oxalyl chloride (or alternate reagent) to yield acid chlorides UU-2 and UU-8. If required, another silyl derived protecting group such as the t-butyldiphenylsilyl group is used, or some other suitable alcohol protecting group. Both UU-2 and UU-8 are reacted with the lithium enolate derived from tert-butylacetate (LDA/THF/-78C) to yield β-ketoesters UU-3 and UU-9. Treatment of UU-3 and UU-9 with H2SO4/acetic anhydride/acetone yields UU-4 and UU-10 [Kaneko, Sato, Sakaki, Abe J. Heterocyclic Chem. (1990) 27:25], both of which react with acid chloride UU-5 hot toluene in the presence of triethylamine to give the final products UU-6 and UU-11. Oxidation of UU-6 and UU-11 with CrO₃ or Swem conditions yield the final products UU-12 and UU-14 both of which when treated with sodium borohydride (or other hydride reagents) give mixtures of diastereomers. Reduction of UU-12 yields UU-6 and UU-13 as a separable mixture of diastereomers. Reduction of UU-14 yields UU-11 and UU-15 as a separable mixture of distereomers. The remaining diastereomeric products are prepared using this same strategy but using the (S) acid chloride UU-16 in place of UU-5.

### CHART VV

Chart W is a modified version of Chart L above. This chart describes the palladium catalyzed allylic alkylation of the cyclic β-ketoester nucleophile of formula VV-1 (wherein, eg., R₁ is phenyl, phenethyl or phenylmethyl; R₂ is phenylmethyl or propyl) utilizing a silyl substituted allylic carbonate of formula VV-2. Desilylation results in the compound of formula VV-4 (wherein, eg., R₁ is phenyl, phenethyl or phenylmethyl; R₂ is phenylmethyl or propyl) and subsequent reduction results in the compound of formula VV-5 (wherein, eg.. R₁ is phenyl, phenethyl or phenylmethyl; R₂ is phenylmethyl or propyl).

### CHART WW

This chart describes a generic procedure for the synthesis of the C-3α branched 5,6-dihydropyrones by alkylation with 1,3-diphenyl allyl alcohol. Thus, boron trifluoride etherate catalyzed reaction of the compound of formula WW-1 (wherein, e.g., R₁ is phenyl, phenethyl or phenylmethyl; R₂ is propyl, phenylmethyl or 2-methylpropyl) with 1,3-diphenyl allyl alcohol (WW-2) provides compounds of the formula WW-3 (wherein, e.g., R₁ is phenyl, phenethyl or phenylmethyl; R₂ is propyl, phenylmethyl or 2-methylpropyl). Subsequent hydrogenation catalyzed by Pd/C provides the compound of formula WW-4 (wherein, e.g., R₁ is phenyl, phenethyl or phenylmethyl; R₂ is propyl, phenylmethyl or 2-methylpropyl).

### CHART XX

This chart is a variation of Chart WW. The only change is that trans-stilbene oxide replaces 1,3-diphenyl allyl alcohol. This chart describes a generic procedure for the synthesis of C-3α branched 5,6-dihydropyrones by alkylation with trans-stilbene oxide. Thus, boron trifluoride etherate catalyzed reaction of the compound of formula XX-1 (wherein, e.g., R₁ is phenethyl; R₂ is propyl) with trans-stilbene oxide (XX-2) provides compounds of formula XX-3 (wherein, e.g., R₁ is phenethyl; R₂ is propyl). Subsequent hydrogenation catalyzed by Pd/C provides the compound of formula XX-4 (wherein, e.g., R₁ is phenethyl; R₂ is propyl).

### CHART YY

Racemic 3-phenylpentanoic acid (YY-4) is prepared from hydrogenation using Pd/C of YY-2, and followed by basic hydrolysis of ester YY-3. The ester YY-2 is prepared by an orthoester Claisen rearrangement on cinnamyl alcohol YY-1 using 1,1,1-triethoxyethane. Acid YY-4 is readily resolved by reacting the acid YY-4 with diethyl phosphorylchloride in the presence of triethylamine, yielding an activated acyl intermediate which is then treated with (S)-α-methylbenzyl amine to yield a mixture of YY-5 and YY-6. YY-5 is obtained diastereomerically pure as a crystalline solid (the other diastereomer YY-6 is in the mother liquors). Treatment of YY-5 with H₃PO₄ at 150-60°C then yields pure (R)-3-phenylpentanoic acid YY-7 (which is the same as CC-1). The (S)-3-Phenylpentanoic acid is obtained following the above procedures if (R)-α-methylbenzyl amine is used to form the diastereomeric amides of the racemic acid YY-4.

### CHART ZZ

Diethyl malonate (ZZ-1) is alkylated with ethyl iodide to give compound of formula ZZ-2, which is further alkylated with benzyl bromide to give compound of formula ZZ-3 (Procedures in Organic Syntheses, Coll. VI:250). Hydrolysis of ZZ-3 gives the racemic acid ZZ-4. The optically active acids, ZZ-5 (which is the same as W-4) and ZZ-6 (which is the same as X4), are obtained from fractional crystallization of the racemic acid ZZ-4 with either the R or the S isomer of α-methybenzyl amine.

### CHART AAA

This chart illustrates the Lewis acid catalyzed coupling reaction between 5,6-dihydro-6,6-disubstituted-2H-pyran-2-ones (AAA-1 wherein, e.g., R₁ is 2-methylpropyl, R₂ is phenylethyl) and benzhydrol (AAA-2) to provide 3-diphenylmethyl derivatives (AAA-3 wherein, e.g., R₁ is phenylethyl, R₂ is 2-methylpropyl).

### CHART BBB

Acetic acid (BBB-1) is treated with two equivalents of lithium diisopropylamide in tetrahydrofuran. To that solution is added cyclopropane carboxaldehyde (commerically available). After aqueous workup, racemic BBB-2 is isolated. (R) BBB-2b and (S) BBB-2a are obtained from fraction crystallization of the racemic acid with either the R or S isomer of α-methylbenzyl amine, ephedrine, brucine, strychnine, quinine, cinchonidine, quinidine or cinchonine. Alernatively, aldol condensation using the Evan's strategy (J. Amer. Chem. Soc. (1981) 103, 2127) would also afford pure enantiomers BBB-2a and BBB-2b. BBB-2a is treated with triethylorthoacetate to yield BBB-3 (Helv. Chim. Acta. (1987) 70, 1320). Upon thermolysis of BBB-3, BBB-4 is obtained (Helv. Chim. Acta. (1987) 70, 1320). Treatment of BBB-4 with BBB-5 in the presence of Cuprous iodide (Tetrahedron Letters 1253 (1984)) gives BBB-6. Treatment of BBB-6 with oxalyl chloride in methylene chloride for several hours yields BBB.7. To a hot toluene solution of BBB-7 is added a toluene solution of triethylamine and BBB-8. The reaction is heated for several hours and the solvent is removed via evaporation when thin layer chromatography (silica gel, ethyl acetate) indicates complete consumption of BBB-8. The crude reaction is diluted with a 10/1 mixture of methanol/water followed by addition of sodium carbonate. This then affords BBB-9. Treatment of BBB-9 with a catalytic amount of p-toluenesulfonic acid and several equivalents of methanol in ether yields BBB-10. Treatment of a methylene chloride solution of BBB-10 with p-cyanobenzenesulfonylchloride in the presence of triethylamine affords BBB-11.

Alternately, treatment of BBB-3 with BBB-5 in the presence of Cuprous iodide (Tetrahedron Letters 1253 (1984)) gives BBB-12. which is the enantiomer of BBB-6, and when carried through the analogous steps as BBB-6 yields BBB-13.

Furthermore, BBB-14 and BBB-15 are substituted for BBB-4 and BBB-3 to prepare the corresponding ethyl stereoisomers.

### CHART CCC

Ketone CCC-1 is treated with (carbethoxymethylene)triphenylphosphorane to yield unsaturated ester CCC-2. Reduction (Red-Al/CuBr, DIBAH/MeCu; NaBH4/resin; NaBH4/Cu2Cl2; ref. Reduction by the Alumino and Borohydrides in Organic Synthesis, J. Seyden-Penne, VCH Publishers, Inc. Lavoisier - Tec & Doc, 1991, p 156) of CCC-2 followed by ester hydrolysis yields CCC-3. (R) CCC-4a and (S) CCC-4b are obtained from fraction crystallization of the racemic acid CCC-3 with either the R or S isomer of α-methylbenzyl amine, ephedrine, brucine, strychnine, quinine, cinchonidine, quinidine or cinchonine. Alternatively, CCC-4a and CCC-4b are obtained via asymmetric reduction (Angew. Chem. Int. Ed. Engl. (1989) 28, 60) followed by basic hydrolysis. Following the protocol described in Chart BBB, CCC-6 is prepared.

### CHART DDD

2-Thienylamine DDD-1 (commerically available) is treated with 2 equivalents of n-butyl lithium in ether at -78°C. After stirring at -78°C for 30 minutes the reaction temperature is raised to 0°C for 30 minutes followed by the addition of sulfur (fine powder). After aqueous workup, DDD-2 is isolated (J. Amer. Chem. Soc. (1955) 77, 5357, 5446; Org. Syn. Vol. VI, 979 (1988)). DDD-2 is treated with p-fluorophenylsulfonyl chloride in methylene chloride in the presence of triethylamine to yield DDD-3. DDD-3 is also available via first reacting DDD-1 with p-fluorobenzenesulfonylchloride followed by treatment of that intermediate with 2 equivalents of n-butyl lithium in ether followed by the addition of sulfur. Treatment of a mixture of DDD-3 and DDD-4 in ether with triethylamine affords DDD-5 (US Patent 4968815). Following the same procedure as found in Chart BBB, DDD-5 is converted to an acid chloride and reacted with (R) 2,2-dimethyl-6-(α-ethylphenethyl)-4H-1,3-dioxin-4-one to afford DDD-6.

As is apparent to those of ordinary skill in the art, the compounds of the present invention can occur in several diastereomeric forms, depending on the configuration around the asymmetric carbon atoms. All such diastereomeric forms are included within the scope of the present invention. Also, the compounds of the present invention can exist in several tautomeric forms, including the particular enol form as depicted in the Formula Chart below by formula I and the keto form of formula II and mixtures thereof. (For formulas I and II, the dashed line indicates that a double bond may be present or absent.) All such tautomeric forms are included within the scope of the present invention. For compounds of the present invention which are 4-hydroxy-pyran-2-ones of formula IV in the Formula Chart below, the enol form predominates. For compounds of the present invention which are 5,6-dihydro-4-hydroxy-pyran-2-ones of formula III in the Formula Chart below, a mixture of the enol and keto forms is commonly expected.

The compounds of the present invention may be in either the free form or the protected form at one or more of the remaining (not Previously protected) carboxyl, amino, hydroxy or other reactive groups. The protecting groups may be any of those known in the art. Examples of nitrogen and oxygen protecting groups are set forth in T.W. Greene, Protecting Groups in Organic Synthesis, Wiley, New York (1981); J.F.W. McOmie, ed., Protective Groups in Organic Chemistry, Plenum Press (1973); and J. Fuhrhop and G, Benzlin, Organic Synthesis, Verlag Chemie (1983). Included among the nitrogen protective groups are tert-butoxycarbonyl (BOC), benzyloxycarbonyl, acetyl, allyl, phthalyl, benzyl, benzoyl, trityl, and the like. Included among the ketone protective groups is 1,3-dioxalane.

The present invention provides for compounds of formula I or pharmacologically acceptable salts and/or hydrates thereof. Pharmacologically acceptable salts refers to those salts which would be readily apparent to a manufacturing pharmaceutical chemist to be equivalent to the parent compound in properties such as formulation, stability, patient acceptance and bioavailablility. Examples of salts of the compounds of the present invention include acidic salts, such as sodium and potassium salts of the compounds of formula I, and basic salts, such as the hydrochloride salt of the compounds of formula I, wherein the R substituents contain a basic moiety.

Those skilled in the art would know how to formulate the compounds of this invention, using pharmaceutically acceptable carriers, into appropriate pharmaceutical dosage forms. Examples of the dosage forms include oral formulations, such as tablets or capsules, or parenteral formulations, such as sterile solutions.

When the compounds in this invention are administered orally, an effective amount is from about 0.1 mg to 100 mg per kg of body weight per day. Either solid or fluid dosage forms can be prepared for oral administration. Solid compositions are prepared by mixing the compounds of this invention with conventional ingredients such as talc, magnesium stearate, dicalcium phosphate, magnesium aluminum silicate, calcium sulfate, starch, lactose, acacia, methyl cellulose, or functionally similar pharmaceutical diluents and carriers. Capsules are prepared by mixing the compounds of this invention with an inert pharmaceutical diluent or carrier and placing the mixture into an appropriately sized hard gelatin capsule. Soft gelatin capsules are prepared by machine encapsulation of a slurry of the compounds of this invention with an acceptable inert oil such as vegetable oil or light liquid petrolatum. Syrups are prepared by dissolving the compounds of this invention in an aqueous vehicle and adding sugar, aromatic flavoring agents and preservatives. Elixirs are prepared using a hydroalcoholic vehicle such as ethanol, suitable sweeteners such as sugar or saccharin and an aromatic flavoring agent. Suspensions are prepared with an aqueous vehicle and a suspending agent such as acacia, tragacanth, or methyl cellulose.

When the compounds of this invention are administered parenterally, they can be given by injection or by intravenous infusion. An effective amount is from about 0.1 mg to 100 mg per kg of body weight per day. Parenteral solutions are prepared by dissolving the compounds of this invention in aqueous vehicle and filter sterilizing the solution before placing in a suitable sealable vial or ampule. Parenteral suspensions are prepared in substantially the same way except a sterile suspension vehicle is used and the compounds of this invention are sterilized with ethylene oxide or suitable gas before it is suspended in the vehicle.

The exact route of administration, dose, or frequency of administration would be readily determined by those skilled in the art and is dependant on the age, weight, general physical condition, or other clinical symptoms specific to the patient to be treated.

Surprisingly and unexpectedly, the compounds of the present invention have been found to decrease the size and weight of the prostate gland in male mammals. This is particularly unexpected in view of the fact that these compounds are structurally quite unlike any known testosterone 5α-reductase inhibitor. Also, the compounds of this invention may have a number of other uses, as described more fully below.

Thus, the present invention provides the use of compounds, such as 3-(α-ethylbenzyl)-6-(α-ethylphenethyl)-4-hydroxy-2H-pyran-2-one, for preventing and treating androgen-dependent diseases, including the prevention and treatment of human and animal prostate lesions, such as benign prostatic hypertrophy and hyperplasia (non-neoplastic enlargements of the prostate) by, for example, maintaining and reducing the size of the prostate gland, and prostatic cancer in male mammals. Also, the present invention provides the use of these compounds to prevent and treat androgen-dependent skin diseases, such as alopecia, hirsutism, particularly female hirsutism, acne vulgaris and seborrhea, in mammals.

By "preventing" is meant avoiding the occurrence of a disease.

By "treating" is meant ameliorating or avoiding the effects of a disease.

By "maintaining" is meant keeping in an existing state.

By "mammals" is meant any warm-blooded vertebrate animal of the class *Mammalia* comprising humans and all other animals that nourish their young with milk secreted by mammary glands and have the skins usually more or less covered with hair. Especially included are human beings, dogs and rats.

By "hypertrophy" is meant the enlargement or overgrowth of an organ or part due to an increase in size of its constituent cells.

By "hyperplasia" is meant the abnormal multiplication or increase in the number of normal cells in normal arrangement in a tissue.

Accordingly, the present invention is also concerned with providing a method of preventing or treating the androgen-dependent diseases of alopecia, hirsutism, acne vulgaris and seborrhea by topical administration, and a method of preventing or treating all of the above conditions as well as benign prostatic hypertrophy and prostate cancer, by topical or parenteral administration, of the compounds of the present invention. Thus, the present invention is concerned with providing suitable topical and parenteral pharmaceutical formulations for use in the novel methods of treatment of the present invention.

The variety of therapeutic dosage forms for different modes of administration and their preparation are hereby incorporated by reference herein from the following: U.S. Patent 4,377,584, col. 10, line 52, through col. 11, line 31; U.S. Patent 4,760,071, col. 6, line 62, through col. 7, line 42; and U.S. Patent 5,017,568, col. 28, lines 3-39.
The amount of the compound of formula I which is effective for the treatment of the above stated conditions ranges from 1 through 1000 mg/kg/day, with 50 through 400 mg/kg/day being preferred and 100 through 400 mg/kg/day being the most preferred. The exact therapeutic dosage form, mode and frequency of administration, and dosage would be readily determined by those skilled in the art and is dependent on the age, weight, general physical condition or other clinical symptoms specific to the patient, human and other mammals, to be treated.

The following five (5) compounds of the present invention were tested in an *in vitro* testosterone 5α-reductase inhibitor assay: 3(α-Ethylbenzyl)-6-(α-ehylphenethyl)-4-hydroxy-2H-pyran-2-one; 6-(1-Benzyl-propyl)-3-cyclopropyl-phenyl-methyl)-4-hydroxy-pyran-2-one; 3-(α-Ethylbenzyl)-6-(α-ethylbenzyl)-4-hydroxy-2H-pyran-2-one; 3-(α-Ethylbenzyl)-6-phenethyl-4-hydroxy-2H-pyran-2-one; 4-Hydroxy-3-(1-phenylpropyl)-6-[1-(tetrahydro-2H-pyran-3-yl)methyl]propyl]-2H-pyran-2-one. This assay is very similar to those described in the following references, except for the use of dog and rat prostates in place of human prostates: Proc. Natl. Acad. Sci. USA. Vol. 90: 5277-5281 (1993); J. Steroid. Biochem. Molec. Biol., Vol. 44, No. 2, pp. 121-131 (1993). All five (5) compounds had activity in this assay at a dose of at least 0.1 mM.

Therefore, and without wishing to be limited to a specific mechanism of action, it was concluded that these compounds and other related compounds of the present invention would be useful for preventing or treating androgen-dependent diseases, such as those described above.

The utility of representative compounds of the present invention has been demonstrated in the biological tests described below:

3-(α-Ethylbenzyl)-6-(α-ethylphenethyl)-4-hydroxy-2H-pyran-2-one was further tested *in vivo* in a four-week oral drug safety and toxicity study in beagle dogs, described in Example 208 below. It was found that, during the conduct of the postdosing evaluation of data from this study, there was a dose-related decrease in prostate size and weight in male dogs. Mean prostatic weights (n=3, 3 dogs in each of groups 1-5) are given in Table I below. Light microscopic examination of high dose and control testes, adrenals and pituitaries did not show drug-related changes.

In a repeat study (groups 6 and 7, of 4 dogs each) the results of which are also given in Table I, 2/4 high dose dogs had lower absolute and relative prostate weights than did the controls, but decreases were marked only in 1/4 dogs.

As demonstrated in this *in vivo* test, the ability of 3-(α-ethylbenzyl)-6-(α-ethylphenethyl)-4-hydroxy-2H-pyran-2-one to reduce the size and weight of the prostate gland, particularly without significant effects on testes and other endocrine glands, indicates that this compound and related compounds of the present invention would be useful to prevent or treat androgen-dependent diseases, such as those described above, especially benign prostatic hypertrophy and hyperplasia in male mammals.

### DESCRIPTION OF THE PREFERRED EMBODIMENTS

In the Preparations and Examples below and throughout this document:
- °C: is degrees Centigrade.
- M: is molar (concentration).
- N: is normal (concentration).
- mL: is milliliter.
- mg: is milligram.
- mmHg: is millimeter of mercury.
- ¹H-NMR: is proton nuclear magnetic resnance spectrum.
- ¹³C-NMR: is carbon nuclear magnetic resonance spectrum.
- δ: is chemical shift (parts per million) relative to TMS.
- CDCl₃: is deuterio-chloroform.
- CD₃OD: is deuterio-methanol.
- FAB MS: is fast-atom-bombardment mass spectroscopy.
- HRMS: is high-resolution mass spectroscopy.
- Anal.: is analytical data.
- Pd/C: is palladium on charcoal.
- THF: is tetrahydrofuran.

The following Preparations and Examples illustrate the present invention:

### PREPARATION 1 Ethyl 5-phenyl-3,5-dioxopentanoate (Formula A-3: X is CH) Refer to Chart A.

To a suspension of 50 mg potassium hydride in 1 mL of dry tetrahydrofuran is added dropwise 130 mg of ethyl acetoacetate (A-1) at 0 °C, followed by addition of 0.7 mL of 1.6 M n-butyl lithium-hexane solution. To the resulting yellow reaction mixture is then added 150 mg of ethyl benzoate (A-2: X is CH), and stirred for another 5 min. The reaction is quenched with 0.5 mL glacial acetic acid, and then diluted with 1 mL water. The tetrahydrofuran is removed under reduced pressure and the aqueous layer is extracted with methylene chloride (2 X 5 mL). The combined organic layers are washed with water (2 mL), dried (magnesium sulfate), and then concentrated. The residue is purified by flash column chromatography (0 to 30 % ethyl acetate in hexane) to give 201 mg of the title product as colorless oil.

Physical characteristics are as follows:
¹H-NMR (CDCl₃) δ 1.29, 3.48, 4.22. 6.30, 7.47, 7.87, 15.81.
¹³C-NMR (CDCl₃) δ 13.8, 45.6, 61.2, 96.5, 126.8, 128.4, 132.4, 133.8, 167.3, 182.3, 189.1.

### PREPARATION 2 4-Hydroxy-6-phenyl-2-pyrone (Formula A-4: X is CH) Refer to Chart A.

The title product of Preparation 1 (700 mg) is heated at 120 °C under reduced pressure (1 mm Hg) for 10 h. The resulting brownish cake is washed with diethyl ether, dried under reduced pressure to give 411 mg of the title product as white solid.
Physical characteristics are as follows:
¹H-NMR (CDCl₃) δ 5.40, 6.78, 7.53, 7.85.

### EXAMPLE 1 4-Hydroxy-6-phenyl-3-(1'-phenyl-propyl)-2-pyrone; also named as 3-(.alpha.-ethylbenzyl)-4-hydroxy-6-phenyl-2H-Pyran-2-one (Formula A-5: X is CH, R is ethyl) Refer to Chart A.

To a solution of 94 mg of the title product of Preparation 2 and 104 mg of (±)-1-phenyl-1-propanol in 3 mL of dioxane is added 0.3 mL of boron trifluoride - diethyl ether at room temperature and allowed to stir for 10 h, and then is quenched with 1 mL of water. The reaction mixture is extracted with methylene chloride (3 X 5 mL) and the combined organic layers are washed with water (2 X 3 mL), brine (3 mL), dried (sodium sulfate) and then concentrated. The residue is purified by column chromatography (20 to 80 % ethyl acetate in hexane) to give 37.5 mg of the title product as a tan solid.

Physical characteristics are as follows:
¹H-NMR (CDCl₃ / CD₃OD) δ 0.94, 2.17, 2.35, 4.05, 4.23, 6.55, 7.17, 7.26, 7.42, 7.50, 7.75.
¹³C-NMR (CDCl₃ / CD₃OD) δ 12.4, 23.8, 41.8, 97.7, 106.1, 124.9, 125.5, 127.5, 127.8, 128.4, 130.2, 130.9, 143.7, 158.0, 165.8.
FAB MS [M]⁺ = 306.

### PREPARATION 3 Ethyl 6-phenyl-3,5-dioxohexanoate (Formula B-3: n is 1) Refer to Chart B.

To a suspension of 580 mg potassium hydride in 15.0 mL of tetrahydrofuran is added 1.82 g of ethyl acetoacetate (B-1) at 0 °C and stirred for 30 min, then is added 9.0 mL of 1.6 M n-butyl lithium-hexane solution and stirred 1 h at 0 °C. To the reaction mixture is added 2.30 g of ethyl phenylacetate (B-2: n is 1) which results in precipitation. and therefore additional 50 ml tetrahydrofuran is added. After stirring for 12 h at room temperature, the reaction is quenched with 10 mL of glacial acetic acid. The tetrahydrofuran is removed under reduced pressure and the aqueous layer is extracted with ethyl acetate (3 X 30 mL). The combined organic layers are washed with water (3 X 10 mL), brine (10 mL), dried (sodium sulfate) and then concentrated. The residue is then purified by flash column chromatography (10 % ethyl acetate in hexane) to afford 1.03 g of the title product as a light yellow oil.

Physical characteristics are as follows:
¹H-NMR (CDCl₃) δ 1.22, 2.18, 3.24, 3.40, 3.54, 3.59, 4.16, 7.22.

### PREPARATION 4 Ethyl 7-phenyl-3,5-dioxoheptanoate (Formula B-3: n is 2) Refer to Chart B.

To a suspension of 580 mg of potassium hydride in 15.0 mL of tetrahydrofuran is added 1.82 g of ethyl acetoacetate (B-1) at 0 °C and stirred for 30 min, then is added 9.0 mL of 1.6 M n-butyl lithium-hexane solution and stirred 1 h at 0 °C. To the reaction mixture is added 2.49 g of ethyl dihydrocinnamate (B-2: n is 2) which results in precipitation, and therefore additional 50 mL of tetrahydrofuran is added. After stirring for 12 h at room temperature, the reaction is quenched with 10 mL of glacial acetic acid. The tetrahydrofuran is removed under reduced pressure and the aqueous layer is extracted with ethyl acetate (30 mL X 3). The combined organic layers are washed with water (3 X 10 mL), brine (10 mL), dried (sodium sulfate) and then concentrated. The residue is then purified by flash column chromatography (10 % ethyl acetate in hexane) to afford 718 mg of the title product as a light yellow oil.

Physical characteristics are as follows:
¹H-NMR (CDCl₃) δ 1.27, 2.28, 2.63, 2.88, 2.95, 3.31, 3.43, 4.19, 7.23.

### PREPARATION 5 6-Benzyl-4-hydroxy-2-pyrone (Formula B-4: n is 1) Refer to Chart B.

The title product of Preparation 3 (510 mg) is heated at 100 °C under reduced pressure (1 mm Hg) for 16 h. The resulting white solid is washed with diethyl ether (10 mL), dried under reduced pressure to give 226 mg of the title product as white solid.

Physical characteristics are as follows:
¹H-NMR (CD₃OD) δ 3.80, 5.32, 5.90, 7.29.
¹³C-NMR (CD₃OD) δ 40.6, 89.9, 102.3, 128.3, 129.9, 130.3, 136.8, 167.3, 168.2, 173.3.
FAB MS [M]⁺ = 202.

### PREPARATION 6 4-Hydroxy-6-phenethyl-2-pyrone (Formula B-4: n is 2) Refer to Chart B.

The title product of Preparation 4 (718 mg) is heated at 100 °C under reduced pressure (1 mm Hg) for 16 h. The resulting white solid is washed with diethyl ether (10 mL), dried under reduced pressure to give 346 mg of the title product as white solid.

Physical characteristics are as follows:
¹H-NMR (CD₃OD) δ 2.73, 2.90, 5.34, 5.86, 7.20.
¹³C-NMR (CD₃OD) δ 33.8, 36.4, 89.9, 102.2, 127.4, 129.4, 129.6, 141.4, 167.3, 168.4, 173.3.
FAB MS [M]⁺ = 216.

### EXAMPLE 2 6-Benzyl-4-hydroxyl-3-(1'-phenylpropyl)-2-pyrone; also named as 6-benzyl-3-(.alpha.-ethylbenzyl)-4-hydroxy-2H-Pyran-2-one (Formula B-5: n is 1) Refer to Chart B.

A mixture of the title product of Preparation 5 (21 mg) and (±)-1-bromo-1-phenylpropane (0.2 mL) are heated at 100 °C for 16 h. The crude reaction mixture is purified by flash column chromatography (10 to 30 % ethyl acetate in hexane) to give 11.2 mg of the title product as yellow solid.

Physical characteristics are as follows:
¹H-NMR (CDCl₃) δ 0.96, 2.17, 3.80, 4.24, 5.93, 7.15-7.45.

### EXAMPLE 3 4-Hydroxy-6-phenethyl-3-(1'-phenylpropyl)-2-pyrone; also named as 3-(.alpha.-ethylbenzyl)-6-phenethyl-4-hydroxy-2H-Pyran-2-one (Formula B-5: n is 2) Refer to Chart B.

A mixture of 22 mg of title product of Preparation 6 and 0.2 mL (±)-1-bromo-1-phenylpropane are heated at 100 °C for 16 h. The crude reaction mixture is purified by flash column chromatography (10 to 30 % ethyl acetate in hexane) to give 14 mg of the title product as yellow solid.

Physical characteristics are as follows:
¹H-NMR (CDCl₃) δ 0.96, 2.17, 2.67, 2.89, 4.25, 5,93, 7.10-7.45.
FAB MS [M]⁺ = 334.

### PREPARATION 7 1-(4'-Bromophenyl)-butanol (Formula C-2: R is OH) Refer to Chart C.

To a solution of 1.90 g of 4-bromobenzaldehyde (C-1) in 20 mL of tetrahydrofuran at 0 °C is added 7.5 mL of 2 M propylmagnesium bromide-tetrahydrofuran solution and stirred for 2 h. The reaction is quenched with 10 mL saturated ammonium chloride solution and 10 mL of water. The aqueous layer is extracted with diethyl ether (3 X 30 mL) and the combined organic layers are washed with brine (20 mL), dried (sodium sulfate), and then concentrated under reduced pressure. The residue is purified by flash column chromatography (5 % ethyl acetate in hexane) to give 1.97 g of the title product as a colorless oil.

Physical characteristics are as follows:
¹H-NMR (CDCl₃) δ 0.93, 1.26-1.42, 1.59-1.76, 4.64, 7.21, 7.46.

### PREPARATION 8 1-Bromo-1-(4'-bromophenyl)-butane (Formula C-2: R is Br) Refer to Chart C.

To a solution of 229 mg of title product of Preparation 7 in 2 mL of diethyl ether is added 2 mL of 48 % hydrobromic acid at room temperature and then stirred for 72 h. The aqueous layer is extracted with diethyl ether (3 X 5 mL) and the combined organic layers are treated with excess solid sodium bicarbonate. After filtration, the organic is concentrated under reduced pressure to give 135 mg of the title product and is used without any further purification.

Physical characteristics are as follows:
¹H-NMR (CDCl₃) δ 0.93, 1.30-1.38, 1.45-1.48, 2.01-2.13, 2.18-2.26, 4.91, 7.25, 7.47.

### EXAMPLE 4 3-[1'-(4"-Bromophenyl)-butyl]-4-hydroxy-6-phenethyl-2-pyrone, also named as 4-hydroxy-6-phenethyl-3-(.alpha.-propyl-p-bromobenzyl)-2H-Pyran-2-one (Formula C-4) Refer to Chart C.

A mixture of 22 mg of title product of Preparation 6 and 115 mg title product of Preparation 8 in 1 mL toluene is refluxed for 16 h. The crude mixture is then purified by gravity column chromatography (10-60 % ethyl acetate in hexane) to give 13.9 mg of the title product as a yellow solid.

Physical characteristics are as follows:
¹H-NMR (CDCl₃) δ 0.92, 1.87-1.95, 2.27-2.35, 2.67, 2.89, 4.64, 5.86, 7.26, 7.53, 7.71.
FAB MS [M]⁺ = 426.

### PREPARATION 9 4-O-Cinnamyl-4-hydroxy-6-methyl-2-pyrone (Formula D-2) Refer to Chart D.

To a solution of 2.522 g of 4-hydroxy-6-methyl-2-pyrone (D-1) in 20 mL of dimethylformamide is added 2.764 g of potassium carbonate at room temperature. The resulting suspension is heated at 90°C for 1 h, and is added 3.942 g of cinnamyl bromide. After stirring at 90 °C for 12 h, the reaction is quenched with 10 % acetic acid (5 mL). The dimethylformamide is removed under reduced pressure and the aqueous is extracted with ethyl acetate (3 x 30 mL). The combined organic layers are layer concentrated, the residue is purified by flash column chromatography (10 to 30 % ethyl acetate in hexane) to give 3.970 g of the title product.

Physical characteristics are as follows:
¹H-NMR (CDCl₃) δ 2.21, 4.65, 5.46, 5.82, 6.32, 6.72, 7.24- 7.42.
¹³C-NMR (CDCl₃) δ 19.8, 26.7, 69.2, 88.1, 100.4, 121.4, 126.0, 126.6, 128.3, 128.4, 128.6, 134.9.
FAB-MS [M-1]⁺ = 241. Anal. Found: C, 76.27; H, 5.74.

### PREPARATION 10 4-Hydroxy-6-methyl-3-(1'-phenyl-2'-propenyl)-2-pyrone (Formula D-3) Refer to Chart D.

A solution of 968 mg of the title product of Preparation 9 in toluene (10 mL) is refluxed for 8 h. The toluene is removed under reduced pressure and the residue is purified by flash column chromatography (50 to 80 % ethyl acetate in hexane) to give 817 mg of the title product.

Physical characteristics are as follows:
¹H-NMR (CDCl₃ / CD₃OD) δ 2.15, 4.97, 5.14, 5.89, 6.53, 7.15-7.31.
¹³C-NMR (CDCl₃ / CD₃OD) δ 19.3, 43.9, 48.9, 100.7, 104.0, 115.8, 126.9, 127.4, 127.8, 137.7, 142.0, 160.5, 166.2.
FAB-MS [M]⁺ = 242. Anal. Found: C, 73.92; H, 5.80.

This compound was also found to have HIV activity.

### PREPARATION 11 4-Hydroxy-6-methyl-3-(1'-phenyl-propyl)-2-pyrone (Formula D-4) Refer to Chart D.

A mixture of 2.48 g of the title product of Preparation 10 and 100 mg of 10% Pd/C in methanol (50 mL) are shaken for 10 h at room temperature under the hydrogen (30 psi) atmosphere. The catalyst is filtered through Celite and methanol is removed under reduced pressure to give 2.501 g of the title product as a white solid.

Physical characteristics are as follows:
¹H-NMR (CDCl₃) δ 0.95, 2.12, 2.18, 2.32, 4.24, 6.13, 7.22, 7.47.
FAB MS [M]⁺ = 244.

### EXAMPLE 5 4-Hydroxy-6-phenethyl-3-(1'-phenylpropyl)-2-pyrone; also named as 3-(.alpha.-ethylbenzyl)-6-phenethyl-4-hydroxy-2H-Pyran-2-one (Formula D-4) Refer to Chart D.

To a solution of 61 mg diisopropylamine in THF (2 mL) is added 0.375 mL 1.6 M n-butyl lithium-hexane solution at -20 °C and stirred 30 min, then is added 49 mg of the title product of Preparation 11 in tetrahydrofuran (3 mL). After stirring at -20 °C for 30 min, the reaction mixture is added 34 mg of benzyl bromide and allowed to stir for additional 30 min, then is quenched with 2 N hydrochloric acid (0.5 mL). The tetrahydrofuran is removed under reduced pressure and the aqueous layer is extracted with ethyl acetate (3 x 5 mL). The combined organic layers are dried (sodium sulfate), concentrated and the residue is purified by gravity column chromatography (20 to 60 % ethyl acetate in hexane) to give 53 mg of the title product as white solid.

Physical characteristics are as follows:
¹H-NMR spectrum is identical to that given in Example 3.

### EXAMPLE 6 6-(4'-Bromophenethyl)-4-hydroxy-3-(1'-phenylpropyl)-2-pyrone; also named as 6-(p-bromophenethyl)-3-(.alpha.-ethylbenzyl)-4-hydroxy-2H-Pyran-2-one (Formula E-2: R is 4-bromo-phenyl-CH₂-) Refer to Chart E.

To a solution of 61 mg diisopropylamine in THF (2 mL) is added 0.375 mL 1.6 M n-butyl lithium-hexane solution at -20 °C and stirred 30 min, then is added 49 mg of E-1 in tetrahydrofuran (3 mL). After stirring at -20 °C for 30 min, the reaction mixture is added 50 mg of 4-bromobenzyl bromide and allowed to stir for additional 30 min, then is quenched with 2 N hydrochloric acid (0.5 mL). The tetrahydrofuran is removed under reduced pressure and the aqueous layer is extracted with ethyl acetate (3 x 5 mL). The combined organic layers are dried (sodium sulfate), concentrated and the residue is purified by gravity column chromatography (20 to 60 % ethyl acetate in hexane) to give 23 mg of the title product as white solid.

Physical characteristics are as follows:
¹H-NMR (CD₃OD) δ 0.92, 2.12-2.26, 2.60, 2.80, 4.22, 6.00, 6.90-7.51.
HRMS found 413.0674.
Anal. Found: C, 64.04; H, 5.28.

### EXAMPLE 7 6-(2'-Fluorophenethyl)-4-hydroxy-3-(1'-phenylpropyl)-2-pyrone; also named as 3-(.alpha.-ethylbenzyl)-6(o-fluorophenethyl)-4-hydroxy-2H-Pyran-2-one (Formula E-2: R is 2-fluoro-phenyl-CH₂-) Refer to Chart E.

To a solution of 61 mg diisopropylamine in tetrahydrofuran (2 mL) is added 0.375 mL 1.6 M n-butyl lithium-hexane solution at -20 °C and stirred 30 min, then is added 49 mg of E-1 in tetrahydrofuran (3 mL). After stirring at -20 °C for 30 min, the reaction mixture is added 38 mg of 2-fluorobenzyl bromide and allowed to stir for additional 30 min, then is quenched with 2 N hydrochloric acid (0.5 mL). The tetrahydrofuran is removed under reduced pressure and the aqueous layer is extracted with ethyl acetate (3 x 5 mL). The combined organic layers are dried (sodium sulfate), concentrated and the residue is purified by gravity column chromatography (20 to 60 % ethyl acetate in hexane) to give 6.1 mg of the title product as white solid.

Physical characteristics are as follows:
¹H-NMR (CDCl₃) δ 0.97, 2.16, 2.69, 2.96, 4.24, 5.80, 7.02-7.45.
FAB-MS [M]⁺ = 352.

### EXAMPLE 8 4-Hydroxy-3-(1'-phenylpropyl)-6-(3'-phenylpropyl)-2-pyrone; also named as 3-(.alpha.-ethylbenzyl)-4-hydroxy-6-(3-phenylpropyl)-2H-Pyran-2-one (Formula E-2: R is phenyl-CH₂CH₂-) Refer to Chart E.

To a solution of 49 mg of E-1 in tetrahydrofuran (2 mL) is added 0.31 mL 1.6 M n-butyl lithium-hexane solution at -20 °C and stirred 30 min, then is added 37 mg of (2-bromoethyl)benzene in tetrahydrofuran (3 mL). After stirring at -20°C for 30 min, the reaction mixture is quenched with 2 N hydrochloric acid (0.5 mL). The tetrahydrofuran is removed under reduced pressure and the aqueous layer is extracted with ethyl acetate (3 x 5 mL). The combined organic layers are dried (sodium sulfate), concentrated and the residue is purified by gravity column chromatography (20 to 60 % ethyl acetate in hexane) to give 21.3 mg of the title product as white solid.

Physical characteristics are as follows:
¹H-NMR (CDCl₃) δ 0.93, 1.87, 2.17, 2.35, 2.59, 4.23, 5.99, 7.08-7.46, 9.55.
FAB-MS [M]⁺ = 348.

### EXAMPLE 9 4-Hydroxy-3-(1'-phenylpropyl)-6-propyl-2-pyrone: also named as 3-(.alpha.-ethylbenzyl)-4-hydroxy-6-propyl-2H-Pyran-2-one (Formula E-2: R is ethyl) Refer to Chart E.

To a solution of 122 mg of E-1 in tetrahydrofuran (4 mL) is added 0.67 mL of 1.6 M n-butyl lithium-hexane solution at -20 °C and stirred 30 min, then is added 78 mg of iodoethane in tetrahydrofuran (6 mL). After stirring at -20 °C for 30 min, the reaction mixture is quenched with 2 N hydrochloric acid (0.5 mL). The tetrahydrofuran is removed under reduced pressure and the aqueous layer is extracted with ethyl acetate (3 X 5 mL). The combined organic layers are dried (sodium sulfate), concentrated and the residue is purified by gravity column chromatography (20 to 60 % ethyl acetate in hexane) to give 39 mg of the tide product as white solid.

Physical characteristics are as follows:
¹HNMR (CD₃OD) δ 0.90-1.05. 1.60, 2.14-2.37, 4.24, 6.00, 7.18-7.48.
FAB-MS [M]⁺ = 372.

### EXAMPLE 10 6-Allyl-4-hydroxy-3-(1'-phenylpropyl)-2-pyrone; also named as 3-(.alpha.-ethylbenzyl)-4-hydroxy-6-(3-butenyl)-2H-Pyran-2-one (Formula E-2: R is CH₂=CHCH₂-) Refer to Chart E.

To a solution of 61 mg of diisopropylamine in tetrahydrofuran (2 mL) is added 0.94 mL of 1.6 M n-butyl lithium-hexane solution at -20 °C and stirred 30 min, then is added 122 mg of E-1 in tetrahydrofuran (3 mL). After stirring at -20 °C for 30 min, the reaction mixture is added 61 mg of allyl bromide and allowed to stir for additional 30 min, then is quenched with 2 N hydrochloric acid. The tetrahydrofuran is removed under reduced pressure and the aqueous layer is extracted with ethyl acetate (3 X 5 mL). The combined organic layers are dried (sodium sulfate), concentrated and the residue is purified by gravity column chromatography (10 to 40 % ethyl acetate in hexane) to give 39 mg of an inseparable mixture (3 : 1) of the title product and 6,6-diallyl-4hydroxy-3-(1'-phenylpropyl)-2-pyrone.

Physical characteristics are as follows:
FAB-MS [M]⁺ = 324 & 364.

### EXAMPLE 11 3-(.alpha.-ethylbenzyl)-4-hydroxy-6-[[(phenylamino)carbonyl]methyl]-2H-Pyran-2-one (Formula F-2) Refer to Chart F.

To a solution of 304 mg diisopropylamine in tetrahydrofuran (5 mL) is added 1.7 mL of 1.6 M n-butyl lithium-hexane solution at -20 °C. The resulting lithium diisopropylamide solution is stirred for 30 min then is added 220 mg of F-1 in tetrahydrofuran (5 mL), the solution turns deep red color instantaneously. The reaction mixture is added 119 mg of phenyl isocyanate and stirred at -20 °C for 1 h, at 0 °C for 2 h, then is quenched with 2 N hydrochloric acid (3 mL). The tetrahydrofuran is removed under reduced pressure, the aqueous layer is extracted with ethyl acetate (3 x 10 mL). The combined organic layers are washed with 1 N hydrochloric acid (5 mL), water (5 mL), dried (sodium sulfate), concentrated. The residue is then purified by gravity column chromatography (2 to 5 % methanol in methylene chloride) to give 247.7 mg.

Physical characteristics are as follows:
¹H-NMR (CD₃OD) δ 0.88, 2.08, 2.25, 3.57, 4.12, 6.19, 7.09-7.51.
¹³C-NMR (CD₃OD) δ 10.1, 22.1, 39.2, 40.3, 100.6, 103.8, 118.2, 122.5, 123.8, 125.8, 126.0, 126.8, 136.0, 142.3, 155.8, 165.8, 164.5, 1647.
HRMS found 363.1470.
Anal. Found: C, 71.20; H, 5.71; N, 3.70.

### EXAMPLE 12 4-Hydroxy-3-(1'-phenyl-2'-propenyl)-6-propyl-2-pyrone; also named as 4-hydroxy-6-phenethyl-3-(.alpha.-vinylbenzyl)-2H-Pyran-2-one (Formula G-2) Refer to Chart G.

To a solution of 121 mg of G-1 in tetrahydrofuran (4 mL) is added 0.67 mL of 1.6 M n-butyl lithium-hexane solution at -20 °C and stirred 30 min, then is added 86 mg of benzyl bromide in tetrahydrofuran (6 mL). After stirring at -20 °C for 30 min, the reaction mixture is quenched with 2 N hydrochloric acid (0.5 mL). The tetrahydrofuran is removed under reduced pressure and the aqueous layer is extracted with ethyl acetate (3 X 5 mL). The combined organic layers are dried (sodium sulfate), concentrated and the residue is purified by gravity column chromatography (10 to 40 % ethyl acetate in hexane) to give 26.9 mg of the title product as white solid.

Physical characteristics are as follows:
¹HNMR (CDCl₃) δ 2.05, 2.71, 2.93, 5.09, 5.30, 5.90, 6.47, 7.13-7.53.
FABMS [M]⁺ = 332.

### EXAMPLE 13 6-(1'-Benzyl-propyl)-4-hydroxy-3-(1'-phenyl-propyl)-2-pyrone; also named as 3-(.alpha.-ethylbenzyl)-6-(.alpha.ethylphenethyl)-4-hydroxy-2H-pyran-2-one (Formula H-4: R₁ is -CH₂-phenyl; R₂ is ethyl) Refer to Chart H.

To a solution of 244 mg of H-3 and 101 mg of diisopropylamine in tetrahydrofuran (25 mL) is added 1.88 mL of 1.6 M n-butyl lithium-hexane solution at 0 °C and allowed to stir for 10 min. To the reaction is added 156 mg of iodoethane and stirred for 10 min, then is added 171 mg of benzyl bromide. The reaction mixture is stirred at 0 °C for 2 h, then is quenched with 2 N hydrochloric acid (2 mL) and water (10 mL). The tetrahydrofuran is removed under reduced pressure and the aqueous layer is extracted with methylene chloride (5 X 30 mL). The combined organic layers are washed with water (10 mL), dried (sodium sulfate), concentrated and the residue is purified by gravity column chromatography (10 to 40 % ethyl acetate in hexane) to give 58 mg of the title product.

Physical characteristics are as follows:
¹H-NMR (CDCl₃) δ 0.76-1.01, 1.50-1.70, 2.05-2.30, 2.45, 2.76-2.96, 4.19-4.33, 5.95, 6.09, 7.00-7.48.
FAB-MS [M]⁺ = 362.

### EXAMPLE 14 6-(1'-Ethyl-propyl)-4-hydroxy-3-(1'-phenyl-propyl)-2-pyrone; also named as 3-(.alpha.-ethylbenzyl)-1-ethylpropyl-4-hydroxy-2H-pyran-2-one (Formula H-4: R₁ is ethyl; R₂ is ethyl) Refer to Chart H.

To a solution of 125 mg of H-3 and 56 mg of diisopropylamine in tetrahydrofuran (10 mL) is added 1.0 mL of 1.6 M n-butyl lithium-hexane solution at 0 °C and allowed to stir for 10 min. To the reaction is added 164 mg of iodoethane. The reaction mixture is stirred at 0 °C for 2 h, then is quenched with 2 N hydrochloric acid (2 mL) and water (10 mL). The tetrahydrofuran is removed under reduced pressure and the aqueous layer is extracted with methylene chloride (5 X 30 mL). The combined organic layers are washed with water (10 mL), dried (sodium sulfate), concentrated and the residue is purified by gravity column chromatography (10 to 40 % ethyl acetate in hexane) to give 89 mg of the title product.

Physical characteristics are as follows:
1H-NMR (CDCl₃) δ 0.80, 0.93, 1.56, 2.06-2.21, 2.27, 4.23, 6.10, 7.14-7.49.
FAB-MS [M]⁺ = 300.

### EXAMPLES 15-25

Following procedures analogous to those described above and using starting materials and reactants readily known and available to one of ordinary skill in organic synthesis, the following additional compounds of the present invention are prepared:
- EXAMPLE 15: 4-Hydroxy-6-(trans-4'-methoxybenzylidene)-methyl-3-(1'-phenylpropyl)-2-pyrone; also named as 3-(.alpha.-ethylbenzyl)-4-hydroxy-6-(p-methoxystyryl)-2H-Pyran-2-one
Physical characteristics are as follows:
¹H-NMR (CD₃OD) δ 0.94, 2.27, 4.61, 5.97, 6.50, 6.62, 7.12-7.56.
FAB-MS [M]⁺ = 362.
- EXAMPLE 16: 3-(.alpha.-ethylbenzyl)-4-hydroxy-6-(2-naphth-2-ylethyl)-2H-Pyran-2-one
Physical characteristics are as follows:
Mass Spectral Data (EI mode) Found: 384.1736.
- EXAMPLE 17: 3-(.alpha.-ethylbenzyl)-4-hydroxy-6-(1-ethyl-2-naphth-2-ylethyl)-2H-Pyran-2-one
Physical characteristics are as follows:
Mass Spectral Data (EI mode) Found: 412.2033.
- EXAMPLE 18: 3-(.alpha.-ethylbenzyl)-4-hydroxy-6-(2-naphth-1-ylethyl)-2H-Pyran-2-one
Physical characteristics are as follows:
Mass Spectral Data (EI mode) Found: 384.1736.
- EXAMPLE 19: 3-(.alpha.-ethylbenzyl)-4-hydroxy-6-(1-ethyl-2-naphth-1-ylethyl)-2H-Pyran-2-one
Physical characteristics are as follows:
Mass Spectral Data (EI mode) Found: 412.2041.
- EXAMPLE 20: 3-(.alpha.-cyclopropylbenzyl)-4-hydroxy-6-methyl-2H-Pyran-2-one
Physical characteristics are as follows:
Mass Spectral Data (EI mode) Found: 256.1116.
- EXAMPLE 21: 3-(.alpha.-cyclopropylbenzyl)-4-hydroxy-6-(3-phenylpropyl)-2H-Pyran-2-one
Physical characteristics are as follows:
Mass Spectral Data (EI mode) Found: 360 (low resolution only).
- EXAMPLE 22: 3-(.alpha.-cyclopropylbenzyl)-6-(1-ethyl propyl)-4-hydroxy-2H-Pyran-2-one
Physical characteristics are as follows:
Mass Spectral Data (EI mode) Found: 312.1725.
- EXAMPLE 23: 3-(.alpha.-cyclopropylbenzyl)-6-(.alpha-benzylphenethyl)-4-hydroxy-2H-Pyran-2-one
Physical characteristics are as follows:
Mass Spectral Data (EI mode) Found: 4362035.
- EXAMPLE 24: 3-(.alpha.-cyclopropylbenzyl)-4-hydroxy-6-phenethyl-2H-Pyran-2-one
Physical characteristics are as follows:
Mass Spectral Data (EI mode) Found: 346.1568.
- EXAMPLE 25: 3.(.alpha.-cyclopropylbenzyl)-6-(1-propylbutyl)-4-hydroxy-2H-Pyran-2-one
Physical characteristics are as follows:
Mass Spectral Data (EI mode) Found: 340.2042.

### PREPARATION 12 Methyl 2-(α-ethylbenzyl)-acetoacetate (Formula 1-3) Refer to Chart I.

To a stirred suspension of 216 mg of sodium hydride in 15 mL of dry tetrahydrofuran at 0 °C is added 0.86 mL of methylacetoacetate of formula I-1. After 15 min. a solution of 1.9 g of α-ethylbenzyl bromide of formula 1-2 in 5 mL of tetrahydrofuran is added. The resulting reaction mixture is heated to 65 °C for 2 days. The cooled mixture is then partitioned between diethyl ether and 1N aqueous hydrochloric acid. The aqueous phase is extracted with two more portions of diethyl ether. The combined organic phase is dried over magnesium sulfate, and then concentrated. The resulting residue is chromatographed on silica gel with 40-50% diethyl ether in hexane to give 0.53 g of diastereomeric title product.

Physical characteristics are as follows:
¹H-NMR δ 7.4-7.1, 3.9-3.8, 3.76, 3.38, 3.3-3.2, 2.3, 1.89, 1.8-1.5, 0.94-0.88, 0.77-0.67.

### PREPARATION 13 Methyl 2-(α-ethylbenzyl)-5-hydroxy-3-oxo-5-phenyl-heptanoate (Formula I-5) Refer to Chart I.

To a stirred suspension of 60 mg of sodium hydride in 5 mL of dry tetrahydrofuran at 0 °C is added a solution of 0.53 g of methyl 2-(α-ethylbenzyl)-acetoacetate of Preparation 12 in 5 mL of tetrahydrofuran. The resulting mixture is allowed to stir at room temperature for 1 hr, and then recooled to 0 °C. A solution of 1.55 mL of 1.6 M *n*-butyllithium in hexane is then slowly added. After 15 min, 0.33 mL of propiophenone of formula I-4 is added. After 30 min, 1 mL of concentrated aqueous hydrochloric acid is added, and the resulting mixture partitioned between water and diethyl ether. The aqueous phase is extracted with two more portions of diethyl ether. The combined organic phase is dried over magnesium sulfate, and then concentrated. The resulting residue was chromatographed on silica gel with 5-15% ethyl acetate in hexane to give 0.48 g of diastereomeric title product.

Physical characteristics are as follows:
¹H-NMR consistent with mixture of tautomers and diastereomers of the title product;
MS-EI: M⁺-29=368, fragments consistent with structure.

### EXAMPLE 26 6-Ethyl-3-(α-ethylbenzyl)-6-phenyl-tetrahydropyran-2,4-dione (Formula I-6) Refer to Chart I.

To a stirred solution of 149 mg of methyl 2-(α-ethylbenzyl)-5-hydroxy-3-oxo-5-phenylheptanoate of Preparation 13 in 10 mL of tetrahydrofuran is added 40 mL of 0.1 N aqueous sodium hydroxide. After 4 hr, the reaction mixture is concentrated to remove tetrahydrofuran and the remaining aqueous phase is washed with two portions of diethyl ether. The aqueous phase is cooled to 0 °C, and then acidified with 2N aqueous hydrochloric acid. It is extracted with four portions of diethyl ether, and the combined organic phase dried over magnesium sulfate, and then concentrated. The residue is chromatographed on silica gel with 30% ethyl acetate in hexane to give 69 mg of tautomeric title product.

Physical characteristics are as follows:
¹H-NMR δ 7.3-7.1. 4.0-3.9. 3.6-3.3. 3.1-2.9, 2.2-1.6, 0.9-0.5;
MS-EI: M⁺ =336.1722 found.

### PREPARATION 14 Methyl 2-(3-phenylpropyl)-acetoacetate (Formula J-3) Refer to Chart J.

To a stirred suspension of 240 mg of sodium hydride in 20 mL of dry tetrahydrofuran at 0°C is added 0.9 mL of methylacetoacetate of formula J-1. After 30 min, a solution of 1.6 mL of 1-bromo-3-phenylpropane of formula J-2 in 5 mL of tetrahydrofuran is added. The resulting reaction mixture is heated to 65 °C overnight. The cooled mixture is then partitioned between diethyl ether and 1N aqueous hydrochloric acid. The aqueous phase is extracted with two more portions of diethyl ether. The combined organic phase is dried over magnesium sulfate, and then concentrated. The resulting residue is chromatographed on silica gel with 40% ethyl acetate in hexane to give 0.75 g of the title product.

Physical characteristics are as follows:
¹H-NMR δ 7.3-7.1, 3.72, 3.44, 2.61, 2.19, 1.92-1.85, 1.67-1.26.

### PREPARATION 15 Methyl 2-(3-phenylpropyl)-5-ethyl-5-hydroxy-3-oxo-heptanoate (Formula J-5) Refer to Chart J.

To a stirred suspension of 84 mg of sodium hydride in 8 mL of dry tetrahydrofuran at 0°C is added a solution of 0.75 g of methyl 2-(3-phenylpropyl)-acetoacetate of Preparation 14 in 5 mL of tetrahydrofuran. The resulting mixture is allowed to stir at room temperature for 30 min, and then recooled to 0 °C. A solution of 2.2 mL of 1.6 M *n*-butyllithium in hexane is then slowly added. After 15 min, 0.37 mL of 3-pentanone of formula J-4 is added. After 30 min, 1 mL of concentrated aqueous hydrochloric acid is added, and the resulting mixture partitioned between water and diethyl ether. The aqueous phase is extracted with two more portions of diethyl ether. The combined organic phase is dried over magnesium sulfate, and then concentrated. The resulting residue is chromatographed on silica gel with 10-25% ethyl acetate in hexane to give 0.435 g of the title product.

Physical characteristics are as follows:
¹H-NMR δ 7.3-7.1, 3.71. 3.5-3.4, 2.7-2.3, 1.9-1.8, 1.6-1.4, 0.86-0.81.

### EXAMPLE 27 6,6-Diethyl-3-(3-phenylpropyl)-tetrahydropyran-2,4-dione (Formula J-6) Refer to Chart J.

To a stirred solution of 435 mg of methyl 2-(3-phenylpropyl)-5-ethyl-5-hydroxy-3-oxo-heptanoate of Preparation 15 in 20 mL of tetrahydrofuran is added 100 mL of 0.125 N aqueous sodium hydroxide. After 4 hr, the reaction mixture is concentrated to remove tetrahydrofuran and the remaining aqueous phase is washed with three portions of diethyl ether. The aqueous phase is cooled to 0 °C, and then acidified with 2N aqueous hydrochloric acid. It is extracted with four portions of diethyl ether, and the combined organic phase dried over magnesium sulfate, and then concentrated. The residue is chromatographed on silica gel with 40% ethyl acetate in hexane to give 316 mg of tautomeric title product.

Physical characteristics are as follows:
¹H-NMR δ 9.34, 7.3-7.1, 3.22, 2.7-2.3, 2.14, 2.05, 1.8.1.6, 0.97-0.85;
MS-EI: M⁺=288.1734 found.

### PREPARATION 16 GENERAL PROCEDURES FOR THE FOLLOWING PREPARATIONS AND EXAMPLES

### A. General Procedure for the Dianion Synthesis of 5,6-Dihydropyrones

Methyl acetoacetate is dissolved in tetrahydrofuran (0.5 M) at 0 °C. Sodium hydride (1.1 equivalent. 60% dispersion in mineral oil) is added and the reaction is stirred 15 minutes at 0 °C. Then *n*-butyl lithium (1.1 equivalents. 1.6 M solution in hexane) is added dropwise and the reaction is stirred 15 minutes at 0 °C. The ketone is dissolved in tetrahydrofuran, then added all at once to the reaction mixture. The reaction is stirred an additional hour, then poured into a saturated ammonium chloride solution. It is extracted with methylene chloride, dried over anhydrous sodium sulfate and evaporated in vacuo. The material obtained is dissolved in tetrahydrofuran (0.3 M) and a 0.1 N sodium hydroxide (1 eq.) solution is added. After stirring one hour, the mixture is extracted with ethyl acetate (1 X). The aqueous layer is adjusted to pH 3 with hydrochloric acid, then extracted with ethyl acetate, dried over anhydrous sodium sulfate and evaporated to afford the desired pyrone product.

### B. General Procedure for the Palladium Catalyzed Allylic Alkylation

Pyrone or 5,6-dihydropyrone (1 eq.), methyl-(1-phenyl-3-trimethylsilyl)-2E-propenyl)carbonate (1.1 eg.), or other appropriately substituted carbonate, palladium acetate (0.05 equivalent) and triphenylphosphine (0.20 equivalents) are suspended in distilled toluene under a carefully flushed atmosphere of nitrogen. The reaction is heated to 70 °C for 1-2 hr. Then the reaction mixture is poured into water, extracted with ethyl acetate and purified by flash column chromatography on silica gel with ethyl acetate/hexane solvent mixture to afford the desired 3-substituted pyrones or 5,6-dihydropyrone.

### C. General Procedure for Protodesilylation

The vinylsilane from B. (1 eq) and *p*-toluene sulfonic acid (0.5 eq) are refluxed in acetonitrile 1-2 hr. The reaction is poured into water, extracted with ethyl acetate, dried over anhydrous sodium sulfate, and evaporated in vacuo to provide the desilylated 3-substituted pyrone or 5,6-dihydropyrone product.

### D. General Procedure for Catalytic Hydrogenation

The olefinic 3-substituted products from C. above are dissolved in methanol, ethanol or a mixture of methanol/tetrahydrofuran. 10% Palladium hydroxide or palladium on carbon is added and the mixture hydrogenated at 40 psi for 4-6 hr. Filtration, evaporation and either flash column chromatography or crystallization give the desired product.

### EXAMPLE 28 Dimethyl 3-[(4-hydroxy-2-oxo-6-phenyl-2H-1-pyran-3-yl)(4-nitrophenyl)methyl]-1,3-propandioate

4-Hydroxy-6-phenyl-2H-pyran-2-one (0.5 g), dimethyl (4-nitrobenzylidene) malonate (0.70 g) and cesium carbonate (0.86 g) are suspended in 7 mL of tetrahydrofuran and refluxed for 2 h. Then the reaction is cooled to room temperature, diluted with water and extracted with methylene chloride. The organic layer is washed with brine, dried over anhydrous sodium sulfate and concentrated *in vacuo.* Purification by flash column chromatography (5% ethyl acetate/methylene chloride) affords 0.16 g of the desired title product as a mixture of diastereomers. (Some of the product may be lost in the aqueous layers, because they are not neutralized.)

Physical characteristics mixture of diastereomers are as follows:
¹H NMR(300 MHz, CD₃OD): δ 8.10, 7.63, 7.36, 5.31, 4.99, 3.69, 3.53, 2.86, 2.70.
HRMS Found: 456.1296.

### EXAMPLE 29 Dimethyl 3-[(4-hydroxy-2-oxo-6-phenyl-2H-1-pyran-3-yl)(3-nitrophenyl)methyl]-1,3-propandioate

Dimethyl (3-nitrobenzylidene)malonate (2.54 g), 1.83 g of 4-hydroxy-6-phenyl-2H-pyran-2-one, and cesium carbonate (3.44 g) are suspended in 24 mL of tetrahydrofuran and refluxed for 3 h. After cooling to room temperature, the reaction is poured into 2 N hydrochloric acid and extracted with ethyl acetate. The organic layers are washed with brine, dried over anhydrous sodium sulfate and concentrated in vacuo. Purification by flash column chromatography (5% methanol/chloroform) affords 4.16 g of the title product as a mixture of diastereomers (foam).

Physical characteristics are as follows:
IR(nujol): 3541-3035(br), 1754, 1738, 1703, 1646, 1529 cm⁻¹.
¹H NMR(300 MHz, CDCl₃): δ 8.11, 7.84, 7.63, 7.21-7.02, 5.11, 5.01, 4.87-4.74, 3.54, 3.54, 3.39, 3.37, 2.74-2.53.
HRMS Found: 456.1296.

### PREPARATION 17 Methyl-(1-phenyl-3-trimethylsilyl-2E-propenyl) carbonate (Formula K-2(c)) Refer to Chart K.

*t*-Butyllithium (116.4 mL, 1.7 M in pentane) is added to 188.4 mL of dried tetrahydrofuran under nitrogen and cooled to -78 °C. (2-Bromovinyl)trimethylsilane(15.18mL) is added dropwise over 10 minutes. The reaction is stirred for 15 min at -78 °C, then 10.00 g of benzaldehyde is added via syringe. The reaction is stirred at -78 °C for 45 min. The reaction is quenched with freshly distilled methyl chloroformate (7.64 mL). The mixture is allowed to warm up to room temperature and the stirring is continued for 2 h at which time it is poured into water and extracted with ethyl acetate, washed with brine, dried over anhydrous sodium sulfate and concentrated in vacuo. Purification by flash column chromatography (1%, then 2% ethyl acetate:hexane) provides 21.5 g of an oil.

Physical characteristics are as follows:
¹H NMR (300MHz, CDCl₃): δ 7.33-7.18, 6.10, 5.99, 5.90, 3.71, 0.03.
HRMS Found: 264.1189.

### PREPARATION 18 Methyl [1-(3-benzyloxyphenyl)-3-trimethylsilyl-2E-propenyl] carbonate (Formula K-2(a) and K-2(b)) Refer to Chart K.

The title product is obtained by following the one-step procedure used for the synthesis of methyl (1-phenyl-3-trimethylsilyl-2E-propenyl) carbonate of Preparation 17 but substituting 3-benzyloxybenzaldehyde (6.58 g) for benzaldehyde.

Purification by flash column chromatography (5% ethyl acetate:hexane) provides 8.18 g of oil.

Physical characteristics are as follows:
¹H NMR (300 MHz, CDCl₃): δ 7.39-7.12, 6.92-6.85, 6.06, 5.96, 5.91, Hz 5.00, 3.71, 0.01.
MS m/e (rel %): 370(85), 279(13), 204(63), 203(69), 189(80), 91(100), 73(37).

### PREPARATION 19 5,6-Dihydro-4-hydroxy-6-phenyl-6-propyl-2H-pyran-2-one (Formula K-1(a)) Refer to Chart K.

Methylacetoacetate (8.0 g) is dissolved in 60 ml tetrahydrofuran and chilled to 0 °C. Sodium hydride (3.03 g of 60% dispersion in mineral oil) is added and the mixture is stirred for 15 minutes at 0°C. *n*-Butylithium (47.4 ml of 1.6 M in hexanes) is added dropwise. After 15 minutes, 11.5 ml of butyrophenone in 5 ml tetrahydrofuran is added at once. The reaction is stirred one hour at 0 °C, then poured into saturated ammonium sulfate solution and extracted with methylene chloride. The extract is dried over anhydrous sodium sulfate and evaporated in vacuo to an oil. The oil is dissolved in 180 ml tetrahydrofuran and 680 ml 0.1 N sodium hydroxide solution is added. After 2 hr of stirring at room temperature, the mixture is extracted with ethyl acetate (1 X). The aqueous layer is adjusted to pH 3 with hydrochloric acid, then extracted with chloroform/methanol. The extract is dried over sodium sulfate and evaporated to give 12.54 g of white solid.

Physical characteristics are as follows:
Mp 130-132 °C
¹H NMR (300 MHz, CDCl₃): δ 7.40-7.25, 3.35, 3.23, 2.91, 2.88, 1.95, 1.33-1.25, 0.87.
IR (mineral oil mull): 1663, 1635, 1592, 1582, 1450 1342, 1332, 1319, 1285, 1263, 1244, cm⁻¹.
MS m/e (rel %): 232(2), 189(50), 149(11), 147(11), 105(100).

### PREPARATION 20 5,6-Dihydro-4-hydroxy-6-phenyl-6-propyl-3-[1-(3-benzyloxyphenyl)-3-trimethylsilyl-2E-propenyl]-2H-pyran-2-one (Formula K-3(a)) Refer to Chart K.

5,6-Dihydro-4-hydroxy-6-phenyl-6-propyl-2H-pyran-2-one of Preparation 19 and starting methyl [1-(3-benzyloxyphenyl)-3-trimethylsilyl-2E-propenyl] carbonate of Preparation 18 (3.50 g) are dissolved in 50 ml of distilled toluene. Palladium acetate (96 mg) and triphenylphosphine (450 mg) are added. The flask is topped with a condenser, carefully flushed with nitrogen and heated to 70 °C for 1.25 hours. The mixture is poured into water, extracted with ethyl acetate, dried over sodium sulfate and evaporated. Flash column chromatography on a silica gel column with 25% ethyl acetate:hexane affords 2.97 g of the title product as a mixture of diastereomers.

Physical characteristics are as follows:
¹H and ¹³C NMR's complicated by presence of diastereomers:
¹H NMR (300 MHz, CDCl₃): δ 7.48-7.34, 6.92, 6.85-5.18, 5.11-4.87, 3.25-2.92, 1.98, 1.54-1.17, 0.91, 0.12 and 0.00.
¹³C NMR (75 MHz, CDCl₃): δ 164.7, 159.2, 158.6, 146.3, 142.8, 142.4, 142.2, 136.5, 134.2, 132.4, 130.0, 129.3, 128.4, 128.3, 127.8, 127.7, 127.5, 127.3, 124.8, 124.7, 120.2, 119.7, 114.4, 113.4. 113.0, 105.3, 82.8. 69.7, 69.5, 46.0, 45.2, 45.0, 37.1, 36.7, 16.5, 13.8, -1.5.
MS m/e (rel %): 525 (4), 508 (3), 393 (7), 261 (6), 205 (6), 131 (6), 91 (100).

### PREPARATION 21 5.6-Dihydro-4-hydroxy-6-phenyl-6-propyl-3-[1-(3-benxyloxyphenyl)-2-propenyl]-2H-pyran-2-one

5,6-Dihydro-4-hydroxy-6-phenyl-6-propyl-3-[1-(3-benzyloxyphenyl)-3-trimethylsilyl-2E-propenyl]-2H-pyran-2-one of Preparation 20 (2.95 g) is desilylated using the general procedure of Preparation 16, C. to afford 2.53 g of the desired title product.

Physical characteristics are as follows:
¹H NMR (300 MHz, CDCl₃): δ 7.34-7.15, 6.90-6.40, 6.35-5.80, 5.28-4.22, 3.10-2.78, 1.84, 1.40-1.05, 0.77. (¹H NMR complicated by presence of diastereomers.)
HRMS Found: 454.2144.

### EXAMPLE 30 5,6-Dihydro-4-hydroxy-6-phenyl-6-propyl-3-[1-(3-hydroxyphenyl)-propyl]-2H-pyran-2-one

5,6-Dihydro-4-hydroxy-6-phenyl-6-propyl-3-[1-(3-benxyloxyphenyl)-2-propenyl]-2H-pyran-2-one (2.41 g) of Preparation 21 is hydrogenated using the general procedure of Preparation 16, D. to provide 1.88 g of the crude product. Flash column chromatography on silica gel with 2% methanol: methylene chloride affords 1.59 g of white solid (mixture of diastereomers). Two successive crystallizations from acetonitrile yield 450 mg of one diastereomer.

Physical characteristics of the cyrstalline diastereomer are as follows:
Mp 191-192 °C.
¹H NMR (300 MHz, CD₃OD): δ 7.40, 7.00, 6.81, 6.62, 3.87, 3.16. 2.25-1.90, 1.51-1.37, 1.30-1.15, 0.95, 0.73.
Analysis Found: C, 74.94; H, 7.38; N, 0.10.
MS m/e (rel %): 366(43), 348(7), 192(34), 175(78), 146(89), 118(100).
IR (mineral oil mull): 1645, 1616, 1599, 1588, 1495, 1448, 1321, 1252, 1225, 1160 cm⁻¹.

### PREPARATION 22 5,6-Dihydro-4-hydroxy-6-cyclohexyl-6-phenyl-2H-pyran-2-one (Formula K-1(b)) Refer to Chart K.

Cyclohexylphenyl ketone (14.92 g) is converted to the title product using the general procedure for synthesis of 5,6-dihydropyrones of Preparation 16, A. to yield 15.34 g of a white solid of the title product.

Physical characteristics are as follows:
Mp 168-170 °C.
¹H NMR (300 MHz, CD₃OD): δ 7.48, 3.19, 2.05-1.75. 1.45-1.05.
¹³C NMR (75 MHz, d₆-DMSO): δ 172.0, 166.4, 141.8, 127.9, 127.2, 125.5, 91.2, 85.1, 47.7, 33.5, 26.7, 26.5, 25.8.
MS m/e (rel %): 272(1), 189(62), 160(5), 147(12), 131(5), 105(100).
IR (mineral oil mull): 1675, 1629, 1598, 1474, 1447, 1349, 1297, 1280, 1265, 1255, 1240, 1215 cm⁻¹.

### PREPARATION 23 5,6-Dihydro-4-hydroxy-6-cyclohexyl-6-phenyl-3-[1-(3-benzyloxyphenyl)-3-trimethylsilyl-2E-propenyl]-2H-pyran-2-one (Formula K-3(b)) Refer to Chart K.

5,6-Dihydro-4-hydroxy-6-cyclohexyl-6-phenyl-2H-pyran-2-one (2.48 g) of Preparation 22 is reacted with methyl [1-(3-benzyloxyphenyl)-3-trimethylsilyl-2E-propenyl] carbonate of Preparation 18 following the general palladium catalyzed alkylation procedure of Preparation 16. B. Flash column chromatography on silica gel with 25% ethyl acetate:hexane affords 2.63 g of the title product as an amorphous solid (mixture of diastereomers).

Physical characteristics are as follows:
Mp 89-91 °C.
¹H NMR (300 MHz, CDCl₃): δ 7.50-7.35, 7.00-6.88, 6.50-5.69, 5.13-4.83, 3.31.2.89. 2.00-1.65, 1.42-0.90, 0.13 and 0.00. (¹H NMR complicated by presence of diastereomers.)
MS m/e (rel %): 566(5), 483(11), 393(5). 261(4), 213(8), 171(17), 129(7), 105(14), 91(100).

### PREPARATION 24 5,6-Dihydro-4-hydroxy-6-cyclohexyl-6-phenyl-3-[1-(3-benzyloxyphenyl)-2E-propenyl]-2H-pyran-2-one

5,6-Dihydro-4-hydroxy-6-cyclohexyl-6-phenyl-3-[1-(3-benzyloxyphenyl)-3-trimethylsilyl-2E-propenyl]-2H-pyran-2-one of Preparation 23 (2.62 g) is desilylated using the general procedure of Preparation 16, C. to provide 2.26 g of product.

Physical characteristics are as follows:
Mp 163-165 °C.
¹H NMR complicated by presence of diastereomers.
¹H NMR (300 MHz, CDCl₃): δ 7.36-7.17, 6.84-6.78, 6.42-5.21, 4.98-4.77, 4.05-3.98, 3.15-2.75, 1.82-1.50, 1.19-0.78.
MS m/e (rel %): 494(5), 411(35), 291(10), 270(4), 224(5), 213(7), 171(15), 105(36), 91(100).

### EXAMPLE 31 5,6-Dihydro-4-hydroxy-6-cyclohexyl-6-phenyl-3-[1-(3-hydroxyphenyl)propyl]-2H-pyran-2-one

5,6-Dihydro-4-hydroxy-6-cyclohexyl-6-phenyl-3-[1-(3-benzyloxyphenyl)-2E-propenyl]-2H-pyran-2-one (2.22 g) of Preparation 24 is hydrogenated using the general procedure of Preparation 16, D. Flash column chromatography on silica gel with 25-50% ethyl acetate:hexane yields 0.97 g of the title product as a foamy solid and a mixture of diastereomers.

Physical characteristics are as follows:
IR (mineral oil mull): 1645, 1616, 1599, 1588, 1448, 1334, 1260, 1255, 1235, 1160 cm⁻¹.
¹H NMR (300 MHz, d₆-DMSO): δ 9.01, 8.99, 7.32-7.20, 6.90-6.32, 3.83-3.45, 3.15-2.98, 1.95-1.50, 1.18-0.69, 0209 and 0.20.
HRMS Found: 406.2144.
Anal. Found: C. 75.36; H, 7.58; N, 0.10.

### PREPARATION 25 5,6-Dihydro-4-hydroxy-6-phenyl-6-propyl-3-(1-phenyl-3-trimethylsilyl-2E-propenyl)-2H-pyran-2-one

5,6-Dihydro-4-hydroxy-6-phenyl-6-propyl-2H-pyran-2-one (2.50 g) of Preparation 19 is treated with methyl (1-phenyl-3-trimethylsilyl-2E-propenyl) carbonate of Preparation 17, using the general procedure for palladium catalyzed alkylation of Preparation 16, B. to afford 6.50 g of crude product. Purification by flash column chromatography on silica gel (25% ethyl acetate:hexane) affords 2.68 g of the title product as a mixture of diastereomers.

Physical characteristics are as follows:
¹H NMR (300 MHz, CDCl₃): δ 7.44-7.05, 6.74-5.62, 5.10-5.03, 3.18-2.93, 2.05-1.90, 1.52-1.20, 0.91 and 0.89, 0.10 and 0.00. (¹H NMR complicated by the presence of diastereomers.)
MS m/e (rel %): 420(6), 402(9), 377(22), 303(14), 287(25), 274(12), 230(13), 184(34), 173(55), 73(100).

### EXAMPLE 32 5,6-Dihydro-4-hydroxy-6-phenyl-6-propyl-3-(1-phenylpropyl)-2H-pyran-2-one

A) 5,6-Dihydro-4-hydroxy-6-phenyl-6-propyl-3-(1-phenyl-3-trimethylsilyl-2E-propenyl)-2H-pyran-2-one (2.65 g) of Preparation 25 is desilylated using the general procedure of Preparation 16, C. to provide 2.21 g of the desired product, which is carried on to the next reaction without any further purification.
   Physical characteristics are as follows:
   MS m/e (rel %): 348(18), 330(19), 305(37), 287(29), 277(17), 230(17), 184(28), 173(49), 146(41), 131(49), 117(100).
B) 5,6-Dihydro-4-hydroxy-6-phenyl-6-propyl-3-(1-phenyl-2E-propenyl)-2H-pyran-2-one of Part A (2.2 g) is hydrogenated using the general procedure of Preparation 16, D. The product is crystallized from acetonitrile to give 947 mg of the desired title product. NMR indicated that one diastereomer had crystallized from the diastereomeric mixture.
   Physical characteristics of one diastereomer is as follows:
   Mp 197-198 °C.
   ¹H NMR (300 MHz, d₆-DMSO): δ 7.35-7.23, 7.12-6.98, 3.72-3.68, 3.07, 2.05-1.65, 1.30-1.10, 1.08-0.90, 0.77, 0.50.
   MS m/e (rel %): 350(5), 332(4), 306(42), 277(29), 173(91), 159(47), 146(52).
   IR (mineral oil mull): 1642, 1603, 1595, 1575, 1448, 1329, 1317, 1276 cm⁻¹.
   Analysis Found: C, 78.50; H, 7.61.

### PREPARATION 26 4-Hydroxy-1-oxaspiro[5,5]undec-3-en-2-one (Formula K-1(c)) Refer to Chart K.

Cyclohexanone (3.89 g) is converted to the title product using the general procedure for dianion synthesis of or 5,6-dihydropyran-2-ones of Preparation 16, A. Crystallization from methanol: diethyl ether provides the desired title product (3.26 g).

Physical characteristics are as follows:
Mp 118-120 °C.
¹H NMR (300 MHz, CDCl₃): δ 3.41, 2.67, 1.89-1.72, 1.60-1.32.
¹³C NMR (75 MHz, CDCl₃): δ 200.7, 167.3, 80.4, 49.4, 44.4, 36.8, 24.5, 21.4.
MS m/e (rel %): 182(41), 139(28), 126(86), 111(11), 98(44), 84(100).
IR (mineral oil mull): 1653, 1615, 1581, 1351, 1325, 1285, 1265, 1256, 1223, 1014 cm⁻¹.
Analysis Found: C, 66.24; H, 7.88, N, 0.17.

### PREPARATION 27 4-Hydroxy-1-oxa-8-[1-phenyl-3-trimethylsilyl-2E-propenyl]spiro[5,5]undec-3-en-2-one (Formula K-3(c)) Refer to Chart K.

4-Hydroxy-1-oxaspiro[5,5]undec-3-en-2-one (1.06 g) is reacted with methyl (1-phenyl-3-trimethylsilyl-2E-propenyl) carbonate of Preparation 17, using the general procedure for palladium catalyzed alkylation of Preparation 16, B. to afford 2.75 g of the crude title product. Flash column chromatography on silica gel with 5% methanol:toluene, followed by similar chromatography with 20% ethyl acetate:hexane, provides 1.26 g of the desired title product.

Physical characteristics are as follows:
¹H NMR (300 MHz, CDCl₃): δ 7.30-7.13, 6.52, 6.43, 5.65, 5.00, 2.45, 1.99-1.82, 1.79-1.20, 0.00.
MS m/e (rel %): 370(6), 352(15), 280(23), 247(19), 231(18), 198(17), 184(14), 173(21), 157(25), 73(100).
IR (mineral oil mull): 1641, 1612, 1602, 1450, 1303, 1287, 1261, 1247 cm⁻¹.

### PREPARATION 28 4-hydroxy-1-oxa-3-(1-phenyl-2-propenyl)spiro-[5,5]-undec-3-en-2-one

4-Hydroxy-1-oxa-8-[1-phenyl-3-trimethylsilyl-2E-propenyl]spiro[5,5]undec-3-en-2-one (1.25 g) of Preparation 27 is desilylated using the general procedure. Flash column chromatography on silica gel with 25% ethyl acetate:hexane provides 660 mg of a foamy solid.

Physical characteristics are as follows:
HRMS Found: 298.1567.
IR (mineral oil mull): 1631, 1493, 1449, 1431, 1338, 1331, 1316, 1304, 1286, 1268, 1244, 1232 cm⁻¹.

### EXAMPLE 33 4-Hydroxy-1-oxa-3-(1-phenylpropyl)spiro-[5,5]-undec-3-en-2-one, sodium salt

4-hydroxy-1-oxa-3-(1-phenyl-2-propenyl)spiro-[5,5]-undec-3-en-2-one (0.63 g) of Preparation 28 is hydrogenated using the general procedure to provide 640 mg of product as an oil. The oil is dissolved in methanol and 0.487 ml of sodium methoxide (25% wt in methanol) is added. The mixture is evaporated in vacuo and crystallized from acetonitrile to afford 375 mg of the title product.

Physical characteristics are as follows:
Mp 201-204°C.°C.
¹H NMR (300 MHz, CD₃OD): δ 7.55, 7.30, 7.16, 4.23, 2.52, 2.46-2.35, 2.23-2.02, 1.96-1.83, 1.78-1.50, 1.08.
¹³C NMR (75 MHz, CD₃OD): δ 183.5, 172.8, 149.0, 129.1, 128.0, 125.2, 99.1, 77.7, 45.6. 42.5, 87.2, 37.0, 26.7, 26.2, 22.9, 13.5.
FAB MS [m+H]⁺ at m/z 323.
IR (mineral oil mull): 1629, 1515, 1450, 1421, 1409, 1365, 1341, 1310 cm⁻¹.
Analysis Found: C, 68.60; H, 7.25.

### EXAMPLE 34 Dihydro-6-methyl-6-phenyl-3-(1-phenyl-2-propenyl)-2H-Pyran-2,4(3H)-dione

Physical characteristics are as follows:
Mp 147-149 °C.

### EXAMPLE 35 Dihydro-3-(1-(3-hydroxypheny))propyl]-6-phenyl-6-phenyl-2H-pyran-2,4(3H)-dione

Physical characteristics of mixture of diastereomers are as follows:
MS m/e (relative intensity): 366(100), 192 (34), 175 (78), 146 (89).

### PREPARATION 29 4-Hydroxy-6-phenethyl-2H-pyran-2-one (Formula M-2) Refer to Chart M.

To a flame-dried flask containing a stirred solution of 0.90 mL of diisopropylamine in 6 mL of anhydrous tetrahydrofuran at -78°C under an argon atmosphere is added 4.0 mL of a 1.6 M solution of n-butyllithium in hexane. The resulting solution is allowed to warm to 0°C for 20 min, and is then treated via cannula with a solution of 378 mg of commercially available 4-hydroxy-6-methyl-2-pyrone of formula M-1 in 15 mL of tetrahydrofuran. The resulting red, thick slurry is slowly treated with 6.0 mL of distilled hexamethylphosphoramide and allowed to stir for 30 min. The red, cloudy solution is then treated with 0.36 mL of benzyl bromide. The reaction quickly becomes a deep orange solution and is allowed to stir at 0°C for an additional 60 min. The mixture is quenched with excess 1 N aqueous hydrochloric acid and the resulting yellow, biphasic mixture is concentrated to remove the tetrahydrofuran. The resulting mixture is partitioned between dichloromethane and water and the acidic aqueous phase is further extracted with additional portions of dichloromethane. The combined organic phases is dried over magnesium sulfate and then concentrated under reduced pressure. The resulting material is diluted with a large volume of diethyl ether and washed with dilute aqueous hydrochloric acid. The ethereal phase is washed with two additional portions of hydrochloric acid, once with brine, dried over magnesium sulfate, and finally concentrated under reduced pressure. The residue is flash column chromatographed on silica gel 60 (230-400 mesh) eluting with 1% acetic acid and 20% to 40% ethyl acetate in dichloromethane to give 440 mg of the title compouund as a tan solid.

Physical characteristics are as follows:
¹H NMR δ 2.7, 3.0, 5.46, 5.84, 7.1-7.3.
TLC R_{f} 0.38 (1% acetic acid and 25% ethyl acetate in dichloromethane.)
Mp 137-138 °C.

### PREPARATION 30 6-(α-ethyl-phenethyl)-4-hydroxy-2H-pyran-2-one (Formula M-3) Refer to Chart M.

To a cold (-78°C) stirred solution of 0.29 ml of diisopropylamine in 4 ml of dry tetrahydrofuran, under argon, is added 1.2 ml of a 1.6 M solution of n-butyllithium in hexane. The solution is warmed to 0°C, kept at that temperature for ten minutes, then cooled to -30°C. Into this solution is cannulated a solution of 189 mg of compound of Preparation 29 in 4 ml of tetrahydrofuran. The resulting heterogeneous mixture is warmed to 0°, and sufficient hexamethylphosphoramide (ca 1 ml) is added to render the mixture mostly homogeneous. After the mixture is stirred for 30 minutes at 0°C, 77 µL of ethyl iodide is added dropwise. After another 90 minutes, the reaction is quenched with excess 1N hydrochloric acid, and tetrahydrofuran is removed under reduced pressure. The residue is extracted with three portions of ethyl acetate, and the combined organic extract washed with dilute hydrochloric acid, dried (magnesium sulfate), and concentrated under reduced pressure. The residue is flash chromatographed on silica gel 60 (230-400 mesh) using 1% acetic acid and 25% ethyl acetate in dichloromethane to provide 182 mg of the title compound.

Physical characteristics are as follows:
¹H NMR δ 0.85, 1.6, 2.6, 2.9, 5.59, 5.86, 7.0-7.3.
FAB MS [m+H]=245.1185.
TLC R_{f} 0.33 (1% acetic acid and 25% ethyl acetate in dichloromethane.)

### EXAMPLE 36 3-(α-Cyclopropyl-meta-(benzyloxycarbonylamino)benzyl)-6-(α-ethyl-phenethyl)-4-hydroxy-2H-pyran-2-one (Formula M-4) Refer to Chart M.

A mixture of 181 mg of the title compound of Preparation 30, 220 mg of the title compound of Preparation 37 (vide infra), 28 mg of p-toluenesulfonic acid monohydrate, and 600 mg of 3Å molecular sieves in 2 ml of benzene is refluxed under argon for 21 hours, then cooled and filtered through Celite. The filtrate is concentrated under reduced pressure, and the residue flash chromatographed on silica gel 60 (230-400 mesh) using 50-100% ethyl acetate in hexane to provide 250 mg of a mixture of materials. This is re-subjected to silica gel chromatography, using 5-20% ethyl acetate in dichloromethane, to afford 154 mg (40%) of the title compound.

Physical characteristics are as follows:
¹H NMR δ 0.26, 0.48, 0.6-7, 0.81, 1.6, 1.8, 2.5, 2.7, 2.9, 3.48, 5.14, 5.86, 6.81, 7.0-7.5, 9.46.
EI HRMS m/z=523.2350.
TLC R_{f} 0.27 (5% ethyl acetate in dichloromethane.)

### PREPARATION 31 3-(α-Cyclopropyl-meta-aminobenzyl)-6-(α-ethyl-phenethyl)4-hydroxy-2H-pyran-2-one (Formula M-5) Refer to Chart M.

A mixture of 146 mg of the title compound of Example 36 and 50 mg of 5% palladium on carbon in 2 ml of methanol is shaken under 40 psi of hydrogen for two hours, then filtered through Celite. The filtrate is concentrated under reduced pressure to give 105 mg (96%) of the title compound.

Physical characteristics are as follows:
¹H NMR δ 0.25, 0.5, 0.65, 0.81, 1.6, 2.5, 2.7, 2.9, 3.4, 5.79, 6.5, 6.8-7.3.
TLC R_{f} 0.38 (30% ethyl acetate in dichloromethane).

### EXAMPLE 37 N-(3-(Cyclopropyl-[6-(1-ethyl-phenethyl)-4-hydroxy-2-oxo-2H-pyran-3-yl]-methyl)-phenyl)-3-(tert-butyloxycarbonylamino)-propionamide (Formula M-6) Refer to Chart M.

To a stirred solution of 50 mg of the title compound of Preparation 31 and 29 mg of *tert*-butyloxycarbonyl-β-alanine in 0.5 ml of dichloromethane is added 22 µL of diisopropylcarbodiimide. The solution is stirred for 18 hours, then flash chromatographed on silica gel 60 (230-400 mesh) using 5-10% methanol and 30% ethyl acetate in dichloromethane. Obtained is 71 mg of product contaminated by byproducts of the coupling reaction. The material is re-chromatographed on silica using 30-70% ethyl acetate in dichloromethane to afford 34 mg (47%) of the title compound as a white solid.

Physical characteristics are as follows:
¹H NMR δ 0.2, 0.5, 0.6, 0.81, 1.41, 1.6, 1.8, 2.4, 2.8, 2.9, 3.3, 3.4, 5.4, 5.8, 7.0-7.3, 7.5.
FAB HRMS [m+H]=561.2995.
TLC R_{f} 0.17 (30% ethyl acetate in dichloromethane.)

### PREPARATION 32 6-(α-Cyclopropylmethyl-cyclopropylethyl)-4-hydroxy-2H-pyran-2-one (Formula N-2) Refer to Chart N.

To a cold (-78°C) stirred solution of 1.5 ml of diisopropylamine in 9 ml of dry tetrahydrofuran, under argon, is added 6.2 ml of a 1.6 M solution of n-butyllithium in hexane. The solution is warmed to 0°C, and into it is cannulated a solution of 378 mg of commercially available 4-hydroxy-6-methyl-2-pyrone of formula N-1 in 8 ml of hexamethylphosphoramide. After 30 minutes at 0°C, 0.32 ml of bromomethylcyclopropane is added; after another ten minutes, a second portion of the same amount is added. The reaction is stirred and allowed to warm to room temperature overnight, then is partitioned between ethyl acetate and excess dilute hydrochloric acid. The organic phase is washed with brine, dried over magnesium sulfate, and concentrated under reduced pressure. The residue is flash chromatographed on silica gel 60 (230-400 mesh) using 1% acetic acid and 25% ethyl acetate in dichloromethane to provide 371 mg of the title compound, along with 206 mg of monoalkylated material.

Physical characteristics are as follows.
¹H NMR δ 0.0, 0.4, 0.6, 1.5, 1.6, 2.2, 5.6, 6.1, 7.2-7.3, 11.5.
EI MS m/z=234.
TLC R_{f} 0.29 (1% acetic acid and 25% ethyl acetate in dichloromethane.)

### EXAMPLE 38 3-(α-Cyclopropyl-meta-(tert-butyloxycarbonylamino)benzyl)-6-(α-cyclopropylmethyl-cyclopropylethyl)-4-hydroxy-2H-pyran-2-one (Formula N-3) Refer to Chart N.

A mixture of 367 mg of the title compound of Preparation 32, 470 mg of the title compound of Preparation 37 (vide infra), 60 mg of p-toluenesulfonic acid monohydrate, and 1 g of 3 Å sieves in 5 ml of benzene is heated with stirring overnight under argon. The mixture is diluted with dichloromethane and ether and filtered through a pad of sodium sulfate. After the solvent is removed under reduced pressure, the residue is flash chromatographed on silica gel 60 (230-400 mesh) using 5-20% ethyl acetate in dichloromethane to afford 399 mg of the title compound.

Physical characteristics are as follows:
¹H NMR δ .06, 0.3, 0.5, 1.4, 1.5, 2.5, 3.5, 5.1, 7.2-7.4.
EI HRMS m/z=513,2513.
TLC R_{f} 0.28 (5% ethyl acetate in dichloromethane.)

### PREPARATION 33 3-(α-Cyclopropyl-meta-aminobenzyl)-6-(α-cyclopropylmethyl-cyclopropylethyl)-4-hydroxy-2H-pyran-2-one (Formula N-4) Refer to Chart N.

A mixture of 391 mg of the title compound of Example 38 and 100 mg of 5% palladium on carbon in 10 ml of methanol is shaken overnight under 40 psi of hydrogen. The mixture is then filtered through Celite, and the filtrate is concentrated under reduced pressure to provide 280 mg of the tide compound.

Physical characteristics are as follows:
¹H NMR δ 0.0, 0.2-0.7, 1.4, 1.6, 1.8, 2.6, 6.8. 7.2-7.4.
TLC R_{f} 0.38 (30% ethyl acetate in dichloromethane.)

### EXAMPLE 39 N-(3-(Cyclopropyl-[6-(2-cyclopropyl-1-cyclopropylmethyl-ethyl)-4-hydroxy-2-oxo-2H-pyran-3-yl]-methyl)-phenyl)-3-indol-1-yl-propionamide (Formula N-5) Refer to Chart N.

A stirred solution of 50 mg of the title compound of Preparation 33 and 27 mg of 3-(1-indolyl)-propionic acid in 1 ml of dichloromethane and 0.1 ml of dimethylforamide is cooled to 0°C, and to this is added 23 µL of diisopropylcarbodiimide. The solution is allowed to warm slowly overnight, and the following day solvent is removed under reduced pressure. The residue is flash chromatographed on silica gel 60 (230-400 mesh) using 0-4% methanol and 20% ethyl acetate in dichloromethane to afford 24 mg of the title compound as a white solid.

Physical characteristics are as follows:
¹H NMR δ -0.06, 0.2-0.7, 1.4, 1.5, 2.6, 3.5, 4.4, 6.06, 6.41, 7.0-7.7, 7.80.
TLC R_{f} 0.41 (20% ethyl acetate in dichloromethane.)

### PREPARATION 34 Cyclopropyl meta-nitrophenyl ketone (Formula O-2) Refer to Chart O.

A 250 ml three necked flask fitted with thermometer and addition funnel is charged with 130 ml of fuming 90% nitric acid. This is cooled to -10°C in a -40°C acetone bath, and into the stirred liquid is added dropwise 21 ml of commercially available cyclopropyl phenyl ketone of formula O-1. The rate of addition is regulated to maintain the reaction temperature at about -10°C. The completed clear yellow solution is stirred for another 10 minutes at -10°C, then poured into 1 L of crushed ice. The precipitated gummy yellow solid is extracted with 700 ml of toluene, and the extract is washed twice with 5% sodium hydroxide solution and once with brine, and dried over magnesium sulfate. After the solvent was removed under reduced pressure, the residue was recrystallized from methanol at -25°C to give 14.6 g of the title compound as dense, pate yellow prisms. The mother liquor contained substantial amounts of the *ortho* isomer.

Physical characteristics are as follows:
¹H NMR δ 1.2, 1.3, 2.7, 7.70, 8.3, 8.4, 8.85.
IR 1664, 1529, 1352, 1225, 1082, 1017, 852, 689 cm⁻¹.
Anal. Found: C, 62.89; H, 4.73; N, 7.32.
EI MS m/z 191.
TLC R_{f} 0.32 (25% ethyl acetate in hexane.)

### PREPARATION 35 meta-Aminophenyl cyclopropyl ketone (Formula O-3) Refer to Chart O.

A solution of 5.76 g of the title compound of Preparation 34 is prepared with the aid of heat in 100 ml of methanol. To this is added 450 mg of 5% platinum on carbon catalyst, and the mixture is stirred vigorously under 1 atmosphere of hydrogen. After 5 hours, the mixture is filtered through a pad of Celite and the filtrate concentrated under reduced pressure to afford 4.89 g of the title compound as a greenish oil.

Physical characteristics are as follows:
¹H NMR δ 1.0. 1.2. 2.6, 3.9, 6.8, 7.2, 7.4.
TLC R_{f} 0.50 (80% ethyl acetate in hexane.)

### PREPARATION 36 meta-Benzyloxycarbonylaminophenyl cyclopropyl ketone (Formula O-4) Refer to Chart O.

To a cold (0°C), stirred solution of 4.89 g of the title compound of Preparation 35 and 6.3 ml of diisopropylethylamine in 90 ml of dichloromethane is added dropwise 4.7 ml of benzyl chloroformate, and the completed solution is allowed to warm to room temperature. After 4 hours, the mixture is washed with dilute hydrochloric acid, and the aqueous phase extracted with two additional portions of dichloromethane. The combined organic phase is dried over magnesium sulfate and concentrated under reduced pressure to a yellow solid. This is triturated with two 30 ml portions of hexane, these being discarded, and the remaining solid is dried under vacuum to afford 8.74 g of the title compound.

Physical characteristics are as follows:
TLC R_{f} 0.45 (5% ethyl acetate in dichloromethane.)

### PREPARATION 37 meta-Benzyloxycarbonylaminophenyl cyclopropyl carbinol (Formula O-5) Refer to Chart O.

To a stirred solution of 8.74 g of compound O-4 in 100 ml of tetrahydrofuran and 100 ml of ethanol is added, in portions, 4.5 g of sodium borohydride. After 3 hours at room temperature, the mixture is cooled in ice for the addition of 100 ml of 1N hydrochloric acid. The mixture is thrice extracted with dichloromethane, and the combined extract dried over magnesium sulfate. Solvent is removed under reduced pressure, and the residue flash chromatographed on silica gel 60 (230-400 mesh) using 40% ethyl acetate in hexane to provide 8.48 g of the title compound as a white crystalline solid. This is optionally recrystallized from ethyl acetate-hexane.

Physical characteristics are as follows:
¹H NMR δ 0.3-0.6, 1.1, 2.35, 3.92, 5.17, 7.1, 7.2-7.4.
IR 1693, 1599, 1559, 1449, 1235, 1054, 697 cm⁻¹.
Anal. Found: C, 72.57; H, 6.51; N. 4.61.

### EXAMPLES 40-76

Following procedures analogous to those described above and using starting materials and reagents readily known and available to one of ordinary skill in the art of organic synthesis, the following additional compounds of the present invention are prepared:
- EXAMPLE 40: 4-Hydroxy-3-(1-phenyl-propyl)-6-(1-propyl-butyl)-pyran-2-one
Physical characteristics are as follows:
Melting point: 132-134 °C.
- EXAMPLE 41: 4-Hydroxy-3-(1-phenyl-allyl)-6-(1-propyl-butyl)-pyran-2-one
Physical characteristics are as follows:
Melting point: 114-116 °C.
- EXAMPLE 42: 3-(5-(Cyclopropyl-phenyl-methyl)-4-hydroxy-6-oxo-6H-pyran-2-yl)-propionic acid tert-butyl ester
Physical characteristics are as follows:
Anal. Found: C, 71.16; H, 7.19.
- EXAMPLE 43: 3-(Cyclopropyl-phenyl-methyl)-6-(2-(3,5-dimethyl-isoxazol-4-yl)-ethyl)-4-hydroxy-pyran-2-one
Physical characteristics are as follows:
Anal. Found: C, 71.07; H, 6.17; N. 4.28.
- EXAMPLE 44: 6-(2-(5-Tert-butyl-(1,2,4)oxadiazol-3-yl)-ethyl)-3-(cyclopropyl-phenyl-methyl)-4-hydroxy-pyran-2-one
Physical characteristics are as follows:
Anal. Found: C, 70.26; H, 6.68, N, 6.10.
HRMS Found: 394.1896.
- EXAMPLE 45: 3-(Cyclopropyl-phenyl-methyl)-4-hydroxy-6-(1-(tetrahydro-furan-3-ylmethyl)-propyl)-pyran-2-one
Physical characteristics are as follows:
Anal. Found: C, 74.51; H, 7.63
HRMS Found: 368.1981.
- EXAMPLE 46: 3-(Cyclopropyl-phenyl-methyl)-4-hydroxy-6-(2-phenyl-1-pyridin-2-ylmethyl-ethyl)-pyran-2-one
Physical characteristics are as follows:
HRMS Found: 437.1989.
- EXAMPLE 47: 3-(Cyclopropyl-phenyl-methyl)-6-(2-(1,3)dioxolan-2-yl-ethyl)-4-hydroxy-pyran-2-one
Physical characteristics are as follows:
HRMS Found: 342.1462.
- EXAMPLE 48: 3-(Cyclopropyl-phenyl-methyl)-4-hydroxy-6-(4-morpholin-4-yl-butyl)-pyran-2-one
Physical characteristics are as follows:
HRMS Found: 3832108.
- EXAMPLE 49: 3-(Cyclopropyl-phenyl-methyl)-4-hydroxy-6-(2-pyridin-2-yl-ethyl)-pyran-2-one
Physical characteristics are as follows:
HRMS Found: 347.1531.
- EXAMPLE 50: 3-(Cyclopropyl-phenyl-methyl)-4-hydroxy-6-(2-(2-methyl-thiazol-4-yl)-ethyl)-pyran-2-one
Physical characteristics are as follows:
HRMS Found: 367.1247.
- EXAMPLE 51: 3-(Cyclopropyl-phenyl-methyl)-4-hydroxy-6-(2-quinolin-2-yl-ethyl)-pyran-2-one
Physical characteristics are as follows:
HRMS Found: 397.1688.
- EXAMPLE 52: 6-(1-(5-Chloro-thiophen-2-ylmethyl)-propyl)-3-(cyclopropyl-phenyl-methyl)-4-hydroxy-pyran-2-one
Physical characteristics are as follows:
HRMS Found: 414.1042.
- EXAMPLE 53: 6-(3-Cloro-propyl)3-(cyclopropyl-phenyl-methyl)-4-hydroxy-pyran-2-one
Physical characteristics are as follows:
Anal. Found: C, 67.61; H, 5.89.
- EXAMPLE 54: 3-(Cyclopropyl-phenyl-methyl)-6-(1-(3,5-dimethyl-isoxazol-4-ylmethyl)-propyl)-4-hydroxy-pyran-2-one
Physical characteristics are as follows:
HRMS Found: 393.1932.
- EXAMPLE 55: 6-(1-(2-(4-Cloro-phenyl)-thiazol-4-ylmethyl)-propyl)-3-(cyclopropyl-phenyl-methyl)-4-hydroxy-pyran-2-one
Physical characteristics are as follows:
Anal. Found: C, 68.25; H, 5.58, N, 2.52.
- EXAMPLE 56: 3-(Cyclopropyl-phenyl-methyl)-4-hydroxy-6-(1-(tetrahydro-furan-2-ylmethyl)-propyl)-pyran-2-one
Physical characteristics are as follows:
HRMS Found: 368.1984.
- EXAMPLE 57: 3-(Cyclopropyl-phenyl-methyl)4-hydroxy-6-(1-thiophen-2-ylmethyl-propyl)-pyran-2-one
Physical characteristics are as follows:
Anal. Found: C, 72.33, H, 6.32.
- EXAMPLE 58: 3-(Cyclopropyl-phenyl-methyl)-4-hydroxy-6-(1-tetrahydro-pyran-4-ylmethyl)-propyl)-pyran-2-one
Physical characteristics are as follows:
HRMS Found: 382.2142.
- EXAMPLE 59: 3-(Cyclopropyl-phenyl-methyl)-6-(1-furan-2-ylmethyl-propyl)4-hydroxy-pyran-2-one
Physical characteristics are as follows:
HRMS Found: 364.1673.
- EXAMPLE 60: 3-(Cyclopropyl-phenyl-methyl)-6-(1-(1,3)dioxolan-2-ylmethyl-propyl)-4-hydroxy-pyran-2-one
Physical characteristics are as follows:
HRMS Found: 370.1775.
- EXAMPLE 61: 3-(Cyclopropyl-phenyl-methyl)-4-hydroxy-6-(1-(tetrahydro-pyran-3-ylmethyl)-propyl)-pyran-2-one
Physical characteristics are as follows:
HRMS Found: 382.2140.
- EXAMPLE 62: 3-(Cyclopropyl-phenyl-methyl)-4-hydroxy-6-(1-(tetrahydro-pyran-2-ylmethyl)-propyl)-pyran-2-one
Physical characteristics are as follows:
HRMS Found: 382.2141.
- EXAMPLE 63: 3-(Cyclopropyl-phenyl-methyl)-4-hydroxy-6-(1-pyridin-2-ylmethyl-propyl)-pyran-2-one
Physical characteristics are as follows:
HRMS Found: 375.1837.
- EXAMPLE 64: 6-(4-Chloro-1-ethyl-butyl)-3-(cyclopropyl-phenyl-methyl)-4-hydroxy-pyran-2-one
Physical characteristics are as follows:
Anal. Found: C, 69.65; H, 7.02; N, 8.26.
HRMS Found: 360.1492.
- EXAMPLE 65: 3-(Cyclopropyl-phenyl-methyl)-6-(3-(1,3)dioxan-2-yl-1-ethyl-propyl)-4-hydroxy-pyran-2-one
Physical characteristics are as follows:
HRMS Found: 398.2094.
- EXAMPLE 66: 6-(3-Chloro-1-ethyl-propyl)-3-(cyclopropyl-phenyl-methyl)-4-hydroxy-pyran-2-one
Physical characteristics are as follows:
HRMS Found: 346.1332.
- EXAMPLE 67: 3-(Cyclopropyl-phenyl-methyl)-6-[ethyl-3-(tetrahydro-pyran-2-yloxy)-propyl]-4-hydroxy-pyran-2-one
Physical characteristics are as follows:
HRMS Found: 412.2237.
- EXAMPLE 68: 3-(Cyclopropyl-phenyl-methyl)-4-hydroxy-6-(1-pyridin-4-ylmethyl-propyl)-pyran-2-one
Physical characteristics are as follows:
HRMS Found: 375.1829.
- EXAMPLE 69: 3-(Cyclopropyl-phenyl-methyl)-6-(1-ethyl-3-morpholin-4-yl-3-oxo-propyl)-4-hydroxy-pyran-2-one
Physical characteristics are as follows:
HRMS Found: 411.2057.
- EXAMPLE 70: 3-(Cyclopropyl-phenyl-methyl)-4-hydroxy-6-(1-pyridin-3-ylmethyl-propyl)-pyran-2-one
Physical characteristics are as follows:
HRMS Found: 375.1827.
- EXAMPLE 71: 3-(Cyclopropyl-phenyl-methyl)-6-(1-ethyl-3-thiophen-1-yl-propyl)-4-hydroxy-pyran-2-one
Physical characteristics are as follows:
HRMS Found: 394.1593.
- EXAMPLE 72: 3-(Cyclopropyl-phenyl-methyl)-4-hydroxy-6-[1-(tetrahydro-pyran-3-ylmethyl)-butyl]-pyran-2-one
Physical characteristics are as follows:
HRMS Found: 396.2298.
- EXAMPLE 73: 3-(Cyclopropyl-phenyl-methyl)-6-(1-ethyl-4-morpholin-4-yl-butyl)-4-hydroxy-pyran-2-one
Physical characteristics are as follows:
HRMS Found: 41.2404.
- EXAMPLE 74: 3-(Cyclopropyl-phenyl-methyl)-6-[1-(2,3-dihydro-benzo[1,4]dioxin-2-ylmethyl)-propyl]-4-hydroxy-pyran-2-one
Physical characteristics are as follows:
HRMS Found: 432.1931.
- EXAMPLE 75: 3-(Cyclopropyl-phenyl-methyl)-4-hydroxy-6-isobutyl-pyran-2-one
Physical characteristics are as follows:
HRMS Found: 298.1566.
- EXAMPLE 76: 3-(Cyclopropyl-phenyl-methyl)-6-[1-(5,6-dihydro-2H-pyran-3-ylmethyl)-propyl]-4-hydroxy-pyran-2-one
Physical characteristics are as follows:
HRMS Found: 380.1987.

### PREPARATION 38 5-Bromo-4-hydroxy-6-methyl-3-(1-phenyl-propyl)-pyran-2-one (Formula P-2) Refer to Chart P.

To a flask containing a suspension of 110 mg of 5-bromo-4-hydroxy-6-methyl-pyran-2-one of formula P-1 (preparation of this material is described in *Syn. Comm.* 1984, *14*, 521) and 30 mg of *p*-toluenesulfonic acid hydrate in 10 mL of benzene is added 0.25 mL of commercial 1-phenyl-1-propanol. The flask is equipped with an addition funnel containing 3 Å molecular sieves (pre-wet with benzene) and a reflux condenser under an argon balloon. The mixture is placed in an 100°C oil bath. After 12 h, the resulting solution is cooled to room temperature and partioned between diethyl ether and excess 1 N aqueous sodium hydroxide. The basic aqueous phase is washed with diethyl ether, acidified to pH1 with 6 N aqueous hydrochloric acid and the resulting precipitant repeatedly extracted chloroform-methanol. The combined organic layers are washed with brine, dried (magnesium sulfate), and concentrated under reduced pressure. The residue is purified by flash column chromatography on silica gel eluting with 100% ethyl acetate to 20% methanol in ethyl acetate to afford 123 mg of the title product as a white solid.

Physical characteristics are as follows:
¹H NMR δ 7.42, 7.22, 7.12, 4.20, 2.32, 2.28, 2.09, 0.90;
EI-MS: [M⁺]=322.0216 found

### EXAMPLE 77 5-Bromo-6-(2-cyclopropyl-cyclopropylmethyl-ethyl)-4-hydroxy-3-(1-phenyl-propyl)-pyran-2-one (Formula P-3) Refer to Chart P.

To a flame-dried flask under an argon atmosphere is added 0.23 mL of distilled diisopropylamine and 1.6 mL of dry tetrahydrofuran. The solution is cooled to -78°C and treated with 1.0 mL (1.6 M in hexane) of *n*-butyllithium. The solution is warmed to 0°C for 15 min, then cooled to -30°C. The lithium diisopropylamine solution is treated with 162 mg of 5-bromo-4-hydroxy-6-methyl-3-(1-phenyl-propyl)-pyran-2-one (the title product of Preparation 38) as a solution in 2.5 mL of tetrahydrofuran. The resulting orange-red solution is stirred for 30 min as the bath temperature rises to -20°C. The solution is then treated with 0.11 mL of commercial (bromomethyl)cyclopropane. The reaction mixture is allowed to warm to 0°C over 3 h. The reaction is then quenched with excess 1 N aqueous hydrochloric acid and partioned between ethyl acetate and water. The aqueous phase is extracted with ethyl acetate and the combined organic layers are dried (magnesium sulfate) and then concentrated under reduced pressure. The residue is purified by flash column chromatography on silica gel eluting with 2% to 10% ethyl acetate in dichloromethane to afford 98 mg of the title product as a tan oil.

Physical characteristics are as follows:
¹H NMR δ 7.45, 7.3-7.1, 6.34, 4.24, 3.31, 2.3-2.1, 1.6-1.2, 0.91, 0.55, 0.35, 0.4- -0.1;
EI-MS: [M⁺]=430.1134 found.

### PREPARATION 39 3-(Cyclopropyl-phenyl-methyl)-4-hydroxy-5-[2-(2-methoxy-ethoxy)-ethyl]-6-methyl-pyran-2-one (Formula Q-2) Refer to Chart Q.

To a flame-dried flask under an argon atmosphere is added 0.60 mL of distilled diisopropylamine and 4.0 mL of dry tetrahydrofuran. The solution is cooled to -78°C and treated with 2.7 mL (1.6 M in hexane) of *n*-butyllithium. The solution is warmed to 0°C for 20 min, then cooled to -40°C. The lithium diisopropylamine solution is treated with 513 mg of 3-(cyclopropyl-phenyl-methyl)-4-hydroxy-6-methyl-pyran-2-one of formula Q-1 (prepared as described in Example 20) as a solution in 17 mL of tetrahydrofuran. The orange dianion is formed over 20 min as the bath temperature rises to -20°C. The solution is then treated with 565 mg of 2-(2-methoxy-ethoxy)-ethyl tosylate as a solution in 2 mL of tetrahydrofuran. The reaction mixture is allowed to slowly warm to 0°C over 2 h. The reaction is quenched with excess 1 N aqueous hydrochloric acid and concentrated under reduced pressure. The residue is partioned between ethyl acetate and water. The aqueous phase is extracted with additional portions of ethyl acetate. The combined organic layers are dried (magnesium sulfate) and then concentrated under reduced pressure. The residue is purified by flash column chromatography on silica gel eluting with 50% to 100% ethyl acetate in hexane to afford 105 mg the title product as a tan oil.

Physical characteristics are as follows:
¹H NMR δ 8.75, 7.50, 7.26, 7.17, 3.68, 3.48, 3.33, 2.66, 2.20, 2.0-1.8, 0.68, 0.50, 0.28;
EI-MS: [M⁺]=358.1777 found.

### EXAMPLE 78 3-(Cyclopropyl-phenyl-methyl)-4-hydroxy-5-[2-(2-methoxy-ethoxy)ethyl]-6-propyl-pyran-2-one (Formula Q-3) Refer to Chart Q.

To a flame-dried flask under an argon atmosphere is added 0.125 mL of distilled diisopropylamine and 1.0 mL of dry tetrahydrofuran. The solution is cooled to -78°C and treated with 0.55 mL (1.6 M in hexane) of *n*-butyllithium. The solution is warmed to 0°C for 15 min, then cooled to -30°C. The lithium diisopropylamine solution is treated with 93 mg of 3-(cyclopropyl-phenyl-methyl)-4-hydroxy-5-[2-(2-methoxy-ethoxy)-ethyl]-6-methyl-pyran-2-one (the title product of Preparation 39) as a solution in 2.5 mL of tetrahydrofuran. The orange dianion is formed over 30 min as the bath temperature rises to -20°C. The solution is then treated with 55 µL of ethyl iodide. The reaction mixture is allowed to warm to 0°C over 2 h. The reaction is quenched with excess 1 N aqueous hydrochloric acid and partioned between ethyl acetate and brine. The aqueous phase is extracted with additional portions of ethyl acetate. The combined organic layers are dried (magnesium sulfate) and then concentrated under reduced pressure. The residue is purified by flash column chromatography on silica gel eluting with 2% to 6% ethyl acetate in dichloromethane to afford 51 mg of the title product as a tan oil.

Physical characteristics are as follows:
¹H NMR δ 8.77, 7.54, 7.3-7.1, 3.67, 3.46, 3.31, 2.66, 2.43, 1.95, 1.66, 0.94, 0.67, 0.49, 0.26;
EI-MS: [M⁺]=386.2097 found.

### PREPARATION 40 3-Benzyl-4-phenyl-but-2-enoic acid, tert-butyl ester (Formula R-2) Refer to Chart R.

To a flame-dried flask under an argon atmosphere is added 400 mg of sodium hydride (60% by weight in oil) and 5 mL of dry benzene. The grey suspension is cooled to 5°C and treated dropwise with 2.0 mL of the reagent *tert*-butyl P,P-dimethylphosphonoacetate to control reaction. After 10 min, the mixture is warmed to room temperature. After an additional 1 h, the tan cloudy solution is cooled to 5°C and treated with 2.1 g of commercial 1,3-diphenylacetone of formula R-1. After 10 min, the reaction mixture is allowed to warm to room temperature and stirred overnight. The resulting orange cloudy suspension is partioned between diethyl ether and cold aqueous phosphate buffer. The aqueous phase is extracted with an additional portion of diethyl ether. The combined organic layers are washed with brine, dried (magnesium sulfate) and then concentrated under reduced pressure. The resulting residue is purified by flash column chromatography on silica gel eluting with 30% to 40% dichloromethane in hexane to afford 2.96 g of the title product as a clear, colorless oil.

Physical characteristics are as follows:
¹H NMR δ 7.4-7.1, 7.10, 5.69, 3.94, 3.29, 1.48;
EI-MS: [M⁺]=308.

### PREPARATION 41 3-Benzyl-4-phenyl-butanoic acid, tert-butyl ester (Formula R-3) Refer to Chart R.

To a Parr bottle containing 200 mg of 5% platinum on carbon is added 2.81 g of *tert*-butyl 3-benzyl-4-phenyl-butenoate (the title product of Preparation 40) as a solution in 30 mL of ethyl acetate. The mixture is hydrogenated under 50 psi of hydrogen gas overnight. The black suspension is filtered through a pad of Celite with ethyl acetate washings and the filtrate concentrated under reduced pressure. The residue is purified by flash column chromatography on silica gel eluting with 50% dichloromethane in hexane to afford 2.41 g of the title product as a white solid. An analytical sample is prepared by recrystallization from methanol.

Physical characteristics are as follows:
Mp 77-78.5°C;
¹H NMR δ 7.3-7.1, 2.60, 2.47, 2.13, 1.43;
Anal. Found: C, 81.16; H, 8.38.

### PREPARATION 42 2-(1-Benzyl-2-phenyl-ethyl)-3,5-dioxo-hexanoic acid, tert-butyl ester (Formula R-4) Refer to Chart R.

To a flame-dried flask under an argon atmosphere is added 0.15 mL of distilled diisopropylamine and 1.0 mL of dry tetrahydrofuran. The solution is cooled to -78°C and treated with 0.65 mL (1.6 M in hexane) of *n*-butyllithium. The solution is warmed to 0°C for 10 min, then re-cooled to -78°C. The lithium diisopropylamine solution is treated with 282 mg of *tert-*butyl 3-benzyl-4-phenyl-butanoate (the title product of Preparation 41) as a solution in 2.5 mL of tetrahydrofuran via cannula. The resulting tan enolate is formed over 20 min. The enolate solution is then treated with 85 µL of distilled diketene. The reaction mixture immediately turns bright yellow and is allowed to warm to 0°C over 1 h. The reaction is maintained at 0°C for 2 h and then quenched by addition to cold dilute 1 M aqueous potassium hydrogen sulfate. The mixture is extracted with three portions of diethyl ether. The combined organic layers are dried (magnesium sulfate) and then concentrated under reduced pressure. The residue is purified by flash column chromatography on silica gel eluting with 10% to 20% ethyl acetate in hexane to afford 126 mg of the tautomeric title compound as a tan oil.

Physical characteristics are as follows:
¹H NMR δ 7.3-7.1, 5.52, 320, 2.8-2.6, 2.03, 1.49;
EI-MS: [M⁺]=394.

### PREPARATION 43 3-(1-Benzyl-2-phenyl-ethyl)-4-hydroxy-6-methyl-pyran-2-one (Formula R-5) Refer to Chart R.

To a flask containing 315 mg of tautomeric *tert*-butyl 2-(1-benzyl-2-phenyl-ethyl)-3,5-dioxo-hexanoate (title product of Preparation 42) is added 3 mL of trifluoroacetic acid. The resulting yellow solution is left to stir at room temperature. After 15 h, the reaction mixture is concentrated under reduced pressure. The residual trifluoroacetic acid is removed by treatment with toluene followed by concentration under reduced pressure twice. The resulting crude acid is isolated as a yellow oil which solidifies upon standing.

To a flask containing the above acid is added 8 mL of acetic anhydride. The material dissolves and a precipitant quickly forms. The mixture is stirred at room temperature overnight. The reaction is treated with methanol and concentrated under reduced pressure. This concentration procedure is repeated with methanol and twice with toluene. The residue is then purified by flash column chromatography on silica gel eluting with 40% to 60% ethyl acetate in hexane to afford 165 mg of the title compound as a white solid.

Physical characteristics are as follows:
¹H NMR δ 7.3-7.0, 5.70, 3.64, 3.16, 2.94, 2.04;
EI-MS: [M⁺]=320;
Anal. Found: C, 78.81; H, 6.19.

### EXAMPLE 79 3-(1-Benzyl-2-phenyl-ethyl)-6-(2-cyclopropyl-1-cyclopropylmethyl-ethyl)-4-hydroxy-pyran-2-one (Formula R-6) Refer to Chart R.

To a flame-dried flask under an argon atmosphere is added 0.23 mL of distilled diisopropylamine and 1.6 mL of dry tetrahydrofuran. The solution is cooled to -78°C and treated with 1.0 mL (1.6 M in hexane) of *n*-butyllithium. The solution is warmed to 0°C for 15 min, then cooled to -35°C. The lithium diisopropylamine solution is treated with 160 mg of 3-(1-benzyl-2-phenyl-ethyl)-4-hydroxy-6-methyl-pyran-2-one (title product of Preparation 43) as a solution in 2.5 mL of tetrahydrofuran. The solution is stirred for 20 min as the bath temperature rises to -25°C. The solution is then treated with 0.12 mL of (bromomethyl)cyclopropane. The reaction mixture is allowed to warm to 0°C over 2 h. The reaction is then quenched with excess 1 N aqueous hydrochloric acid and partioned with diethyl ether. The aqueous phase is extracted with two additional portions of diethyl ether. The combined organic layers are dried (magnesium sulfate) and then concentrated under reduced pressure. The residue is purified by flash column chromatography on silica gel eluting with 20% to 40% ethyl acetate in hexane to afford 56 mg of the title product as a clear, colorless oil.

Physical characteristics are as follows:
¹H NMR δ 7.3-7.0, 6.05, 3.62, 3.22, 3.02, 2.52, 1.46, 0.5-0.2. 0.0- -0.15;
EI-MS: [M⁺]=428.2359 found.

### EXAMPLES 80-123

Following procedures analogous to those described above and using starting materials and reagents readily known and available to one of ordinary skill in the art of organic synthesis, the following additional compounds of the present invention, having the mass spectral data indicated in Table III below, are prepared:
- EXAMPLE 80: 3-(Cyclopropyl-phenyl-methyl)-4-hydroxy-6-(1-isobutyl-3-methyl-butyl)-pyran-2-one
- EXAMPLE 81: 6-(2-Cyclopropylmethyl-ethyl)-3-(cyclopropyl-phenyl-methyl)-4-hydroxy-pyran-2-one
- EXAMPLE 82: 3-Dicyclopropylmethyl-4-hydroxy-6-phenethyl-pyran-2-one
- EXAMPLE 83: 6-(1-Cyclopropyl-ethyl)-3-dicyclopropylmethyl-4-hydroxy-pyran-2-one
- EXAMPLE 84: 6-(1-Cyclopropyl-1-cyclopropylmethyl-ethyl)-3-dicyclopropylmethyl-4-hydroxy-pyran-2-one
- EXAMPLE 85: 6-(1-Cyclohexylmethyl-propyl)-3-(cyclopropyl-phenyl-methyl)-4-hydroxy-pyran-2-one
- EXAMPLE 86: 6-(1-Benzyl-propyl)-3-(cyclopropyl-phenyl-methyl)-4-hydroxy-pyran-2-one
- EXAMPLE 87: 6-(2-Cyclopropyl-1-cyclopropylmethyl-ethyl)-4-hydroxy-3(1-phenyl-propyl)-pyran-2-one
- EXAMPLE 88: 6-(1-Benzyl-2-cyclopropyl-ethyl)-3-(cyclopropyl-phenyl-methyl)-4-hydroxy-pyran-2-one
- EXAMPLE 89: 6-(2-Cyclopropyl-ethyl)-3-(cyclopropyl-phenyl-methyl)-4-hydroxy-pyran-2-one
- EXAMPLE 90: 6-(1-Cyclopropylmethyl-propyl)-3-(cyclopropyl-phenyl-methyl)-4-hydroxy-pyran-2-one
- EXAMPLE 91: 3-(Cyclopropyl-phenyl-methyl)-4-hydroxy-6-(4-phenyl-butyl)-pyran-2-one
- EXAMPLE 92: 3-(Cyclohexy)-cyclopropyl-methyl)-6-(2-cyclopropyl-1-cyclopropylmethyl-ethyl)-4-hydroxy-pyran-2-one
- EXAMPLE 93: 3-(Cyclopropyl-phenyl-methyl)-6-(1-ethyl-4-phenyl-butyl)-4-hydroxy-pyran-2-one
- EXAMPLE 94: 6-(3-Cyclohexyl-propyl)-3-(cyclopropyl-phenyl-methyl)-4-hydroxy-pyran-2-one
- EXAMPLE 95: 6-(3-Cyclohexyl-1-ethyl-propyl)-3-(cyclopropyl-phenyl-methyl)4-hydroxy-pyran-2-one
- EXAMPLE 96: 6-(2-Cyclopropyl-ethyl)-4-hydroxy-3-(1-phenyl-propyl)-pyran-2-one
- EXAMPLE 97: 6-(1-Allyl-but-3-enyl)-3-(cyclopropyl-phenyl-methyl)-4-hydroxy-pyran-2-one
- EXAMPLE 98: 6-But-3-enyl-3-(cyclopropyl-phenyl-methyl)-4-hydroxy-pyran-2-one
- EXAMPLE 99: 3-(Cyclopropyl-phenyl-methyl)-6-(1-ethyl-3-phenyl-propyl)-4-hydroxy-pyran-2-one
- EXAMPLE 100: 5-Bromo-6-(2-cyclopropyl-ethyl)-4-hydroxy-3-(1-phenyl-propyl)-pyran-2-one
- EXAMPLE 101: 6-(1-Benzyl-2-phenyl-ethyl)-4-hydroxy-3-(1-phenyl-propyl)-pyran-2-one
- EXAMPLE 102: 3-(Cyclopropyl-phenyl-methyl)-4-hydroxy-6-(3-(2-methoxy-ethoxy)-propyl)-pyran-2-one
- EXAMPLE 103: 3-(Cyclopropyl-phenyl-methyl)-4-hydroxy-5-(2-(2-methoxy-ethoxy)-ethyl)-6-(3-(2-methoxy-ethoxy)-pyran-2-one
- EXAMPLE 104: 3-(Cyclopropyl-phenyl-methyl)-4-hydroxy-6-propyl-pyran-2-one
- EXAMPLE 105: 5-Bromo-4-hydroxy-6-phenylethyl-3(1-phenyl-propyl)-pyran-2-one
- EXAMPLE 106: 3-(Cyclopropyl-phenyl-methyl)-6-(1-ethyl-3-(2-methoxy-ethoxy)-propyl)-4-hydroxy-pyran-2-one
- EXAMPLE 107: 6-(1-Benzyl-3-(2-methoxy-ethoxy)-propyl)-3-(cyclopropyl-phenyl-methyl)-4-hydroxy-pyran-2-one
- EXAMPLE 108: 3-(Cyclopropyl-phenyl-methyl)-4-hydroxy-6-(3-(2-methoxy-ethoxy)-ethoxy)-propyl)-pyran-2-one
- EXAMPLE 109: 5-Bromo-4-hydroxy-3-(1-phenyl-propyl)-6-propyl-pyran-2-one
- EXAMPLE 110: 3-(Cycloprpyl-phenyl-methyl)-6-(1-ethyl-propyl)-4-hydroxy-5-(2-(2-methoxy-ethoxy)ethyl)-pyran-2-one
- EXAMPLE 111: 6-(1-Benzyl-propyl)-5-bromo-4-hydroxy-3-(1-phenyl-propyl)-pyran-2-one
- EXAMPLE 112: 6-(2-Cyclopropyl-1-cyclopropylmethyl-ethyl)-3-(cydopropyl-phenyl-methyl)-4-hydroxy-5-(2-(2-methoxy-ethoxy)-ethoxy)-ethyl)-pyran-2-one
- EXAMPLE 113: 12-Hydroxy-11-(1-phenyl-allyl)-1,4,9-trioxa-dispiro[4.2.5.2]pemadec-11-en-10-one
- EXAMPLE 114: 12-Hydroxy-11-(1-phenyl-propyl)-1,4,9-trioxa-dispiro[4.2.5.2]pentadec-11-en-10-one
- EXAMPLE 115: 4-Hydroxy-3-(1-phenyl-propyl)-1-oxa-spiro[5.5]undec-3-ene-2,9-dione
- EXAMPLE 116: 4,9-Dihydroxy-3-(1-phenyl-propyl)-1-oxa-spiro[5.5]undec-3-en-2-one
- EXAMPLE 117: 3-(Cyclopropyl-phenyl-methyl)-6-(2-furan-2-yl-2-hydroxy-ethyl)-4-hydroxy-pyran-2
Physical characteristics are as follows:
MS: m/z (relative intensity) 352 (3, parent), 334 (27), 256 (100), 131 (30), 121 (36), 118 (21).
- EXAMPLE 118: 3-(Cyclopropyl-phenyl-methyl-4-hydroxy-6-(4,4,4-trifluoro-butyl)-pyran-2-one
Physical characteristics are as follows:
MS: m/z (relative intensity) 352 (87, parent), 261 (78), 235 (80), 131 (88), 144 (53), 115 (68).
- EXAMPLE 119: 6-[2-(1-Cyclohexyl-1H-tetrazol-5-yl)-ethyl]-3-(cyclopropyl-phenyl-methyl)-4-hydroxy-2-one
Physical characteristics are as follows:
MS: m/z (relative intensity) 420 (48, parent), 221 (45), 172 (46), 125 (46), 115 (52), 91 (71), 85 (100), 83 (45), 69 (56).
- EXAMPLE 120: 6,6-Dibenzyl-4-hydrpxy-3-(1-phenyl-propyl)-5.6-dihydro-pyran-2-one
Physical characteristics are as follows:
MS: 412.2032 Found.
¹H-NMR: 7.5-7.0, 4.11, 3.1-2.8, 2.42, 2.09, 0.95.
- EXAMPLE 121: 4-Hydroxy-3-(1-phenyl-allyl)-1,9-dioxa-spiro[5,5]undec-3-en-2-one
Physical characteristics are as follows:
MS m/e (relative intensity): 117 (100), 129 (65), 175 (36), 256 (14), 282 (22), 300 (11).
NMR (CDCl₃, TMS): 7.3-7.5, 6.8, 6.3, 5.0-5.5, 3.7-3.9, 2.5, 1.5-1.9.
- EXAMPLE 122: 4-Hydroxy-6-methyl-3-(3-phenyl-prop-2-enyl)-2H-Pyran-2-one
Physical characteristics are as follows:
¹H-NMR: 7.4-7.1, 6.4-6.1, 6.0. 3.15, 2.14
- EXAMPLE 123: 4-Hydroxy-3-(1-phenyl-propyl)-1-oxa-spiro[5.5]undec-3-ene-2,9-dione monooxime
Physical characteristics are as follows:
Mp 209-211 °C (decomposed).
MS: m/e (relative intensity): 329 (28), 267 (7), 212 (7), 176 (19), 159 (40), 119 (84), 91 (100).

### PREPARATION 44 4-Hydroxy-3-(1-phenylcyclobutyl)-6-phenethyl)-2H-pyran-2-one (Formula S-3) Refer to Chart S.

This compound is prepared by the procedures described in Example 1 above.

Physical characteristics are as follows:
M.P.: 215-216 °C.
Anal. found: C, 79.37; H, 6.33.

### EXAMPLE 124 4-Hydroxy-3-(1-phenylcyclobutyl)-6-[1-(phenylmethyl)propyl]-2H-pyran-2-one (Formula S-4) Refer to Chart S.

This compound is prepared by procedures described in Example 6 above.

Physical characteristics are as follows:
EI HRMS: found 374.1877.
¹H NMR(CDCl₃): δ 0.79, 1.5-1.6, 1.7-2.0, 2.1, 2.47, 2.7-2.9, 5.97, 7.0-7.3, 7.7.

### PREPARATION 45 4-Hydroxy-1-oxaspiro[5.7]tridec-3-en-2-one (Formula T-3) Refer to Chart T.

This compound is prepared by procedures described in Preparation 16A above.

Physical characteristics are as follows:
M.P.: 128-130°C.
Anal. found: C, 68.44: H, 8.7.

### PREPARATION 46 4-Hydroxy-3(1-phenyl-3-trimethylsilyl-2E-propenyl)-1-oxaspiro(5.7)tridec-3-en-2-one (Formula T-4) Refer to Chart T.

This compound is prepared by procedures described in Preparation 16B above.

Physical characteristics are as follows:
¹H NMR(CDCl₃): δ 0.7, 1.0-2.1, 2.4-2.5, 3.5, 4.3, 4.4, 5.0, 5.6-5.9, 6.4-6.6, 7.1-7.3.

### EXAMPLE 125 4-Hydroxy-3-(1-phenyl-2-propenyl)-1-oxaspiro[5.7]tridec-3-en-2-one (Formula T-5) Refer to Chart T.

This compound is prepared by procedures described in Preparation 16C above.

Physical characteristics are as follows:
M.P.: 152-153 °C.
EI HRMS: found 326.1894.

### EXAMPLE 126 4-Hydroxy-3-(1-phenylpropyl)-1-oxaspiro[5.7]tridec-3-en-2-one (Formula T-6) Refer to Chart T.

This compound is prepared by procedures described in Preparation 16D above.

Physical characteristics are as follows:
M.P.: 166.5-167 °C.
EI HRMS: found 328.2039.

### EXAMPLES 127-130

Following procedures analogous to those described in Chart L, the following compounds of the present invention are prepared:
- EXAMPLE 127: 4-Hydroxy-3-(1-phenylallyl)-1-oxa-spiro[5.6]-dodec-3-en-2-one
Physical characteristics are as follows:
M.P.: 120-121 °C.
- EXAMPLE 128: 4-Hydroxy-3-(1-phenylallyl)-1-oxa-spiro[5.4]-dec-3-en-2-one
Physical characteristics are as follows:
M.P.: 115-117 °C.
- EXAMPLE 129: 7-Benzyl-4-hydroxy-3-(1-phenylallyl)-1-oxa-spiro[5.5]undec-3-en-2-one
Physical characteristics (of mixture of diastereomers at 1-phenylallyl center) are as follows:
MS m/e (rel %): 388, 370, 297, 279, 172, 171, 117, 115.
HRMS found: 388.2033.
- EXAMPLE 130: 4-Hydroxy-3-(1-phenylpropyl)-1-oxa-spiro[5.4]-dec-3-en-2-one
Physical characteristics are as follows:
M.P.: 158-161 °C.

### EXAMPLES 131-145

Following procedures analogous to those described above, the following compounds of the present invention are prepared:
- EXAMPLE 131: 3-(Cyclopropylphenylmethyl)-6-[1-ethyl-3-(4- morpholinyl)propyl]-4-hydroxy-2H-pyran-2-one
Physical characteristics are as follows:
HRMS found = 397.2253.
- EXAMPLE 132: 3-(Cyclopropylphenylmethyl)-β-ethyl-4-hydroxy-2-oxo-, phenylmethyl ester 2H-pyran-6-propanoic acid
Physical characteristics are as follows:
HRMS found = 432.1926.
- EXAMPLE 133: 3-(Cyclopropylphenylmethyl)-4-hydroxy-6-[2-methyl-1-(phenylmethyl)propyl]-2H-pyran-2-one
Physical characteristics are as follows:
HRMS found = 389.2108.
- EXAMPLE 134: 3-(Cyclopropylphenylmethyl)-4-hydroxy-6-[2-methyl-1-[(tetrahydro-2H-pyran-3-yl)methyl]propyl]-2H-pyran-2-one
Physical characteristics are as follows:
HRMS found = 396.2309.
- EXAMPLE 135: 4-Hydroxy-3-(1-phenylpropyl)-6-[1-[(tetrahydro-2H-pyran-3-yl)methyl]propyl]-2H-pyran-2-one
Physical characteristics are as follows:
HRMS found = 370.2139.
- EXAMPLE 136: 3-(Cyclopropylphenylmethyl)-6-(1-ethyl-4,4,4-trifluorobutyl)-4-hydroxy-2H-pyran-2-one
Physical characteristics are as follows:
HRMS found = 380.1588
Anal. Found: C, 66.34; H, 6.27.
- EXAMPLE 137: 3-[2-[3-(Cyclopropylphenylmethyl)-4-hydroxy-2-oxo-2H-pyran-6-yl]butyl]-1-[(4-methylphenyl)sulfonyl]-piperidine
Physical characteristics are as follows:
HRMS found = 536.2482
Anal. Found: C, 69.40; H, 7.04; N, 2.21.
- EXAMPLE 138: 2-[2-[3-(Cyclopropylphenylmethyl)-4-hydroxy-2-oxo-2H-pyran-6-yl]butyl]-1-[(4-methylphenyl)sulfonyl]-pyrrolidine
Physical characteristics are as follows:
HRMS found = 521.2229
Anal. Found: C, 68.45; H, 6.81; N, 2.35.
- EXAMPLE 139: 3-(Cyclopropylphenylmethyl)-4-hydroxy-6-(3,3,3-trifluoropropyl)-2H-pyran-2-one
Physical characteristics are as follows:
HRMS found = 338.1143
Anal. Found: C, 63.22; H, 5.28.
- EXAMPLE 140: 2-[2-[3-(Cyclopropylphenylmethyl)-4-hydroxy-2-oxo-2H-pyran-6-yl]butyl]-1-[(4-methylphenyl)sulfonyl]-piperidine
Physical characteristics are as follows:
HRMS found = 535.2398
Anal. Found: C, 68.74; H, 7.10; N, 2.36.
- EXAMPLE 141: 4-[2-[3-(Cyclopropylphenylmethyl)-4-hydroxy-2-oxo-2H-pyran-6-yl]butyl]-1-[(4-methylphenyl)sulfonyl]-piperidine
Physical characteristics are as follows:
HRMS found = 535.2394.
- EXAMPLE 142: 4-[2-[3-(Cyclopropylphenylmethyl)-4-hydroxy-2-oxo-2H-pyran-6-yl]butyl]-1-(phenylmethyl)-2-pyrrolidinone
Physical characteristics are as follows:
HRMS found = 471.2414.
- EXAMPLE 143: 6-(Cyclopentylmethyl)-3-(cyclopropylphenylmethyl)-4-hydroxy-2H-pyran-2-one
Physical characteristics are as follows:
HRMS found = 324.1736
Anal. Found: C, 77.49; H, 7.37.
- EXAMPLE 144: 3-(Cyclopropylphenylmethyl)4-hydroxy-6-[(tetrahydro-2H-pyran-4-yl)methyl]-2H-pyran-2-one
Physical characteristics are as follows:
HRMS found = 340.1672
Anal. Found: C, 73.90; H, 7.55.
- EXAMPLE 145: 3-(Cyclopropylphenylmethyl)-6-(3-fluoropropyl)-4-hydroxy-2H-pyran-2-one
Physical characteristics are as follows:
HRMS found = 302.1311
Anal. Found: C, 70.85; H, 6.36.

### EXAMPLES 146-153

The following procedures analogous to those described above, the following compounds of the present invention are prepared:
- EXAMPLE 146: 3-(α-cyclopropylbenzyl)-4-hydroxy-6-(α-ethyl-β-hydroxyphenethyl)-2H-pyran-2-one (Formula TT-4) Refer to Chart TT.
Physical characteristics are as follows:
MP 73-90°C
- EXAMPLE 147: 3-(α-cyclopropylbenzyl)-4-hydroxy-6-(β-hydroxy-p-methylphenethyl)-2H-pyran-2-one. Refer to Chart TT.
Physical characteristics are as follows:
MP 87-9°C
- EXAMPLE 148: 3-(α-cyclopropylbenzyl)-4-hydroxy-6-(β-hydroxy-p-fluorophenethyl)-2H-pyran-2-one. Refer to Chart TT.
Physical characteristics are as follows:
MP 68-73°C
- EXAMPLE 149: 3-(α-cyclopropylbenzyl)-4-hydroxy-6-(β-hydroxy-p-chlorophenethyl)-2H-pyran-2-one. Refer to Chart TT.
Physical characteristics are as follows:
MP 76-79°C
- EXAMPLE 150: 3-(α-cyclopropylbenzyl)-4-hydroxy-6-(β-hydroxy-m-chlorophenethyl)-2H-pyran-2-one. Refer to Chart TT.
Physical characteristics are as follows:
MP 62-75°C
- EXAMPLE 151: 3-(α-cyclopropylbenzyl)-4-hydroxy-6-(β-hydroxy-o-chlorophenethyl)-2H-pyran-2-one. Refer to Chart TT.
Physical characteristics are as follows:
MP 65-78°C
- EXAMPLE 152: 3-(α-cyclopropylbenzyl)-4-hydroxy-6-(2-(furan-3-yl)-2-hydroxyethyl)-2H-pyran-2-one. Refer to Chart TT.
Physical characteristics are as follows:
MP 66-78°C
- EXAMPLE 153: 3-(α-cyclopropylbenzyl)-4-hydroxy-6-(2-(thiophen-3-yl)-2-hydroxyethyl)-2H-pyran-2-one. Refer to Chart TT.
Physical characteristics are as follows:
MP 71-89°C

### EXAMPLES 154-167

The following procedures are analogous to those described in Chart H, but using Q-1 (see Chart Q) as the starting material, the following compounds are prepared:
- EXAMPLE 154: 3-(α-cyclopropylbenzyl)-4-hydroxy-6-(α-ethyl-p-flurorphenethyl)-2H-pyran-2-one
Physical characteristics are as follows:
MP 52-63°C
- EXAMPLE 155: 3-(α-cyclopropylbenzyl)-4-hydroxy-6-(α-ethyl-p-chlorophenethyl)-2H-pyran-2-one
Physical characteristics are as follows:
MP 61-77°C
- EXAMPLE 156: 3-(α-cyclopropylbenzyl)-4-hydroxy-6-(α-ethyl-m-chlorophenethyl)-2H-pyran-2-one
Physical characteristics are as follows:
MP 45-56°C
- EXAMPLE 157: 3-(α-cyclopropylbenzyl)-4-hydroxy-6-(α-ethyl-o-chlorophenethyl)-2H-pyran-2-one
Physical characteristics are as follows:
MP 168-74°C
- EXAMPLE 158: 3-(α-cyclopropylbenzyl)-4-hydroxy-6-(α-ethyl-p-bromophenethyl)-2H-pyran-2-one
Physical characteristics are as follows:
MP 64-75°C
- EXAMPLE 159: 3-(α-cyclopropylbenzyl)-4-hydroxy-6-(α-ethyl-m-bromophenethyl)-2H-pyran-2-one
Physical characteristics are as follows:
MP 54-66°C
- EXAMPLE 160: 3-(α-cyclopropylbenzyl)-4-hydroxy-6-(α-ethyl-p-triflurormethylphenethyl)-2H-pyran-2-one
Physical characteristics are as follows:
MP 180-6°C
- EXAMPLE 161: 3-(α-cyclopropylbenzyl)-4-hydroxy-6-(α-ethyl-m-triflurormethylphenethyl)-2H-pyran-2-one
Physical characteristics are as follows:
MP 47-61°C
- EXAMPLE 162: 3-(α-cyclopropylbenzyl)-4-hydroxy-6-(α-ethyl-o-triflurormethylphenethyl)-2H-pyran-2-one
Physical characteristics are as follows:
MP 180-4°C
- EXAMPLE 163: 3-(α-cyclopropylbenzyl)-4-hydroxy-6-(α-ethyl-p-methoxyphenethyl)-2H-pyran-2-one
Physical characteristics are as follows:
MP 49-61°C
- EXAMPLE 164: 3-(α-cyclopropylbenzyl)-4-hydroxy-6-(α-ethyl-m-methoxyphenethyl)-2H-pyran-2-one
Physical characteristics are as follows:
MP 42-57°C
- EXAMPLE 165: 3-(α-cyclopropylbenzyl)-4-hydroxy-6-(p-fluorophenethyl)-2H-pyran-2-one
Physical characteristics are as follows:
oil
- EXAMPLE 166: 3-(α-cyclopropylbenzyl)-4-hydroxy-6-(p-chlorophenethyl)-2H-pyran-2-one
Physical characteristics are as follows:
MP 64-78°C
- EXAMPLE 167: 3-(α-cyclopropylbenzyl)-4-hydroxy-6-(p-bromophenethyl)-2H-pyran-2-one
Physical characteristics are as follows:
MP 56-68°C

### PREPARATION 47 5,6-Dihydro-4-hydroxy-6-phenyl-6-phenylmethyl-2H-pyran-2-one (Formula VV-1; R₁ = phenyl, R₂ = phenylmethyl) Refer to Chart VV.

Deoxybenzoin (3.1 g) is converted to the title compound using the general procedure for synthesis of 5,6-dihydropyrones of Preparation 16, A. Flash chromatography of the residue with hexanes/ether (2:1, 1:1) followed by ether gives 3.6 g of the title compound as a white solid.

Physical characteristics are as follows:
MP 89-92 °C.
¹H NMR (300 MHz, CDCl₃): δ 2.75-2.89, 3.09-3.19, 3.28-3.39, 7.06-7.08, 7.25-7.28, 7.35-7.39.
M/S m/e (rel %): 190(8), 189 (65), 147 (10), 105 (100), 77 (30).
Anal. Found: C, 77.09; H, 5.78.

### PREPARATION 48 5,6-Dihydro-4-Hydroxy-6-phenethyl-6-propyl-2H-pyran-2-one (Formula VV-1; R₁ = phenethyl, R₂ propyl) Refer to Chart VV.

1-Phenyl-3-hexanone (2.0 g) is converted to the title compound using the general procedure for synthesis of 5,6-dihydropyrones of Preparation 16, A. to yield 1.96 g of the title compound as a light yellow oil.

Physical characteristics are as follows:
¹H NMR (300 MHz, CDCl₃): δ 0.96, 1.21, 1.48, 1.72, 1.98, 2.73, 3.43, 7.15-7.32.
Anal. Found: C, 73.77; H, 7.96.

### PREPARATION 49 5,6-Dihydro-4-hydroxy-6-phenylmethyl-6-propyl-2H-pyran-2-one (Formula VV-1; R₁ = phenylmethyl, R₂ = propyl) Refer to Chart VV.

1-Phenyl-2-pentanone (2 g) is converted to the title compound using the general procedure for synthesis of 5,6-dihydropyrones of Preparation 16, A. to yield 2.1 g of the title compound as a white solid.

Physical characteristics are as follows:
MP 101-103 °C.
¹H NMR (300 MHz, CDCl₃): δ 0.97, 1.51, 1.72, 2.62, 2.84-2.91, 3.11-3.27, 7.12-7.34.

### EXAMPLE 168 5,6-Dihydro-4-hydroxy-6-phenyl-6-(phenylmethyl)-3-(1-phenyl-2-propenyl)-2H-pyran-2-one (Formula VV-4; R₁ = phenyl, R₂ = phenylmethyl) Refer to Chart VV.

5,6-Dihydro-4-hydroxy-6-phenyl-6-phenylmethyl-2H-pyran-2-one (0.2 g) of Preparation 47 is reacted with methyl (1-phenyl-3-trimethylsilyl-2E-propenyl) carbonate (VV-2) of Preparation 17, using the general procedure for palladium catalyzed alkylation of Preparation 16, B. Desilylation using the general procedure in Preparation 16, C. provides 0.14 g of the tide compound as a white foam.

Physical characteristics are as follows:
¹H NMR complicated by presence of diastereomers.
¹H NMR (300 MHz, CDCl₃): δ 2.79-3.39, 4.31, 4.83-5.15, 5.34, 5.90-5.99, 6.03, 6.23-6.31, 6.50-6.54, 6.87-7.34.
HRMS found: 397.1794.

### EXAMPLE 169 5,6-Dihydro-4-hydroxy-6-phenyl-6-(phenylmethyl)-3-(1-phenylpropyl)-2H-pyran-2-one (Formula VV-5; R₁ = phenyl, R₂ = phenylmethyl) Refer to Chart VV.

The title compound of Example 168, 5,6-Dihydro-4-hydroxy-6-phenyl-6-(phenylmethyl)-3-(1-phenyl-2-propenyl)-2H-pyran-2-one, (47 mg) is hydrogenated using the general procedure in Preparation 16, D, utilizing 10% palladium/carbon as catalyst, to yield 47 mg of the title compound as a white foam.

Physical characteristics are as follows:
¹H NMR complicated by presence of diastereomers.
¹H NMR (300 MHz, CDCl₃): δ 0.42, 0.95, 1.30-2.10, 2.77, 2.98, 3.14, 3.33, 3.91, 4.13, 5.48, 5.03, 6.86-6.92, 6.93-7.07, 7.08-7.49.
HRMS found: 399.1598.

### EXAMPLE 170 3-(1,3-Diphenyl-2-propenyl)-5,6-dihydro-4-hydroxy-6-(2-phenylethyl)-6-propyl-, (E)-2H-pyran-2-one (Formula WW-3; R₁ = phenethyl, R₂ = propyl) Refer to Chart WW.

To a solution of 5,6-dihydro-4-hydroxy-6-phenethyl-6-propyl-2H-pyran-2-one (100 mg) of Preparation 48 and 1,3-diphenyl allyl alcohol (WW-2) (161 mg) in dioxane, under argon, is added boron trifluoride-diethyl ether (237 µL). The reaction mixture is stirred at room temperature for 5 min. and then quenched with water. Ether is added and the combined organic layers are extracted with 0.1 N sodium hydroxide (3 x 10 mL). The combined aqueous layers are cooled to 0°C and acidified to pH 1 by the dropwise addition of 2 N hydrochloric acid. The milky solution is extracted with methylene chloride (3x 15 mL) and the combined organic layers are washed with sat. sodium chloride, dried (sodium sulfate) and evaporated under reduced pressure to give 155 mg of the title compound as a white foam.

Physical characteristics are as follows:
¹H NMR complicated by presence of diastereomers.
¹H NMR (300 MHz. CDCl₃): δ 0.90-1.02, 1.38-1.53, 1.73-2.18, 2.49-2.75, 5.23, 6.36-6.75, 7.04-7.41.
MS m/e (rel %): 453 (20), 331 (17), 201 (39), 175 (33), 173 (16), 115 (30), 105 (19), 91 (100).

### EXAMPLE 171 3-(1,3-Diphenylpropyl)-5,6-dihydro-4-hydroxy-6-(2-phenylethyl)-6-propyl-, 2H-pyran-2-one (Formula WW-4; R₁ = phenethyl, R₂ = propyl) Refer to Chart WW.

The title compound of Example 170, 3-(1,3-Diphenyl-2-propenyl)-5,6-dihydro-4-hydroxy-6-(2-phenylethyl)-6-propyl-, (E)-2H-pyran-2-one, (100 mg) is hydrogenated using the general procedure in Preparation 16, D, utilizing 10% palladium/carbon as catalyst, to yield 100 mg of the title compound as a white foam.

Physical characteristics are as follows:
¹H NMR complicated by presence of diastereomers.
¹H NMR (300 MHz, CDCl₃): δ 0.72-1.03. 1.17-2.78. 4.36, 5.84, 6.97-7.45.
HRMS found: 455.2609.

### EXAMPLE 172 3-(1,3-Diphenyl-2-propenyl)-5,6-dihydro-4-hydroxy-6-phenyl-6-(phenylmethyl)-2H-pyran-2-one (Formula WW-3; R₁ = phenyl, R₂ = phenylmethyl) Refer to Chart WW.

To a solution of 5,6-dihydro-4-hydroxy-6-phenyl-6-phenylmethyl-2H-pyran-2-one (200 mg) of Preparation 47 and 1,3-diphenyl allyl alcohol (WW-2) (300 mg) in dioxane, under argon, is added boron trifluoride-diethyl ether (440 µL). The reaction mixture is stirred at room temperature for 5 min. and then quenched with water. Ether is added and the combined organic layers are extracted with 0.1 N sodium hydroxide (3x 10 mL). The combined aqueous layers are cooled to 0°C and acidified to pH 1 by the dropwise addition of 2 N hydrochloric acid. The milky solution is extracted with methylene chloride (3x 15 mL) and the combined organic layers are washed with sat. sodium chloride, dried (sodium sulfate) and evaporated under reduced pressure to give the title compound as a white foam.

Physical characteristics are as follows:
MP 181-185 °C (dec).
¹H NMR complicated by presence of diastereomers.
¹H NMR (300 MHz, CDCl₃): δ 2.82-2.88, 3.04-3.16, 3.30-3.39, 4.97, 5.09, 5.61, 5.66, 5.87, 6.18-6.26, 6.41, 6.52-5.60, 6.96-7.40.
MS m/e (rel %): 454 (27), 363 (44), 233 (39), 205 (28), 193 (100), 115 (94), 91 (75).
Anal. Found: C, 83.43; H, 5.90.

### EXAMPLE 173 3-(1,2-Diphenylethenyl)-5,6-dihydro-4-hydroxy-6-(2-phenylethyl)-6-propyl-, (E)-2H-pyran-2-one (Formula XX-2; R₁ = phenethyl, R₂ = propyl) Refer to Chart XX.

To a solution of 5,6-dihydro-4-hydroxy-6-phenethyl-6-propyl-2H-pyran-2-one (100 mg) of Preparation 48 and stilbene oxide (151 mg) in dioxane, under argon, is added boron trifluoride-diethyl ether (237 µL). The reaction mixture is stirred at room temperature for 16 hr. and then quenched with water. Ether is added and the combined organic layers are extracted with 0.1 N sodium hydroxide (3x 10 mL). The combined aqueous layers are cooled to 0°C and acidified to pH 1 by the dropwise addition of 2 N hydrochloric acid. The milky solution is extracted with methylene chloride (3x 15 mL) and the combined organic layers are washed with sat. sodium chloride, dried (sodium sulfate) and evaporated under reduced pressure to give the tide compound as a white foam.

Physical characteristics are as follows:
¹H NMR complicated by presence of diastereomers.
Physical characteristics are as follows:
¹H NMR (300 MHz, CDCl₃): δ 0.96, 1.29-1.46, 1.54-1.71, 1.80-2.04, 2.38, 2.59-2.71, 4.92, 5.78, 6.61, 7.13-7.41.
MS m/e (rel %): 439 (22), 438 (66), 247 (31), 220 (100), 105 (29), 91 (71).
HRMS Found: 439.2261.

### EXAMPLE 174 5,6-Dihydro-4-hydroxy-6-(2-phenylethyl)-3-(1-phenyl-2-propenyl)-6-propyl-2H-pyran-2-one (Formula VV-4; R₁ = phenethyl, R₂ = propyl) Refer to Chart VV.

5,6 Dihydro-4-hydroxy-6-phenethyl-6-propyl-2H-pyran-2-one (0.3 g) of Preparation 48 is reacted with methyl (1-phenyl-3-trimethylsilyl-2E-propenyl) carbonate (VV-2) of Preparation 17, using the general procedure for palladium catalyzed alkylation of Preparation 16, B. Desilylation using the general procedure in Preparation 16, C. provides 0.24 g of the title compound as a white foam.

Physical characteristics are as follows:
¹H NMR complicated by presence of diastereomers.
¹H NMR (300 MHz, CDCl₃): δ 0.87-1.04, 1.34-155, 1.68-1.77, 1.77-2.14, 2.45-2.77, 5.06, 5.40-5.47, 6.30-6.45, 6.56, 6.62, 7.08, 7.19-7.38.
HRMS Found: 377.2128.

### EXAMPLE 175 5,6-Dihydro-4-hydroxy-6-(2-phenylethyl)-3-(1-phenylpropyl)-6-propyl-2H-pyran-2-one (Formula VV-5; R₁ = phenethyl, R₂ = propyl) Refer to Chart VV.

The title compound of Example 174, 5,6-Dihydro-4-hydroxy-6-(2-phenylethyl)-3-(1-phenyl-2-propenyl)-6-propyl-2H-pyran-2-one, (69 mg) is hydrogenated using the general procedure in Preparation 16, D, utilizing 10% palladium/carbon as catalyst, to yield 66 mg of the title compound as a white foam.

Physical characteristics are as follows:
¹H NMR complicated by presence of diastereomers.
¹H NMR (300 MHz, CDCl₃): δ 0.87-1.13, 1.25-2.20, 2.33-2.54, 2.60-2.78, 4.23, 5.68, 7.13-7.42.
HRMS Found: 379.2264.

### EXAMPLE 176 3-(1,3-Diphenyl-2-propenyl)-5,6-dihydro-4-hydroxy-6-(phenylmethyl)-6-propyl-2H-pyran-2-one (Formula WW-3; R₁ = phenylmethyl, R₂ = propyl) Refer to Chart WW.

To a solution of 5,6-dihydro-4-hydroxy-6-phenylmethyl-6-propyl-2H-pyran-2-one (200 mg) of Preparation 49 and 1,3-diphenyl allyl alcohol (WW-2) (341 mg) in dioxane, under argon, is added boron trifluoride-diethyl ether (200 µL). The reaction mixture is stirred at room temperature for 5 min. and then quenched with water. Ether is added and the combined organic layers are extracted with 0.1 N sodium hydroxide (3x 10 mL). The combined aqueous layers are cooled to 0°C and acidified to pH 1 by the dropwise addition of 2 N hydrochloric acid. The milky solution is extracted with methylene chloride (3x 15 mL) and the combined organic layers are washed with sat. sodium chloride, dried (sodium sulfate) and evaporated under reduced pressure to give the title compound as a white foam.

Physical characteristics are as follows:
¹H NMR complicated by presence of diastereomers.
¹H NMR (300 MHz, CDCl₃): δ 0.90, 1.40-1.76, 2.37-2-52, 2.95-3.18, 5.12, 5.24, 6.28-6.71, 7.11-7.45.
HRMS Found: 439.2274.

### EXAMPLE 177 3-(1,3-Diphenylpropyl)-5,6-dihydro-4-hydroxy-6-(phenylmethyl)-6-propyl-2H-pyran-2-one (Formula WW-4; R₁ = phenylmethyl, R₂ = propyl) Refer to Chart WW.

The title compound of Example 176, 3-(1,3-Diphenyl-2-propenyl)-5,6-dihydro-4-hydroxy-6-(phenylmethyl)-6-propyl-2H-pyran-2-one, (62 mg) is hydrogenated using the general procedure in Preparation 16, D, utilizing 10% palladium/carbon as catalyst, to yield 62 mg of the title compound as a white foam.

Physical characteristics are as follows:
¹H NMR complicated by presence of diastereomers.
¹H NMR (300 MHz, CDCl₃): δ 0.90, 1.35-1-78, 2.36-2.61, 2.63-2.80, 2.95-3.10, 4.34, 5.71, 6.88-7.46.
HRMS Found: 441.2430.

### EXAMPLE 178 3-(α-Cyclopropyl-meta-(phenylsulfonylamino)benzyl)-6(α-ethylphenethyl)-4-hydroxy-2H-pyran-2-one (Formula M-7 wherein R₁ is phenyl) Refer to Chart M.

To a cold (0°) stirred solution of 65 mg of amine (M-5) is added 27 µL of pyridine, followed by 23 µL of benzenesulfonyl chloride. The solution is stirred and allowed to warm slowly to room temperature. After 18 hours, it is diluted with ethyl acetate, and the solution washed with dilute aqueous hydrochloric acid and brine, and dried over magnesium sulfate. The solution is concentrated under reduced pressure, and the residue flash chromatographed on silica gel using 10-15% ethyl acetate in dichloromethane to provide 66.7 mg of title compound as a white foam.

Physical characteristics are as follows:
¹H NMR δ 0.098, 0.24, 0.45, 0.6, 0.78, 1.55, 1.95, 2.49, 2.75, 2.84, 3.40, 5.84, 6.87, 7.0-7.5, 7.70.
IR 3253, 2964, 2661, 1572, 1414, 1284, 1158, 731 cm⁻¹
TLC R_{f} 0.23 (10% ethyl acetate in dichloromethane).
HRMS m+ at m/z 529.1927; calc'd 529.1923.

### EXAMPLES 179-185

Utilizing procedures analogous to those described above in Example 178, the following additional compounds of the present invention are prepared:
- EXAMPLE 179: 3-(α-Cyclopropyl-*meta*-(propylsulfonylamino)benzyl)-6-(α-ethylphenethyl)-4-hydroxy-2H-pyran-2-one
Physical characteristics are as follows:
M+ 495.2074.
- EXAMPLE 180: 3-(α-Cyclopropyl-*meta*-((E)-2-phenylethenylsulfonylamino)benzyl)-6-(α-ethylphenethyl)-4-hydroxy-2H-pyran-2-one
Physical characteristics are as follows:
M+ 555.2088.
- EXAMPLE 181: 3-(α-Cyclopropyl-*meta*-(4-bromophenylsulfonylamino)benzyl)-6-(α-ethylphenethyl)-4-hydroxy-2H-pyran-2-one
Physical characteristics are as follows:
M+ 607.1023.
- EXAMPLE 182: 3-(α-Cyclopropyl-*meta*-(2,5-dichlorophenylsulfonylamino)benzyl)-6-(α-ethylphenethyl)-4-hydroxy-2H-pyran-2-one
Physical characteristics are as follows:
M+ 597.1143.
- EXAMPLE 183: 3-(α-Cyclopropyl-*meta*-(4-*tert*-butylphenylsulfonylamino)benzyl)-6-(α-ethylphenethyl)-4-hydroxy-2H-pyran-2-one
Physical characteristics are as follows:
M+ 585.2558.
- EXAMPLE 184: 3-(α-Cyclopropyl-*meta*-(4-cyanophenylsulfonylamino)benzyl)-6-(α-ethylphenethyl)-4-hydroxy-2H-pyran-2-one
Physical characteristics are as follows:
M+ 554.
- EXAMPLE 185: 3-(α-Cyclopropyl-*meta*-(4-methoxyphenylsulfonylamino)benzyl)-6-(α-ethylphenethyl)4-hydroxy-2H-pyran-2-one
Physical characteristics are as follows:
M+ 559.

### EXAMPLE 186 3-α-Ethylbenzyl-6-α-ethylbenzyl-4-hydrozy-2H-pyran-2-one

Physical characteristics are as follows:
¹H NMR (300 MHz, CDCL₃) δ 7.44, 7.23-7.09, 6.08, 4.17, 3.31, 2.24-2.10, 1.95, 1.75, 0.89, 0.74.

### PREPARATION 50 4-Hydroxy-6-methyl-3-(α-methylbenzyl)-2H-pyran-2-one (Formula Y-5) Refer to Chart Y. Following the procedure of Chart Y, 0.743 grams of Y-5 was obtained.

Physical characteristics are as follows:
H-NMR: 1.64, 2.12, 4.50, 5.99, 7.2, 7.35.

### PREPARATION 51 4-Hydroxy-3-(α-(R)-ethylbenzyl)-6-methyl-2H-pyran-2-one (Formula Z-5) Refer to Chart Z. Following the procedure of Chart Z, 0.160 grams of Z-5 was obtained.

Physical characteristics are as follows:
H-NMR: 0.92, 2.1, 2.2, 4.2, 6.1, 7.2. 7.4.
- EXAMPLE 187: 3-([R]-α-ethylbenzyl)-4-hydroxy-6-([R]-α-ethylphenethyl)-2H-pyran-2-one (Formula AA-5) Refer to Chart AA.
- EXAMPLE 188: 3-([R]-α-ethylbenzyl)-4-hydroxy-6-([S]-α-ethylphenethyl)2H-pyran-2-one (Formula BB-5) Refer to Chart BB.
- EXAMPLE 189: 3-([S]-α-ethylbenzyl)-4-hydroxy-6-([R]-α-ethylphenethyl)-2H-pyran-2-one (Formula CC-5) Refer to Chart CC.
- EXAMPLE 190: 3-([S]-α-ethylbenzyl)-4-hydroxy-6-([S]-α-ethylphenethyl)-2H-pyran-2-one (Formula DD-5) Refer to Chart DD.
- EXAMPLE 191: 3-([R]-α-ethylbenzyl)-4-hydroxy-6-([R]-α-ethylphenethyl)-2H-pyran-2-one (Formula EE-5) Refer to Chart EE.
- EXAMPLE 192: 3-([R]-α-ethylbenzyl)-4-hydroxy-6-([S]-α-ethylphenethyl)-2H-pyran-2-one (Formula FF-3) Refer to Chart FF.
- EXAMPLE 193: 3-([S]-α-ethylbenzyl)-4-hydroxy-6-([R]-α-ethylphenethyl)-2H-pyran-2-one (Formula GG-5) Refer to Chart GG.
- EXAMPLE 194: 3-([S]-α-ethylbenzyl)-4-hydroxy-6-([S]-α-ethylphenethyl)-2H-pyran-2-one (Formula HH-3) Refer to Chart HH.
- EXAMPLE 195: Refer to Chart II.
3-(α-cyclopropyl((5-methoxymethylhydroxymethylether)furfur-2-yl))-4-hydroxy-6-(α-ethylphenethyl)-2H-pyran-2-one (Formula II-7)

Physical characteristics are as follows:
H-NMR: 0.3, 0.4-0.7, 0.9, 1.3, 1.6.2.5, 2.9, 3.3, 3.8, 4.4, 4.6, 5.65, 5.68, 6.2, 6.3, 7.1-7.3.
3-(α-cyclopropyl((5-hydroxymethyl)furfur-2-yl))-4-hydroxy-6-(α-ethylphenethyl)-2H-pyran-2-onc (Formula II-8)
- EXAMPLE 196: Refer to Chart JJ.
3-(α-cyclopropyl((5-methoxymethyl)furfur-2-yl))-4-hydroxy-6-(α-ethylphenethyl)-2H-pyran-2-one (Formula JJ-2)
3-(α-cyclopropyl((5-azidomethyl)furfur-2-yl))-4-hydroxy-6-(α-ethylphenethyl)-2H-pyran-2-one (Formula JJ-3)
3-(α-cyclopropyl((5-aminomethyl)furfur-2-yl))-4-hydroxy-6-(α-ethylphenethyl)-2H-pyran-2-one (Formula JJ-4)
3-(α-cyclopropyl((5-[N-accetyl]aminomethyl)furfur-2-yl))-4-hydroxy-6-(α-ethylphenethyl)-2H-pyran-2-one (Formula JJ-5)
3-(α-cyclopropyl((5-[N-phenylsulphonyl]aminomethyl)furfur-2-yl))4-hydroxy-6-(α-ethylphenethyl)-2H-pyran-2-one (Formula JJ-6)

The following compounds are prepared using the procedures of Chart JJ, but with a substituted sulphonyl halide:
3-(α-cyclopropyl((5-[N-(o-fluoro)phenylsulphonyl]aminomethyl)furfur-2-yl))-4-hydroxy-6-(α-ethylphenethyl)-2H-pyran-2-one
3-(α-cyclopropyl((5-[N-(p-fluoro)phenylsulphonyl]aminomethyl)furfur-2-yl))-4-hydroxy-6-(α-ethylphenethyl)-2H-pyran-2-one
3-(α-cyclopropyl((5-[N-(m-fluoro)phenylsulphonyl]aminomethyl)furfur-2-yl))-4-hydroxy-6-(α-ethylphenethyl)-2H-pyran-2-one
3-(α-cyclopropyl((5-[N-(m-fluoro)phenylsulphonyl]aminomethyl)furfur-2-yl))-4-hydroxy-6-(α-ethylphenethyl)-2H-pyran-2-one
3-(α-cyclopropyl((5-[N-(o-cyano)phenylsulphonyl]aminomethyl)furfur-2-yl))-4-hydroxy-6-(α-ethylphenethyl)-2H-pyran-2-one
3-(α-cyclopropyl((5-[N-(p-cyano)phenylsulphonyl]aminomethyl)furfur-2-yl))-4-hydroxy-6-(α-ethylphenethyl)-2H-pyran-2-one
3-(α-cyclopropyl((5-[N-(m-cyano)phenylsulphonyl]aminomethyl)furfur-2-yl))-4-hydroxy-6-(α-ethylphenethyl)-2H-pyran-2-one
3-(α-cyclopropyl((5-[N-(m-cyano)phenylsulphonyl]aminomethyl)furfur-2-yl))-4-hydroxy-6-(α-ethylphenethyl)-2H-pyran-2-one
3-(α-cyclopropyl((5-(4-carboethoxy-1,2,3-triazol-1-yl)methyl)furfur-2-yl))-4-hydroxy-6-(α-ethylphenethyl)-2H-pyran-2-one (Formula JJ-7)
3-(α-cyclopropyl((5-(4-carboxylic-1,2,3-triazol-1-yl)methyl)furfur-2-yl))-4-hydroxy-6-(α-ethylphenethyl)-2H-pyran-2-one (prepared by hydrolyzing JJ-7 to its corresponding carboxylic acid)
- EXAMPLE 197: Refer to Chart KK.
3-(α-cyclopropyl((5-(N-(1-nitro-2-methylthioethen-2-yl)aminomethyl)furfur-2-yl))-4-hydroxy-6-(α-ethylphenethyl)-2H-pyran-2-one (Formula KK-3)
3-(α-cyclopropyl((5-(N-(1-nitro-2-[N-isopropyl])ethen-2-yl)aminomethyl)furfur 2-yl))-4-hydroxy-6-(α-ethylphenethyl)-2H-pyran-2-one (Formula KK-4)
3-(α-cyclopropyl((5-(N-(N-cyano, methylthioimino)aminomethyl)furfur-2-yl))-4-hydroxy-6-(α-ethylphenethyl)-2H-pyran-2-one (Formula KK-6)
3-(α-cyclopropyl((5-(N-(N-cyano, N'-isopropylguanidine)aminomethyl)furfur-2-yl))-4-hydroxy-6-(α-ethylphenethyl)-2N-pyran-2-one (Formula KK-7)
- EXAMPLE 198: Refer to Chart LL.
3-(α-cyclopropyl((5-(N-benzylcarbamate)aminomethyl)furfur-2-yl))-hydroxy-6-(α-ethylphenethyl)-2H-pyran-2-one (Formula LL-6)
3-(α-cyclopropyl((5-aminomethyl)furfur-2-yl))-4-hydroxy-6-(α-ethylphenethyl)-2H-pyran-2-one (Formula LL-7)
- EXAMPLE 199: Refer to Chart MM.
3-(α-cyclopropyl((5-(N-benzylcarbamate)aminomethyl)thiophen-2-ylmethyl))-4-hydroxy-6-(α-ethylphenethyl)-2H-pyran-2-one (Formula MM-6)
3-(α-cyclopropyl((5-aminomethyl)thiophen-2-ylmethyl))-hydroxy-6-(α-ethylphenethyl)-2H-pyran-2-one (Formula MM-7)
3-(α-cyclopropyl((5-(N-phenylsulphonyl)aminomethyl)thiophen-2-ylmethyl))4-hydroxy-6-(α-ethylphenethyl)-2H-pyran-2-one (Formula MM-8)
- EXAMPLE 200: Refer to Chart NN.
3-(α-cyclopropylfurfur-2-yl))-4-hydroxy-6-(α-ethylphenethyl)-2H-pyran-2-one (Formula NN-5)
3-(α-cyclopropyl(5-N-phenylsulphonyl)furfur-2-yl))-4-hydroxy-6-(α-ethylphenethyl)-2H-pyran-2-one (Formula NN-7)
- EXAMPLE 201: Refer to Chart OO.
3-(α-cyclopropyl((5-methoxymethylhydroxymethylether)thiophen-2-ylmethyl))-4-hydroxy-6-(α-ethylphenethyl)-2H-pyran-2-one (Formula OO-7)
3-(α-cyclopropyl((5-hydroxymethyl)thiophen-2-ylmethyl))-4-hydroxy-6-(α-ethylphenethyl)-2H-pyran-2-one (Formula OO-8)
- EXAMPLE 202: Refer to Chart PP.
3-(α-cyclopropyl((5-methoxymethyl)thiophen-2-ylmethyl))-4-hydroxy-6-(α-ethylphenethyl)-2H-pyran-2-one (Formula PP-2)
3-(α-cyclopropyl((5-azidomethyl)thiophen-2-ylmethyl)-4-hydroxy-6-(α-ethylphenethyl)-2H-pyran-2-one (Formula PP-3)
3-(α-cyclopropyl((5-aminomethyl)thiophen-2-ylmethyl))-4-hydroxy-6-(α-ethylphenethyl)-2H-pyran-2-one (Formula PP-4)
3-(α-cyclopropyl((5-[N-acetyl]aminomethyl)thiophen-2-ylmethyl))-4-hydroxy-6-(α-ethylphenethyl)-2H-pyran-2-one (Formula PP-5)
3-(α-cyclopropyl((5-[N-phenylsulphonyl]aminomethyl)thiophen-2-ylmethyl))-4-hydroxy-6-(α-ethylphenethyl)-2H-pyran-2-one (Formula PP-6)
3-(α-cyclopropyl((5-(4-carboethoxy-1,2,3-triazol-1-yl)methyl)thiophen-2-ylmethyl))-4-hydroxy-6-(α-ethylphenethyl)-2H-pyran-2-one (Formula PP-7)
3-(α-cyclopropyl((5-(4-carboxylic-1,2,3-triazol-1-yl)methyl)thiophen-2-ylmethyl))-4-hydroxy-6-(α-ethylphenethyl)-2H-pyran-2-one (prepared by hydrolyzing PP-7 to its corresponding carboxylic acid).
- EXAMPLE 203: 3-(α-cyclopropyl((5-hydroxymethyl)thiophen-2-ylmethyl))-4-hydroxy-6-(α-ethylphenethyl)-2H-pyran-2-one (Formula QQ-5) Refer to Chart QQ.
- EXAMPLE 204: Refer to Chart RR.
3-(α-cyclopropyl((5-(N-(1-nitro-2-methylthioethen-2-ylmethyl)aminomethyl)thiophen-2-yl))-4-hydroxy-6-(α-ethylphenethyl)-2H-pyran-2-one (Formula RR-3)
3-(α-cyclopropyl((5-(N-(1-nitro-2-[N-isopropyl])ethen-2-yl)aminomethyl)thiophen-2-ylmethyl))-4-hydroxy-6-(α-ethylphenethyl)-2H-pyran-2-one (Formula RR4)
3-(α-cyclopropyl((5-(N-(N-cyano, methylthioimino)aminomethyl)thiophen-2-ylmethyl))-4-hydroxy-6-(α-ethylphenethyl)-2H-pyran-2-one (Formula RR-6)
3-(α-cyclopropyl((5-(N-(N-cyano, N'-isopropylguanidine)aminomethyl)thiophen-2-ylmethyl))-4-hydroxy-6-(α-ethylphenethyl)-2H-pyran-2-one (Formula RR-7)
- EXAMPLE 205: Refer to Chart SS.
3-(α-cyclopropylthiophen-2-ylmethyl))-4-hydroxy-6-(α-ethylphenethyl)-2H-pyran-2-one (Formula SS-5)
3-(α-cyclopropyl(5-N-phenylsulphonyl)thiophen-2-ylmethyl))-4-hydroxy-6-(α-ethylphenethyl)-2H-pyran-2-one (Formula SS-7)
- EXAMPLE 206: Refer to Chart TT.
3-(α-cyclopropylbenzyl)-4-hydroxy-6-(β-hydroxyphenethyl)-2H-pyran-2-one (Formula TT-2)
3-(α-cyclopropylbenzyl)-4-hydroxy-6-(α-ethyl-β-hydroxyphenethyl)-2H-pyran-2-one (Formula TT-4)
- EXAMPLE 207: Refer to Chart UU.
3-(α[S]-Ethylbenzyl)-4-hydroxy-6-(α[R]-ethyl-β[S]-hydroxyphenethyl)-2H-pyran-2-one (Formula UU-6)
3-(α[S]-Ethylbenzyl)-4-hydroxy-6-(α[S]-ethyl-β[R]-hydroxyphenethyl)-2H-pyran-2-one (Formula UU-11)
3-(α[S]-Ethylbenzyl)-4-hydroxy-6-(α[R]-ethyl-β[R]-hydroxyphenethyl)-2H-pyran-2-one (Formula UU-13)
3-(α[S]-Ethylbenzyl)-4-hydroxy-6-(α[S]-ethyl-β[S]-hydroxyphenethyl)-2H-pyran-2-one (Formula UU-14)

The following compounds are diastereomers prepared from UU-16:
3-(α[R]-Ethylbenzyl)-4-hydroxy-6-(α[R]-ethyl-β[S]-hydroxyphenethyl)-2H-pyran-2-one
3-(α[R]-Ethylbenzyl)-4-hydroxy-6-(α[S]-ethyl-β[R]-hydroxyphenethyl)-2H-pyran-2-one
3-(α[R]-Ethylbenzyl)-4-hydroxy-6-(α[R]-ethyl-β[R]-hydroxyphenethyl)-2H-pyran-2-one
3-(α[R]-Ethylbenzyl)-4-hydroxy-6-(α[S]-ethyl-β[S]-hydroxyphenethyl)-2H-pyran-2-one

The following compounds are prepared by the procedures of Chart UU, but with UU-16 wherein the ethyl group is replaced with a cyclopropyl group:
3-(α[S]-Cyclopropylbenzyl)-4-hydroxy-6-(α[R]-ethyl-β[S]-hydroxyphenethyl)-2H-pyran-2-one
3-(α[S]-Cyclopropylbenzyl)-4-hydroxy-6-(α[S]-ethyl-β[R]-hydroxyphenethyl)-2H-pyran-2-one
3-(α[S]-Cyclopropylbenzyl)-4-hydroxy-6-(α[R]-ethyl-β[R]-hydroxyphenethyl)-2H-pyran-2-one
3-(α[S]-Cyclopropylbenzyl)-4-hydroxy-6-(α[S]-ethyl-β[S]-hydroxyphenethyl)-2H-pyran-2-one
3-(α[R]-Cyclopropylbenzyl)-4-hydroxy-6-(α[R]-ethyl-β[S]-hydroxyphenethyl)-2H-pyran-2-one
3-(α[R]-Cyclopropylbenzyl)-4-hydroxy-6-(α[S]-ethyl-β[R]-hydroxyphenethyl)-2H-pyran-2-one
3-(α[R]-Cyclopropylbenzyl)4-hydroxy-6-(α[R]-ethyl-β[R]-hydroxyphenethyl)-2H-pyran-2-one
3-(α[R]-Cyclopropylbenzyl)-4-hydroxy-6-(α[S]-ethyl-β[S]-hydroxyphenethyl)-2H-pyran-2-one

### EXAMPLE 208 3-(α-Ethylbenzyl)-6-(α-ethylphenethyl)4-hydroxy-2H-pyran-2-one: A Four Week Oral Drug Safety and Toxicity Study in Beagle Dogs

Five groups of three (3) male and three (3) female young adult beagle dogs were given 3-(α-Ethylbenzyl)-6-(α-ethylphenethyl)-4-hydroxy-2H-pyran-2-one in 95% ethyl alcohol orally in gelatin capsules at doses of 0 (95% ethyl alcohol vehicle control), 50, 100, 200 or 400 mg/kg/day for twenty-eight (28) consecutive days. Daily oral doses were divided into two (2) equal doses with at least seven (7) hours between the morning and afternoon dose. Parameters evaluated included twice daily clinical observations; daily evaluation of food and water consumption; twice weekly body weights; pretest and dosage day (dd) 15 or 16 and 24 or 25 electrocardiograms: pretest and dd 23 or 24 ophthalmology examinations; pretest and dd 1, 8, 14, 21 and 28 plasma drug levels; pretest and dd 8, 14, and 28 hemograms and serum chemistries; pretest and dd 14 and 28 urinalyses; terminal body weights, gross necropsy observations and absolute and relative organ weights; and histopathologic evaluation of organs and tissues.

Among other observations, daily oral doses of 50 to 400 mg/kg/day were associated with a dose related decrease in prostate size and weight. Table IV below shows that doses of 50 to 400 mg/kg/day, and particularly doses of 100 to 400 mg/kg/day, caused decreased prostate gland size and weight.

### PREPARATION 52 5,6-Dihydro-4-hydroxy-6-(2-methylpropyl)-6-phenylethyl-2H-pyran-2-one (Formula AAA-1; R₁ = 2-methylpropyl, R₂ = phenylethyl) Refer to Chart AAA.

5-Methyl-1-phenyl-3-hexanone (4.0 g) is converted to the title compound using the general procedure for synthesis of 5,6-dihydropyrones of Preparation 16, A. to yield 4.0 g of the title compound as a white solid.

Physical characteristics are as follows:
MP 63-65 °C.
¹H NMR (300 MHz, CDCl₃): δ 0.98, 1.65, 1.83-1.91, 1.93-2.06, 2.66-2.75, 3.42, 7.14-7.32.

### EXAMPLE 209 2H-Pyran-2-one, 3-diphenylmethyl-5,6-dihydro-4-hydroxy-6-(2-methylpropyl)-6-(2-phenylethyl)- (Formula AAA-3; R₁ = phenylethyl, R₂ = 2-methylpropyl) Refer to Chart AAA.

To a solution of 5,6-Dihydro-4-hydroxy-6-(2-methylpropyl)-6-phenylethyl-2H-pyran-2-one (150 mg) of Preparation 52 and benzhydrol (AAA-2) (202 mg) in dioxane, under Argon, is added [boron trifluoride etherate] (68 µL). The reaction mixture is stirred at room temperature for 12 hr. and then quenched with water. Ether is added and the combined organic layers are extracted with 0.1 N sodium hydroxide (3x 10 mL). The combined aqueous layers are cooled to 0°C and acidified to pH 1 using 2 N hydrochloric acid dropwise. The milky solution is extracted with methylene chloride (3x 15 mL) and the combined organic layers are washed with sat. sodium chloride, dried (sodium sulfate) and evaporated under reduced pressure to give the title compound as a light yellow foam.

Physical characteristics are as follows:
¹H NMR (300 MHz, CDCl₃): δ 0.94, 1.63-1.92, 1.97-2.22, 2.51-2.82 5.77, 5.82. 7.10-7.39.
HRMS Found: 440.2351

### EXAMPLE 210 2H-Pyran-2-one, 3-(1,3-diphenyl-2-propenyl)-5,6-dihydro-4-hydroxy-6-(2-methylpropyl)-6-(2-phenylethyl)- (Formula WW-3; R₁ = phenylethyl, R₂ = 2-methylpropyl) Refer to Chart WW.

To a solution of 5,6-Dihydro-4-hydroxy-6-(2-methylpropyl)-6-phenylethyl-2H-pyran-2-one (250 mg) of Preparation 52 and 1.3-diphenyl allyl alcohol (WW-2) (383 mg) in dioxane, under Argon, is added [boron trifluoride etherate] (112 µL). The reaction mixture is stirred at room temperature for 5 min. and then quenched with water. Ether is added and the combined organic layers are extracted with 0.1 N sodium hydroxide (3x 10 mL). The combined aqueous layers are cooled to 0°C and acidified to pH 1 using 2 N hydrochloric acid dropwise. The milky solution is extracted with methylene chloride (3x 15 mL) and the combined organic layers are washed with sat. sodium chloride, dried (sodium sulfate) and evaporated under reduced pressure to give the title compound as a white foam.

Physical characteristics are as follows:
¹H NMR complicated by presence of diastereomers.
¹H NMR (300 MHz, CDCl₃): δ 0.91-1.00, 1.57-2.19, 2.49-2.73, 5.25, 6.42-6.49, 6.63-6.71, 7.05-7.43.
HRMS Found: 467.2591

### EXAMPLE 211 2H-Pyran-2-one, 3-(1.3-diphenyl-2-propyl)-5,6-dihydro-4-hydroxy-6-(2-methylpropyl)-6-(2-phenylethyl)- (Formula WW-4; R₁ = phenylethyl, R₂ = 2-methylpropyl) Refer to Chart WW.

3-(1,3-Diphenyl-2-propenyl)-5,6-dihydro-4-hydroxy-6-(2-methylpropyl)-6-(2-phenylethyl)-2H-pyran-2-one, of Example 210 (100 mg) is hydrogenated using the general procedure in Preparation 16, D, utilizing 10% Pd/C as catalyst, to yield 99 mg of the title compound as a white foam.

Physical characteristics are as follows:
¹H NMR complicated by presence of diastereomers,
¹H NMR (300 MHz, CDCl₃): δ 0.64-1.05, 1.52-2.18, 2.26-2.75, 4.37, 5.74, 6.95-7.48.
HRMS Found: 468.2664

Following procedures analogous to those described above and using starting materials and reagents readily known and available to one of ordinary skill in the art of organic synthesis, the following additional compounds of the present invention are prepared:

### EXAMPLE 212 3-(α-Cyclopropyl-meta-[(phenylaminocarbonyl)amino]benzyl)-6-(α-ethylphenethyl)-4-hydroxy-2H-pyran-2-one

Physical characteristics are as follows:
FAB HRMS: 509.2451 (found).

### EXAMPLE 213 3-(α-Cyclopropyl-meta-[(benzylaminocarbonyl)amino]benzyl)-6-(α-ethylphenethyl)-4-hydroxy-2H-pyran-2-one

Physical characteristics are as follows:
FAB HRMS: 523.2600 (found).

### EXAMPLE 214 3-(α-Cyclopropyl-meta-[(4-methoxyphenylaminocarbonyl)amino]benzyl)-6-(α-ethylphenethyl)-4-hydroxy-2H-pyran-2-one

Physical characteristics are as follows:
¹H NMR: δ 0.15, 0.38, 0.57, 0.76, 1.51, 1.78, 2.41, 2.69, 2.81, 3.26, 3.67, 5.69, 6.72, 6.9-7.4.

### EXAMPLE 215 3-(α-Cyclopropyl-meta-[(4-bromophenylaminocarbonyl)amino]benzyl)-6-(α-ethylphenethyl)-4-hydroxy-2H-pyran-2-one

Physical characteristics are as follows:
¹H NMR: δ 0.15, 0.36, 0.57, 0.76, 1.51, 1.70, 2.40, 2.65-2.85, 3-25, 5.71, 6.8-7.6.

### EXAMPLE 216 3-(α-Cyclopropyl-meta-[(phenylsulfonylaminocarbonyl)amino]benzyl)-6-(α-ethylphenethyl)-4-hydroxy-2H-pyran-2-one

Physical characteristics are as follows:
¹H NMR: δ 0.11, 0.19, 0.40, 0.53, 0.81, 1.59, 1.81, 2.45, 2.7-2.85, 3.29, 5.7, 6.8-8.0.

### EXAMPLE 217 2H-Pyran-2-one, 6-butyl-5,6-dihydro-3-(1,3-diphenyl-2-propenyl)-4-hydroxy-6-phenylmethyl.

Physical characteristics are as follows:
EI-MS: (M+)=452
¹H NMR: 0.8, 1.35, 1.6, 2.49, 2.98, 5.07, 6.5, 6.9, 7.1-7.4.

### EXAMPLE 218 2H-Pyran-2-one, 6-butyl-5,6-dihydro-3-(1,3-diphenylpropyl)-4-hydroxy-6-phenylmethyl.

Physical characteristics are as follows:
¹H NMR: 0.85, 1.1-1.3, 1.6, 2.3-2.6, 2.94, 4.23, 7.0-7.5.

### EXAMPLE 219 2H-Pyran-2-one, 6-butyl-5,6-dihydro-hydroxy-6-phenylmethyl-3-(1-phenyl-2-propenyl)-

Physical characteristics are as follows:
¹H NMR: 0.89, 1.1-1.7, 2.48, 3.0, 4.9-5.3, 6.3-6.6, 7.0-7.4.

### EXAMPLE 220 2H-Pyran-2-one, 6-butyl-5,5-dihydro-4-hydroxy-6-phenylmethyl-3-(1-phenylpropyl)-

Physical characteristics are as follows:
¹H NMR: 0.8-1.0, 1.2-1.7, 1.9-2.3, 2.45, 2.97, 4.05, 7.0-7.5.

### EXAMPLE 221 3-(α-Cyclopropyl-meta-(tert-butyloxycarbonyl-L-alanylamino)benzyl)-6-(α-ethylphenethyl)-4-hydroxy-2H-pyran-2-one

Physical characteristics are as follows:
(M + Na)+ 583.2801.

### EXAMPLE 222 3-(α-Cyclopropyl-meta-(N-α-tert-butyloxycarbonyl-N-im-p-toluenesulfonyl-L-histidylamino)benzyl)-6-(α-ethylphenethyl)-4-hydroxy-2H-pyran-2-one

Physical characteristics are as follows:
(M + H)+ 781.3268.

### EXAMPLE 223 3-(α-Cyclopropyl-meta-(4-bromophenylbenzoylamino)benzyl)-6-(α-ethylphenethyl)-4-hydroxy-2H-pyran-2-one

Physical characteristics are as follows:
M+ 571.1358.

### EXAMPLE 224 3-(α-Cyclopropyl-meta-(4-chlorophenylsulfonylamino)benzyl)-6-(α-ethylphenethyl)-4-hydroxy-2H-pyran-2-one

Physical characteristics are as follows:
M+ 563.1528.

### EXAMPLE 225 3-(α-Cyclopropyl-meta-(N-benzyloxycarbonyl-O-α-benzyl-L-glutamylamino)benzyl)-6-(α-ethylphenethyl)4-hydroxy-2H-pyran-2-one

Physical characteristics are as follows:
(M + H)+ 743.3330.

### EXAMPLE 226 3-(α-Cyclopropyl-meta-(N-α-tert-butyloxycarbonyl-L-histidylamino)benzyl)-6-(α-ethylphenethyl)-4-hydroxy-2H-pyran-2-one

Physical characteristics are as follows:
(M + H)+ 627.3186.

### EXAMPLE 227 3-(α-Cyclopropyl-meta-(4-cyanophenylbenzoylamino)benzyl)-6-(α-ethylphenethyl)4-hydroxy-2H-pyran-2-one

Physical characteristics are as follows:
(M + H)+ 519.2277.

### EXAMPLE 228 3-(α-Cyclopropyl-meta-(L-glutamylamino)benzyl)-6-(α-ethylphenethyl)-4-hydroxy-2H-pyran-2-one

### EXAMPLE 229 3-(α-Cyclopropyl-meta-(8-quinolinesulfonylamino)benzyl)-6-(α-ethylphenethyl)-4-hydroxy-2H-pyran-2-one

Physical characteristics are as follows:
M+ 580.

### EXAMPLE 230 3-(α-Cyclopropyl-meta-(3-(1-indolyl)propanoylamino)benzyl)-6-(α-ethylphenethyl)-4-hydroxy-2H-pyran-2-one

Physical characteristics are as follows:
M+ 560.

### EXAMPLE 231 3-(α-Cyclopropyl-meta-(2-pyridylacetylamino)benzyl)-6-(α-ethylphenethyl)-4-hydroxy-2H-pyran-2-one

Physical characteristics are as follows:
M+ 508.

### EXAMPLE 232 2H-Pyran-2-one, 3-(α-cyclopropyl-[5-(methoxymethoxy-methyl)-thiophen-2-yl]-methyl)-5,6-dihydro-4-hydroxy-6-(2-methylpropyl)-6-(2-phenylethyl)-

Physical characteristics are as follows:
¹H NMR (300 MHz, CDCl₃): δ 0.33, 0.46, 0.65, 0.92, 1.54-1.68, 1.70-1.84, 1.90-2.18, 3.37, 3.57, 4.65, 6.79, 6.86, 7.10-7.28.
HRMS Found: 423.2002.

### EXAMPLE 233 2H-Pyran-2-one, 5,6-dihydro-4-hydroxy-6-(2-methylpropyl)-6-(2-phenylethyl)-3-(1-phenyl-2-propenyl)-

Physical characteristics are as follows:
¹H NMR complicated by presence of diastereomers.
¹H NMR (300 MHz, CDCl₃, CD₃OD): δ 0.95-0.98, 1.60-1.71, 1.73-1.89, 1.90-2.14, 2.56-2.72, 3.46, 4.91, 5.11-5.20, 6.44-6.59, 7.11-7.33.
HRMS Found: 391.2273.

### EXAMPLE 234 2H-Pyran-2-one, 5,6-dihydro-4-hydroxy-6-(2-methylpropyl)-6-(2-phenylethyl)-3-(1-phenylpropyl)-

Physical characteristics are as follows:
¹H NMR complicated by presence of diastereomers.
¹H NMR (300 MHz, CDCl₃): δ 0.88-1.02, 1.54-2.36, 2.59, 3.36, 4.09, 7.03-7.28, 7.41-7.43.
HRMS Found: 393.2430.

### EXAMPLE 235 2H-Pyran-2-one, 5,6-dihydro-3-diphenylmethyl-4-hydroxy-6,6-di-(2-phenylethyl)-

Physical characteristics are as follows:
¹H NMR complicated by presence of diastereomers.
¹H NMR (300 MHz, CDCl₃): δ 2.09-2.24, 2.63-2.74, 5.79, 5.87, 7.10-7.35.
HRMS Found: 489.2430.

### EXAMPLE 236 2H-Pyran-2-one, 5,6-dihydro-4-hydroxy-6,6-di-(2-phenylethyl)-3-(1,3-diphenyl-2-propenyl)-

Physical characteristics are as follows:
¹H NMR (300 MHz, CDCl₃): δ 2.03-2.27, 2.66-2.82, 5.24, 6.43, 6.68, 7.10-7.43.
HRMS Found: 515.2586.

### EXAMPLE 237 2H-Pyran-2-one, 5,6-dihydro-4-hydroxy-6,6-di-(2-phenylethyl)-3-(1,3-diphenylpropyl)-

Physical characteristics are as follows:
¹H NMR complicated by presence of diastereomers.
¹H NMR (300 MHz, CDCl₃): δ 1.52-1.91, 2.03-2.25, 2.31-2.78, 4.36, 5.96, 7.08-7.41.
HRMS Found: 517.2743.

### EXAMPLE 238 2H-Pyran-2-one, 5,6-dihydro-4-hydroxy-3-(1,3-diphenyl-2-propenyl)-6-(3-phenylpropyl)-6-propyl-

Physical characteristics are as follows:
¹H NMR complicated by presence of diastereomers.
¹H NMR (300 MHz, CDCl₃): δ 0.84-0.93, 2.21-1.42, 1.56-1.85, 2.49-2.64, 5.17, 6.32-6.44, 6.48-6.66, 7.09-7.41.

### EXAMPLE 239 2H-Pyran-2-one, 5,6-dihydro-4-hydroxy-3-(1,3-diphenylpropyl)-6-(3-phenylpropyl)-6-propyl-

Physical characteristics are as follows:
¹H NMR complicated by presence of diastereomers.
¹H NMR (300 MHz, CDCl₃): δ 0.79-1.05, 1.16-1.87, 2.23-2.74, 4.31, 5.64, 7.05-7.48.

### EXAMPLE 240 2H-Pyran-2-one, 3-(α-cyclopropyl-[5-(methoxymethoxy-methyl)-thiophen-2-yl]-methyl)-5,6-dihydro-4-hydroxy-6-(2-phenylethyl)-6-propyl-

Physical characteristics are as follows:
¹H NMR complicated by presence of diastereomers.
¹H NMR (300 MHz, CDCl₃): δ 0.38, 0.51, 0.72, 0.95, 1.37-1.51, 1.62-2.21, 2.44-2.58, 2.61-2.72, 3.39, 3.40, 3.80, 4.67, 6.51, 6.65, 6.88, 6.99, 7.16-7.30.

### EXAMPLE 241 2H-Pyran-2-one, 3-(α-cyclopropyl-[5-(methoxymethoxy-methyl)-thiophen-2-yl]-methyl)-5,6-dihydro-4-hydroxy-6-(3-phenylpropyl)-6-propyl-

Physical characteristics are as follows:
¹H NMR complicated by presence of diastereomers.
¹H NMR (300 MHz, CDCl₃): δ 0.36-0.58, 0.71, 0.90, 123-1.42, 1.61-1.83, 2.47-2.62, 3.40, 3.43, 3.75, 4.68, 6.41, 6.86, 6.88, 6.92, 7.10-7.21, 7.26-7.32.

### EXAMPLE 242 2H-Pyran-2-one, 5,6-dihydro-3-diphenylmethyl-4-hydroxy-6-(3-phenylpropyl)-6-propyl-

Physical characteristics are as follows:
¹H NMR (300 MHz, CDCl₃): δ 0.88, 1.22-1.38, 1.59-1.82, 2.48, 2.58, 5.71, 5.76, 7.07-7.39.

### EXAMPLE 243 3-(α-Cyclopropylbenzyl)-6-[1-cyclopropylmethyl-2-(tetrahydropyran-3-yl)ethyl]-4-hydroxy-2H-pyran-2-one

Physical characteristics are as follows:
ms: found m/z = 408.

### EXAMPLE 244 3-(α-Cyclopropyl-meta-(ethylsulfonylamino)benzyl-6-[1-cyclopropylmethyl-2-(tetrahydropyran-3-yl)ethyl]-4-hydroxy-2H-pyran-2-one

Physical characteristics are as follows:
ms: found m/z = 515.

### EXAMPLE 245 3-(α-Cyclopropyl-meta-(4-methylphenylsulfonylamino)benzyl-6-[1-cyclopropylmethyl-2-(tetrahydropyran-3-yl)ethyl]-4-hydroxy-2H-pyran-2-one

Physical characteristics are as follows:
ms: found m/z = 577.

### EXAMPLE 246 3-(α-Cyclopropyl-meta-(4-methylphenylsulfonylamino)benzyl-6-(α-ethyl-phenethyl)-4-hydroxy-2H-pyran-2-one

Physical characteristics are as follows:
ms: found m/z = 543.

### EXAMPLE 247 3-(α-Cyclopropyl-meta-(ethylsulfonylamino)benzyl-6-(α-ethyl-phenethyl)-4-hydroxy-2H-pyran-2-one

Physical characteristics are as follows:
ms: found m/z = 481.

### EXAMPLE 248 3-(α-Cyclopropyl-meta-(3-pyridylacetylamino)benzyl)-6-(α-ethylphenethyl)-4-hydroxy-2H-pyran-2-one

Physical characteristics are as follows:
¹H NMR δ 0.22, 0.48, 0.64, 0.82, 1.60, 1.89, 2.48, 2.8, 2.9, 3.32, 5.69, 7.1, 7.2, 7.5, 7.8, 8.4, 8.5 ppm.

### EXAMPLE 249 3-(α-Cyclopropyl-meta-(4-pyridylacetylamino)benzyl)-6-(α-ethylphenethyl)-4-hydroxy-2H-pyran-2-one

Physical characteristics are as follows:
¹H NMR δ 0.22, 0.48, 0.64, 0.82, 1.6, 1.9, 2.5, 2.8, 2.9, 3.3, 5.71, 7.05, 7.2, 7.3, 7.5, 8.40 ppm.

### PREPARATION 53 (4S,5R) 3-Butyryl-4-methyl-5-phenyl-2-oxazolidinone (Formula W-1) Refer to Chart W.

(4S,5R) 4-Methyl-5-phenyl-2-oxazolidinone (10.0 g) is added to tetrahydrofuran (115 mL) and cooled to -78°C. To that solution is added n-butyl lithium (38.8 mL, 1.6 M in hexane) and the resulting reaction stirred at -78°C for 1 hour. To that solution is added butyryl chloride (6.75 mL) and the reaction stirred for an additional 1.5 hours. The reaction is warmed to room temperature and quenched via addition of water (100 mL). The reaction is extracted with ethyl acetate, dried (sodium sulfate) and solvent removed in vacuco to yield the crude product. The residue is dissolved in hexane and placed in the freezer overnight. Two crops of crystalls are obtained (8.5 g, 4.5 g) to yield a total of 13.0 grams of the title product.

### PREPARATION 54 (4S,5R) 3-(α[R]-Benzyl)butyryl-4-methyl-5-phenyl-2-oxazolidinone (Formula W-3) Refer to Chart W.

(4S,5R) 3-Butyryl-4-methyl-5-phenyl-2-oxazolidinone of Preparation 53 (4.0 g) is cooled to -78°C in tetrahydrofuran (54 mL) under a nitrogen atmosphere. Lithium diisopropylamide (LDA, 2.0 M) is added dropwise over several minutes and stirring continued for 30 minutes. The reaction temperature is then adjusted to 0°C and benzyl bromide (2.15 mL) is added. The reaction is then allowed to warm to room temperature and after 30 minutes at room temperature, water (150 mL) is added and the reaction is extracted with ethyl acetate. The combined ethyl acetate extracts are dried and solvent removed in vacuo to yield a pate yellow oil. The oil is dissolved in toluene and applied to a silica gel column eluting with hexane to yield 4.4 g of the desired title product as an oil. [α]D = -69°.

### PREPARATION 55 (R) α-ethyl hydrocinnamic acid (Formula W-4) Refer to Chart W.

(4S,5R) 3-(α[R]-Benzyl)butyryl-4-methyl-5-phenyl-2-oxazolidinone of Preparation 54 (3.48 g) is added to a 3:1 mixture of tetrahydrofuran/water (35 mL). To that solution is added a lithium hydroxide/hydrogen peroxide solution (from 10.3 mL of a **@** 10 M solution of hydrogen peroxide and 911 mg of lithium hydroxide monohydrate) and the resulting solution stirred for 3 hours. Water (50 mL) is added and the reaction is then extrated with ethyl acetate. The aqueous is acidified with 2N hydrochloric acid and extracted with methylene chloride to yield 1.71 g of pure title product as a colorless liquid. [α]D = -36°.

### PREPARATION 56 (R) 2,2-Dimethyl-6-(α-ethylphenethyl)-4H-1,3-dioxin-4-one (Formula W-7) Refer to Chart W.

(R) α-Ethyl hydrocinnamic acid of Preparation 55 (1.58 g) is added to methylene chloride (5 mL) followed by the addition of oxalyl chloride (0.82 mL, 1.05 equiv) at room temperature. After stirring for 4 hours, the solvent and excess oxalyl chloride is removed via evaporation to yield W-8.

tert-Butyl acetate (2.4 mL, 17.8 mmol) is added to a tetrahydrofuran solution (10 mL) of lithium diisopropylamide (8.9 mL, 2.0 M LDA) dropwise at -78°C. After stirring for 30 minutes, this lithium enolate (W-9) is added to the above acid chloride (W-8) (in tetrahydrofuran (5 mL)) dropwise via cannular at -78°C. After 20 minutes at -78°C, water (5 mL) is added and the reaction warmed to 0°C. 1N Hydrochloric acid (10 mL) is added slowly at 0°C followed by ethyl acetate (20 mL). The organic layer is separated and the aqueous extracted with several addition times with ethyl acetate. The combined extracts are dried and solvent removed in vacuo to yield 3.00 g of tert-butyl 5-phenyl-4[R]-ethyl-3-oxo-pentanoate (W-10). Sulfuric acid (0.44 mL) is added dropwise to a mixture of tert-butyl 5-phenyl-4[R]-ethyl-3-oxo-pentanoate (3.0 g), acetone (1.5 mL) and acetic anhydride (3 mL) with stirring at 0°C overnight. The reaction is then poured into saturated sodium bicarbonate (50 mL) and ice. The mixture is stirred and warmed to room temperture for 30 minutes and then extracted with ether (2 X 30 mL). The combined ether extracts are dried over magnesium sulfate and solvent removed in vacuo to yield 2.81 g of crude title product. Silica gel chromatography (acetone/ethyl acetate/hexane; 1:3:16) affords 2.10 g of the desired product as a colorless oil.

Physical characteristics are as follows:
H-NMR: 0.92(m), 1.60(s), 1.61(s), 2.41(m), 2.77, 2.80, 5.13, 7.12-7.27.

### EXAMPLE 250 (3S,6R) 3-(α-ethylbenzyl)-6-(α-ethylphenethyl)-4-hydroxy-2H-pyran-2-one (Formula CC-5) Refer to Chart CC.

To (R) 3-phenylvaleric acid (CC-1) (Chemistry Letters (1981) 913-16) (400 mg) in anhydrous methylene chloride (5 mL) is added oxalyl chloride (0.21 mL) and the resulting solution heated at reflux for 2 hours. The reaction is then cooled to room temperature and the solvent and excess oxalyl chloride removed in vacuo to yield the crude acid chloride (CC-2). The acid chloride is dissolved in toluene (10 mL) and then heated to reflux. To that solution, at reflux, is added a mixture of triethylamine (202 mg) and (R) 2,2-dimethyl-6-(α-ethylphenethyl)-4H-1,3-dioxin-4-one (350 mg) (CC-3) in toluene (1.5 mL) dropwise. A long needle is inserted via rubber septa just above the solvent to sweep acetone from the reaction. The reaction is maintained at reflux for 2.5 hours. The reaction is cooled to room temperature and the triethylamine hydrochloride is removed via filtration. The filtrate is evaporated in vacuo and the residue, containing CC-4, is added to a methanol/water mixture (6:1; 35 mL) and 0.5 g of sodium carbonate added and the reaction stirred at room temperature overnight. The methanol is removed in vacuo and a saturated sodium carbonate solution (20 mL) added to the crude reaction mixture. That material is extracted with hexane (2 X 20 mL) which contains the methyl ester (R) α-ethyl hydrocinnamic acid (CC-6). The aqueous layer is made acidic by adding 1N hydrochloric acid at 0°C to a pH @ 3. That aqueous is extracted with ethyl acetate (2 X 30 mL), dried (sodium sulfate) and solvent removed in vacuo to yield 359 mg of crude title product. Chromatography (silica gel; 15% ethyl acetate/methylene chloride) affords 299.9 mg of material which is recrystallized to yield 210.0 mg of product. An additional 20 mg of product is recovered from the mother liquiors to yield a total of 230.0 mg of the title product.

Physical characteristics are as follows:
MP 141-2°C.

### PREPARATION 57 (4R, 5S) 3-Butyryl-4-methyl-5-phenyl-2-oxazolidinone (Formula X-1) Refer to Chart X.

(4R,5S) 4-Methyl-5-phenyl-2-oxazolidinone (10.0 g) is added to tetrahydrofuran (115 mL) and cooled to -78°C. To that solution is added n-butyl lithium (38.8 mL, 1.6 M in hexane) and the resulting reaction stirred at -78°C for 1 hour. To that solution is added butyryl chloride (6.75 mL) and the reaction stirred for an additional 1.5 hours. The reaction is warmed to room temperature and quenched via addition of water (100 mL). The reaction is extracted with ethyl acetate, dried (sodium sulfate) and solvent removed in vacuco to yield the crude title product (X-1). The residue is dissolved in hexane and placed in the freezer overnight. Two crops of crystals are obtained (11.5 g. 1.7 g) to yield a total of 13.2 grams of product.

Physical characteristics are as follows:
H-NMR (CDCl3) 0.88, 0.91, 0.98, 1.00, 1.03, 1.68, 1.70, 1.73, 1.75, 2.88, 2.91, 2.92, 2.93, 2.95, 2.97, 4.74, 4.77, 4.79, 4.74, 4.77, 4.79, 5.66, 5.68, 7.26-42.

### PREPARATION 58 (4R,5S) 3-(α[S]-Benzyl)butyryl-4-methyl-5-phenyl-2-oxazolidinone (Formula X-3) Refer to Chart X.

(4R,5S) 3-Butyryl-4-methyl-5-phenyl-2-oxazolidinone of Preparation 57 (11.5g) is cooled to -78°C in tetrahydrofuran (100 mL) under a nitrogen atmosphere. Lithium diisopropylamide (LDA, prepared from 33.5 mL of 1.6 M n-butyl lithium and 7.83 mL of diisopropylamine; total volume of tetrahydrofuran is 80 mL) is added dropwise over several minutes and stirring continued for 30 minutes. The reaction temperature is then adjusted to 0°C and benzyl bromide (8.3 mL) is added. The reaction is then allowed to warm to room temperature and after 30 minutes at room temperature the reaction is quenched with 0.25 M hydrochloric acid. The reaction is extracted with ethyl acetate (3 X 100 mL). The combined ethyl acetate extracts are dried and solvent removed in vacuo to yield a pale yellow oil. The oil is dissolved in toluene and applied to a silica gel column, eluting with hexane (500 mL) and then 15% ethyl acetate/hexane to yield 13.8 g of the desired title product as an oil.

Physical characteristics are as follows:
H-NMR (CDCl3) 0.619, 0.641, 0.024, 0.949, 0.973, 1.56, 1.58, 1.60, 1.62, 1.72, 1.75, 1.78, 1.80, 2.74, 2.76, 2.79, 2.81, 2.97, 2.99, 3.01, 3.04, 4.16, 4.16, 4.20, 4.71, 4.74, 4.76, 5.59, 5.61, 7.16-7.42,

### PREPARATION 59 (S) α-ethyl hydrocinnamic acid (Formula X-4) Refer to Chart X.

(4R,5S) 3-(α[S]-Benzyl)butyryl-4-methyl-5-phenyl-2-oxazolidinone of Preparation 58 (1.50 g) is added to a 3:1 mixture of tetrahydrofuran/water (15 mL). To that solution is added a lithium hydroxide/hydrogen peroxide solution (from 4.45 mL of an @ 10 M solution of hydrogen peroxide and 393 mg of lithium hydroxide monohydrate) and the resulting solution stirred for 3 hours. Water (50 mL) is added and the reaction is then extrated with ethyl acetate. The aqueous is acidified with 2N hydrochloric acid and extracted with methylene chloride to yield 0.746 g of pure title product as a colorless liquid. [α]D = +34°.

### PREPARATION 60 (S) 2,2-Dimethyl-6-(α-ethylphenethyl)-4H-1,3-dioxin-4-one (Formula X-7) Refer to Chart X.

Following the procedure described for the preparation of (R) 2,2-dimethyl-6-(α-ethylphenethyl)-4H-1,3-dioxin-4-one of Preparation 56, 6.5 grams of the title product is prepared.

Physical characteristics are as follows:
H-NMR: 0.92(m), 1.60(s), 1.16(s), 2.41(m), 2.77, 2.80, 5.13, 7.12-7.27.

### EXAMPLE 251 (3S,6S) 3-(α-ethylbenzyl)-6-(α-ethylphenethyl)-4-hydroxy-2H-pyran-2-one (Formula DD-5) Refer to Chart DD.

Following the procedure described for the preparation of (3S,6R) 3-(α-ethylbenzyl)-6-(α-ethylphenethyl)-4-hydroxy-2H-pyran-2-one of Example 250, 1.40 grams of the title product is prepared.

Physical characteristics are as follows:
MP 162-164°C.

### EXAMPLE 252 Sodium (3S,6R) 3-(α-ethylbenzyl)-6-(α-ethylphenethyl)-2H-pyran-2-one 4-oxide

(3S,6R) 3-(α-ethylbenzyl)-6-(α-ethylphenethyl)-4-hydroxy-2H-pyran-2-one of Example 250 (36.2 mg) is added to 1.0 mL of tetrahydrofuran. To that solution is added 0.4 mL of 0.25 N sodium hydroxide at room temperature. That mixture is stirred for 30 minutes and the tetrahydrofuran and water are removed in vacuo. The resulting salt is washed with ethyl ether and dried in the vacuum oven over night. This affords 37.5 mg of the desired sodium salt, title product.

Physical characteristics are as follows:
MP 210°C (decomposes).
H-NMR (DMSO-d6) 0.739 (multiplet), 0.758 (multiplet), 1.431 (multiplet), 1.896 (multiplet), 2.298 (multiplet), 2.658 (part of an ABX), 2.759 (part of an ABX), 3.982 (triplet), 5.199 (singlet), 6.99-7.37 (multiplet).

### EXAMPLE 253 (3R,6R) 3-(α-ethylbenzyl)-6-(α-ethylphenethyl)-4-hydroxy-2H-pyran-2-one (Formula AA-5) Refer to Chart AA.

Physical characteristics are as follows:
MP 161-3°C.

### EXAMPLE 254 (3R,6S) 3-(α-ethylbenzyl)-6-(α-ethylphenethyl)-4-hydroxy-2H-pyran-2-one (Formula BB-5) Refer to Chart BB.

Physical characteristics are as follows:
H-NMR: 0.83 (m), 0.95 (m), 1.62 (m), 2.13 (m), 2.49 (m), 2.80 (m), 2.88 (m), 4.26 (m), 5.7 (s), 7.02-7.43 (m).

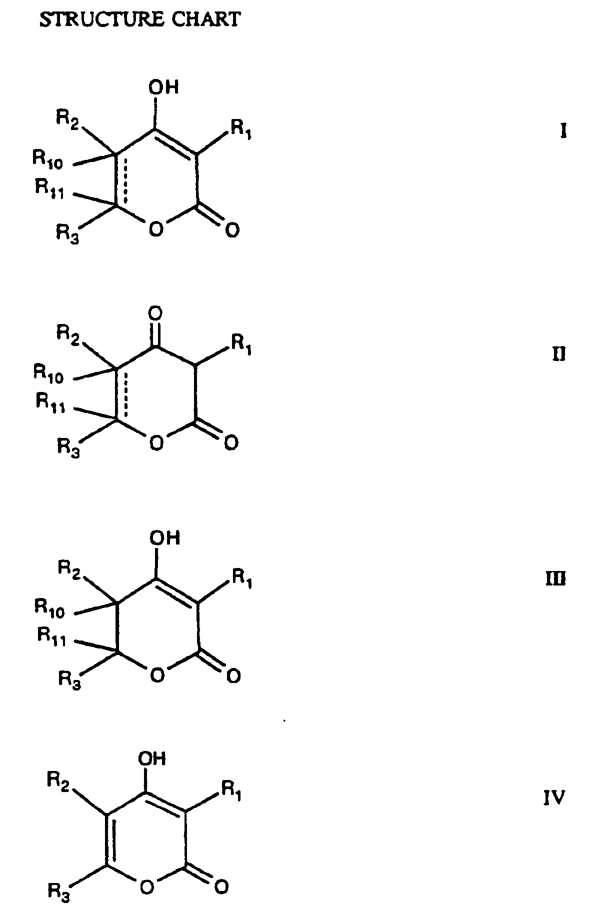

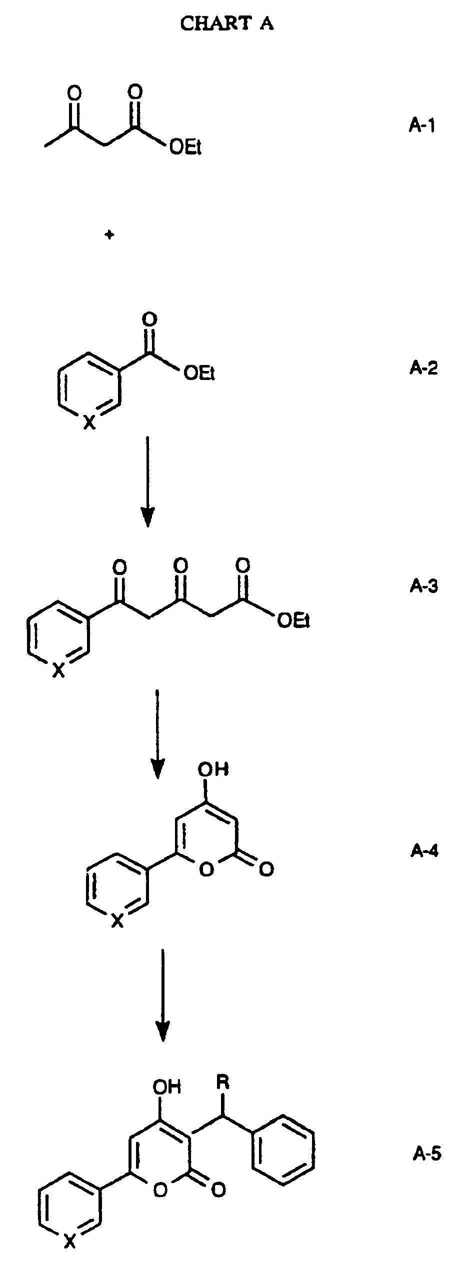

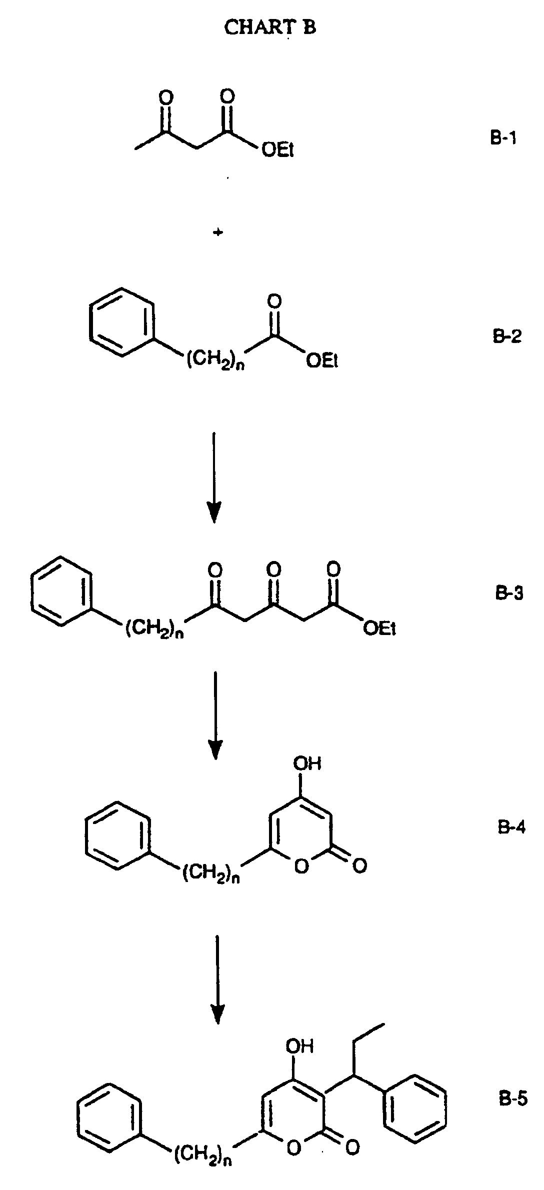

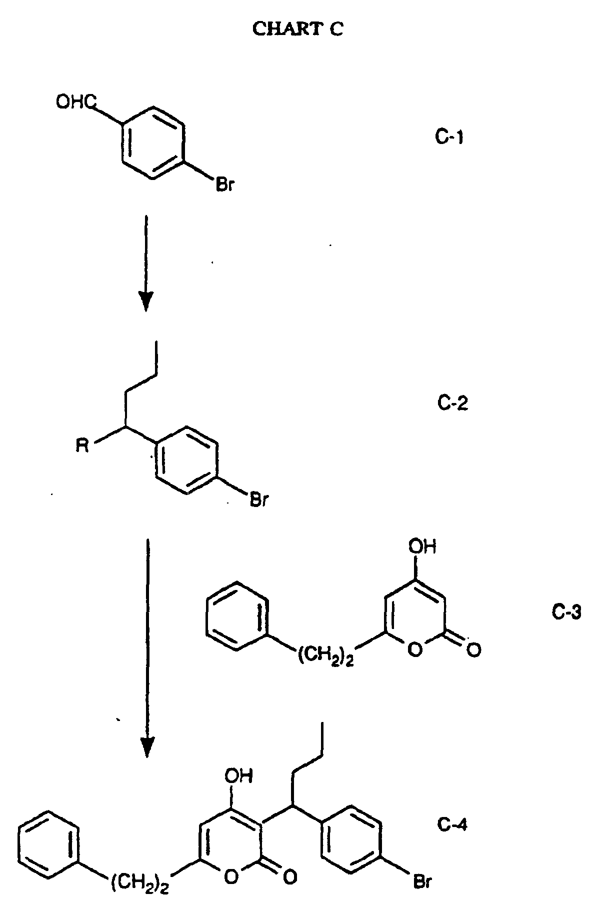

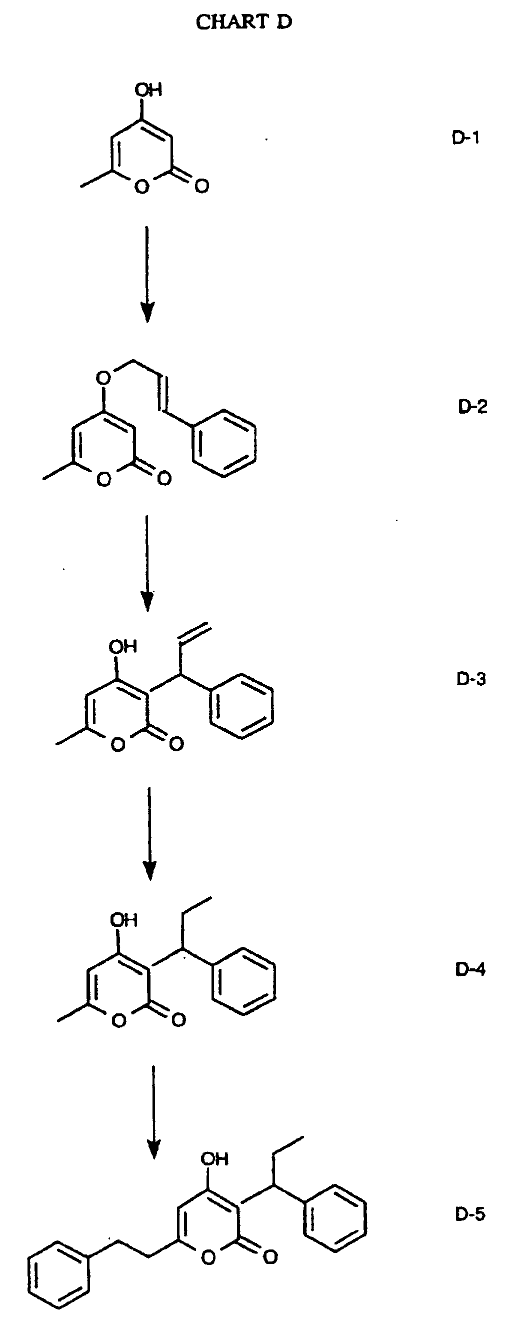

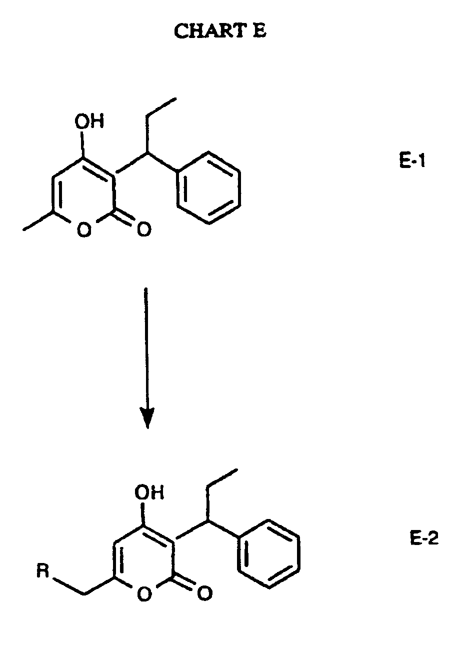

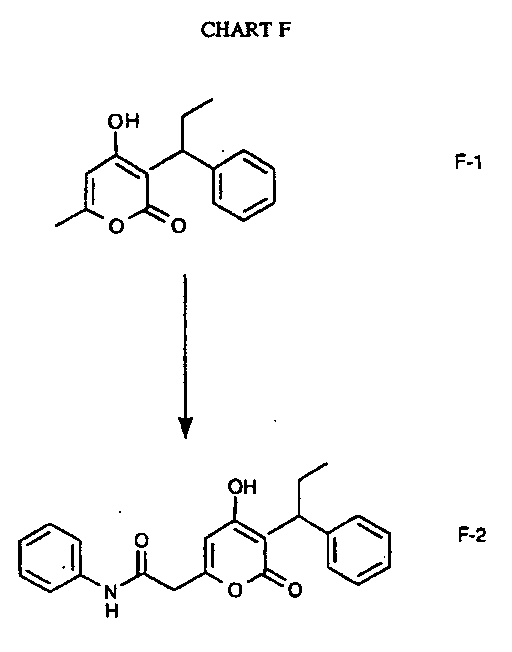

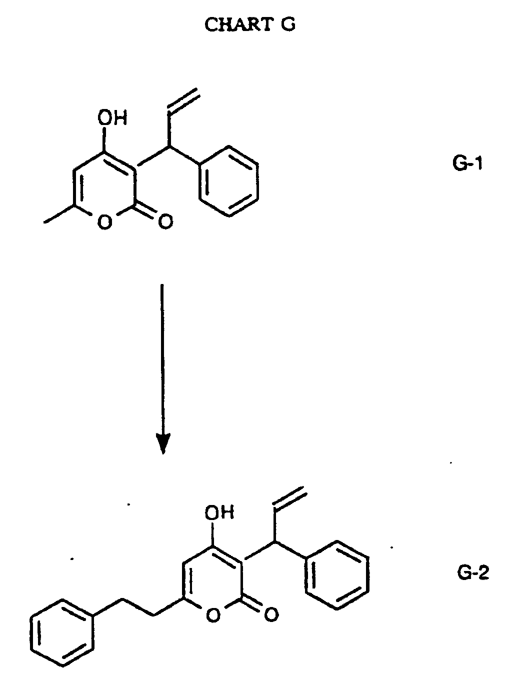

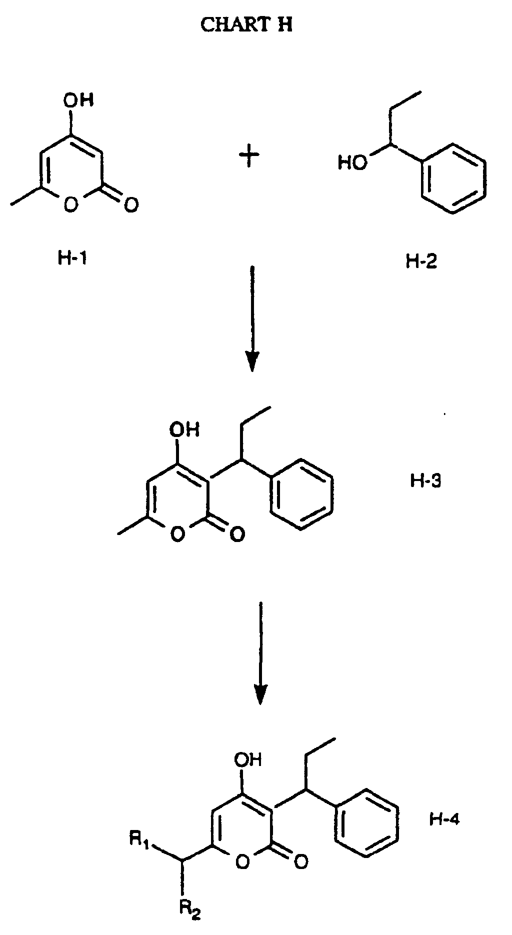

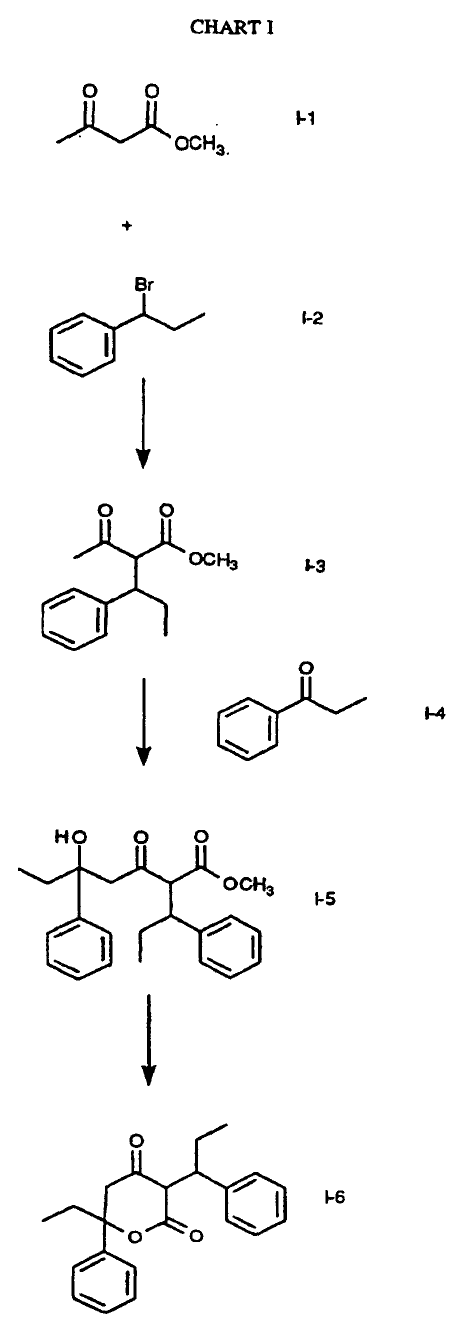

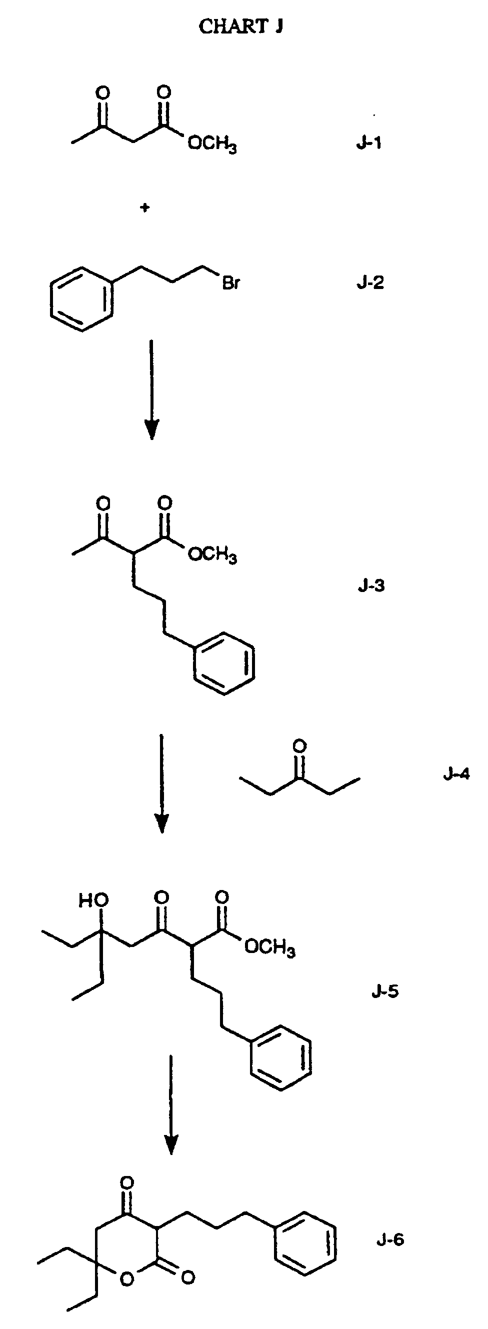

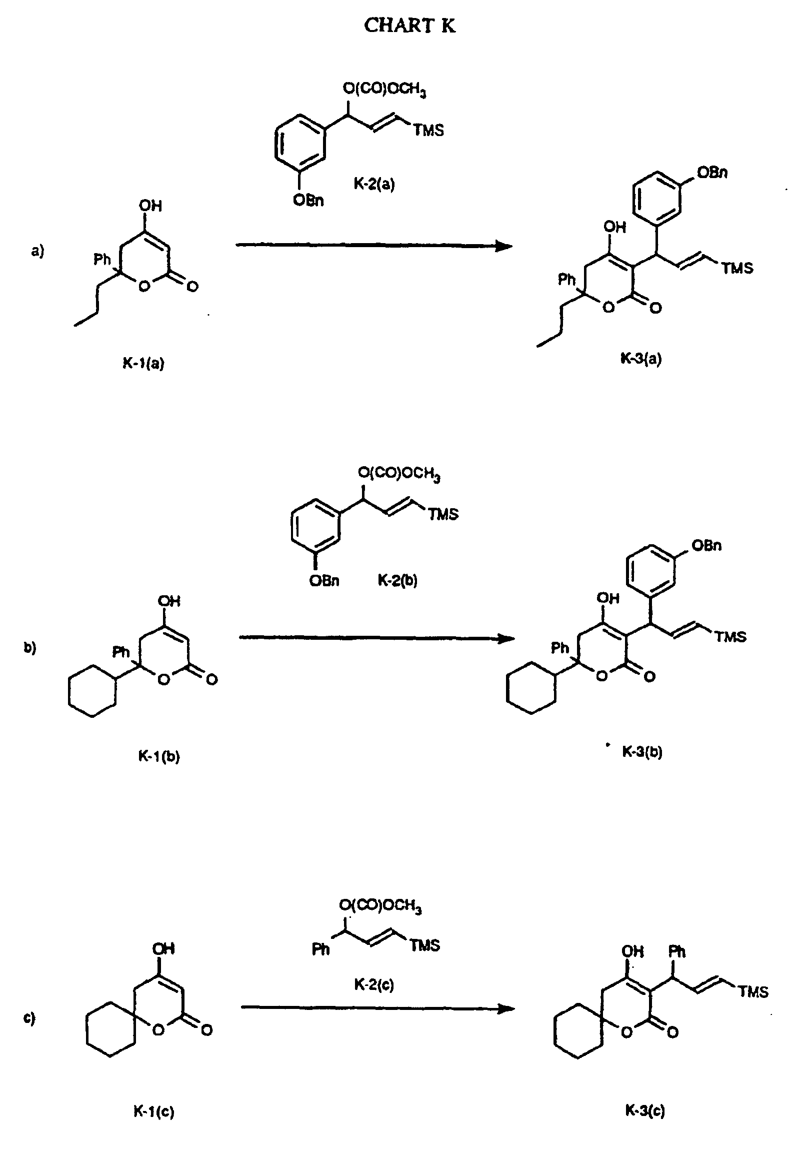

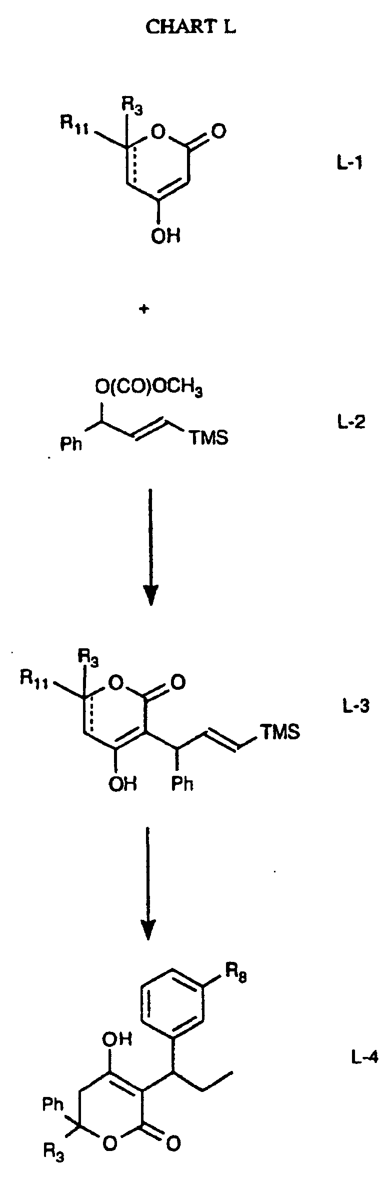

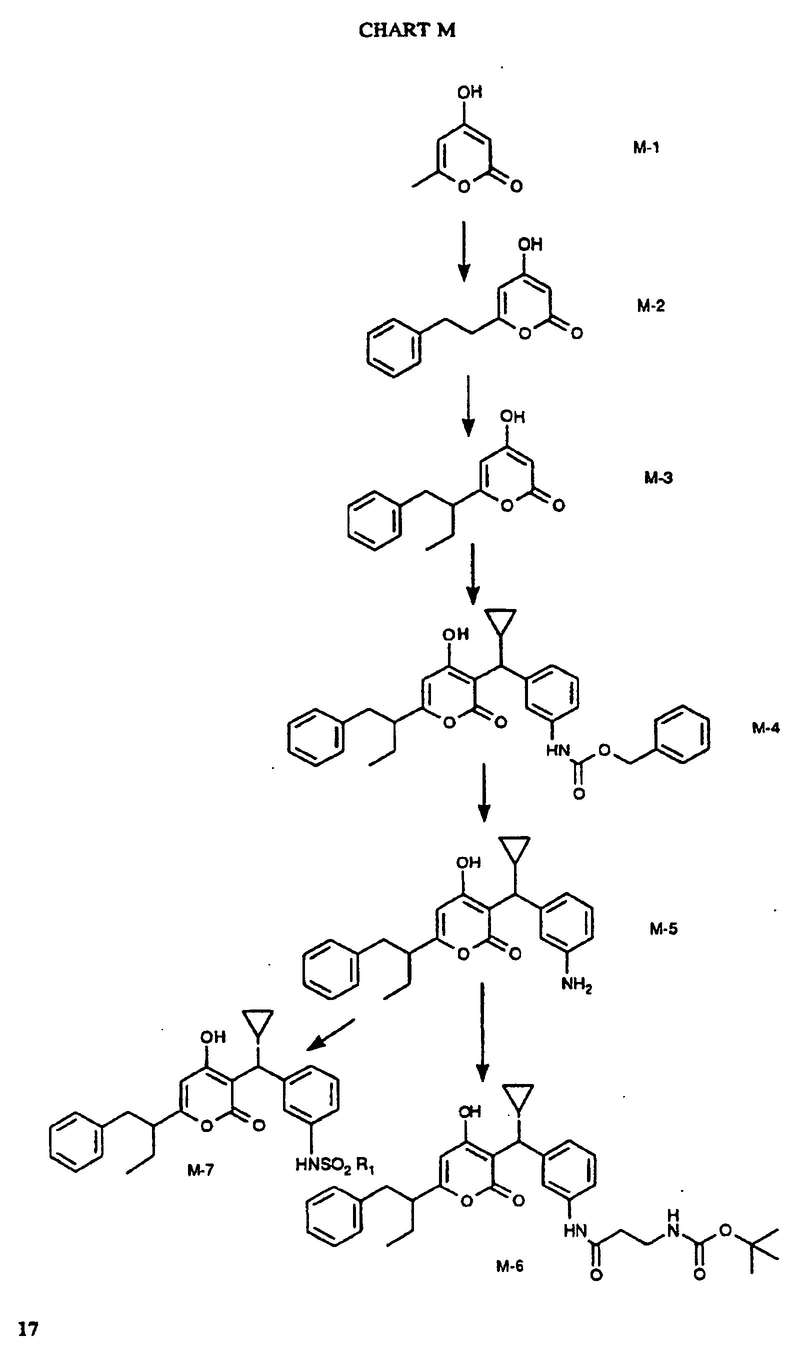

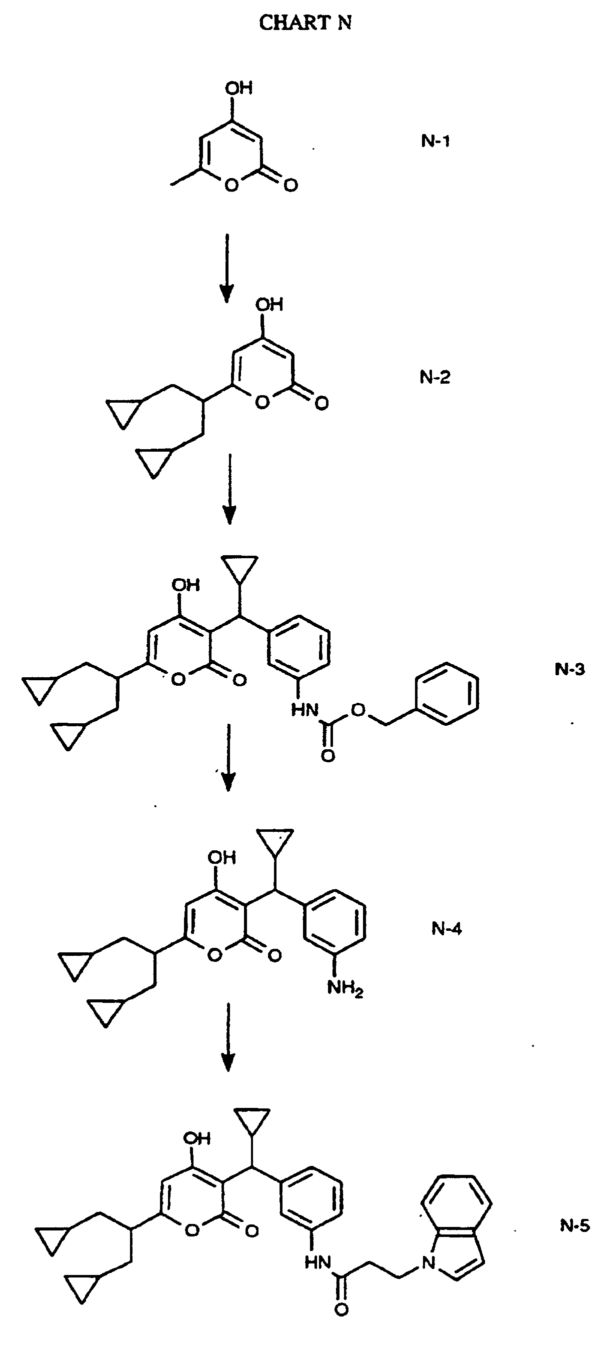

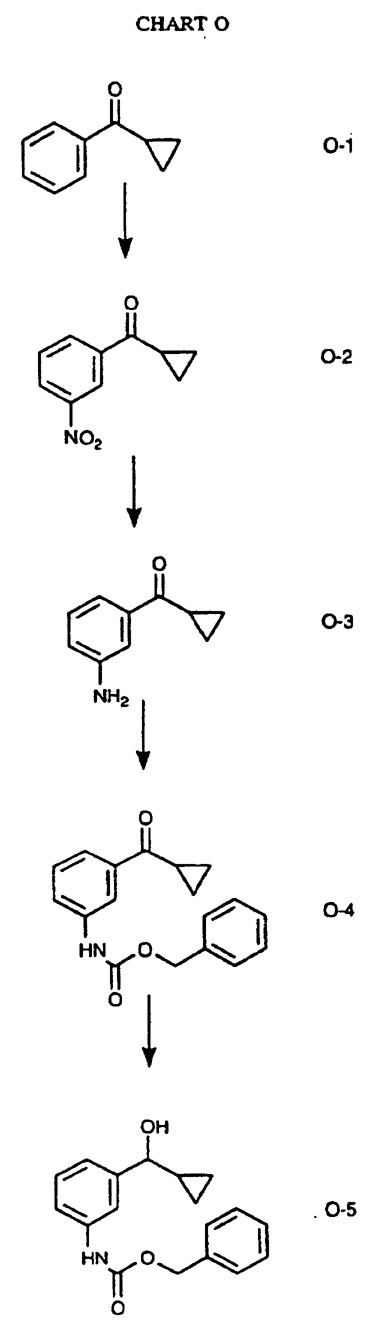

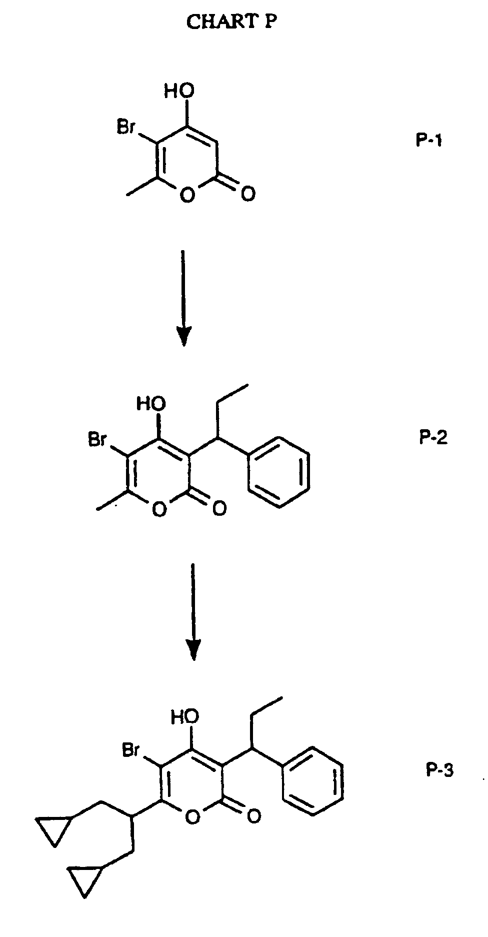

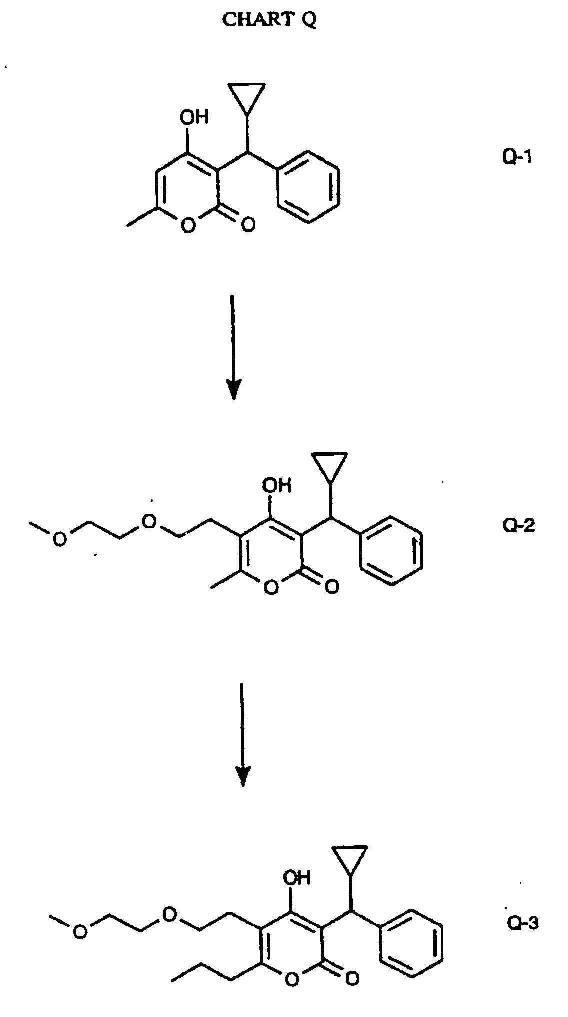

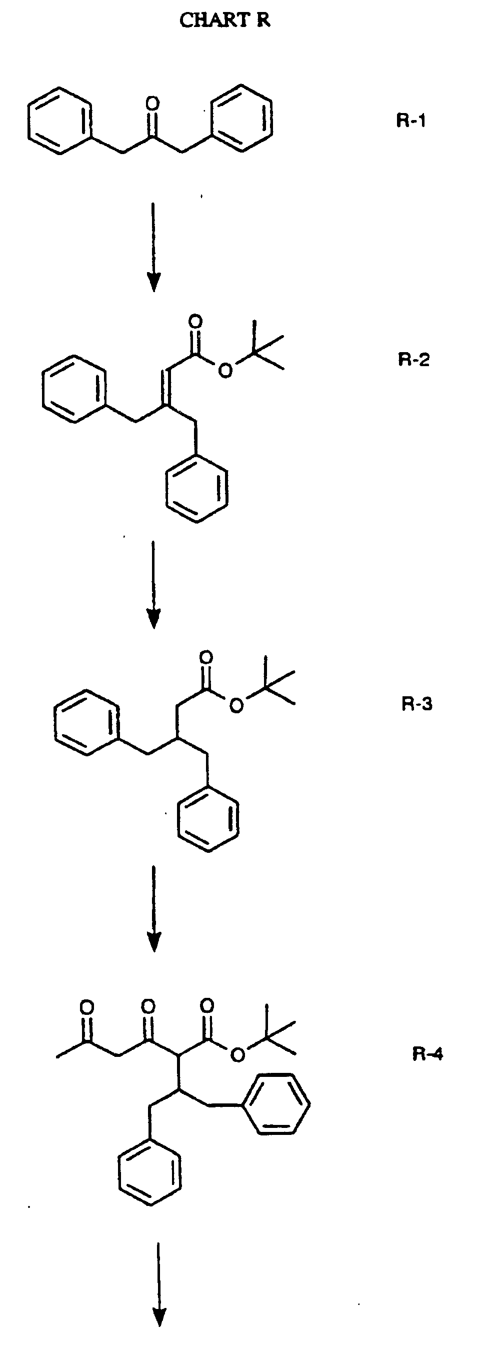

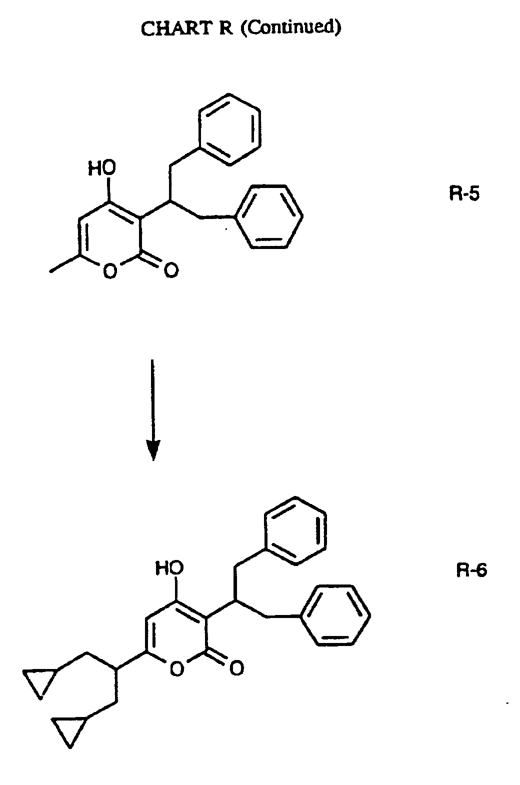

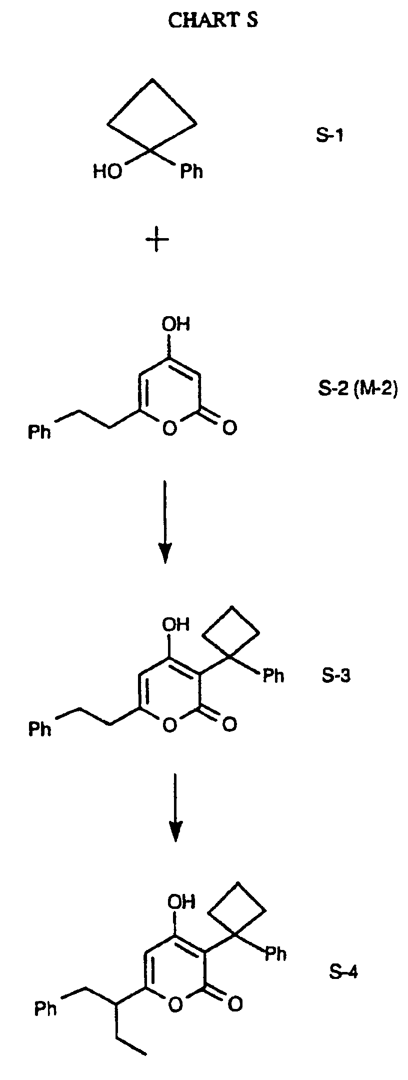

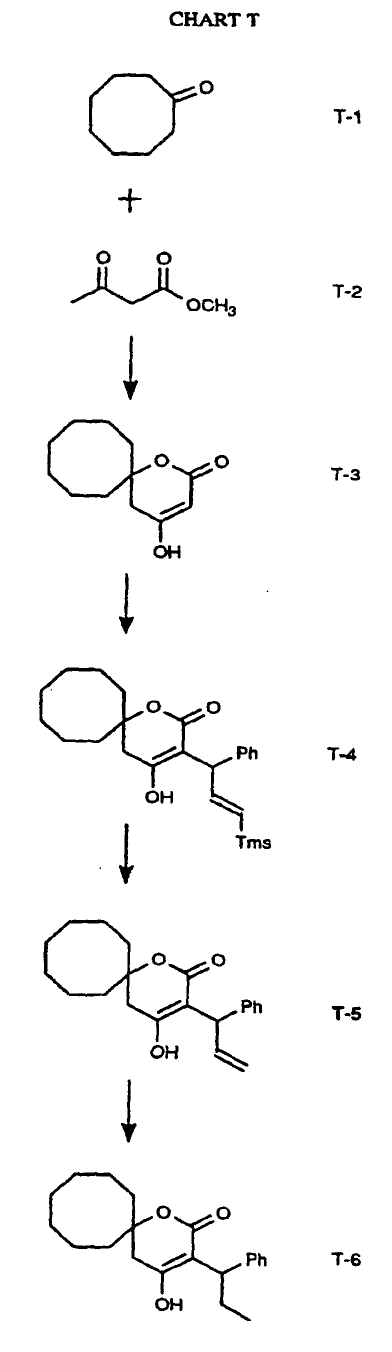

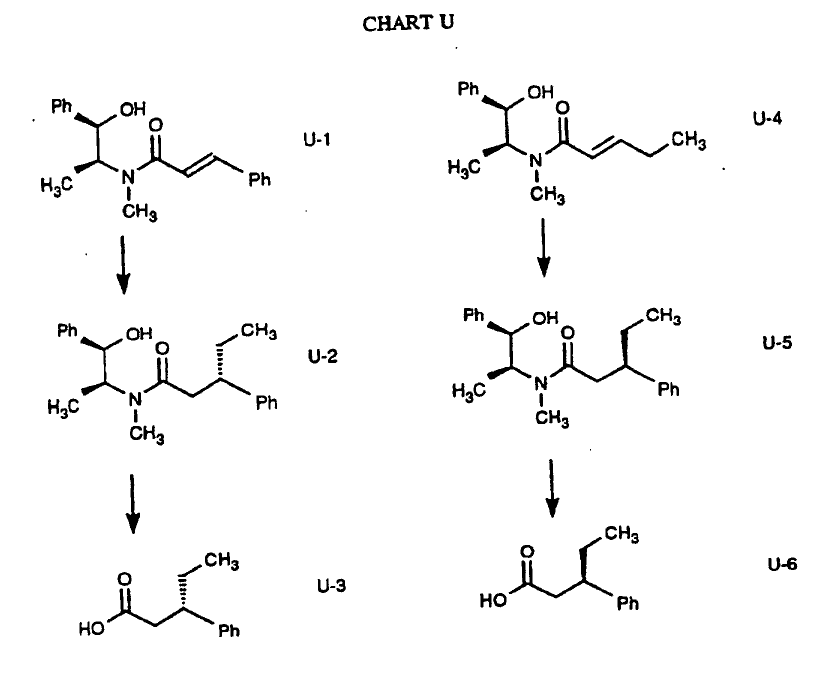

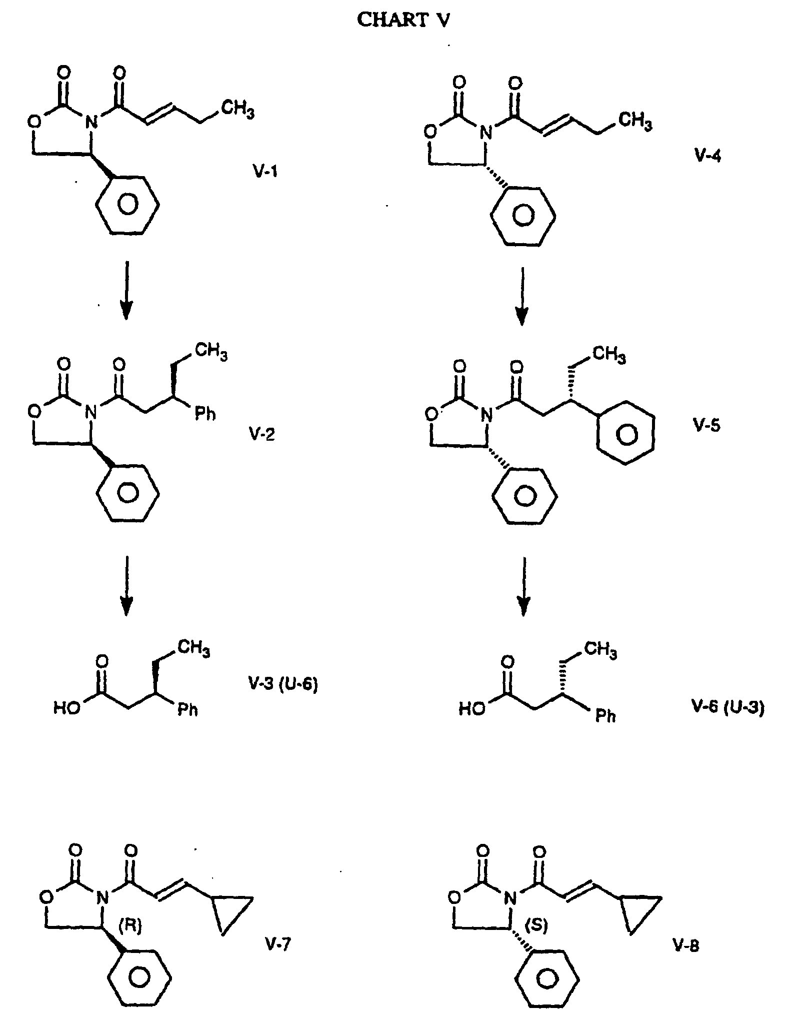

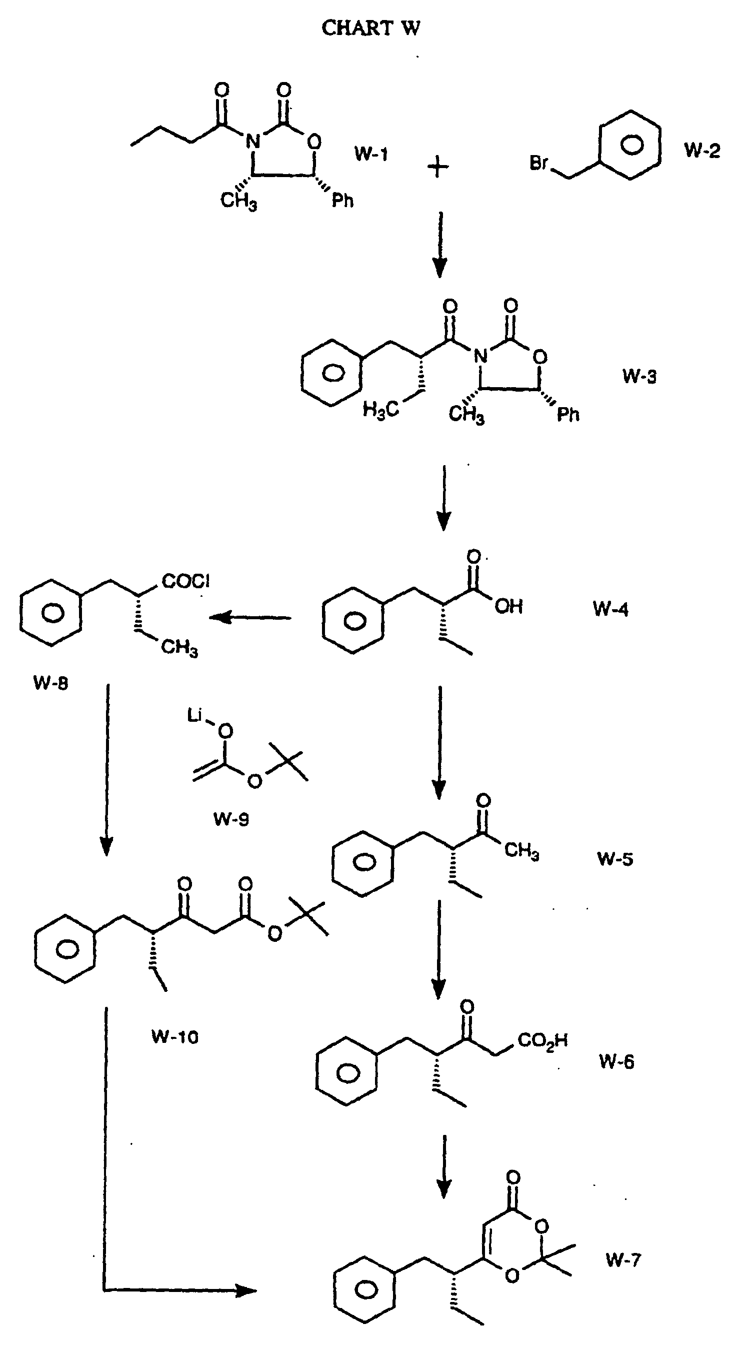

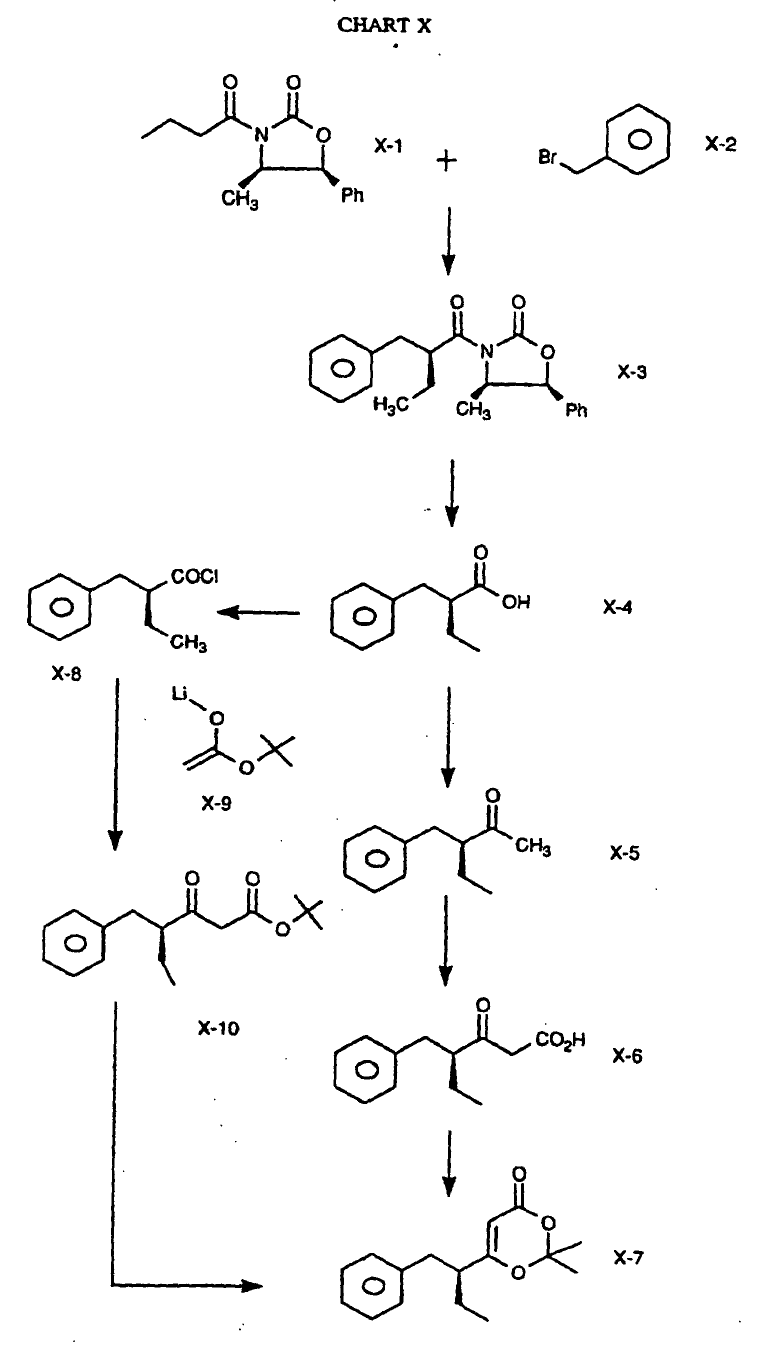

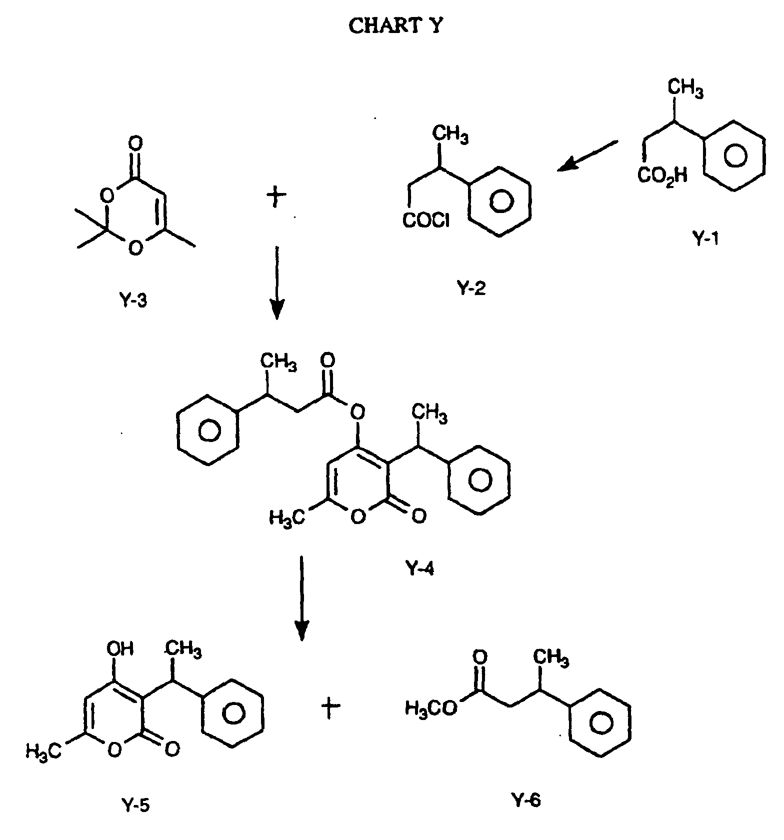

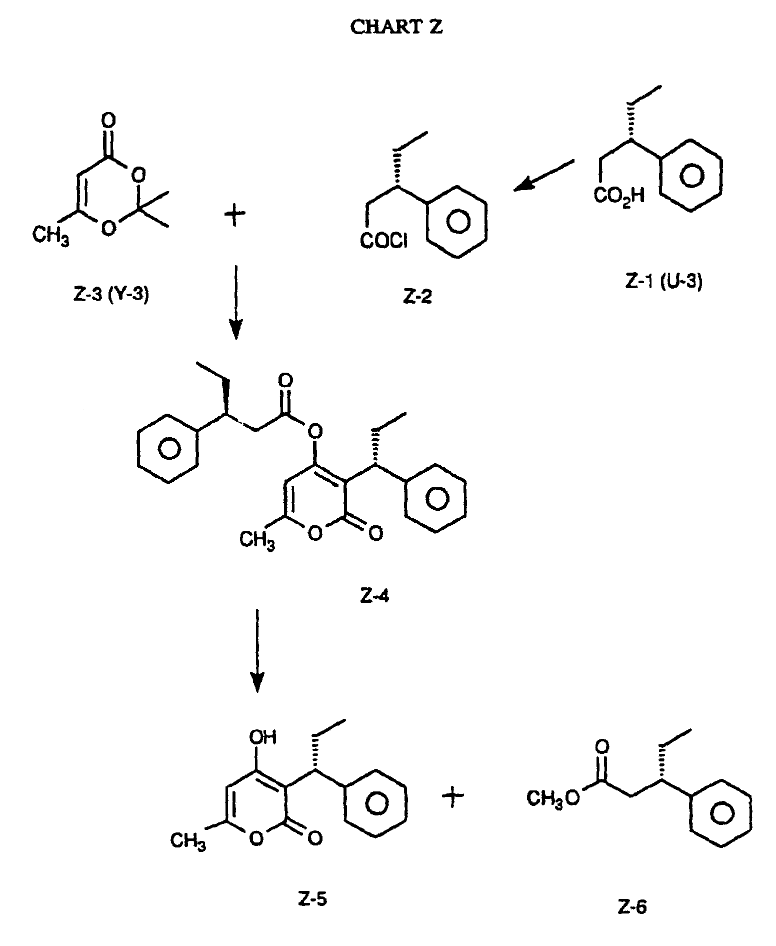

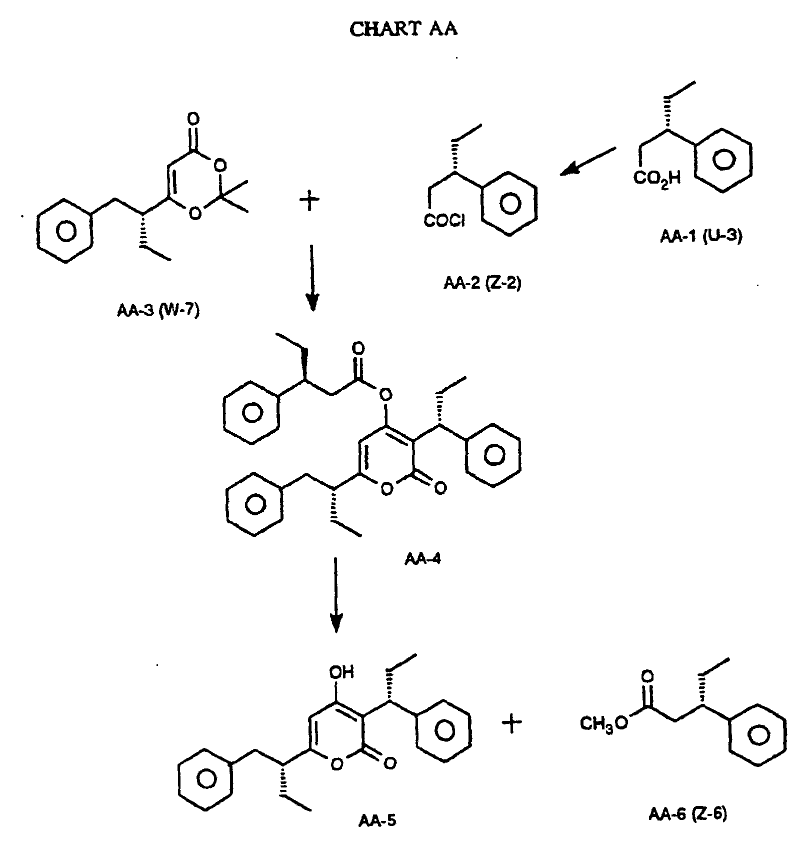

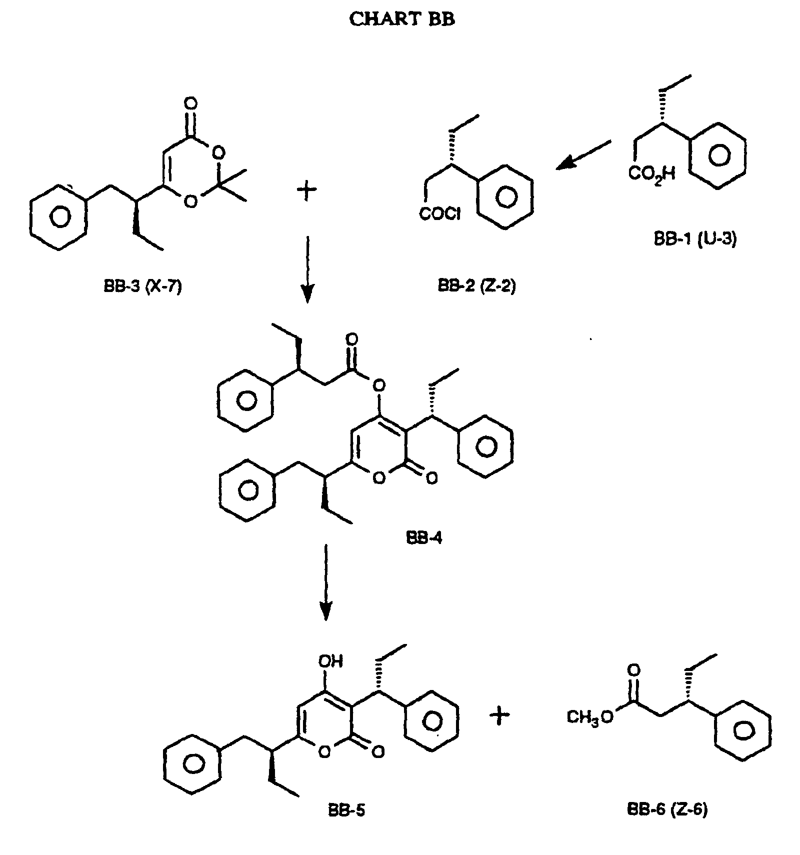

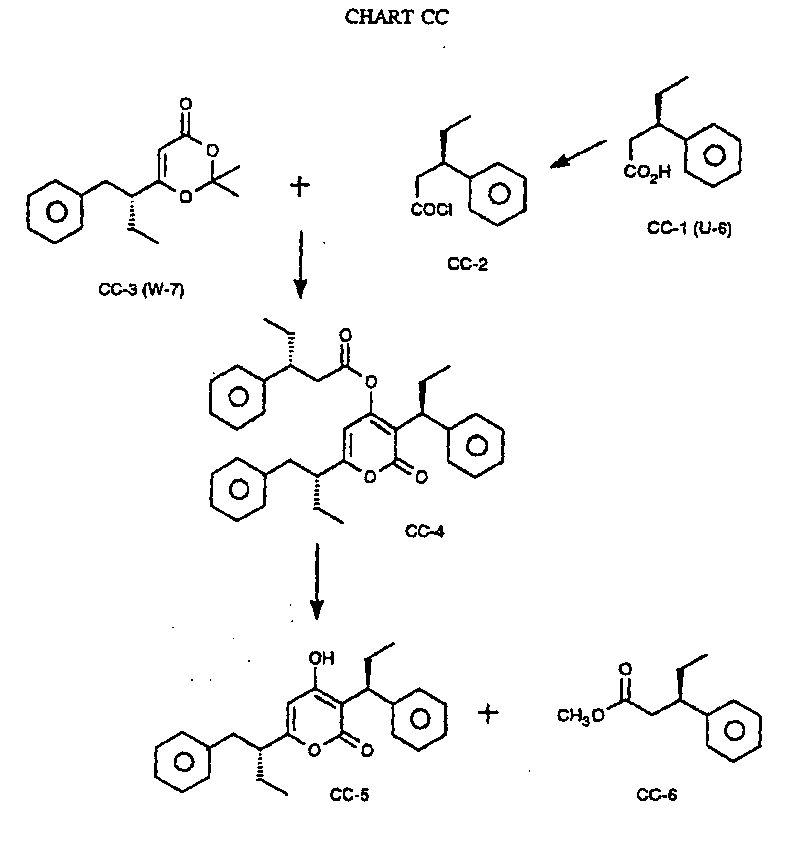

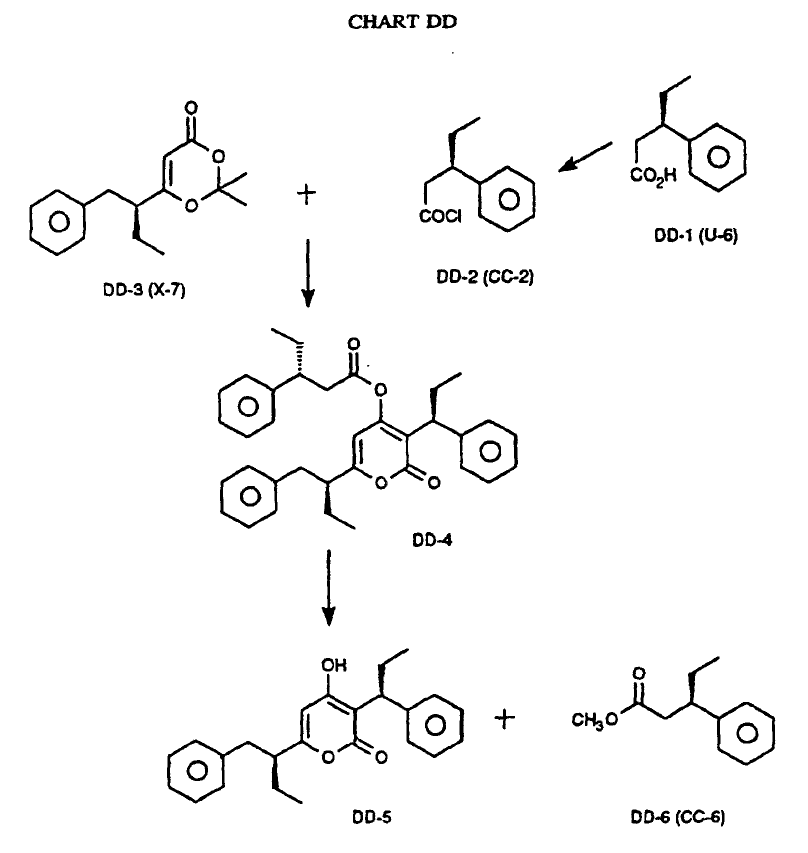

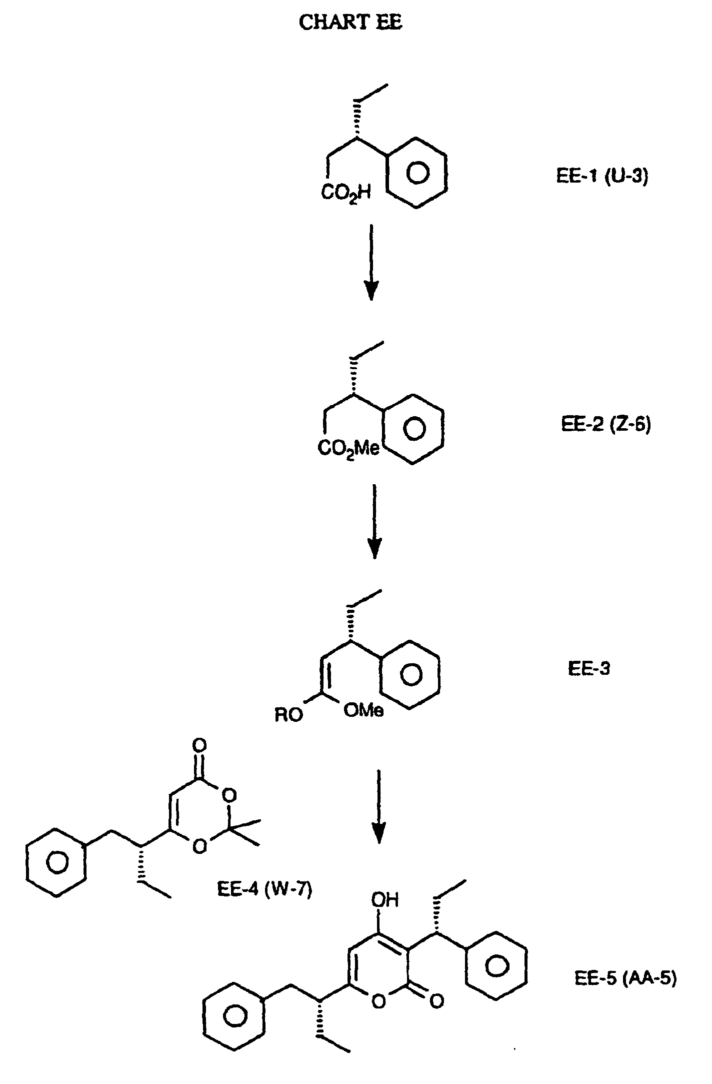

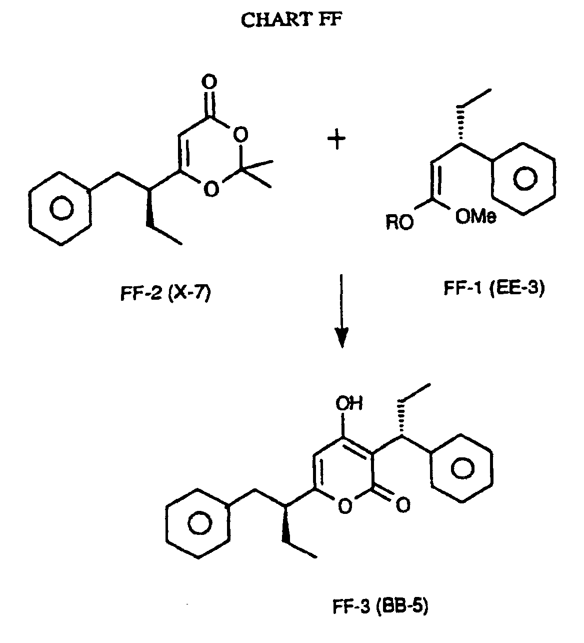

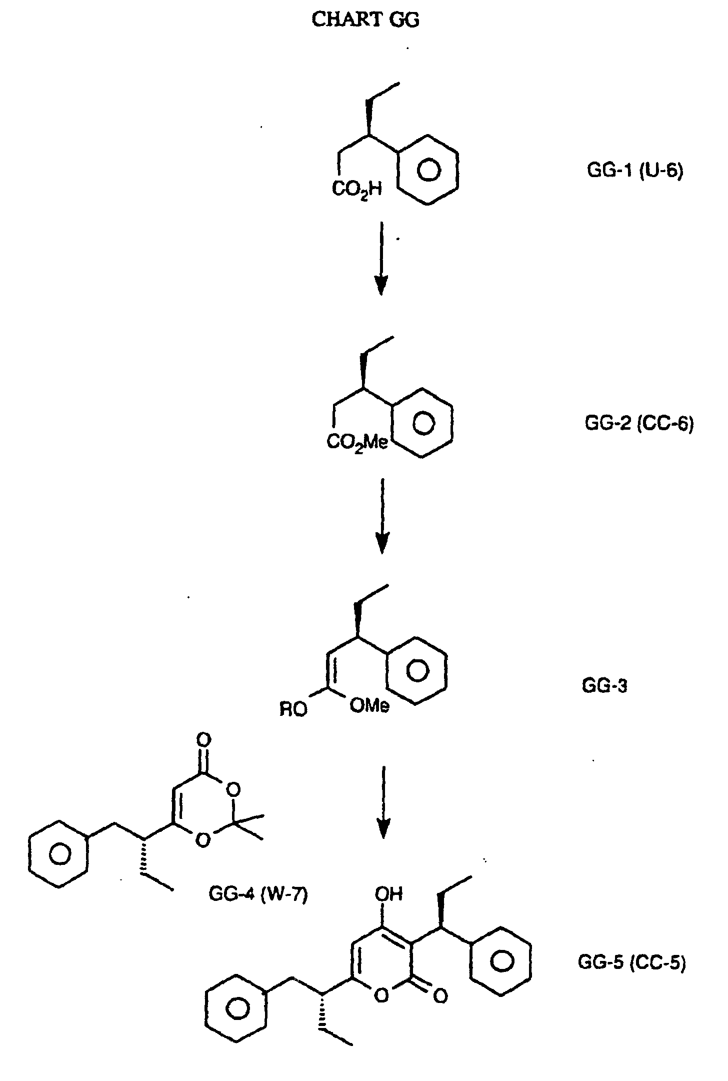

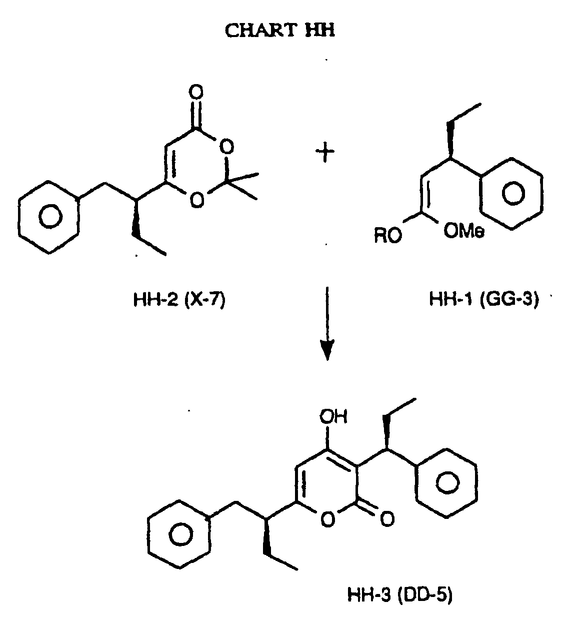

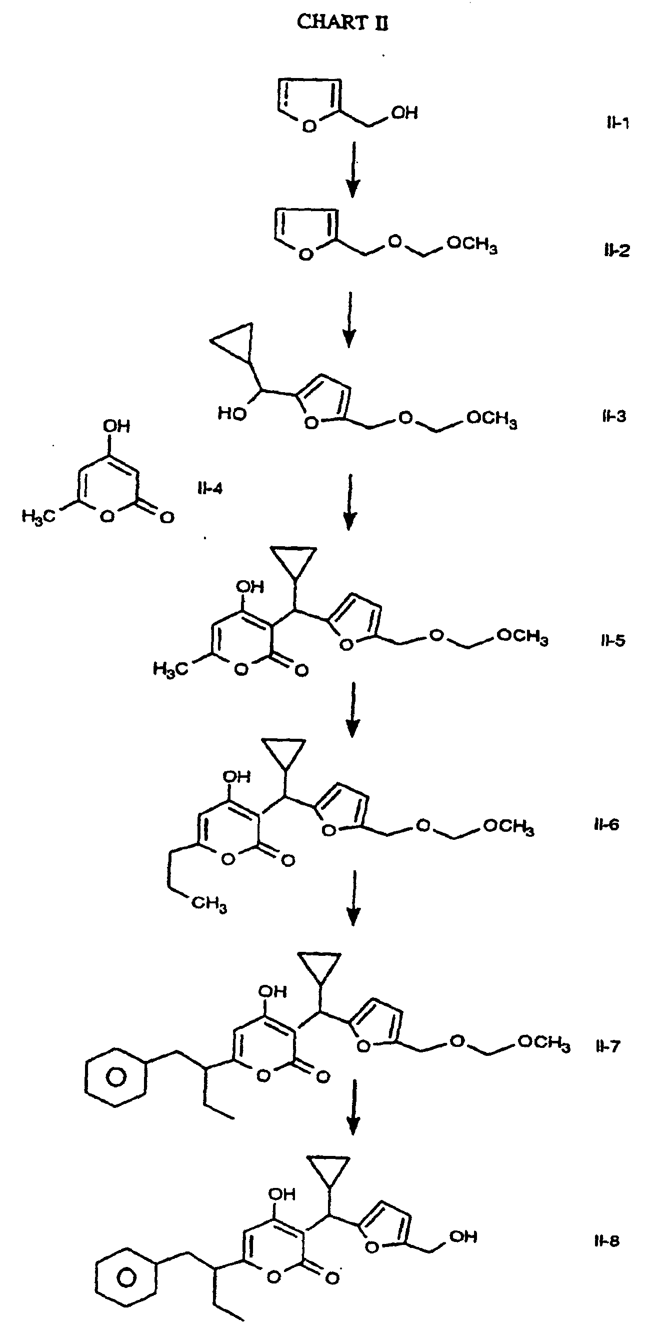

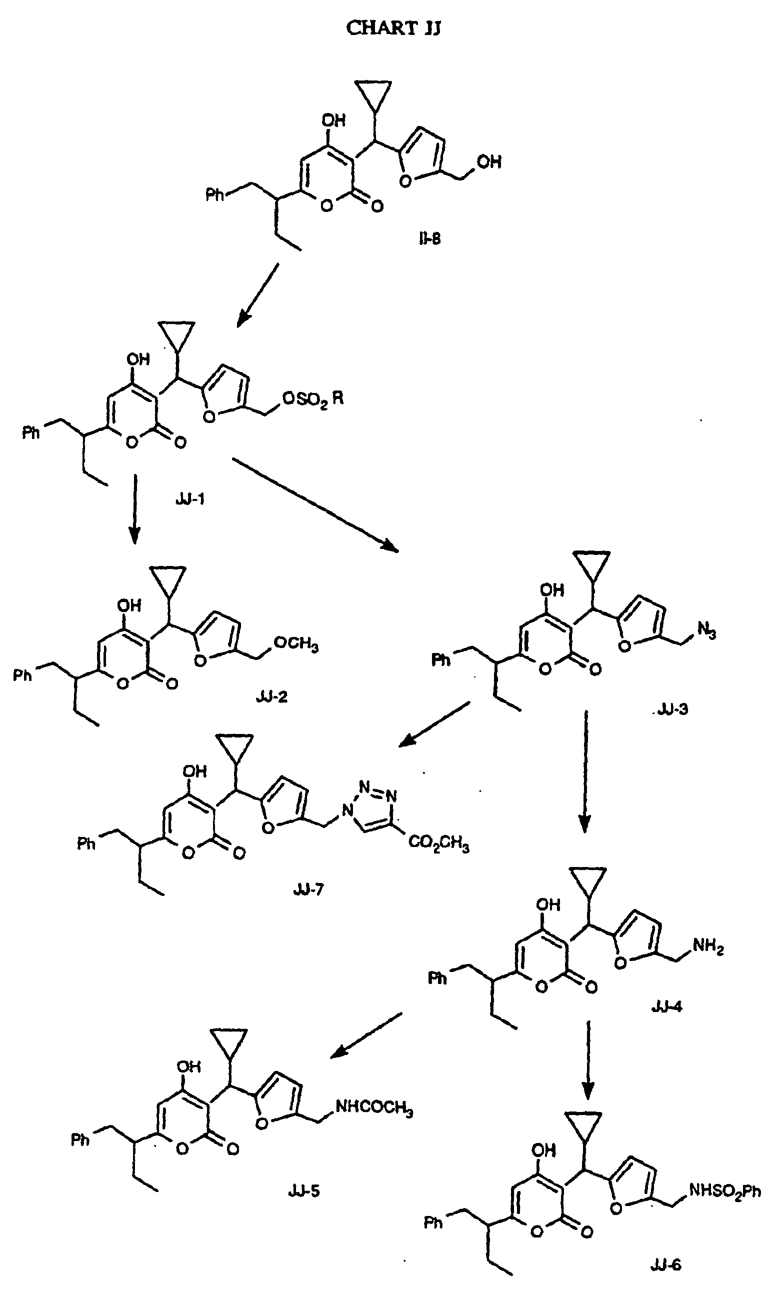

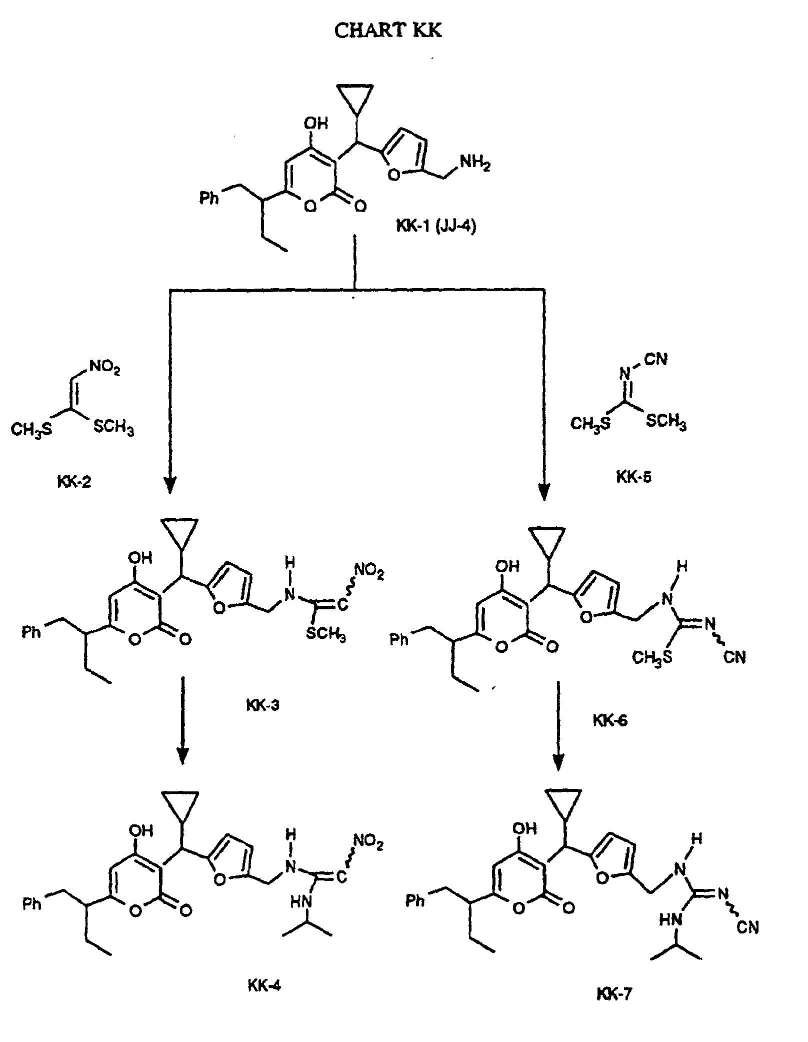

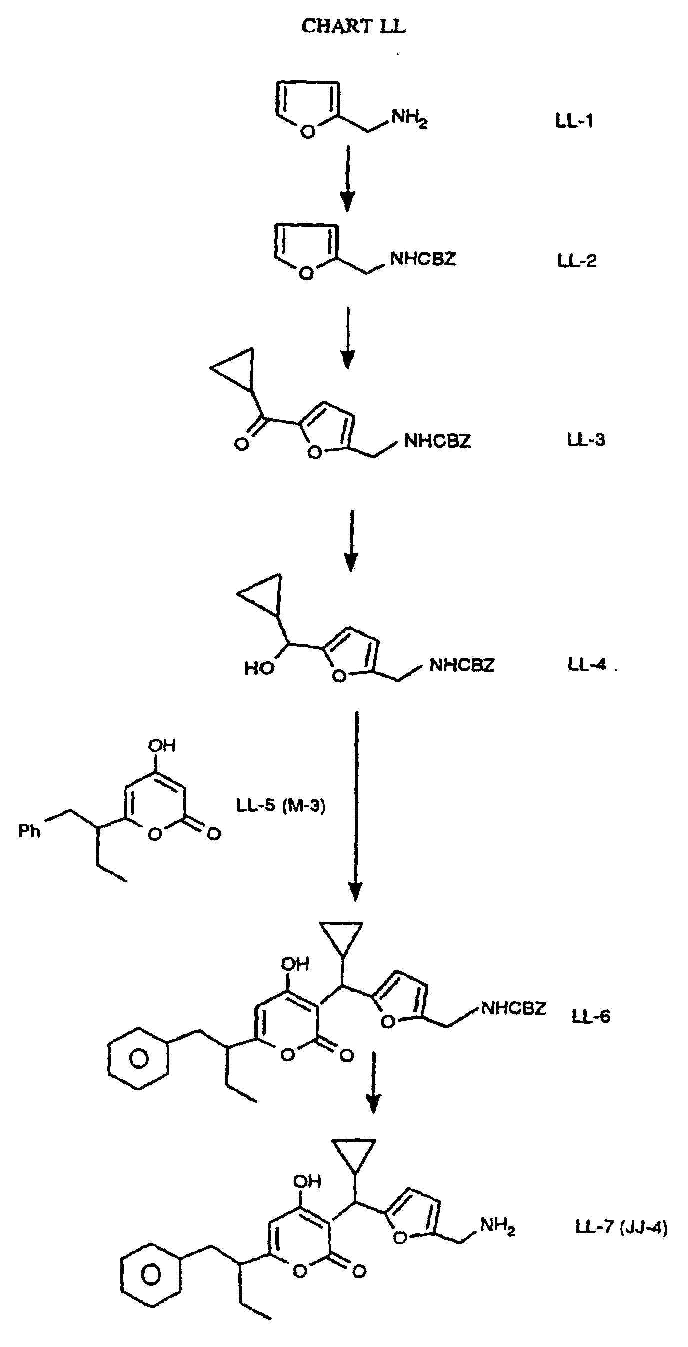

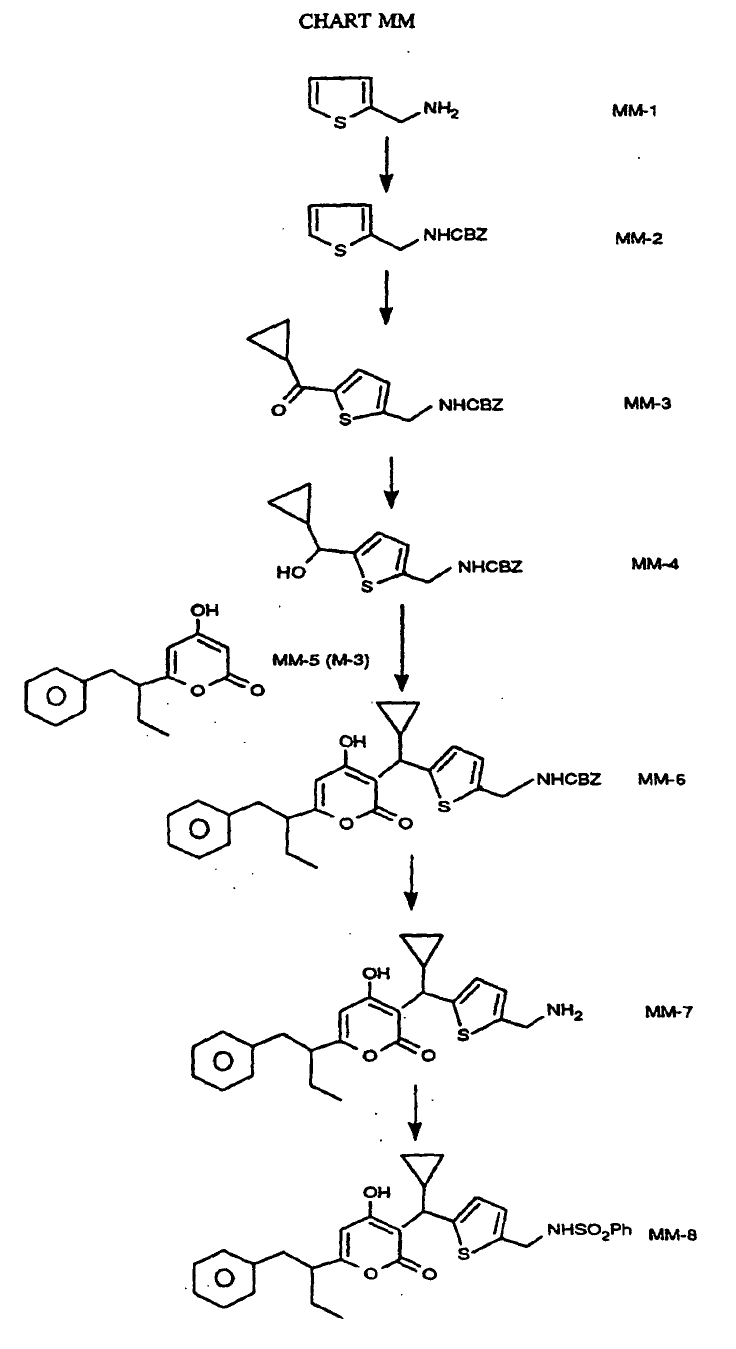

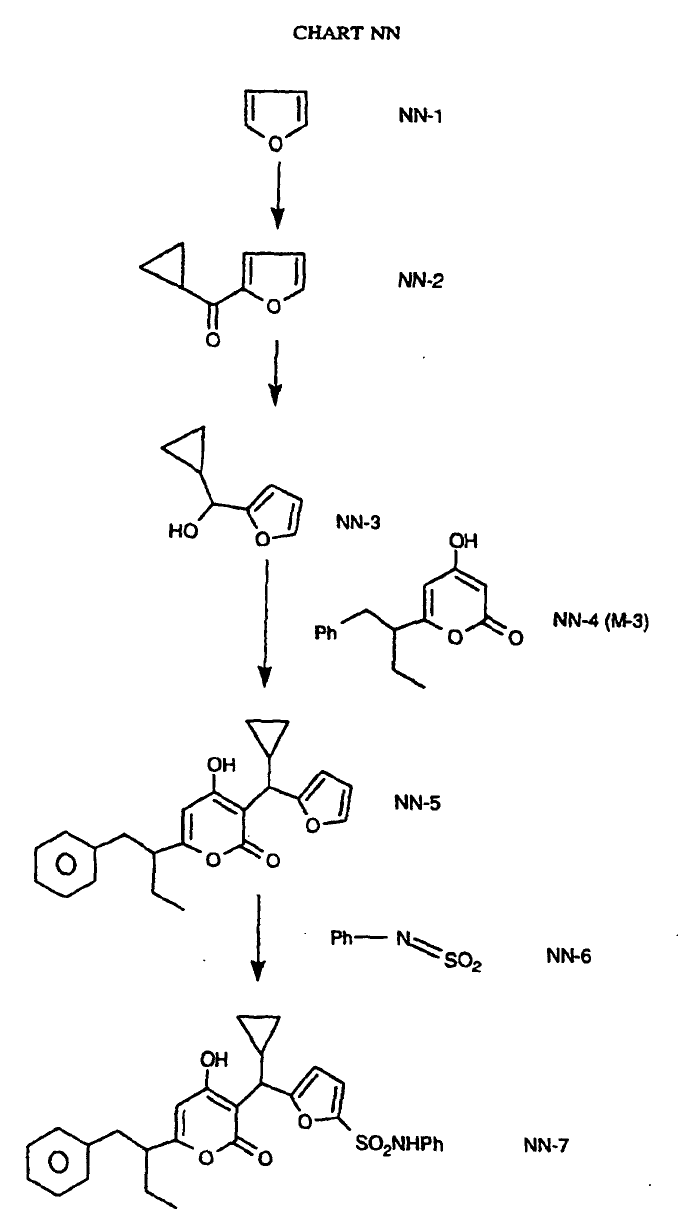

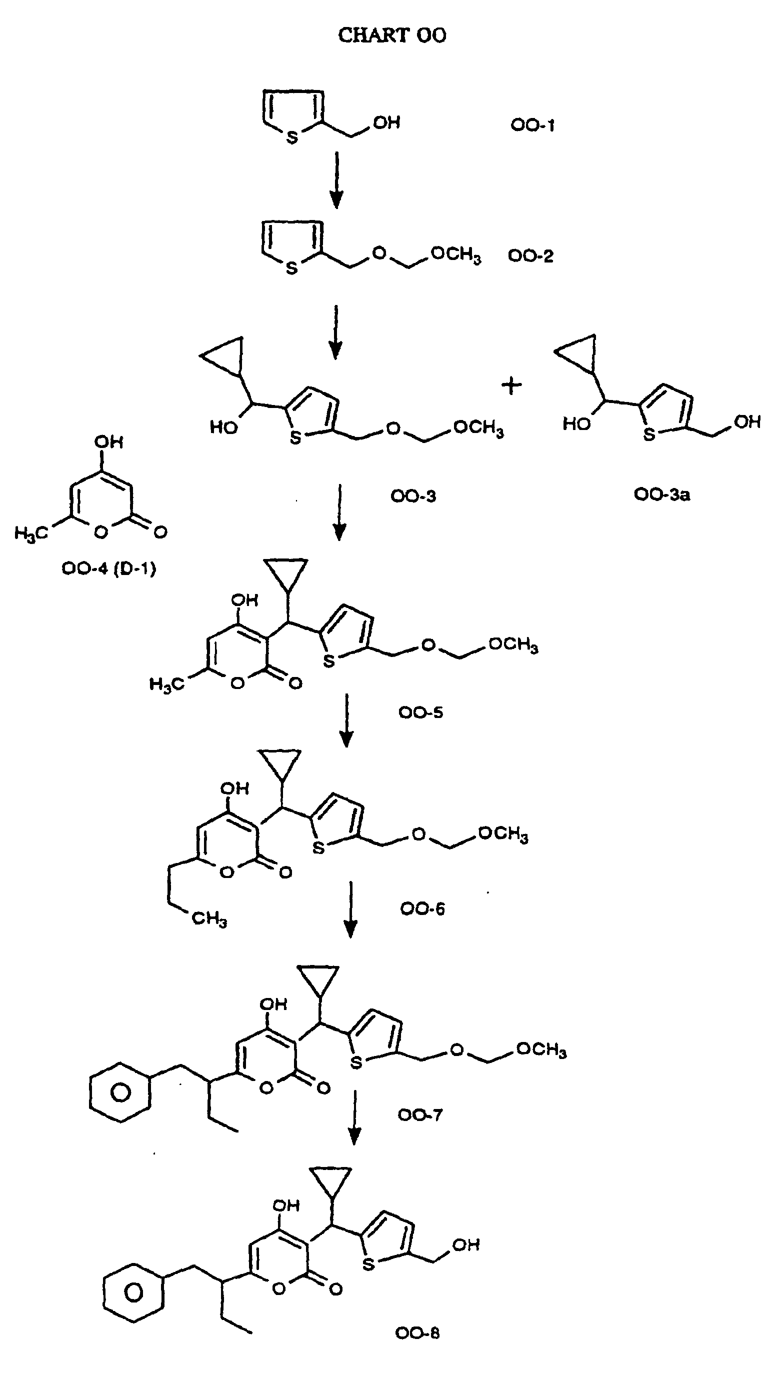

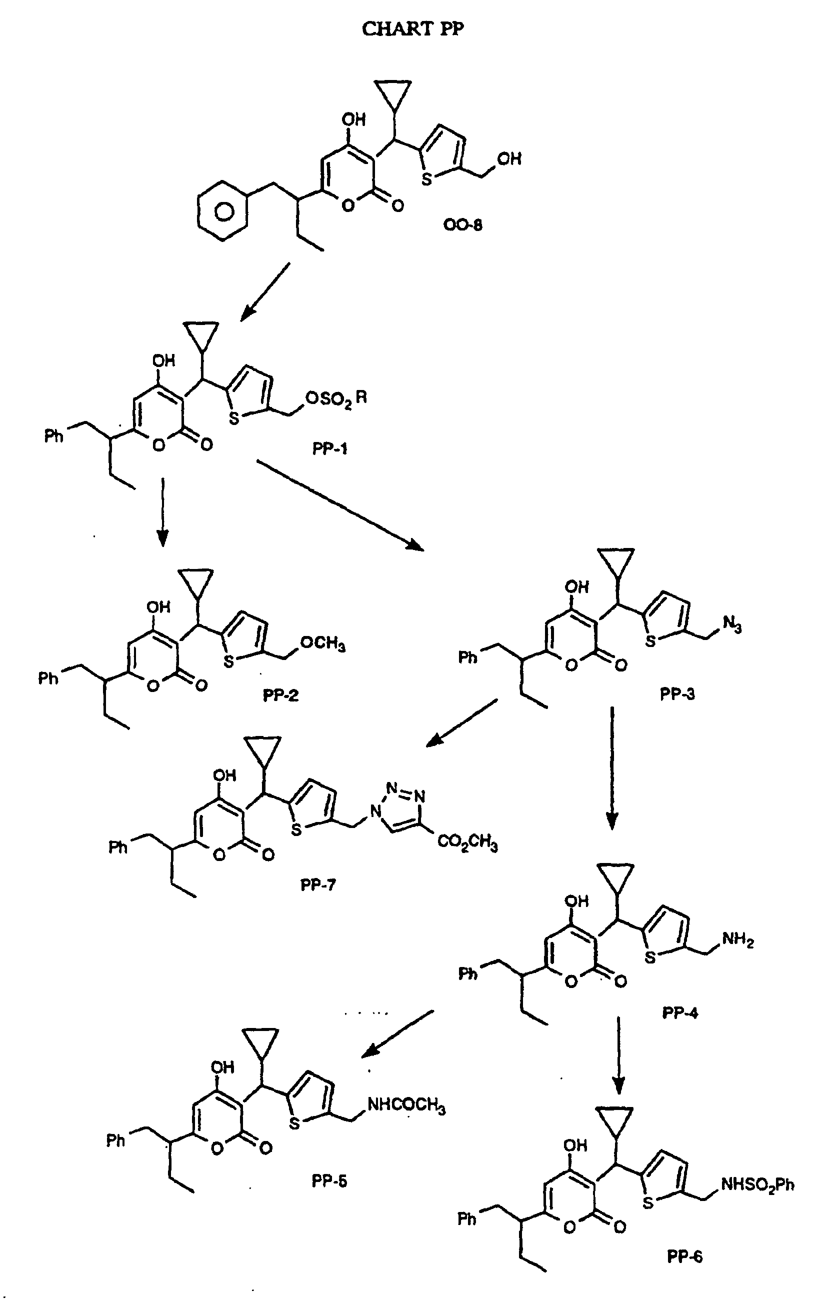

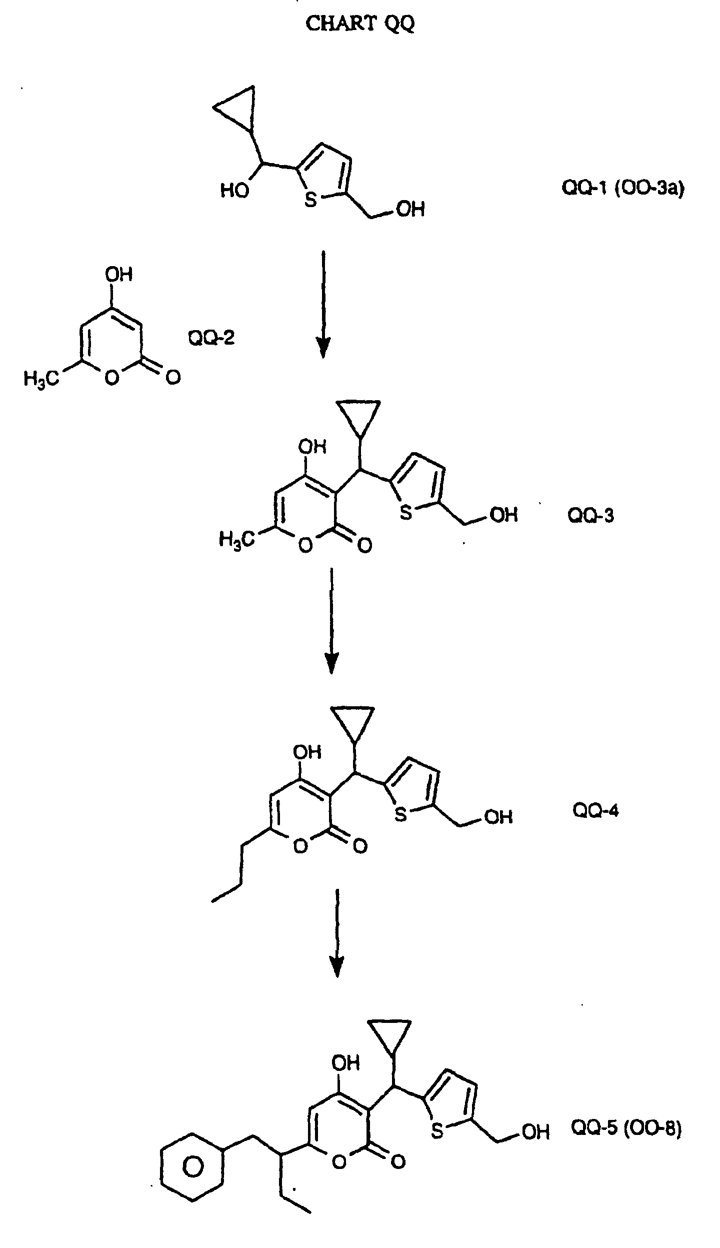

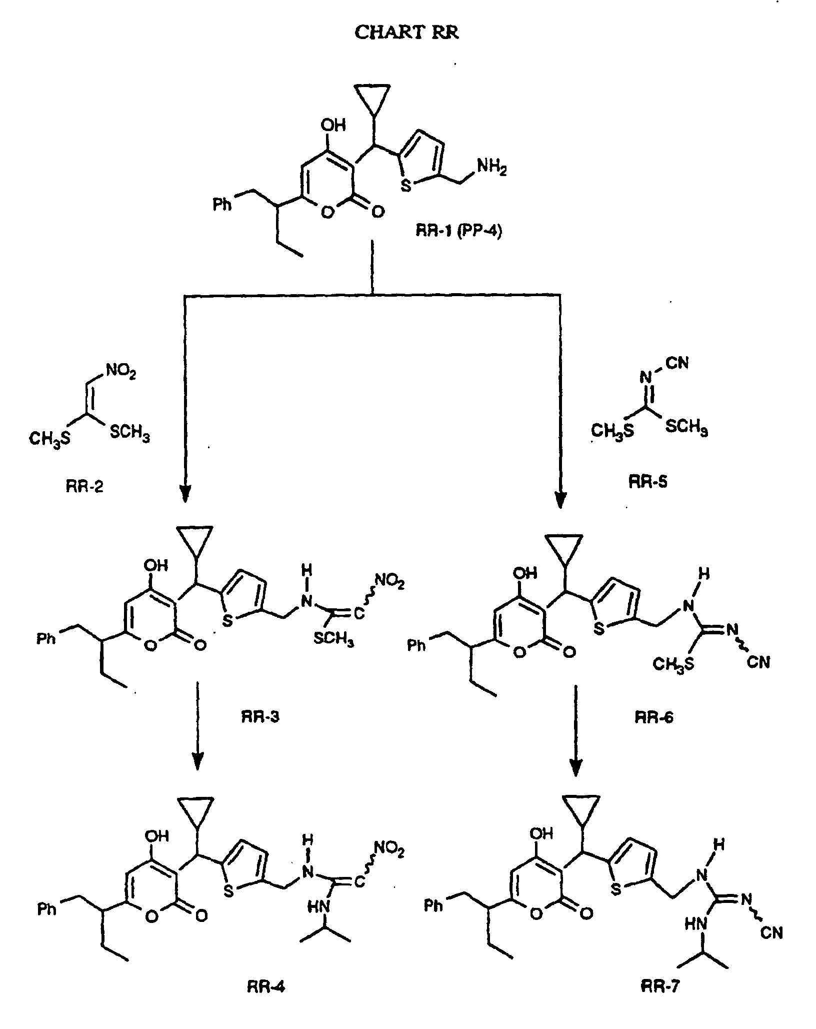

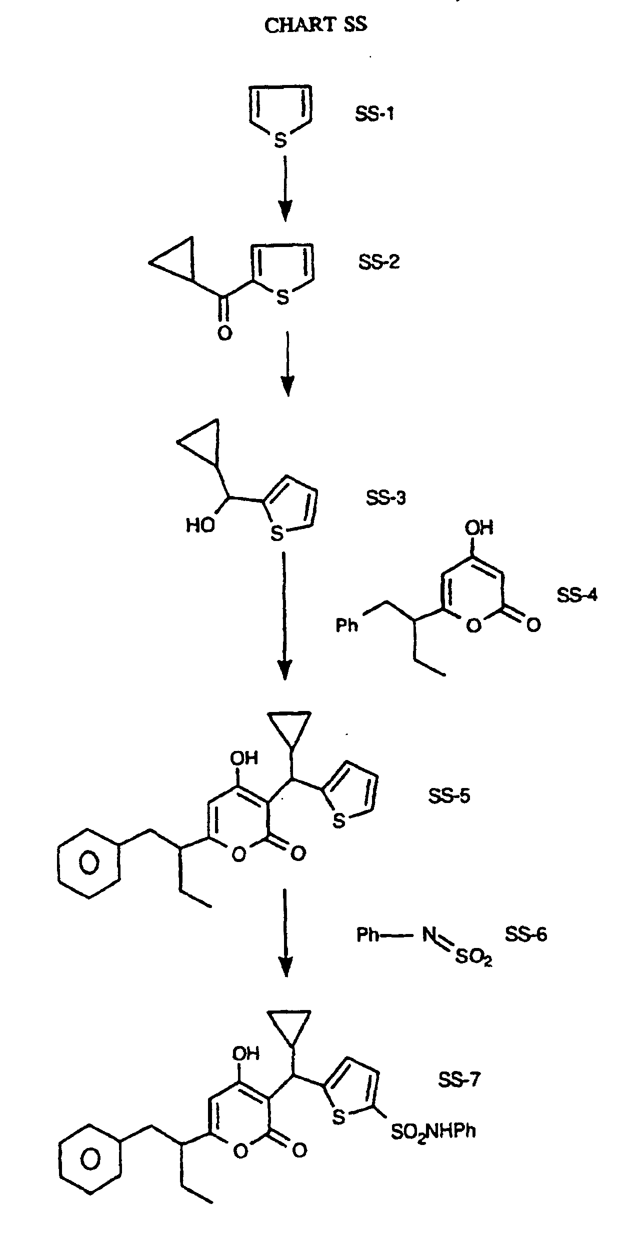

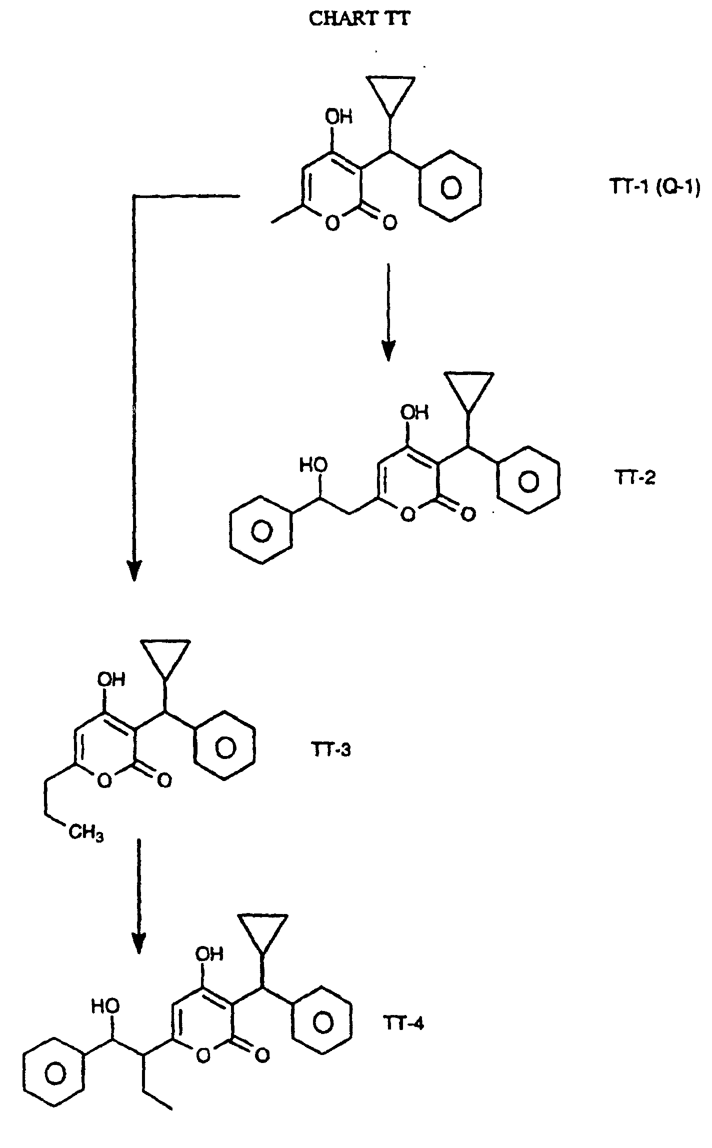

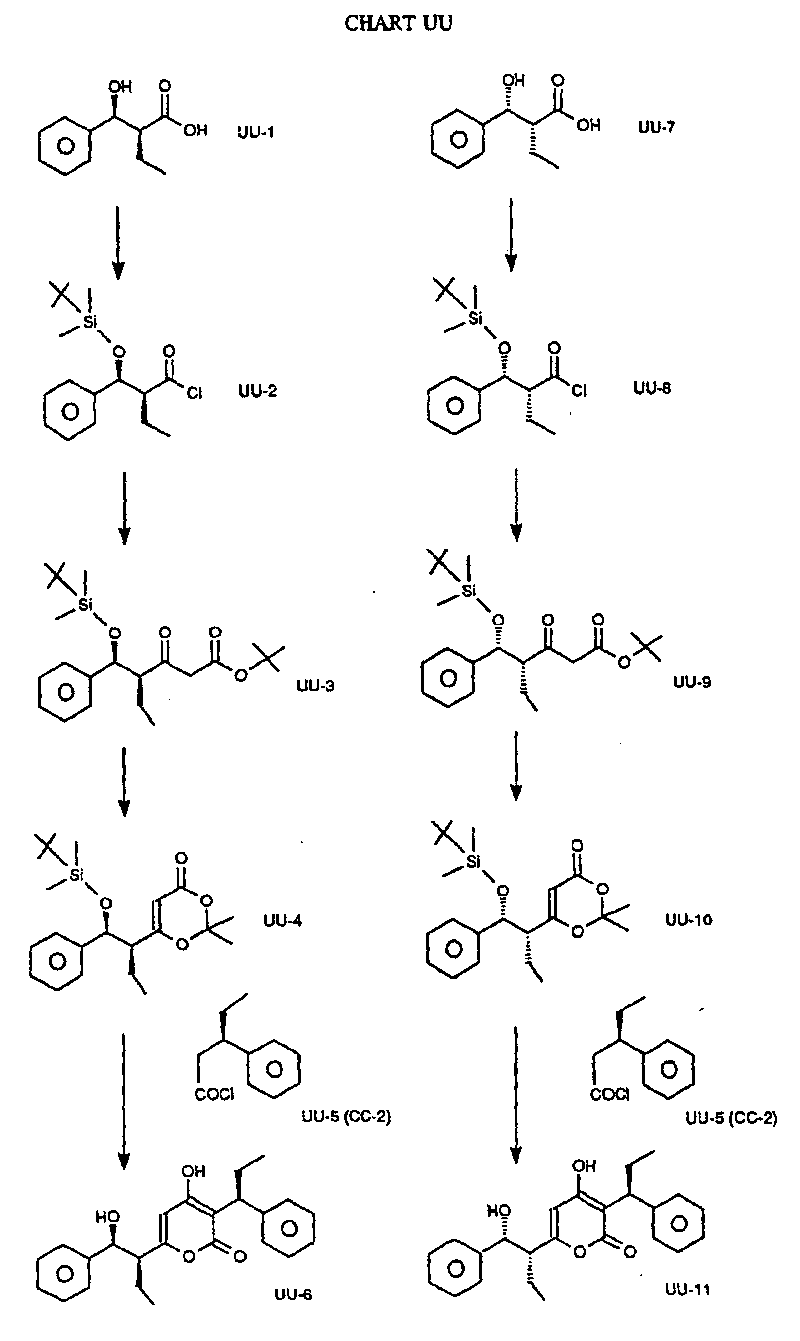

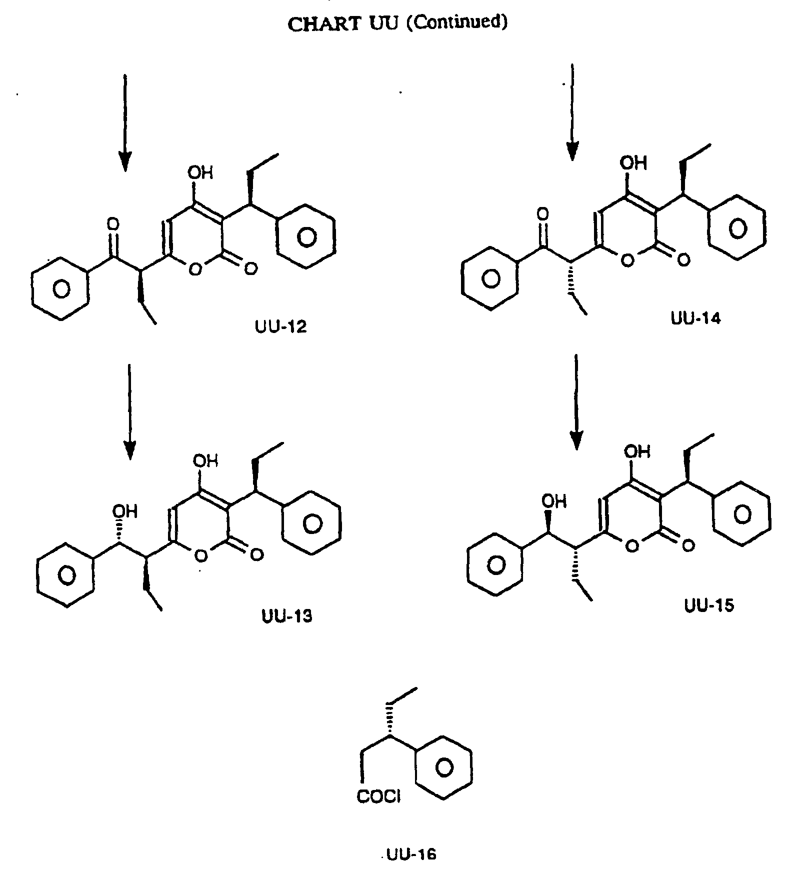

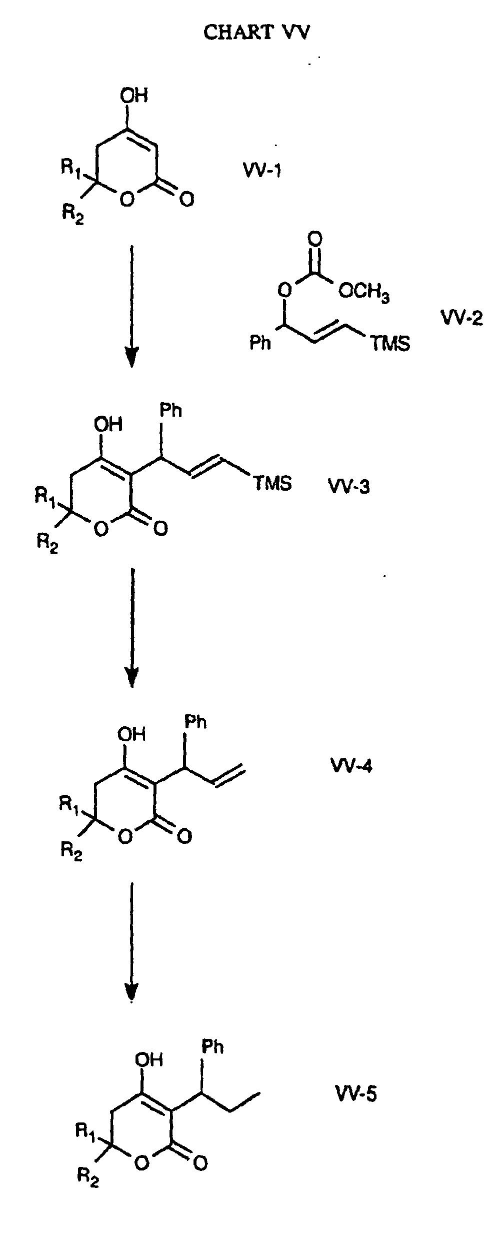

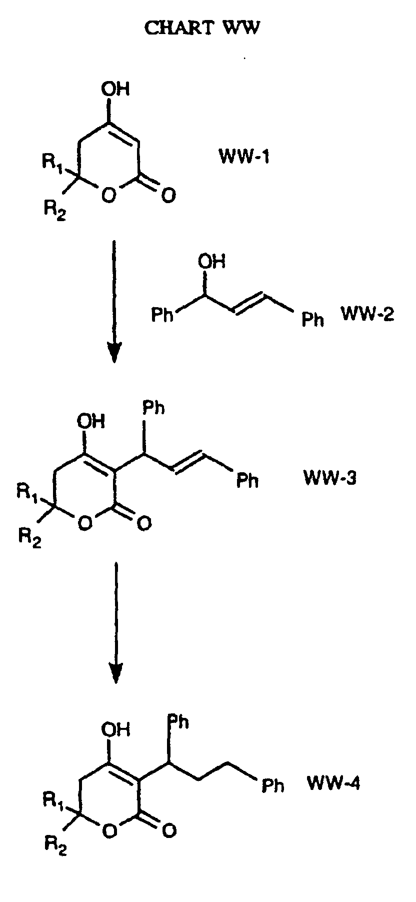

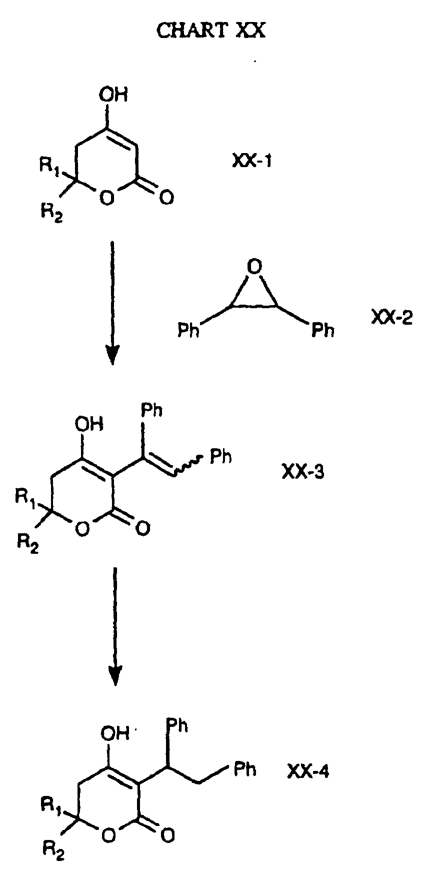

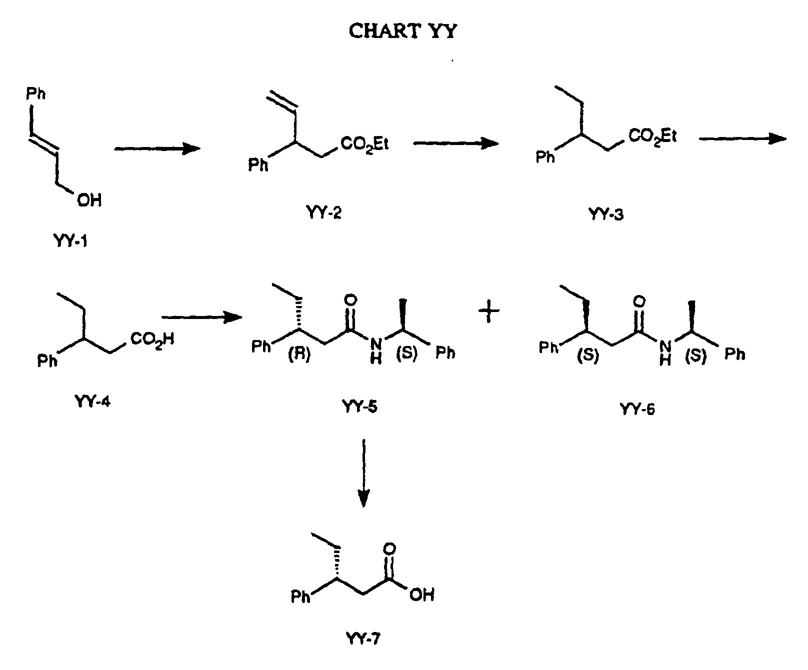

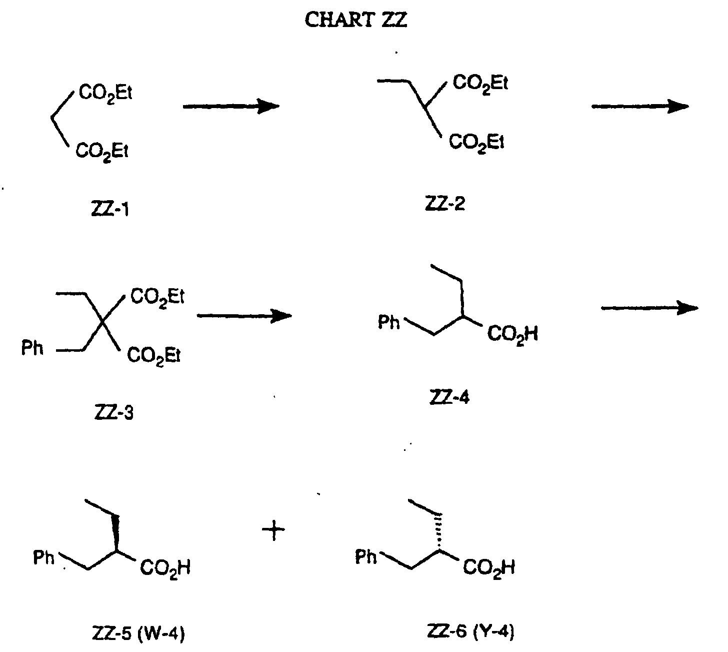

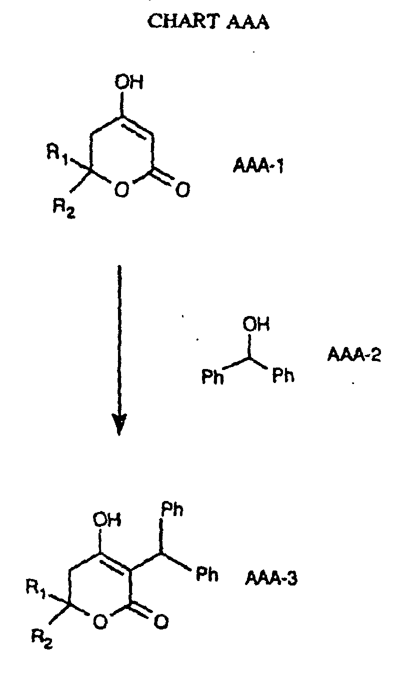

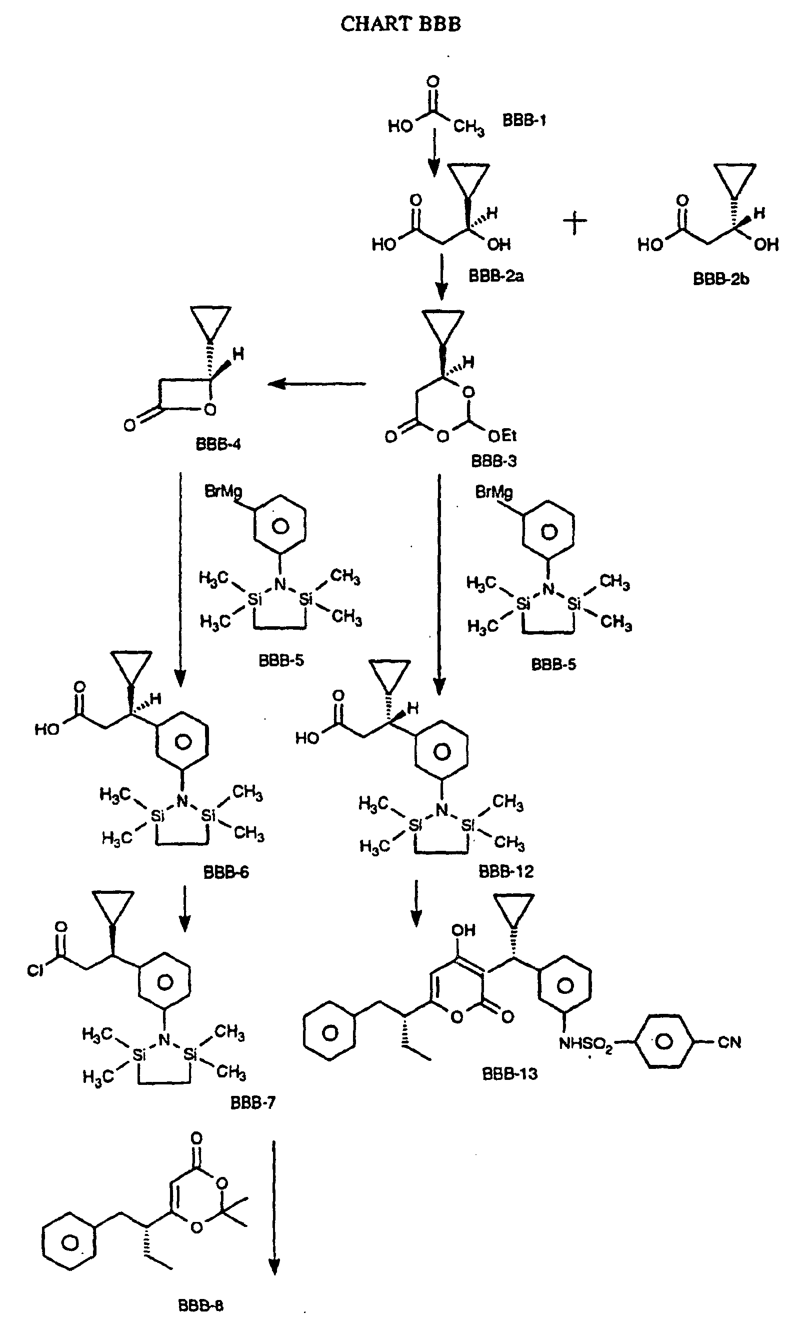

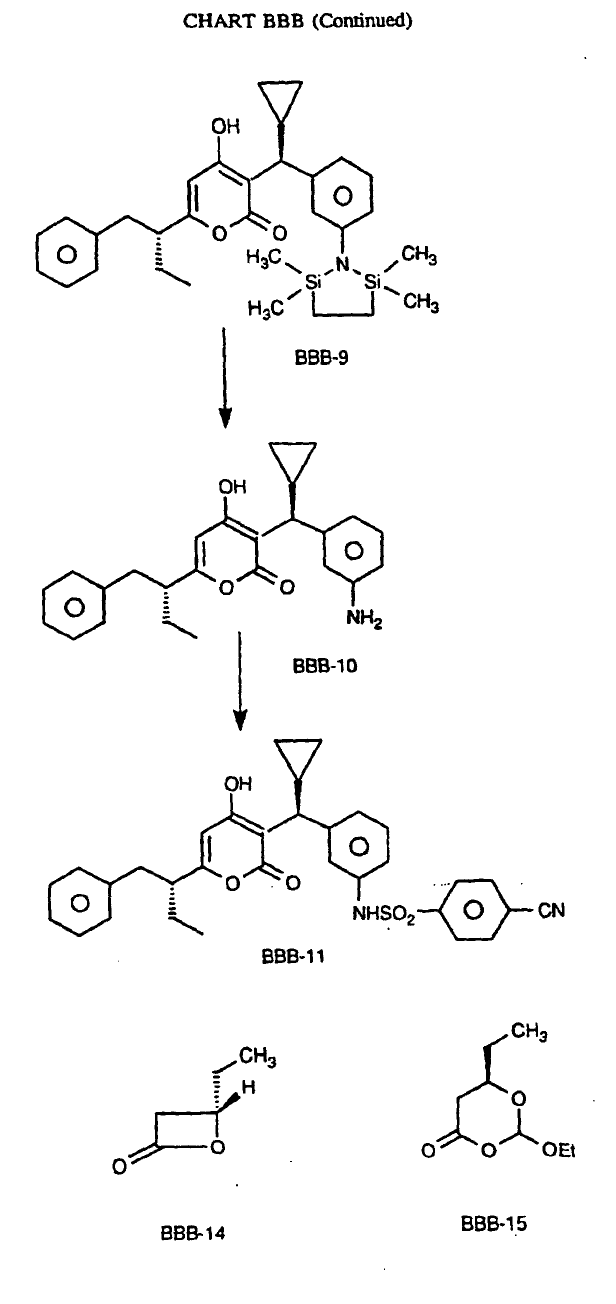

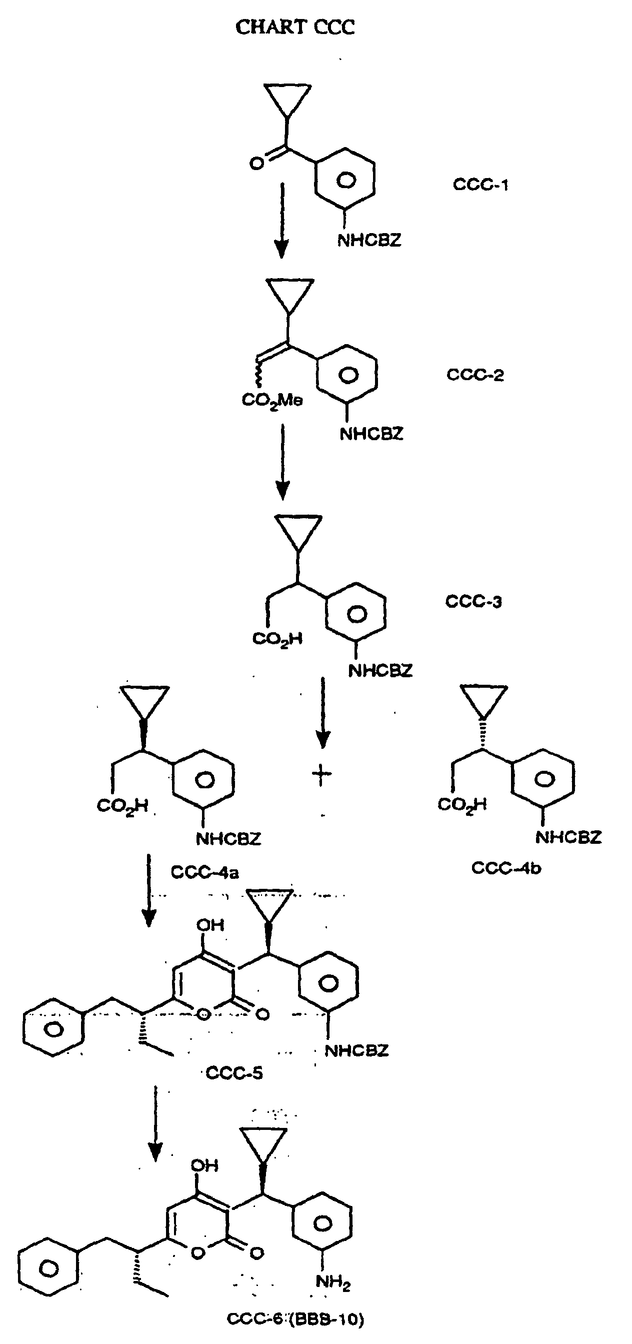

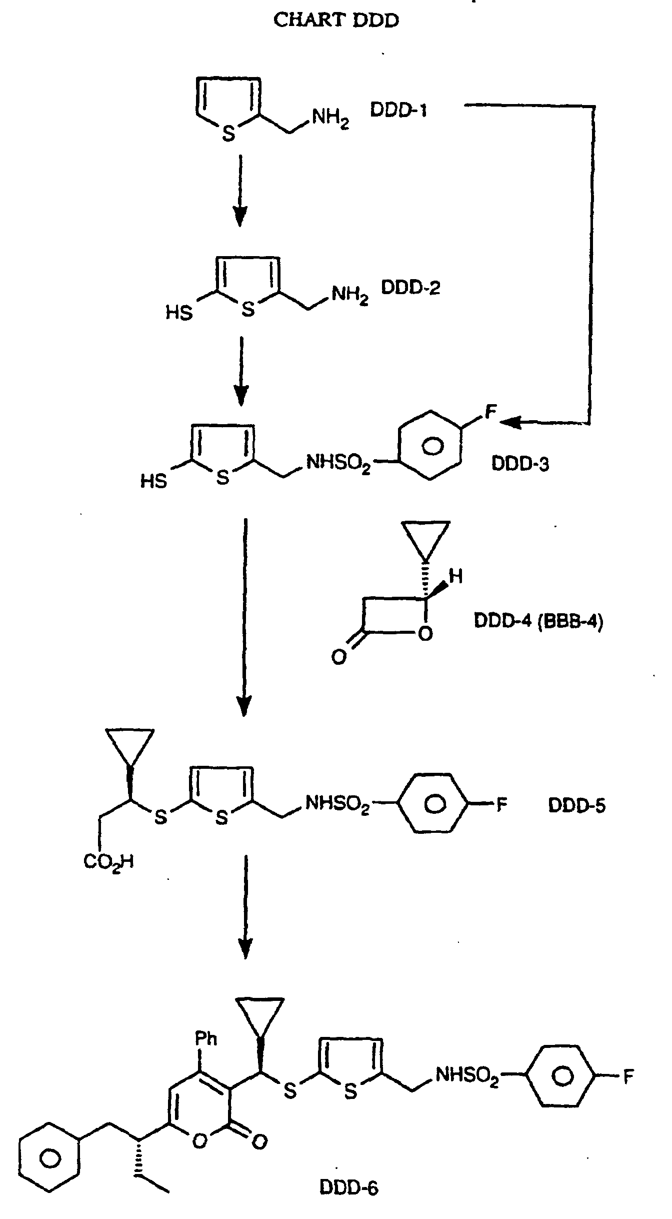

**TABLE I**

| MEAN ABSOLUTE AND RELATIVE (%) PROSTATE WEIGHTS IN MALE DOGS | | | |
|---|---|---|---|
| Group No. | Dose (mg/kg/day) | Relative Weight* | Absolute Weight (GMs) |
| 1 | 0 | 0.096 | 9.55 |
| 2 | 50 | 0.088 | 8.87 |
| 3 | 100 | 0.074 | 7.55 |
| 4 | 200 | 0.046 | 4.58 |
| 5 | 400 | 0.044 | 4.43 |
| 6 | 0 | 0.076 | 7.98 |
| 7 | 400 | 0.055 | 5.65 |

| | | | |
|---|---|---|---|
| *% body weight | | | |

## Claims

1. Use of a compound of formula I 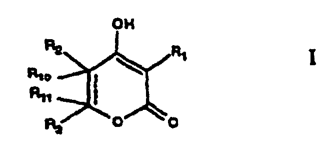 to prepare a medicament for preventing or treating benign prostatic hypertrophy or hyperplasla, prostatic cancer, alopecia, hirsutism, acne vulgaris and seborrhea in a mammal in need of such prevention or treatment;
wherein R₁ is
a) -(CH₂)ₙ-CH(R₅)-(CH₂)ₘ-R₄,
b) -CH(aryl)-CH[C(O)-O-C₁-C₆ alkyl]₂,
c) -C(C₃-C₅cycloalkyl)-(CH₂)ₙ-R₄,
d) C-(akyl)=CH-aryl,
e) -CH(R₅)-S-(CH₂)ₘ-R4, or
f) -(CH₂)ₚ-aryl;
wherein R₂ is
a) hydrogen,
b) halo,
c) C₁-C₆ alkyl-[O-(CH₂)₂]_{q}-(CH₂)ₙ-,
d) C₁-C₆ alkyl, or
e) -(CH₂)ₙ-CH(R₅)-CH₂)ₘ-R₄;
wherein R₃ is
a) C₁-C₁₀ alkyl optionally substituted by zero (0) to five (5) halo,
b) C₂-C₁₀ alkenyl,
c) R₄-(CH₂)ₘ-CH(R₆)-(H₂)ₙ₋,
d) R₄-(CH₂)ₚ-,
e) R₄-CH=CH-,
f) CH₂=CH-(CH₂)ₚ-,
g) R₄(CH₂)ₘX₁C(O)(CH₂)ₙ-,
h) R₄(CH₂)ₘC(O)X₁(CH₂)ₙ-,
i) aryl,
j) het,
k) C₃-C₇ cycloalkyl.
l) C₁-C₆ alkyl-O-C(O)-(CH₂)ₙ-,
m) C₁-C₆ alkyl-[O-(CH₂)₂]_{q}-(CH₂)ₙ-,
n) R₄-CH(R₆)-CH(R₆)-,
p) R₁₂-(CH₂)ₘ-X₂-(CH₂)ₙ-(R₇)HC-, or
q) R₁₅-(CH₂)ₘ-X₂-(CH₂)ₙ-(R₇)HC-;
wherein R₄ is
a) aryl,
b) het,
c) C₃-C₇ cycloalkyl,
d) C₂-C₁₀ alkenyl,
e) C₁-C₆alkyl-[O-(CH₂)₂]_{q}-(CH₂)ₙ-.
f) halo,
g) het-O-,
h) het-C(O)-,
i) aryl-(CH₂)ₙ-O-C(O)-, or
j) trifluoromethyl;
wherein R₅ is
a) C₁-C₁₀ alkyl,
b) C₂-C₁₀ alkenyl,
c) C₃-C₇ cycloalkyl,
d) -(CH₂)ₙ-aryl,
e) -(CH₂)ₚ-het, or
f) -(CH₂)ₙ-CH=CH-aryl;
wherein R₆ is
a) C₁-C₁₀ alkyl,
b) R₄-C₁-C₅ alkyl,
c) -(CH₂)ₙ-C₃-C₇ cycloalkyl,
d) -(CH₂)ₚ-CH-CH₂,
e) -(CH₂)ₚ-aryl,
f) -(CH₂)ₚ-het, or
g) hydroxy-;
wherein X₁ is -NR₇-;
wherein R₇ is
a) hydrogen, or
b) C₁-C₅ alkyl;
wherein aryl is
a) phenyl substitued by zero (0) to three(3) R₈,
b) naphthyl substitued by zero (0) to three(3) R₈,
c) biphenyl substituted by zero (0) to three (3) R_{8,} or
d) perhalophenyl:
wherein het is a 5- or 6-membered saturated or unsaturated ring containing from one (1) to four (4) heteroatoms selected from the group consisting of nitrogen, oxygen and sulfur, and including any bicyclic group in which any of the above heterocyclic rings is fused to a benzene ring, C₃-C₈ cycloalkyl, or another heterocycle; and if chemically feasible, the nitrogen and sulfur atoms may be in the oxidized forms; and substituted by zero (0) to three (3) R₉;
wherein R₈ and R₉ are independently
a) C₁-C₈ alkyl substituted by zero (0) to three (3) halo,
b) C₂₋C₈ alkenyl,
c) hydroxy,
d) hydroxy-C₁-C₅ alkyl,
e) -(CH₂)ₙ-O-C₁-C₅ alkyl substituted by zero (0) to three (3) hydroxy,
f) (CH₂)ₙ-O-C₂-C₇ alkenyl substituted by zero (0) to three (3) hydroxy,
g) halo,
h) amino,
i) amino-C₁-C₅ alkyl.
j) mono-or di-C₁-C₅ alkylamino,
k) -C(O)-C₁-C₅ alkyl,
l) -CHO,
m) -COOH,
n) -COOC₁-C₅alkyl,
o) -CON(R₇)₂,
p) C₃-C₇ cycloalkyl,
q) nitro,
r) -CN,
s) -SO₃H,
t) -SO₂NH₂,
n) -O[(CH₂)₂O]_{q}-CH₃,
v) -[CH₂-O]_{q}-C₁-C₃ alkyl,
w) -(CH₂)ₙ-NHC(O)-O-(CH₂)ₚ-R_{12,}
x) -(CH₂)ₙ-NHC(O)-O-(CH₂)ₚ-R_{15,}
y) -(CH₂)ₙ- R₁₂,
Z) -SO₂- R₁₂,
a1) -(CH₂)ₙ-X₂-(CH₂)ₙ-R₁₂,
b1) -(CH₂)ₙ-X₂-(CH₂)ₙ-R₁₅,
c1) -(CH₂)ₙ-X₂-CH=CH-R₁₂,
d1) -(CH₂)ₙ-X₂-CH=CH-R₁₅,
e1) -(CH₂)ₙ-X₂-C₁-C₁₀alkyl substituted by zero (0) to three (3) halo,
f1) -(CH₂)ₙ-X₂-C₂-C₅ alkenyl,
g1) -X₂-(CH₂)ₚ-CH(NH₂)(COOH),
h1) -NHCONH-SO₂- R₁₂,
i1) -X₂-(CH₂)ₚ-NH-C(O)-O-C₁-C₆ alkyl,
j1) -X₂-CH(X₃)-NH-C(O)-O-C₁-C₆ alkyl,
k1) -X₂-(CH₂)ₚ-CH[NH-C(O)-O-(CH₂)ₚ-R₁₂]-C(O)-O-CH₂)ₚ-R₁₂,
l1) -(CH₂)ₙ-X₄-N(C₁-C-₃ alkyl)₂,
m1) -(CH₂)ₙ-X₄-NHR₁₂,
n1) -NH-AA-P₁,
o1) -(CH₂)ₚ-N₃,
p1) -(CH₂)ₚ-R₁₂,
q1) -(CH₂)ₚ-R₁₅,
r1) -(CH₂)ₙ-NHC(SCH₃)=CHNO₂,
s1) -(CH₂)ₙNHC(NHR₇)=CHNO₂,
t1) -(CH₂)ₙ-NHC(SCH₃)=NCN, or
u1) -(CH₂)ₙ-NHC(NHR₇)=NCN;
wherein X₂ is
a) -NH-C(O)-,
b) -NH-SO₂-,
c) NH-C(O)-NH-, or
d) -SO₂-NH-;
wherein X₃ is
a) C₁-C₆ alkyl, or
b) -(CH₂)ₚ-R₁₅;
wherein X₄ is
a) -NH-C(O)-, or
b) -NH-SO₂-;
wherein R₁₀ is hydrogen;
wherein R₁₁ is
a) hydrogen,
b) C₁-C₆ alkyl,
c) -(CH₂)ₙ-aryl,
d) -(CH₂)ₙ-C₃-C₇ cycloalkyl, or
e) -(CH₂)ₙ-het;
or wherein R₁₀ and R₁₁ taken together form a double bond;
or wherein R₃ and R₁₁ taken together form
a) C₃-C₈ cycloalkyl substituted by zero (0) to three (3) hydroxy, =N-OH, =O (oxo), or protected form thereof, or substituted at the α-position by R₁₄; or
b) a 5- or 6-membered saturated ring containing one (1) or two (2) oxygen atoms;
wherein R₁₂ is
a) phenyl substituted by zero (0) to three (3) R₁₃, or
b) naphthyl substituted by zero (0) to three (3) R₁₃;
wherein R₁₃ is
a) C₁-C₁₀ alkyl substituted by zero (0) to three (3) halo,
b) hydroxy,
c) hydroxy-C₁-C₅ alkyl,
d) -(CH₂)ₙ-O-C₁-C₅ alkyl substituted by zero (0) to three (3) hydroxy or halo,
e) -(CH₂)ₙ-O-C₂-C₇ alkenyl substituted by zero (0) to three (3) hydroxy or halo,
f) halo,
g) amino,
h) amino C₁-C₅ alkyl,
i) mono-or di-C₁-C₅ alkylamino,
j) -C(O)-C₁-C₅ alkyl,
k) -CHO,
l) -COOH,
m) -CON(R₇)₂,
n) -NHCOC₁-C₃ alkyl,
o) -NHOH,
p) nitro,
q) -CN,
r) -(CH₂)ₙ-phenyl,
s) -COOC₁-C₅alkyl, or
t) -SO₂-phenyl substituted by zero (0) to three (3) C₁-C₅ alkyl,
u) -(CH₂)ₙ-X₄-phenyl, or
v) -(CH₂)ₙ-N=N-phenyl substituted by zero (0) or one (1) -N(C₁-C₃ alkyl)₂;
wherein R₁₄ is
a) -(CH₂)ₙ-aryl,
b) -C₁-C₆alkyl, or
c) -(CH₂)ₙ-C₄-C₇cycloalkyl;
wherein R₁₅ is a 5- or 6-membered saturated or unsaturated ring containing from one (1) to four (4) heteroatoms selected from the group consisting of nitrogen, oxygen and sulfur; and including any bicyclic group in which any of the above heterocyclic rings is fused to a benzene ring, C₃-C₈ cycloalkyl, or another heterocycle; and substituted by zero (0) to three (3) R₁₃:
wherein AA is an amino acid residue;
wherein P₁ is hydrogen or a nitrogen protecting group;
wherein m and n are independently zero (0) to five (5) inclusive;
wherein p is one (1) to five (5) inclusive;
wherein q is one (1) to five (5) inclusive; and
pharmaceutically acceptable salts thereof.

2. The use of claim 1, wherein the size of the prostate in a male mammal is reduced or maintained.

3. The use of claim 2
wherein R₁ is -(CH₂)ₙ-CH(R₅)-(CH₂)ₘ-R₄;
wherein R₂ is hydrogen;
wherein R₃ is
a) R₄-(CH₂)ₘ-CH(R₆)-(CH₂)ₙ-, or
b) R₄-(CH₂)ₚ-;
wherein R₄ is
a) phenyl, or
b) tetrahydropyranyl;
wherein R₅ is
a) propyl, or
b) cyclopropyl;
wherein R₆ is ethyl;
wherein m is zero (0) or one (1);
wherein n is zero (0);
wherein p is two (2);
wherein R₁₀ and R₁₁ taken together form a double bond.

4. The use of claim 1 or 2, wherein the compound is selected from the group consisting of:
3-(.alpha.-ethylbenzyl)-4-hydroxy-6-phenyl-2H-Pyran-one;
6-benzyl-3-(.alpha.-ethylbenzyl)-4-hydroxy-2H-Pyran-2-one;
3-(.alpha.-ethylbenzyl)-6-phenethyl-4-hydroxy-2H-Pyran-2-one;
4-hydroxy-6-phenethyl-3-(.alpha.-propyl-p-bromobenzyl)-2H-Pyran-2-one;
6-(p-bromophenethyl)-3-(.alpha.-ethylbenzyl)-4-hydroxy-2H-Pyran-2-one;
3-(alpha-ethylbenzyl)-6-(o-fluorophenethyl)-4-hydroxy-2H-Pyran-2-one:
3-(.alpha.-ethylbenzyl)-4-hydroxy-6-(3-phenylpropyl-2H-Pyran-2-one;
3-(.alpha.-ethylbenzyl)-4-hydroxy-6-propyl-2H-Pyran-2-one;
3-(.alpha.-ethylbenzyl)-4-hydroxy-6-(3-butenyl)-2H-Pyran-2-one;
3-(.alpha.-ethylbenzyl)-4-hydroxy-6-[[(phenylamino)carbonyl]methyl]-2H-Pyran-2-one;
4-hydroxy-6-phenethyl-3-(.alpha.-vinylbenzyl)-2H-Pyran-2-one;
3-(.alpha.-ethylbenzyl)-6-(.alpha.-ethylphenethyl)-4-hydroxy-2H-Pyran-2-one;
3-(.alpha.-ethylbenzyl)-1-ethylpropyl-4-hydroxy-2H-Pyran-2-one;
3-(.alpha.-ethylbenzyl)-4-hydroxy-6-(p-methoxystyryl)-2H-Pyran-2-one;
3-(.alpha.-ethylbenzyl)4-hydroxy-6-(2-naphth-2-ylethyl)-2H-Pyran-2-one;
3-(.alpha-ethylbenzyl)-4-hydroxy-6-(1-ethyl-2 naphth-2-ylethyl)-2H-Pyran-2-one;
3-(.alpha.-ethylbenzyl)-4-hydroxy-6-(2-naphth-1-ylethyl)-2H-Pyran-2-one;
3-(.alpba.-elhylbenzyl)-4-hydroxy-6-(1-ethyl-2-naphth-1-ylethyl)-2H-Pyran-2-one;
3-(.alpha.-cyclopropylbenzyl)-4-hydroxy-6-(3-phenylpropyl)-2H-Pyran-2-one;
3-(.alpha.-cyclopropylbenzyl)-6-(1-ethyl propyl)-4-hydroxy-2H-Pyran-2-one;
3-(.alpha.-cyclopropylbenzyl)-6-(.alpha.-benzylphenethyl)-4-hydroxy-2H-Pyran-2-one;
3-(.alpha.-cyclopropylbenzyl)-4-hydroxy-6-phenethyl-2H-Pyran-2-one;
3-(.alpha.-cyclopropylbenzyl)-6-(1-propylbutyl)-4-hydroxy-2H-Pyran-2-one;
3-(.alpha.-cyclopropylbenzyl)-4-hydroxy-6-methyl-2H-Pyran-2-one;
4-Hydroxy-6-methyl-3-(3-phenyl-prop-2-enyl)-2H-pyran-2-one;
Dimethyl 3-[(4-hydroxy-2-oxo-6-phenyl-2H-1-pyran-3-yl)(4-nitrophenyl)-1,3-propandioate;
Dimethyl 3-[(4-hydroxy-2-oxo-6-phenyl-2H-1-pyran-3-yl)(3-nitrophenyl)-1,3-propandioate;
6-Ethyl-3-(α-ethylbenzyl)-6-phenyl-tetrahydropyran-2,4-dione;
5,6-Dihydro-4-hydroxy-6-cyclohexyl-6-phenyl-3-[1-(3-hydroxyphenyl)propyl]-2H-pyran-2-one;
4-hydroxy-1-oxa-3-(1-phenylpropyl)spiro-[5,5]-undeo-3-en-2-one, sodium salt;
6,6-Diethyl-3-(3-phenylpropyl)-tetrahydropyran-2,4-dione;
Dihydro-6-methyl-6-phenyl-3-(1-phenyl-2-propenyl)-2H-Pyran-2,4(3H)-dione;
Dihydro-3-(1-(3-hydroxyphenyl)propyl]-6-phenyl-6-propyl-2H-pyran-2,4(3H)-dione;
5,6-Dihydro-4-hydroxy-6-phenyl-6-propyl-3-(1-phenylpropyl)-2H-pyran-2-one;
5,6-Dihydro-4-hydroxy-6-phenyl-6-propyl-3-[1-(3-hydroxyphenyl)-propyl]-2H-pyran-2-one;
12-Hydroxy-11-(1-phenyl-allyl)-1,4,9-trioxa-dispiro[4.2.5.2]pentadec-11-en-10-one;
12-Hydroxy-11-(1-phenyl-propyl)-1,4,9-trioxa-dispiro[4.2.5.2] pentadec-11-en-10-one;
4-Hydroxy-3-(1-phenyl-propyl)-1-oxa-spiro[5-5]undec-3-ene-2,9-dione;
6,6-Dibenzyl-4-hydroxy-3-(1-phenyl-propyl)-5,6-dihydro-pyran-2-one;
4-Hydroxy-3-(1-phenyl-allyl)-1,9-dioxa-spiro[5,5]undec-3-en-2-one;
4,9-Dihydroxy-3-(1-phenyl-propyl)-1-oxa-spiro[5,5]undec-3-en-2-one;
N-(3-Cyclopropyl-[6-(1-ethyl-phenethyl)-4-hydroxy-2-oxo-5,6-dihydro-2H-pyran-3-yl]-methyl)-phenyl)-3-(*tert*-butyloxycarbonylamino)-propionamide;
N-(3-{Cyclopropyl-[6-(2-cyclopropyl-1-cyclopropylmethyl-ethyl)-4-hydroxy-2-oxo-5,6-dihydro-2H-pyran-3-yl]-methyl}-phenyl)-3-indol-1-yl-propionamide;
3-(Cyclopropyl-phenyl-methyl)4-hydroxy-6-(1-(tetrahydro-furan-3-ylmethyl)-propyl)-pyran-2-one;
6-(1-(5-Chloro-thiophen-2-ylmethyl)-propyl)-3-(cyclopropyl-phenyl-methyl)-4-hydroxy-pyran-2-one;
3-(Cyclopropyl-phenyl-methyl)-6-(1-(3,5-dimethyl-isoxazol-4-ylmethyl)-propyl)-4-hydroxy-pyran-2-one:
3-(Cyclopropyl-phenyl-methyl)-4-hydroxy-6-(1-(tetrahydro-furan-2-ylmethyl)-propyl)-pyran-2-one:
3-(Cyclopropyl-phenyl-methyl)-4-hydroxy-6-(1-thiophen-2-ylmethyl-propyl)-pyran-2-one;
3-(Cyclopropyl-phenyl-methyl)-4-hydroxy-6-(1-tetrahydro-pyran-4-ylmethyl)-propyl)-pyran-2-one;
3-(Cyclopropyl-phenyl-methyl)-6-(1-furan-2-ylmethyl-propyl)4-hydroxy-pyran-2-one;
3-(Cyclopropyl-phenyl-methyl)-6-(1-(1,3)dioxolan-2-ylmethyl-propyl)-4-hydroxy-pyran-2-one;
3-(Cyclopropyl-phenyl-methyl)-4-hydroxy-6-(1-(tetrahydro-pyran-3-ylmethyl)propyl)-pyran-2-one;
3-(Cyclopropyl-phenyl-methyl)-4-hydroxy-6-(1-(tetrahydro-pyran-2-ylmethyl)-propyl)-pyran-2-one;
3-(Cyclopropyl-phenyl-methyl)-4-hydroxy-6-(1-pyridin-2-ylmethyl-propyl)-pyran-2-one;
6-(4-Chloro-1-ethyl-butyl)-3-(cyclopropyl-phenyl-methyl)-4-hydroxy-pyran-2-one;
6-(3-Chloro-1-ethyl-propyl)-3-(cyclopropyl-phenyl-methyl)-4-hydroxy-pyran-2-one;
3-(Cyclopropyl-phenyl-methyl)-6-[ethyl-3-(tetrahydro-pyran-2-yloxy)-propyl]-4-hydroxy-pyran-2-one;
3-(Cyclopropyl-phenyl-methyl)-4-hydroxy-6-(1-pyridin-3-ylmethyl-propyl)-pyran-2-one;
3-(Cyclopropyl-phenyl-methyl)-6-(1-ethyl-3-thiophen-3-yl-propyl)-4-hydroxy-pyran-2-one;
3-(Cyclopropyl-phenyl-methyl)-4-hydroxy-6-[1-(tetrahydro-pyran-3-ylmethyl)-butyl]-pyran-2-one;
5-Bromo-6-(2-cyclopropyl-cyclopropylmethyl-ethyl)-4-hydroxy-3-(1-phenyl-propyl)-pyran-2-one;
3-(1-Benzyl-2-phenyl-ethyl)-6-(2-cyclopropyl-1-cyclopropylmethyl-ethyl)-4-hydroxy-pyran-2-one;
6-(2-Cyclopropylmethyl-ethyl)-3-(cyclopropyl-phenyl-methyl)-4-hydroxy-pyran-2-one;
6-(1-Allyl-but-3-enyl)-3-(cyclopropyl-phenyl-methyl)-4-hydroxy-pyran-2-one; 3-(Cyclopropyl-phenyl-methyl)-6-(1-ethyl-3-(2-methoxy-ethoxy)-propyl)-4-hydroxy-pyran-2-one;
6-(1-Benzyl-3-(2-methoxy-ethoxy)-propyl)-3-(cyclopropyl-phenyl-methyl)-4-hydroxy-pyran-2-one;
3-(α-Cyclopropyl-*meta*-(benzyloxycarbonylamino)benzyl)-6-(α-ethyl-phenethyl)-4-hydroxy-2H-pyran-2-one;
3-(α-Cyclopropyl-*meta*-(*tert*-butyloxycarbonylamino)benzyl)-6-(α-cyclopropylmethyl-cyclopropylethyl)-4-hydroxy-2H-pyran-2-one;
4-Hydroxy-3-(1-phenyl-propyl)-6-(1-propyl-butyl)-pyran-2-one;
4-Hydroxy-3-(1-phenyl-alkyl)-6-(1-propyl-butyl)-pyran-2-one;
3-(5-(Cyclopropyl-phenyl-methyl)-4-hydroxy-6-oxo-6H-pyran-2-yl)-propionic acid tert-butyl ester;
3-(Cyclopropyl-phenyl-methyl)-6-(2-(3,5-dimethyl-isoxazol-4-yl)-ethyl)-4-hydroxy-pyran-2-one;
6-(2-(5-tert-Butyl-(1,2,4)oxadiazol-3-yl)-ethyl)-3-(cyclopropyl-phenyl-methyl)4-hydroxy-pyran-2-one;
3-(Cyclopropyl-phenyl-methyl)-4-hydroxy-6-(2-phenyl-1-pyridin-2-ylmethyl-ethyl)-pyran-2-one;
3-(Cyclopropyl-phenyl-methyl)-6-(2-(1,3)dioxolan-2-yl-ethyl)-4-hydroxy-pyran-2-one;
3-(Cyclopropyl-phenyl-methyl)-4-hydroxy-6-(4-morpholin-4-yl-butyl)-pyran-2-one;
3-(Cyclopropyl-phenyl-methyl)-4-hydroxy-6-(2-pyridin-2-yl-ethyl)-pyran-2-one;
3-(Cyclopropyl-phenyl-methyl)-4-hydroxy-6-(2-(2-methyl-thiazol-4-yl)-ethyl)-pyran-2-one;
3-(Cyclopropyl-phenyl-methyl)-4-hydroxy-6-(2-quinolin-2-yl-ethyl)-pyran-2-one;
6-(3-Chloro-propyl)-3-(cyclopropyl-phenyl-methyl)-4-hydroxy-pyran-2-one;
6-(1-(2-(4-Chloro-phenyl)-thiazol-4-ylmethyl)-propyl)-3-(cyclopropyl-phenyl-methyl)-4-hydroxy-pyran-2-one;
3-(Cyclopropyl-phenyl-methyl)-6-(3-(1,3)dioxan-2-yl-1-ethyl-propyl)-4-hydroxy-pyran-2-one;
3-(Cyclopropyl-phenyl-methyl)-4-hydroxy-6-(1-pyridin-4-ylmethyl-propyl)-pyran-2-one;
3-(Cyclopropyl-phenyl-methyl)-6-(1-ethyl-3-morpholin-4-yl-3-oxo-propyl)-4-hydroxy-pyran-2-one;
3-(Cyclopropyl-phenyl-methyl)-6-(1-ethyl-4-morpholin-4-yl-butyl)4-hydroxy-pyran-2-one;
3-(Cyclopropyl-phenyl-methyl)-6-[1-(2,3-dihydro-benzo[1,4]dioxin-2-ylmethyl)-propyl]-4-hydroxy-pyran-2-one;
3-(Cyclopropyl-phenyl-methyl)-4-hydroxy-6-isobutyl-pyran-2-one;
Cyclopropyl-phenyl-methyl)-6-[1-(5,6-dihydro-2H-pyran-3-ethyl)-propyl]-hydroxy-pyran-2-one;
3-(Cyclopropyl-phenyl-methyl)-4-hydroxy-5-(2-(2-methoxy-ethoxy)-ethyl)-6-propyl-pyran-2-one;
3-(Cyclopropyl-phenyl-methyl)-4-hydroxy-6-(1-isobutyl-3-methyl-butyl)-pyran-2-one;
3-Dicyclopropylmethyl-4-hydroxy-6-phenethyl-pyran-2-one;
6-(1-Cyclopropyl-ethyl)-3-dicyclopropylmethyl-4-hydroxy-pyran-2-one;
6-(1-Cyclopropyl-1-cyclopropylmethyl-ethyl)-3-dicyclopropylmethyl-4-hydroxy-pyran-2-one;
6-(1-Cyclohexylmethyl-propyl)-3-(cyclopropyl-phenyl-methyl)-4-hydroxy-pyran-2-one;
6-(1-Benzyl-propyl)-3-(cyclopropyl-phenyl-methyl)-4-hydroxy-pyran-one;
6-(2-Cyclopropyl-1-cyclopropylmethyl-ethyl)-4-hydroxy-3(1-phenyl-propyl)-pyran-2-one;
6-(1-Benzyl-2-cyclopropyl-ethyl)-3-(cyclopropyl-phenyl-methyl)-4-hydroxy-pyran-2-one;
6-(2-Cyclopropyl-ethyl)-3-(cyclopropyl-phenyl-methyl)-4-hydroxy-pyran-2-one;
6-(1-Cyclopropylmethyl-propyl)-3-(cyclopropyl-phenyl-methyl)-4-hydroxy-pyran-2-one;
3-(Cyclopropyl-phenyl-methyl)-4-hydroxy-6-(4-phenyl-butyl)-pyran-2-one;
3-(Cyclohexyl-cyclopropyl-methyl)-6-(2-cyclopropyl-1-cyclopropylmethyl-ethyl)-4-hydroxy-pyran-2-one;
3-(Cyclopropyl-phenyl-methyl)-6-(1-ethyl-4-phenyl-butyl)-4-hydroxy-pyran-2-one;
6-(3-Cyclohexyl-propyl)-3-(cyclopropyl-phenyl-methyl)-4-hydroxy-pyran-2-one;
6-(3-Cyclohexyl-1-ethyl-propyl)-3-(cyclopropyl-phenyl-methyl-4-hydroxy-pyran-2-one;
6-(2-Cyclopropyl-ethyl)-4-hydroxy-3-(1-phenyl-propyl)-pyran-2-one;
6-But-3-enyl-3-(cyclopropyl-phenyl-methyl)-4-hydroxy-pyran-2-one;
3-(Cyclopropyl-phenyl-methyl)-6-(1-ethyl-3-phenyl-propyl)-4-hydroxy-pyran-2-one;
5-Bromo-6-(2-cyclopropyl-ethyl)-4-hydroxy-3-(1-Phenyl-propyl)-pyran-2-one;
6-(1-Benzyl-2-phenyl-ethyl)-4-hydroxy-3-(1-phenyl-propyl)-pyran-2-one;
3-(Cyclopropyl-phenyl-methyl)-4-hydroxy-6-(3-(2-methoxy-ethoxy)-propyl)-pyran-2-one;
3-(Cyclopropyl-phenyl-methyl)-4-hydroxy-5-(2-(2-methoxy-ethoxy)-ethyl)-6-(3-(2-methoxy-ethoxy)-pyran-2-one;
3-(Cyclopropyl-phenyl-methyl)-4-hydroxy-6-propyl-pyran-2-one;
5-Bromo-4-hydroxy-6-phenylethyl-3(1-phenyl-propyl)-pyran-2-one;
3-(Cyclopropyl-phenyl-methyl)-4-hydroxy-6-(3-(2-methoxy-ethoxy)-ethoxy)-propyl)-pyran-2-one;
5-Bromo-4-hydroxy-3-(1-phenyl-propyl)-6-propyl-pyran-2-one;
3-(Cycloprpyl-phenyl-methyl)-6-(1-ethyl-propyl)-4-hydroxy-5-(2-(2-methoxy-ethoxy)ethyl)-pyran-2-one;
6-(1-Benzyl-propyl)-5-bromo-4-hydroxy-3-(1-phenyl-propyl)-pyran-2-one;
6-(2-Cyclopropyl-1-cyclopropylmethyl-ethyl)-3-(cyclopropyl-phenyl-methyl)-4-hydroxy-5-(2-(2-methoxy-ethoxy)-ethoxy)-ethyl)-pyran-2-one;
3-(Cyclopropyl-phenyl-methyl)-6-(2-furan-2-yl-2-hydroxy-ethyl)-4-hydroxy-pyran-2-one;
3-(Cyclopropyl-phenyl-methyl)-4-hydroxy-6-(4,4,4-trifluoro-butyl)-pyran-2-one;
6-[2-(1-cyclohexyl-1H-tetrazol-5-yl)-ethyl]-3-(cyclopropyl-phenyl-methyl)-4-hydroxy-2-one;
4-Hydroxy-3-(1-phenyl-propyl)-1-oxa-spiro[5.5]undec-3-ene-2,9-dione monooxime;
5,6-Dihydro-4-hydroxy-6-phenyl-6-(phenylmethyl)-3-(1-phenyl-2-propenyl)-2H-pyran-2-one;
5,6-Dihydro-4-hydroxy-6-phenyl-6-(phenylmethyl)-3-(1-phenylpropyl)-2H-pyran-2-one;
3-(1,3-Diphenyl-2-propenyl)-5,6-dihydro-4-hydroxy-6-(2-phenylethyl)-6-propyl-, (E)-2H-pyran-2-one;
3-(1,3-Diphenyl-2-propyl)-5,6-dihydro-4-hydroxy-6-(2-phenylethyl)-6-propyl-, (E)-2H-pyran-2-one;
3-(1,3-Diphenyl-2-propenyl)-5,6-dihydro-4-hydroxy-6-phenyl-6-(phenylmethyl)-2H-pyran-2-one;
3-(1,2-Diphenylethenyl)-5,6-dihydro-4-hydroxy-6-(2-phenylethyl)-6-propyl-, (E)-2H-pyran-2-one;
5,6-Dihydro-4-hydroxy-6-(2-phenylethyl)-3-(1-phenyl-2-propenyl)-6-propyl-2H-pyran-2-one;
5,6-Dihydro-4-hydroxy-6-(2-phenylethyl)-3-(1-phenylpropyl)-6-propyl-2H-pyran-2-one;
3-(1,3-Diphenyl-2-propenyl)-5,6-dihydro-4-hydroxy-6-(phenylmethyl)-6-propyl-2H-pyran-2-one;
3-(1,3-Diphenylpropyl)-5,6-dihydro-4-hydroxy-6-(phenylmethyl)-6-propyl-2H-pyran-2-one;
4-Hydroxy-3-(1-phenylallyl)-1-oxa-[5.6]-dodec-3-en-2-one;
4-Hydroxy-3-(1-phenylallyl)-1-oxa-spiro[5.4]-dec-3-en-2-one;
7-Benzyl-4-hydroxy-3-(1-phenylallyl)-1-oxa-spiro[5.5]undec-3-en-2-one;
4-Hydroxy-3-(1-phenylpropyl)-1-oxa-spiro[5.4]-dec-3-en-2-one;
4-Hydroxy-3-(1-phenyl-2-propenyl)-1-oxaspiro[5.7]tridec-3-en-2-one;
4-Hydroxy-3-(1-phenylpropyl)-1-oxaspiro[5.7]tridec-3-en-2-one;
3-(α-cyclopropylbenzyl)-4-hydroxy-6-(α-ethyl-β-hydroxyphenethyl)-2H-pyran-2-one;
3-(α-cyclopropylbenzyl)-4-hydroxy-6-(β-hydroxy-p-methylphenethyl)-2H-pyran-2-one;
3-(α-cyclopropylbenzyl)-4-hydroxy-6-(β-hydroxy-p-fluorophenethyl)-2H-pyran-2-one;
3-(α-cyclopropylbenzyl)-4-hydroxy-6-(β-hydroxy-p-chlorophenethyl)-2H-pyran-2-one;
3-(α-cyclopropylbenzyl)-4-hydroxy-6-(β-hydroxy-m-chlorophenethyl)-2H-pyran-2-one;
3-(α-cyclopropylbenzyl)-4-hydroxy-6-(β-hydroxy-o-chlorophenethyl)-2H-pyran-2-one;
3-(α-cyclopropylbenzyl)-4-hydroxy-6-(2-(furan-3-yl)-2-hydroxyethyl)-2H-pyran-2-one;
3-(α-cyclopropylbenzyl)-4-hydroxy 6-(2-(thiophen-3-yl)-2-hydroxyethyl)-2H-pyran-2-one;
3-(α-cyclopropylbenzyl)-4-hydroxy-6-(α-ethyl-p-fluorphenethyl)-2H-pyran-2-one;
3-(α-cyclopropylbenyl)-4-hydroxy-6-(α-ethyl-p-chlorophenyl)-2H-pyran-2-one;
3-(α-cyclopropylbenzyl)-4-hydroxy-6-(α-ethyl-m-chlorophenethyl)-2H-pyran-2-one;
3-(α-cyclopropylbenzyl)-4-hydroxy-6-(α-ethyl-o-chlorophenethyl)-2H-Pyran-2-one;
3-(α-cyclopropylbenzyl)-4-hydroxy-6-(α-ethyl-p-bromophenethyl)-2H-pyran-2-one;
3(α-cyclopropylbenzyl)-4-hydroxy-6-(α-ethyl-m-bromophenethyl)-2H-pyran-2-one;
3-(α-cyclopropylbenzyl)-4-hydroxy-6-(α-ethyl-p-triflurormethylphenethyl)-2H-pyran-2-one;
3-(α-cyclopropylbenzyl)-4-hydroxy-6-(α-ethyl-m-triflurormethylphenethyl)-2H-pyran-2-one;
3-(α-cyclopropylbenzyl)-4-hydroxy-6-(α-ethyl-o-trifluormethylphenethyl)-2H-pyran-2-one;
3-(α-cyclopropylbenzyl)-4-hydroxy-6-(α-ethyl-p-methoxyphenethyl)-2H-pyran-2-one:
3-(α-cyclopropylbenzyl)-4-hydroxy-6-(α-ethyl-m-methoxyphenethyl)-2H-pyran-2-one;
3-(α-cyclopropylbenzyl)-4-hydroxy-6-(p-fluorophenethyl)-2H-pyran-2-one;
3-(α-cyclopropylbenzyl)-4-hydroxy-6-(p-chlorophenethyl)-2H-pyran-2-one;
3-(α-cyclopropylbenzyl)-4-hydroxy-6-(p-bromophenethyl)-2H-pyran-2-one;
3-(Cyclopropylphenylmethyl)-6-[1-ethyl-3-(4-morpholinyl)propyl]-4-hydroxy-2H-pyran-2-one;
3-(Cyclopropylphenylmethyl)-β-ethyl-4-hydroxy-2-oxo-, phenylmethyl ester 2H-pyran-6-propanoic acid;
3-(Cyclopropylphenylmethyl)-4-hydroxy-6-[2-methyl-1-(phenylmethyl)propyl]-2H-pyran-2-one;
3-(Cyclopropylphenylmethyl)-4-hydroxy-6-[2-methyl-1-[(tetrahydro-2H-pyran-3-yl)methyl]propyl]-2H-pyran-2-one;
4-Hydroxy-3-(1-phenylpropyl)-6-[1-[(tetrahydro-2H-pyran-3-yl)methyl]propyl]-2H-pyran-2-one;
3-(Cyclopropylphenylmethyl)-6-(1-ethyl-4,4,4-trifluorobutyl)-4-hydroxy-2H-pyran-2-one;
3-[2-[3-(Cyclopropylphenylmethyl)-4-hydroxy-2-oxo-2H-pyran-6-yl]butyl]-1-[(4-methylphenyl)sulfonyl]-piperidine;
2-[2-[3-(Cyclopropylphenylmethyl)-4-hydroxy-2-oxo-2H-pyran-6-yl]butyl]-1-[(4-methylphenyl)sulfonyl]-pyrrolidine;
3-(Cyclopropylphenylmethyl)-4-hydroxy-6-(3,3,3-trifluoropropyl)-2H-pyran-2-one;
2-[2-[3-(Cyclopropylphenylmethyl)-4-hydroxy-2-oxo-2H-pyran-6-yl]butyl]-1-[(4-methylphenyl)sulfonyl]-piperidine;
4-[2-[3-(Cyclopropylphenylmethyl)-4-hydroxy-2-oxo-2H-pyran-6-yl]butyl]-1-[(4-methylphenyl)sulfonyl]-piperidine;
4-[2-[3-(Cyclopropylphenylmethyl)-4-hydroxy-2-oxo-2H-pyran-6-yl]butyl]-1-(phenylmethyl)-2-pyrrolidinone;
6-(Cyclopentylmethyl)-3-(cyclopropylphenylmethyl)-4-hydroxy-2H-pyran-2-one;
3-(Cyclopropylphenylmethyl)4-hydroxy-6-[(tetrahydro-2H-pyran-4-yl)methyl]-2H-pyran-2-one;
3-(Cyclopropylphenylmethyl)-6-(3-fluoropropyl)-4-hydroxy-2H-pyran-2-one;
4-Hydroxy-3-(1-phenylcyclobutyl)-6-[1-(phenylmethyl)propyl]-2H-pyran-2-one;
3-(α-Ethylbenzyl)-6-(α-ethylbenzyl)-4-hydroxy-2H-pyran-2-one;
3-(α-Cyclopropyl-*meta*-(phenylsulfonylamino)benzyl)-6-(α-ethylphenethyl)-4-hydroxy-2H-pyran-2-one;
3-(α-Cyclopropyl-*meta*-(propylsulfonylamino)benzyl)-6-(α-ethylphenethyl)-4-hydroxy-2H-pyran-2-one;
3-(α-Cyclopropyl-*meta*-((E)-2-phenylethenylsulfonylamino)benzyl)-6-(α-ethylphenethyl)-4-hydroxy-2H-pyran-2-one;
3-(α-Cyclopropyl-*meta*-(4-bromophenylsulfonylamino)benzyl)-6-(α-ethylphenethyl)-4-hydroxy-2H-pyran-2-one;
3-(α-Cyclopropyl-*meta*-(2,5-dichlorophenylsulfonylamino)benzyl)-6-(α-ethylphenethyl)-4-hydroxy-2H-pyran-2-one;
3-(α-Cyclopropyl-*meta*-(4-*tert*-butylphenylsulfonylamino)benzyl)-6-(α-ethylphenethyl)-4-hydroxy-2H-pyran-2-one;
3-(α-Cyclopropyl-*meta*-(4-cyanophenylsulfonylamino)benzyl)-6-(α-ethylphenethyl)-4-hydroxy-2H-pyran-2-one;
3-(α-Cyclopropyl-*meta*-(4-methoxyphenylsulfonylamino)benzyl)-6-(α-ethylphenethyl)-4-hydroxy-2H-pyran-2-one;
6-Butyl-5,6-dihydro-3-(1,3-diphenyl-2-propenyl)-4-hydroxy-6-phenylmethyl-2H-pyran-2-one;
6-Butyl-5,6-dihydro-3-(1,3-diphenylpropyl)-4-hydroxy-6-phenylmethyl-2H-pyran-2-one;
6-Butyl-5,6-dihydro-4-hydroxy-6-phenylmethyl-3-(1-phenyl-2-propenyl)-2H-pyran-2-one;
6-Butyl-5,6-dihydro-4-hydroxy-6-phenylmethyl-3-(1-phenylpropyl)-2H-pyran-2-one;
3-(α-Cyclopropyl-[5-(methoxymethoxy-methyl)-thiophen-2-yl]-methyl)-5,6-dihydro-4-hydroxy-6-(2-methylpropyl)-6-(2-phenylethyl)-2H-pyran-2-one;
5,6-Dihydro-4-hydroxy-6-(2-methylpropyl)-6-(2-phenylethyl)-3-(1-phenyl-2-propenyl)-2H-pyran-2-one;
5,6-dihydro-hydroxy-6-(2-methylpropyl)-6-(2-phenylethyl)-3-(1-phenylpropyl)-2H-pyran-2-one;
5,6-Dihydro-3-diphenylmethyl-4-hydroxy-5,6-di-(2-phenylethyl)-2H-pyran-2-one;
5,6-Dihydro-4-hydroxy-6,6-di-(2-phenylethyl)-3-(1,3-diphenyl-2-propenyl)-2H-pyran-2-one;
5,6-Dihydro-hydroxy-6,6-di-(2-phenylethyl)-3-(1,3-diphenylpropyl)-2H-pyran-2-one;
5,6-Dihydro-4-hydroxy-3-(1,3-diphenyl-2-propenyl)-6-(3-phenylpropyl)-6-propyl-2H-pyran-2-one;
5,6-Dihydro-4-hydroxy-3-(1,3-diphenylpropyl)-6-(3-phenylpropyl)-6-propyl-2H-pyran-2-one;
3-(α-Cyclopropyl-[5-(methoxymethoxy-methyl)-thiophen-2-yl]-methyl)-5,6-dihydro-4-hydroxy-6-(2-phenylethyl)-6-propyl-2H-pyran-2-one;
3-(α-Cyclopropyl-[5-(methoxymethoxy-methyl)-thiophen-2-yl]-methyl)-5,6-dihydro-4-hydroxy-6-(3-phenylpropyl)-6-propyl-2H-pyran-2-one;
5,6-Dihydro-3-diphenylmethyl-4-hydroxy-6-(3-phenylpropyl)-6-propyl-2H-pyran-2-one;
3-Diphenylmethyl-5,6-dihydro-4-hydroxy-6-(2-methylpropyl)-6-(2-phenylethyl)-2H-pyran-2-one;
3-(1,3-Diphenyl-2-propenyl)-5,6-dihydro-4-hydroxy-6-(2-methylpropyl)-6-(2-phenylethyl)-2H-pyran-2-one;
3-(1,3-Diphenyl-2-propyl)-5,6-dihydro-4-hydroxy-6-(2-methylpropyl-6-(2-phenylethyl)-2H-pyran-2-one;
3-(α-Cyclopropyl-*meta*-[(phenylaminocarbonyl)amino]benzyl)-6-(α-ethylphenethyl)-4-hydroxy-2H-pyran-2-one;
3-(α-Cyclopropyl-*meta*-[(4-methoxyphenylaminocarbonyl)amino]benzyl)-6-(α-ethylphenethyl)-4-hydroxy-2H-pyran-2-one;
3-(α-Cyclopropyl-*meta*-[(benzylaminocarbonyl)amino]benzyl)-6-(α-ethylphenethyl)-4-hydroxy-2H-pyran-2-one;
3-(α-Cyclopropyl-*meta*-[(4-bromophenylaminocarbonyl)amino]benzyl)-6-(α-ethylphenethyl)-4-hydroxy-2H-pyran-2-one;
3-(α-Cyclopropyl-*meta*-[(phenylsulfonylaminocarbonyl)amino]benzyl)-6-(α-ethylphenethyl)-4-hydroxy-2H-pyran-2-one;
3-(α-Cyclopropyl-*meta*-(N-α-*tert*-butyloxycarbonyl-N-im-p-toluenesulfonyl-L-histidylamino)benzyl)-6-(α-ethylphenethyl)-4-hydroxy-2H-pyran-2-one;
3-(α-Cyclopropyl-*meta*-(*tert*-butyloxycarbonyl-L-alanylamino)benzyl)-6-(α-ethylphenethyl)-4-hydroxy-2H-pyran-2-one;
3-(α-Cyclopropyl-*meta*-(4-bromophenylbenzoylamino)benzyl)-6-(α-ethylphenethyl)-4-hydroxy-2H-pyran-2-one;
3-(α-Cyclopropyl-*meta*-(N-α-*tert*-butyloxycarbonyl-L-histidylamino)benzyl)-6-(α-ethylphenethyl)-4-hydroxy-2H-pyran-2-one;
3-(α-Cyclopropyl-*meta*-(N-benzyloxycarbonyl-O-α-benzyl-L-glutamylamino)benzyl)-6-(α-ethylphenethyl)-4-hydroxy-2H-pyran-2-one;
3-(α-Cyclopropyl-*meta*-(4-cyanophenylbenzoylamino)benzyl)-6-(α-ethylphenethyl)-4-hydroxy-2H-pyran-2-one;
3-(α-Cyclopropyl-*meta*-(L-glutamylamino)benzyl)-6-(α-ethylphenethyl)-4-hydroxy-2H-pyran-2-one;
3-(α-Cyclopropyl-*meta*-(3-(1-indolyl)propanoylamino)benzyl)-6-(α-ethylphenethyl)-4-hydroxy-2H-pyran-2-one;
3-(α-Cyclopropyl-*meta*-(2-pyridylacetylamino)benzyl)-6-(α-ethylphenethyl)-4-hydroxy-2H-pyran-2-one;
3-(α-Cyclopropylbenzyl)-6-[1-cyclopropylmethyl-2-(tetrahydropyran-3-yl)ethyl]-4-hydroxy-2H-pyran-2-one;
3-(α-Cyclopropyl-*meta*-(3-pyridylacetylamino)benzyl)-6-(α-ethylphenethyl)-4-hydroxy-2H-pyran-2-one;
3-(α-Cyclopropyl-*meta*-(4-pyridylacetylamino)benzyl)-6-(α-ethylphenethyl)-4-hydroxy-2H-pyran-2-one;
3-(α-Cyclopropyl-*meta*-(4-chlorophenylsulfonylamino)benzyl)-6-(α-ethylphenethyl)-4-hydroxy-2H-pyran-2-one;
3(α-Cyclopropyl-*meta*-(8-quinolinesulfonylamino)benzyl)-6-(α-ethylphenethyl)-4-hydroxy-2H-pyran-2-one;
3-(α-Cyclopropyl-*meta*-(ethylsulfonylamino)benzyl-6-[1-cyclopropylmethyl-2-(tetrahydropyran-3-yl)ethyl]-4-hydroxy-2H-pyran-2-one;
3-(α-Cyclopropyl-*meta*-(4-methylphenylsulfonylamino)benzyl-6-(α-ethyl-phenethyl)-4-hydroxy-2H-pyran-2-one;
3-(α-Cyclopropyl-*meta*-(4-methylphenylsulfonylamino)benzyl-6-[1-cyclopropylmethyl-2-(tetrahydropyran-3-yl)ethyl]-4-hydroxy-2H-pyran-2-one;
3-(α-Cyclopropyl-*meta*-(ethylsulfonylamino)benzyl-6-(α-ethyl-phenethyl)-4-hydroxy-2H-pyran-2-one;
(3S,6R) 3-(α-ethylbenzyl)-6-(α-ethylphenethyl)-4-hydroxy-2H-pyran-2-one;
(3S,6S) 3-(α-ethylbenzyl)-6-(α-ethylphenethyl)-4-hydroxy-2-pyran-2-one;
Sodium (3S,6R) 3-(α-ethylbenzyl)-6-(α-ethylphenethyl)-2H-pyran-2-one 4-oxide;
(3R,6R) 3-(α-ethylbenzyl)-6-(α-ethylphenethyl)-4-hydroxy-2H-pyran-2-one; and
(3R,6S) 3-(α-ethylbenzyl)-6-(α-ethylphenethyl)-4-hydroxy-2H-pyran-2-one.

5. The use of claim 4, wherein the compound is selected from the group consisting of:
3-(α-Ethylbenzyl)-6-(α-ethylphenethyl)-4-hydroxy-2H-pyran-2-one;
6-(1-Benzyl-propyl)-3-(cyclopropyl-phenyl-methyl)-4-hydroxy-pyran-2-one;
3-(α-Ethylbenzyl)-6-(α-ethylphenmethyl)-4-hydroxy-2H-pyran-2-one;
3-(α-Ethylbenzyl)-6-phenethyl-4-hydroxy-2H-pyran-2-one; and
4-Hydroxy-3-(1-phenylpropyl)-6-[(tetrahydro-2H-pyran-3-yl)methyl]propyl]-2H-pyran-2-one.

6. The use of claim 5, wherein the compound is to be administered in an amount of 100 to 400 mg/kg/day.

## Patentansprüche

1. Verwendung einer Verbindung der Formel I 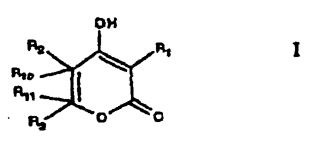 zur Herstellung eines Medikaments zur Prävention oder Behandlung von benigner Prostatahypertrophie oder -hyperplasie, Prostatakrebs, Alopezie, Hirsutismus, Akne vulgaris und Seborrhoe bei einem eine derartige Prävention oder Behandlung benötigenden Säuger;
wobei R₁
a) -(CH₂)ₙ-CH(R₅)-(CH₂)ₘ-R₄,
b) -CH(Aryl)-CH[C(O)-O-C₁-C₆-alkyl]₂,
c) -C(C₃-C₅-Cycloalkyl)-(CH₂)ₙ-R₄,
d) -C(Aryl)=CH-aryl,
e) -CH(R₅)-S-(CH₂)ₘ-R₄ oder
f) -(CH₂)ₚ-Aryl bedeutet;
wobei R₂
a) Wasserstoff,
b) Halogen,
c) C₁-C₆-Alkyl-[O-(CH₂)₂]_{q}-(CH₂)ₙ-,
d) C₁-C₆-Alkyl oder
e) -(CH₂)ₙ-CH(R₅)-(CH₂)ₘ-R₄ bedeutet;
wobei R₃
a) C₁-C₁₀-Alkyl, das optional mit null (0) bis fünf (5) Halogenen substituiert ist,
b) C₂-C₁₀-Alkenyl,
C) R₄-(CH₂)ₘ-CH(R₆)-(CH₂)ₙ-,
d) R₄-(CH₂)ₚ-,
e) R₄-CH=CH-,
f) CH₂=CH-(CH₂)ₚ-,
g) R₄(CH₂)ₘX₁C(O)(CH₂)ₙ-,
h) R₄(CH₂)ₘC(O)X₁(CH₂)ₙ-,
i) Aryl,
j) Het,
k) C₃-C₇-Cycloalkyl,
l) C₁-C₆-Alkyl-O-C(O)-(CH₂)ₙ-,
m) C₁-C₆-Alkyl-[O-(CH₂)₂]_{q}-(CH₂)ₙ-,
n) R₄-CH(R₆)-CH(R₆)-,
p) R₁₂-(CH₂)ₘ-X₂-(CH₂)ₙ-(R₇)HC- oder
q) R₁₅-(CH₂)ₘ-X₂-(CH₂)ₙ-(R₇)HC- bedeutet;
wobei R₄
a) Aryl,
b) Het,
c) C₃-C₇-Cycloalkyl,
d) C₂-C₁₀-Alkenyl,
e) C₁-C₆-Alkyl-[O-(CH₂)₂]_{q}-(CH₂)ₙ-,
f) Halogen,
g) Het-O-,
h) Het-C(O)-,
i) Aryl-(CH₂)ₙ-O-C(O)- oder
j) Trifluormethyl bedeutet;
wobei R₅
a) C₁-C₁₀-Alkyl,
b) C₂-C₁₀-Alkenyl,
c) C₃-C₇-Cycloalkyl,
d) -(CH₂)ₙ-Aryl,
e) -(CH₂)ₚ-Het oder
f) -(CH₂)ₙ-CH=CH-Aryl bedeutet;
wobei R₆
a) C₁-C₁₀-Alkyl,
b) R₄-C₁-C₅-Alkyl,
c) -(CH₂)ₙ-C₃-C₇-Cycloalkyl,
d) -(CH₂)ₚ-CH=CH₂,
e) -(CH₂)ₚ-Aryl,
f) -(CH₂)ₚ-Het oder
g) Hydroxy bedeutet;
wobei X₁ -NR₇- bedeutet;
wobei R₇
a) Wasserstoff oder
b) C₁-C₅-Alkyl bedeutet;
wobei Aryl
a) Phenyl, das mit null (0) bis drei (3) Resten R₈ substituiert ist,
b) Naphthyl, das mit null (0) bis drei (3) Resten R₈ substituiert ist,
c) Biphenyl, das mit null (0) bis drei (3) Resten R₈ substituiert ist, oder
d) Perhalogenphenyl bedeutet;
wobei Het einen 5- oder 6-gliedrigen gesättigten oder ungesättigten Ring, der ein (1) bis vier (4) Heteroatome, die aus der aus Stickstoff, Sauerstoff und Schwefel bestehenden Gruppe ausgewählt sind, enthält, bedeutet und jede bicyclische Gruppe, in der einer der obigen heterocyclischen Ringe an einen Benzolring, ein C₃-C₈-Cycloalkyl oder einen anderen Heterocyclus ankondensiert ist, umfasst; und, falls chemisch möglich, die Stickstoff- und Schwefelatome in den oxidierten Formen vorhanden sein können; und mit null (0) bis drei (3) Resten R₉ substituiert sein kann;
wobei R₈ und R₉ unabhängig voneinander
a) C₁-C₈-Alkyl, das mit null (0) bis drei (3) Halogenen substituiert ist,
b) C₂-C₈-Alkenyl,
c) Hydroxy,
d) Hydroxy-C₁-C₅-alkyl,
e) -(CH₂)ₙ-O-C₁-C₅-Alkyl, das mit null (0) bis drei (3) Hydroxy substituiert ist,
f) -(CH₂)ₙ-O-C₂-C₇-Alkenyl, das mit null (0) bis drei (3) Hydroxy substituiert ist,
g) Halogen,
h) Amino,
i) Amino-C₁-C₅-alkyl,
j) Mono- oder Di-C₁-C₅-alkylamino,
k) -C(O)-C₁-C₅-Alkyl,
l) -CHO,
m) -COOH,
n) -COO-C₁-C₅-Alkyl,
o) *-*CON(R₇)₂,
p) C₃-C₇-Cycloalkyl,
q) Nitro,
r) -CN,
s) -SO₃H,
t) -SO₂NH₂,
u) -O[(CH₂)₂-O]_{q}-CH₃,
v) -[CH₂-O]_{q}-C₁-C₃-Alkyl,
w) -(CH₂)ₙ-NHC(O)-O-(CH₂)ₚ-R₁₂,
x) -(CH₂)ₙ-NHC(O)-O-(CH₂)ₚ-R₁₅,
y) -(CH₂)ₙ-R₁₂,
z) -SO₂-R₁₂,
a1) -(CH₂)ₙ-X₂-(CH₂)ₙ-R₁₂,
b1) -(CH₂)ₙ-X₂-(CH₂)ₙ-R₁₅,
c1) -(CH₂)ₙ-X₂-CH=CH-R₁₂,
d1) -(CH₂)ₙ-X₂-CH=CH-R₁₅,
e1) -(CH₂)ₙ-X₂-C₁-C₁₀-Alkyl, das mit null (0) bis drei (3) Halogenen substituiert ist,
f1) -(CH₂)ₙ-X₂-C₂-C₅-Alkenyl,
g1) -X₂-(CH₂)ₚ-CH(NH₂) (COOH),
h1) -NHCONH-SO₂-R₁₂,
i1) -X₂-(CH₂)ₚ-NH-C(O)-O-C₁-C₆-Alkyl,
j1) -X₂-CH(X₃)-NH-C(O)-O-C₁-C₆-Alkyl,
k1) -X₂-(CH₂)ₚ-CH[NH-C(O)-O-(CH₂)ₚ-R₁₂]-C(O)-O-(CH₂)ₚ-R₁₂,
l1) -(CH₂)ₙ-X₄-N(C₁-C₃-Alkyl)₂,
m1) -(CH₂)ₙ-X₄-NHR₁₂,
n1) -NH-AA-P₁,
o1) -(CH₂)ₚ-N₃,
p1) -(CH₂)ₚ-R₁₂,
q1) -(CH₂)ₚ-R₁₅,
r1) -(CH₂)ₙ-NHC(SCH₃)=CHNO₂,
s1) -(CH₂)ₙ-NHC(NHR₇)=CHNO₂,
t1) -(CH₂)ₙ-NHC(SCH₃)=NCN oder
u1) -(CH₂)ₙ-NHC(NHR₇)=NCN bedeuten;
wobei X₂
a) -NH-C(O)-,
b) -NH-SO₂-,
c) -NH-C(O)-NH- oder
d) -SO₂-NH- bedeutet;
wobei X₃
a) C₁-C₆-Alkyl oder
b) -(CH₂)ₚ-R₁₅ bedeutet;
wobei X₄
a) -NH-C(O)- oder
b) -NH-SO₂- bedeutet;
wobei R₁₀ Wasserstoff bedeutet;
wobei R₁₁
a) Wasserstoff,
b) C₁-C₆-Alkyl,
c) -(CH₂)ₙ-Aryl,
d) -(CH₂)ₙ-C₃-C₇-Cycloalkyl oder
e) -(CH₂)ₙ-Het bedeutet;
oder wobei R₁₀ und R₁₁ zusammengenommen eine Doppelbindung bilden;
oder wobei R₃ und R₁₁ zusammengenommen
a) C₃-C₈-Cycloalkyl, das mit null (0) bis drei (3) Resten von Hydroxy, =N-OH, =O (Oxo) oder einer geschützten Form derselben substituiert ist oder an der α-Position mit R₁₄ substituiert ist; oder
b) einen 5- oder 6-gliedrigen gesättigten Ring, der ein (1) oder zwei (2) Sauerstoffatome enthält, bilden;
wobei R₁₂
a) Phenyl, das mit null (0) bis drei (3) Resten R₁₃ substituiert ist, oder
b) Naphthyl, das mit null (0) bis drei (3) Resten R₁₃ substituiert ist, bedeutet;
wobei R₁₃
a) C₁-C₁₀-Alkyl, das mit null (0) bis drei (3) Halogenen substituiert ist,
b) Hydroxy,
c) Hydroxy-C₁-C₅-alkyl,
d) -(CH₂)ₙ-O-C₁-C₅-Alkyl, das mit null (0) bis drei (3) Resten Hydroxy oder Halogen substituiert ist,
e) -(CH₂)ₙ-O-C₂-C₇-Alkenyl, das mit null (0) bis drei (3) Resten Hydroxy oder Halogen substituiert ist,
f) Halogen,
g) Amino,
h) Amino-C₁-C₅-alkyl,
i) Mono- oder Di-C₁-C₅-alkylamino,
j) -C(O)-C₁-C₅-Alkyl,
k) -CHO,
l) -COOH,
m) -CON(R₇)₂,
n) -NHCO-C₁-C₃-Alkyl,
o) -NHOH,
p) Nitro,
q) -CN,
r) -(CH₂)ₙ-Phenyl,
s) -COO-C₁-C₅-Alkyl oder
t) -SO₂-Phenyl, das mit null (0) bis drei (3) Resten C₁-C₅-Alkyl substituiert ist,
u) -(CH₂)ₙ-X₄-Phenyl oder
v) -(CH₂)ₙ-N=N-Phenyl, das mit null (0) oder einem (1) -N(C₁-C₃-Alkyl)₂ substituiert ist, bedeutet;
wobei R₁₄
a) -(CH₂)ₙ-Aryl,
b) -C₁-C₆-Alkyl oder
c) -(CH₂)ₙ-C₄-C₇-Cycloalkyl bedeutet;
wobei R₁₅ einen 5- oder 6-gliedrigen gesättigten oder ungesättigten Ring, der ein (1) bis vier (4) Heteroatome, die aus der aus Stickstoff, Sauerstoff und Schwefel bestehenden Gruppe ausgewählt sind, enthält, bedeutet und jede bicyclische Gruppe, in der einer der obigen heterocyclischen Ringe an einen Benzolring, ein C₃-C₈-Cycloalkyl oder einen anderen Heterocyclus ankondensiert ist, umfasst; und mit null (0) bis drei (3) Resten R₁₃ substituiert sein kann;
wobei AA einen Aminosäurerest bedeutet;
wobei P₁ Wasserstoff oder eine Stickstoffschutzgruppe bedeutet;
wobei m und n unabhängig voneinander null (0) bis einschließlich fünf (5) bedeuten;
wobei p eins (1) bis einschließlich fünf (5) bedeutet;
wobei q eins (1) bis einschließlich fünf (5) bedeutet;
und pharmazeutisch akzeptabler Salze derselben.

2. Verwendung nach Anspruch 1, wobei die Größe der Prostata bei einem männlichen Säuger verringert oder beibehalten wird.

3. Verwendung nach Anspruch 2,
wobei R₁-(CH₂)ₙ-CH(R₅)-(CH₂)ₘ-R₄ bedeutet,
wobei R₂ Wasserstoff bedeutet;
wobei R₃
a) R₄-(CH₂)ₘ-CH(R₆)-(CH₂)ₙ- oder
b) R₄-(CH₂)ₚ- bedeutet;
wobei R₄
a) Phenyl oder
b) Tetrahydropyranyl bedeutet;
wobei R₅
a) Propyl oder
b) Cyclopropyl bedeutet;
wobei R₆ Ethyl bedeutet;
wobei m null (0) oder eins (1) bedeutet;
wobei n null (0) bedeutet;
wobei p zwei (2) bedeutet;
wobei R₁₀ und R₁₁ zusammengenommen eine Doppelbindung bilden.

4. Verwendung nach Anspruch 1 oder Anspruch 2, wobei die Verbindung ausgewählt ist aus der Gruppe von:
3-(α-Ethylbenzyl)-4-hydroxy-6-phenyl-2H-pyran-2-on;
6-Benzyl-3-(α-ethylbenzyl)-4-hydroxy-2H-pyran-2-on;
3-(α-Ethylbenzyl)-6-phenethyl-4-hydroxy-2H-pyran-2-on;
4-Hydroxy-6-phenethyl-3-(α-propyl-p-brombenzyl)-2H-pyran-2-on;
6-(p-Bromphenethyl)-3-(α-ethylbenzyl)-4-hydroxy-2H-pyran-2-on;
3-(α-Ethylbenzyl)-6-(o-fluorphenethyl)-4-hydroxy-2H-pyran-2-on;
3-(α-Ethylbenzyl)-4-hydroxy-6-(3-phenylpropyl)-2H-pyran-2-on;
3-(α-Ethylbenzyl)-4-hydroxy-6-propyl-2H-pyran-2-on;
3-(α-Ethylbenzyl)-4-hydroxy-6-(3-butenyl)-2H-pyran-2-on;
3-(α-Ethylbenzyl)-4-hydroxy-6-[[(phenylamino)carbonyl]methyl]-2H-pyran-2-on;
4-Hydroxy-6-phenethyl-3-(α-vinylbenzyl)-2H-pyran-2-on; 3-(α-Ethylbenzyl)-6-(α-ethylphenethyl)-4-hydroxy-2H-pyran-2-on;
3-(α-Ethylbenzyl)-1-ethylpropyl-4-hydroxy-2H-pyran-2-on;
3-(α-Ethylbenzyl)-4-hydroxy-6-(p-methoxystyryl)-2H-pyran-2-on;
3-(α-Ethylbenzyl)-4-hydroxy-6-(2-naphth-2-ylethyl)-2H-pyran-2-on;
3-(α-Ethylbenzyl)-4-hydroxy-6-(1-ethyl-2-naphth-2-ylethyl)-2H-pyran-2-on;
3-(α-Ethylbenzyl)-4-hydroxy-6-(2-naphth-1-ylethyl)-2H-pyran-2-on;
3-(α-Ethylbenzyl)-4-hydroxy-6-(1-ethyl-2-naphth-1-ylethyl)-2H-pyran-2-on;
3-(α-Cyclopropylbenzyl)-4-hydroxy-6-(3-phenylpropyl)-2H-pyran-2-on;
3-(α-Cyclopropylbenzyl)-6-(1-ethylpropyl)-4-hydroxy-2H-pyran-2-on;
3-(α-Cyclopropylbenzyl)-6-(α-benzylphenethyl)-4-hydroxy-2H-pyran-2-on;
3-(α-Cyclopropylbenzyl)-4-hydroxy-6-phenethyl-2H-pyran-2-on;
3-(α-Cyclopropylbenzyl)-6-(1-propylbutyl)-4-hydroxy-2H-pyran-2-on;
3-(α-Cyclopropylbenzyl)-4-hydroxy-6-methyl-2H-pyran-2-on;
4-Hydroxy-6-methyl-3-(3-phenyl-prop-2-enyl)-2H-pyran-2-on;
Dimethyl-3-[(4-hydroxy-2-oxo-6-phenyl-2H-1-pyran-3-yl)(4-nitrophenyl)-1,3-propandioat;
Dimethyl-3-[(4-hydroxy-2-oxo-6-phenyl-2H-1-pyran-3-yl)(3-nitrophenyl)-1,3-propandioat;
6-Ethyl-3-(α-ethylbenzyl)-6-phenyl-tetrahydropyran-2,4-dion;
5,6-Dihydro-4-hydroxy-6-cyclohexyl-6-phenyl-3-[1-(3-hydroxyphenyl)propyl]-2H-pyran-2-on;
4-Hydroxy-1-oxa-3-(1-phenylpropyl)spiro-[5,5]-undec-3-en-2-on-natriumsalz;
6,6-Diethyl-3-(3-phenylpropyl)-tetrahydropyran-2,4-dion;
Dihydro-6-methyl-6-phenyl-3-(1-phenyl-2-propenyl)-2H-pyran-2,4(3H)-dion;
Dihydro-3-[1-(3-hydroxyphenyl)propyl]-6-phenyl-6-propyl-2H-pyran-2,4(3H)-dion;
5,6-Dihydro-4-hydroxy-6-phenyl-6-propyl-3-(1-phenylpropyl)-2H-pyran-2-on;
5,6-Dihydro-4-hydroxy-6-phenyl-6-propyl-3-[1-(3-hydroxyphenyl)-propyl]-2H-pyran-2-on;
12-Hydroxy-11-(1-phenyl-allyl)-1,4,9-trioxadispiro[4.2.5.2]pentadec-11-en-10-on;
12-Hydroxy-11-(1-phenyl-propyl)-1,4,9-trioxadispiro[4.2.5.2]pentadec-11-en-10-on;
4-Hydroxy-3-(1-phenyl-propyl)-1-oxa-spiro[5.5]undec-3-en-2,9-dion;
6,6-Dibenzyl-4-hydroxy-3-(1-phenyl-propyl)-5,6-dihydro-pyran-2-on;
4-Hydroxy-3-(1-phenyl-allyl)-1,9-dioxaspiro[5.5]undec-3-en-2-on;
4,9-Dihydroxy-3-(1-phenyl-propyl)-1-oxaspiro[5.5]undec-3-en-2-on;
N-(3-Cyclopropyl-[6-(1-ethyl-phenethyl)-4-hydroxy-2-oxo-5,6-dihydro-2H-pyran-3-yl]-methyl)-phenyl)-3-(tert-butyloxycarbonylamino)-propionamid;
N-(3-{Cyclopropyl-[6-(2-cyclopropyl-1-cyclopropylmethyl-ethyl)-4-hydroxy-2-oxo-5,6-dihydro-2H-pyran-3-yl]-methyl}-phenyl)-3-indol-1-ylpropionamid;
3-(Cyclopropyl-phenyl-methyl)-4-hydroxy-6-(1-(tetrahydro-furan-3-ylmethyl)-propyl)-pyran-2-on;
6-(1-(5-Chloro-thiophen-2-ylmethyl)-propyl)-3-(cyclopropyl-phenyl-methyl)-4-hydroxy-pyran-2-on;
3-(Cyclopropyl-phenyl-methyl)-6-(1-(3,5-dimethylisoxazol-4-ylmethyl)-propyl)-4-hydroxy-pyran-2-on;
3-(Cyclopropyl-phenyl-methyl)-4-hydroxy-6-(1-(tetrahydro-furan-2-ylmethyl)-propyl)-pyran-2-on;
3-(Cyclopropyl-phenyl-methyl)-4-hydroxy-6-(1-thiophen-2-ylmethyl-propyl)-pyran-2-on;
3-(Cyclopropyl-phenyl-methyl)-4-hydroxy-6-(1-tetrahydro-pyran-4-ylmethyl)-propyl)-pyran-2-on;
3-(Cyclopropyl-phenyl-methyl)-6-(1-furan-2-ylmethylpropyl)-4-hydroxy-pyran-2-on;
3-(Cyclopropyl-phenyl-methyl)-6-(2-(1,3)dioxolan-2-ylmethyl-propyl)-4-hydroxy-pyran-2-on;
3-(Cyclopropyl-phenyl-methyl)-4-hydroxy-6-(1-tetrahydro-pyran-3-ylmethyl)-propyl)-pyran-2-on;
3-(Cyclopropyl-phenyl-methyl)-4-hydroxy-6-(1-tetrahydro-pyran-2-ylmethyl)-propyl)-pyran-2-on;
3-(Cyclopropyl-phenyl-methyl)-4-hydroxy-6-(1-pyridin-2-ylmethyl-propyl)-pyran-2-on;
6-(4-Chlor-1-ethyl-butyl)-3-(cyclopropyl-phenylmethyl)-4-hydroxy-pyran-2-on;
6-(3-Chlor-1-ethyl-propyl)-3-(cyclopropyl-phenylmethyl)-4-hydroxy-pyran-2-on;
3-(Cyclopropyl-phenyl-methyl)-6-[ethyl-3-(tetrahydropyran-2-yloxy)-propyl-4-hydroxy-pyran-2-on;
3-(Cyclopropyl-phenyl-methyl)-4-hydroxy-6-(1-pyridin-3-ylmethyl-propyl)-pyran-2-on;
3-(Cyclopropyl-phenyl-methyl)-6-(1-ethyl-3-thiophen-3-yl-propyl)-4-hydroxy-pyran-2-on;
3-(Cyclopropyl-phenyl-methyl)-4-hydroxy-6-[1-(tetrahydro-pyran-3-ylmethyl)-butyl]-pyran-2-on;
5-Brom-6-(2-cyclopropyl-cyclopropylmethyl-ethyl)-4-hydroxy-3-(1-phenyl-propyl)-pyran-2-on;
3-(1-Benzyl-2-phenyl-ethyl)-6-(2-cyclopropyl-1-cyclopropylmethyl-ethyl)-4-hydroxy-pyran-2-on;
6-(2-Cyclopropylmethyl-ethyl)-3-(cyclopropyl-phenylmethyl)-4-hydroxy-pyran-2-on;
6-(1-Allyl-but-3-enyl)-3-(cyclopropyl-phenyl-methyl)-4-hydroxy-pyran-2-on;
3-(Cyclopropyl-phenyl-methyl)-6-(1-ethyl-3-(2-methoxy-ethoxy)-propyl)-4-hydroxy-pyran-2-on;
6-(1-Benzyl-3-(2-methoxy-ethoxy)-propyl)-3-(cyclopropyl-phenyl-methyl)-4-hydroxy-pyran-2-on;
3-(α-Cyclopropyl-meta-(benzyloxycarbonylamino)benzyl)-6-(α-ethyl-phenethyl)-4-hydroxy-2H-pyran-2-on;
3-(α-Cyclopropyl-meta-(tertbutyloxycarbonylamino)benzyl)-6-(α-cyclopropylmethylcyclopropylethyl)-4-hydroxy-2H-pyran-2-on;
4-Hydroxy-3-(1-phenyl-propyl)-6-(1-propyl-butyl)-pyran-2-on;
4-Hydroxy-3-(1-phenyl-allyl)-6-(1-propyl-butyl)-pyran-2-on;
3-(5-Cyclopropyl-phenyl-methyl)-4-hydroxy-6-oxo-6H-pyran-2-yl)-propionsäure-tert-butylester;
3-(Cyclopropyl-phenyl-methyl)-6-(2-(3,5-dimethylisoxazol-4-yl)-ethyl)-4-hydroxy-pyran-2-on;
6-(2-(5-tert-Butyl-(1,2,4)oxadiazal-3-yl)-ethyl)-3-(cyclopropyl-phenyl-methyl)-4-hydroxy-pyran-2-on;
3-(Cyclopropyl-phenyl-methyl)-4-hydroxy-6-(2-phenyl-1-pyridin-2-ylmethyl-ethyl)-pyran-2-on;
3-(Cyclopropyl-phenyl-methyl)-6-(2-(1,3)dioxolan-2-yl-ethyl)-4-hydroxy-pyran-2-on;
3-(Cyclopropyl-phenyl-methyl)-4-hydroxy-6-(4-morpholin-4-yl-butyl)-pyran-2-on;
3-(Cyclopropyl-phenyl-methyl)-4-hydroxy-6-(2-pyridin-2-yl-ethyl)-pyran-2-on;
3-(Cyclopropyl-phenyl-methyl)-4-hydroxy-6-(2-(2-methyl-thiazol-4-yl)-ethyl)-pyran-2-on;
3-(Cyclopropyl-phenyl-methyl)-4-hydroxy-6-(2-chinolin-2-yl-ethyl)-pyran-2-on;
6-(3-Chlor-propyl)-3-(cyclopropyl-phenyl-methyl)-4-hydroxy-pyran-2-on;
6-(1-(2-(4-Chlor-phenyl)-thiazol-4-ylmethyl)-propyl)-3-cyclopropyl-phenyl-methyl)-4-hydroxy-pyran-2-on;
3-(Cyclopropyl-phenyl-methyl)-6-(3-(1,3)dioxan-2-yl-1-ethyl-propyl)-4-hydroxy-pyran-2-on;
3-(Cyclopropyl-phenyl-methyl)-4-hydroxy-6-(1-pyridin-4-ylmethyl-propyl)-pyran-2-on;
3-(Cyclopropyl-phenyl-methyl)-6-(1-ethyl-3-morpholin-4-yl-3-oxo-propyl)-4-hydroxy-pyran-2-on;
3-(Cyclopropyl-phenyl-methyl)-6-(1-ethyl-4-morpholin-4-yl-butyl)-4-hydroxy-pyran-2-on;
3-(Cyclopropyl-phenyl-methyl)-6-[1-(2,3-dihydro-benzo[1,4]dioxin-2-ylmethyl)-propyl]-4-hydroxy-pyran-2-on;
3-(Cyclopropyl-phenyl-methyl)-4-hydroxy-6-isobutyl-pyran-2-on;
Cyclopropyl-phenyl-methyl)-6-[1-(5,6-dihydro-2H-pyran-3-ethyl)-propyl]-4-hydroxy-pyran-2-on;
3-(Cyclopropyl-phenyl-methyl)-4-hydroxy-5-(2-(2-methoxy-ethoxy)-ethyl)-6-propyl-pyran-2-on;
3-(Cyclopropyl-phenyl-methyl)-4-hydroxy-6-(1-isobutyl-3-methyl-butyl)-pyran-2-on;
3-Dicyclopropylmethyl-4-hydroxy-6-phenethyl-pyran-2-on;
6-(1-Cyclopropyl-ethyl)-3-dicyclopropylmethyl-4-hydroxy-pyran-2-on;
6-(2-Cyclopropyl-2-cyclopropylmethyl-ethyl)-3-dicyclopropylmethyl-4-hydroxy-pyran-2-on;
6-(1-Cyclohexylmethyl-propyl)-3-(cyclopropyl-phenyl-methyl)-4-hydroxy-pyran-2-on;
6-(1-Benzyl-propyl)-3-(cyclopropyl-phenyl-methyl)-4-hydroxy-pyran-2-on;
6-(2-Cyclopropyl-1-cyclopropylmethyl-ethyl)-4-hydroxy-3-(1-phenyl-propyl)-pyran-2-on;
6-(1-Benzyl-2-cyclopropyl-ethyl)-3-(cyclopropyl-phenyl-methyl)-4-hydroxy-pyran-2-on;
6-(2-Cyclopropyl-ethyl)-3-(cyclopropyl-phenyl-methyl)-4-hydroxy-pyran-2-on;
6-(1-Cyclopropylmethyl-propyl)-3-(cyclopropyl-phenyl-methyl)-4-hydroxy-pyran-2-on;
3-(Cyclopropyl-phenyl-methyl)-4-hydroxy-6-(4-phenyl-butyl)-pyran-2-on;
3-(Cyclohexyl-cyclopropyl-methyl)-6-(2-cyclopropyl-1-cyclopropylmethyl-ethyl)-4-hydroxy-pyran-2-on;
3-(Cyclopropyl-phenyl-methyl)-6-(1-ethyl-4-phenyl-butyl)-4-hydroxy-pyran-2-on;
6-(3-Cyclohexyl-propyl)-3-(cyclopropyl-phenyl-methyl)-4-hydroxy-pyran-2-on;
6-(3-Cyclohexyl-1-ethyl-propyl)-3-(cyclopropyl-phenyl-methyl)-4-hydroxy-pyran-2-on;
6-(2-Cyclopropyl-ethyl)-4-hydroxy-3-(1-phenyl-propyl)-pyran-2-on;
6-But-3-enyl-3-(cyclopropyl-phenyl-methyl)-4-hydroxy-pyran-2-on;
3-(Cyclopropyl-phenyl-methyl)-6-(1-ethyl-3-phenyl-propyl)-4-hydroxy-pyran-2-on;
5-Brom-6-(2-cyclopropyl-ethyl)-4-hydroxy-3-(1-phenyl-propyl)-pyran-2-on;
6-(1-Benzyl-2-phenyl-ethyl)-4-hydroxy-3-(1-phenyl-propyl)-pyran-2-on;
3-(Cyclopropyl-phenyl-methyl)-4-hydroxy-6-(3-(2-methoxy-ethoxy)-propyl-pyran-2-on;
3-(Cyclopropyl-phenyl-methyl)-4-hydroxy-5-(2-(2-methoxy-ethoxy)-ethyl)-6-(3-(2-methoxy-ethoxy)-pyran-2-on;
3-(Cyclopropyl-phenyl-methyl)-4-hydroxy-6-propyl-pyran-2-on;
5-Brom-4-hydroxy-6-phenylethyl-3-(1-phenyl-propyl)-pyran-2-on;
3-(Cyclopropyl-phenyl-methyl)-4-hydroxy-6-(3-(2-methoxy-ethoxy)-ethoxy)-propyl)-pyran-2-on;
5-Brom-4-hydroxy-3-(1-phenyl-propyl)-6-propyl-pyran-2-on;
3-(Cyclopropyl-phenyl-methyl)-6-(1-ethyl-propyl)-4-hydroxy-5-(2-(2-methoxy-ethoxy)ethyl)-pyran-2-on;
6-(1-Benzyl-propyl)-5-brom-4-hydroxy-3-(1-phenyl-propyl)-pyran-2-on;
6-(2-Cyclopropyl-1-cyclopropylmethyl-ethyl)-3-(cyclopropyl-phenyl-methyl)-4-hydroxy-5-(2-(2-methoxy-ethoxy)-ethoxy)-ethyl)-pyran-2-on;
3-(Cyclopropyl-phenyl-methyl)-6-(2-furan-2-yl-2-hydroxy-ethyl)-4-hydroxy-pyran-2-on;
3-(Cyclopropyl-phenyl-methyl)-4-hydroxy-6-(4,4,4-trifluor-butyl)-pyran-2-on;
6-[2-(1-Cyclohexyl-1H-tetrazol-5-yl)-ethyl]-3-cyclopropyl-phenyl-methyl)-4-hydroxy-2-on;
4-Hydroxy-3-(1-phenyl-propyl)-1-oxa-spiro[5.5]undec-3-en-2,9-dionmonooxim;
5,6-Dihydro-4-hydroxy-6-phenyl-6-(phenylmethyl)-3-(1-phenyl-2-propenyl)-2H-pyran-2-on;
5,6-Dihydro-4-hydroxy-6-phenyl-6-(phenylmethyl)-3-(1-phenylpropyl)-2H-pyran-2-on;
3-(1,3-Diphenyl-2-propenyl)-5,6-dihydro-4-hydroxy-6-(2-phenylethyl)-6-propyl-(E)-2H-pyran-2-on;
3-(1,3-Diphenyl-2-propyl)-5,6-dihydro-4-hydroxy-6-(2-phenylethyl)-6-propyl-(E)-2H-pyran-2-on;
3-(1,3-Diphenyl-2-propenyl)-5,6-dihydro-4-hydroxy-6-phenyl-6-(phenylmethyl)-2H-pyran-2-on;
3-(1,2-Diphenylethenyl)-5,6-dihydro-4-hydroxy-6-(2-phenylethyl)-6-propyl-(E)-2H-pyran-2-on;
5,6-Dihydro-4-hydroxy-6-(2-phenylethyl)-3-(1-phenyl-2-propenyl)-6-propyl-2H-pyran-2-on;
5,6-Dihydro-4-hydroxy-6-(2-phenylethyl)-3-(1-phenylpropyl)-6-propyl-2H-pyran-2-on;
3-(1,3-Diphenyl-2-propenyl)-5,6-dihydro-4-hydroxy-6-(phenylmethyl)-6-propyl-2H-pyran-2-on;
3-(1,3-Diphenylpropyl)-5,6-dihydro-4-hydroxy-6-(phenylmethyl)-6-propyl-2H-pyran-2-on;
4-Hydroxy-3-(1-phenylallyl)-1-oxa-spiro[5.6]-dodec-3-en-2-on;
4-Hydroxy-3-(1-phenylallyl)-1-oxa-spiro[5.4]-dec-3-en-2-on;
7-Benzyl-4-hydroxy-3-(1-phenylallyl)-1-oxa-spiro[5.5]-undec-3-en-2-on;
4-Hydroxy-3-(1-phenylpropyl)-1-oxa-spiro[5.4]-dec-3-en-2-on;
4-Hydroxy-3-(1-phenyl-2-propenyl)-1-oxaspiro[5.7]tridec-3-en-2-on;
4-Hydroxy-3-(1-phenylpropyl)-1-oxaspiro[5.7]tridec-3-en-2 -on;
3-(α-Cyclopropylbenzyl)-4-hydroxy-6-(α-ethyl-β-hydroxyphenethyl)-2H-pyran-2-on;
3-(α-Cyclopropylbenzyl)-4-hydroxy-6-(β-hydroxy-p-methylphenethyl)-2H-pyran-2-on;
3-(α-Cyclopropylbenzyl)-4-hydroxy-6-(β-hydroxy-p-fluorphenethyl)-2H-pyran-2-on;
3-(α-Cyclopropylbenzyl)-4-hydroxy-6-(β-hydroxy-p-chlorphenethyl)-2H-pyran-2-on;
3-(α-Cyclopropylbenzyl)-4-hydroxy-6-(β-hydroxy-m-chlorphenethyl)-2H-pyran-2-on;
3-(α-Cyclopropylbenzyl)-4-hydroxy-6-(β-hydroxy-o-chlorphenethyl)-2H-pyran-2-on;
3-(α-Cyclopropylbenzyl)-4-hydroxy-6-(2-(furan-3-yl)-2-hydroxyethyl)-2H-pyran-2-on;
3-(α-Cyclopropylbenzyl)-4-hydroxy-6-(2-(thiophen-3-yl)-2-hydroxyethyl)-2H-pyran-2-on;
3-(α-Cyclopropylbenzyl)-4-hydroxy-6-(α-ethyl-p-fluorphenethyl)-2H-pyran-2-on;
3-(α-Cyclopropylbenzyl)-4-hydroxy-6-(α-ethyl-p-chlorphenethyl)-2H-pyran-2-on;
3-(α-Cyclopropylbenzyl)-4-hydroxy-6-(α-ethyl-m-chlorphenethyl)-2H-pyran-2-on;
3-(α-Cyclopropylbenzyl)-4-hydroxy-6-(α-ethyl-o-chlorphenethyl)-2H-pyran-2-on;
3-(α-Cyclopropylbenzyl)-4-hydroxy-6-(α-ethyl-p-bromphenethyl)-2H-pyran-2-on;
3-(α-Cyclopropylbenzyl)-4-hydroxy-6-(α-ethyl-m-bromphenethyl)-2H-pyran-2-on;
3-(α-Cyclopropylbenzyl)-4-hydroxy-6-(α-ethyl-p-trifluormethylphenethyl)-2H-pyran-2-on;
3-(α-Cyclopropylbenzyl)-4-hydroxy-6-(α-ethyl-m-trifluormethylphenethyl)-2H-pyran-2-on;
3-(α-Cyclopropylbenzyl)-4-hydroxy-6-(α-ethyl-o-trifluormethylphenethyl)-2H-pyran-2-on;
3-(α-Cyclopropylbenzyl)-4-hydroxy-6-(α-ethyl-p-methoxyphenethyl)-2H-pyran-2-on;
3-(α-Cyclopropylbenzyl)-4-hydroxy-6-(α-ethyl-m-methoxyphenethyl)-2H-pyran-2-on;
3-(α-Cyclopropylbenzyl)-4-hydroxy-6-(p-fluorphenethyl)-2H-pyran-2-on;
3-(α-Cyclopropylbenzyl)-4-hydroxy-6-(p-chlorphenethyl)-2H-pyran-2-on;
3-(α-Cyclopropylbenzyl)-4-hydroxy-6-(p-bromphenethyl)-2H-pyran-2-on;
3-(Cyclopropylphenylmethyl)-6-[1-ethyl-3-(4-morpholinyl)propyl]-4-hydroxy-2H-pyran-2-on;
3-(Cyclopropylphenylmethyl)-β-ethyl-4-hydroxy-2-oxo-2H-pyran-6-propansäure-phenylmethylester;
3-(Cyclopropylphenylmethyl)-4-hydroxy-6-[2-methyl-1-(phenylmethyl)propyl)-2H-pyran-2-on;
3-(Cyclopropylphenylmethyl)-4-hydroxy-6-[2-methyl-1-[(tetrahydro-2H-pyran-3-yl)methyl]propyl]-2H-pyran-2-on;
4-Hydroxy-3-(1-phenylpropyl)-6-[1-[(tetrahydro-2H-pyran-3-yl)methyl]propyl]-2H-pyran-2-on;
3-(Cyclopropylphenylmethyl)-6-(1-ethyl-4,4,4-trifluorbutyl)-4-hydroxy-2H-pyran-2-on;
3-[2-[3-(Cyclopropylphenylmethyl)-4-hydroxy-2-oxo-2H-pyran-6-yl]butyl]-1-[(4-methylphenyl)sulfonyl]-piperidin;
2-[2-[3-(Cyclopropylphenylmethyl)-4-hydroxy-2-oxo-2H-pyran-6-yl]butyl]-1-[(4-methylphenyl)sulfonyl]-pyrrolidin;
3-(Cyclopropylphenylmethyl)-4-hydroxy-6-(3,3,3-trifluorpropyl)-2H-pyran-2-on;
2-[2-[3-(Cyclopropylphenylmethyl)-4-hydroxy-2-oxo-2H-pyran-6-yl]butyl]-1-[(4-methylphenyl)sulfonyl]-piperidin;
4- [2-[3-(Cyclopropylphenylmethyl)-4-hydroxy-2-oxo-2H-pyran-6-yl]butyl]-1-[(4-methylphenyl)sulfonyl]-piperidin;
4-[2-[3-(Cyclopropylphenylmethyl)-4-hydroxy-2-oxo-2H-pyran-6-yl]butyl]-1-(phenylmethyl)-2-pyrrolidinon;
6-(Cyclopentylmethyl)-3-(cyclopropylphenylmethyl)-4-hydroxy-2H-pyran-2-on;
3-(Cyclopropylphenylmethyl)-4-hydroxy-6-[(tetrahydro-2H-pyran-4-yl)methyl]-2H-pyran-2-on;
3-(Cyclopropylphenylmethyl)-6-(3-fluorpropyl)-4-hydroxy-2H-pyran-2-on;
4-Hydroxy-3-(1-phenylcyclobutyl)-6-[1-(phenylmethyl)propyl]-2H-pyran-2-on;
3-(α-Ethylbenzyl)-6-(α-ethylbenzyl)-4-hydroxy-2H-pyran-2-on;
3-(α-Cyclopropyl-meta-(phenylsulfonylamino)benzyl)-6-(α-ethylphenethyl)-4-hydroxy-2H-pyran-2-on;
3-(α-Cyclopropyl-meta-(propylsulfonylamino)benzyl)-6-(α-ethylphenethyl)-4-hydroxy-2H-pyran-2-on;
3-(α-Cyclopropyl-meta-((E)-2-phenylethenylsulfonylamino)benzyl)-6-(αethylphenethyl)-4-hydroxy-2H-pyran-2-on;
3-(α-Cyclopropyl-meta-(4-bromphenylsulfonylamino)benzyl)-6-(α-ethylphenethyl)-4-hydroxy-2H-pyran-2-on;
3-(α-Cyclopropyl-meta-(2,5-dichlorphenylsulfonylamino)benzyl)-6-(α-ethylphenethyl)-4-hydroxy-2H-pyran-2-on;
3-(α-Cyclopropyl-meta-(4-tert-butylphenylsulfonylamino)benzyl)-6-(α-ethylphenethyl)-4-hydroxy-2H-pyran-2-on;
3-(α-Cyclopropyl-meta-(4-cyanophenylsulfonylamino)benzyl)-6-(α-ethylphenethyl)-4-hydroxy-2H-pyran-2-on;
3-(α-Cyclopropyl-meta-(4-methoxyphenylsulfonylamino)benzyl)-6-(α-ethylphenethyl)-4-hydroxy-2H-pyran-2-on;
6-Butyl-5,6-dihydro-3-(1,3-diphenyl-2-propenyl)-4-hydroxy-6-phenylmethyl-2H-pyran-2-on;
6-Butyl-5, 6-dihydro-3-(1,3-diphenylpropyl)-4-hydroxy-6-phenylmethyl-2H-pyran-2-on;
6-Butyl-5,6-dihydro-4-hydroxy-6-phenylmethyl-3-(1-phenyl-2-propenyl)-2H-pyran-2-on;
6-Butyl-5,6-dihydro-4-hydroxy-6-phenylmethyl-3-(1-phenylpropyl)-2H-pyran-2-on;
3-(α-Cyclopropyl-[5-(methoxymethoxy-methyl)-thiophen-2-yl]-methyl)-5,6-dihydro-4-hydroxy-6-(2-methylpropyl)-6-(2-phenylethyl)-2H-pyran-2-on;
5,6-Dihydro-4-hydroxy-6-(2-methylpropyl)-6-(2-phenylethyl)-3-(1-phenyl-2-propenyl)-2H-pyran-2-on;
5,6-Dihydro-4-hydroxy-6-(2-methylpropyl)-6-(2-phenylethyl)-3-(1-phenylpropyl)-2H-pyran-2-on;
5,6-Dihydro-3-diphenylmethyl-4-hydroxy-6,6-di-(2-phenylethyl)-2H-pyran-2-on;
5,6-Dihydro-4-hydroxy-6,6-di-(2-phenylethyl)-3-(1,3-diphenyl-2-propenyl)-2H-pyran-2-on;
5,6-Dihydro-4-hydroxy-6,6-di-(2-phenylethyl)-3-(1,3-diphenylpropyl)-2H-pyran-2-on;
5,6-Dihydro-4-hydroxy-3-(1,3-diphenyl-2-propenyl)-6-(3-phenylpropyl)-6-propyl-2H-pyran-2-on;
5,6-Dihydro-4-hydroxy-3-(1,3-diphenylpropyl)-6-(3-phenylpropyl)-6-propyl-2H-pyran-2-on;
3-(α-Cyclopropyl-[5-(methoxymethoxy-methyl)-thiophen-2-yl]-methyl)-5,6-dihydro-4-hydroxy-6-(2-phenylethyl)-6-propyl-2H-pyran-2-on;
3-(α-Cyclopropyl-[5-(methoxymethoxy-methyl)-thiophen-2-yl]-methyl)-5,6-dihydro-4-hydroxy-6-(3-phenylpropyl)-6-propyl-2H-pyran-2-on;
5,6-Dihydro-3-diphenylmethyl-4-hydroxy-6-(3-phenylpropyl)-6-propyl-2H-pyran-2-on;
3-Diphenylmethyl-5,6-dihydro-4-hydroxy-6-(2-methylpropyl)-6-(2-phenylethyl)-2H-pyran-2-on;
3-(1,3-Diphenyl-2-propenyl)-5,6-dihydro-4-hydroxy-6-(2-methylpropyl)-6-(2-phenylethyl)-2H-pyran-2-on;
3-(1,3-Diphenyl-2-propyl)-5,6-dihydro-4-hydroxy-6-(2-methylpropyl)-6-(2-phenylethyl)-2H-pyran-2-on;
3-(α-Cyclopropyl-meta-[(phenylaminocarbonyl)amino]benzyl)-6-(α-ethylphenethyl)-4-hydroxy-2H-pyran-2-on;
3-(α-Cyclopropyl-meta-[(4-methoxyphenylaminocarbonyl)amino]benzyl)-6-(α-ethylphenethyl)-4-hydroxy-2H-pyran-2-on;
3-(α-Cyclopropyl-meta-[(benzylaminocarbonyl)amino]benzyl)-6-(α-ethylphenethyl)-4-hydroxy-2H-pyran-2-on;
3-(α-Cyclopropyl-meta-[(4-bromphenylaminocarbonyl)amino]benzyl)-6-(α-ethylphenethyl)-4-hydroxy-2H-pyran-2-on;
3-(α-Cyclopropyl-meta-[(phenylsulfonylaminocarbonyl)amino]benzyl)-6-(α-ethylphenethyl)-4-hydroxy-2H-pyran-2-on;
3-(α-Cyclopropyl-meta-(N-α-tert-butyloxycarbonyl-N-im-p-toluolsulfonyl-L-histidylamino)benzyl)-6-(α-ethylphenethyl)-4-hydroxy-2H-pyran-2-on;
3-(α-Cyclopropyl-meta-(tert-butyloxycarbonyl-L-alanylamino)benzyl)-6-(α-ethylphenethyl)-4-hydroxy-2H-pyran-2-on;
3-(α-Cyclopropyl-meta-(4-bromphenylbenzoylamino)benzyl)-6-(α-ethylphenethyl)-4-hydroxy-2H-pyran-2-on;
3-(α-Cyclopropyl-meta-(N-α-tert-butyloxycarbonyl-L-histidylamino)benzyl)-6-(α-ethylphenethyl)-4-hydroxy-2H-pyran-2-on;
3-(α-Cyclopropyl-meta-(N-benzyloxycarbonyl-O-α-benzyl-L-glutamylamino)benzyl)-6-(α-ethylphenethyl)-4-hydroxy-2H-pyran-2-on;
3-(α-Cyclopropyl-meta-(4-cyanophenylbenzoylamino)benzyl)-6-(α-ethylphenethyl)-4-hydroxy-2H-pyran-2-on;
3-(α-Cyclopropyl-meta-(L-glutamylamino)benzyl)-6-(α-ethylphenethyl)-4-hydroxy-2H-pyran-2-on;
3-(α-Cyclopropyl-meta-(3-(1-indolyl)propanoylamino)benzyl)-6-(α-ethylphenethyl)-4-hydroxy-2H-pyran-2-on;
3-(α-Cyclopropyl-meta-(2-pyridylacetylamino)benzyl)-6-(α-ethylphenethyl)-4-hydroxy-2H-pyran-2-on;
3-(α-Cyclopropylbenzyl)-6-[1-cyclopropylmethyl-2-(tetrahydropyran-3-yl)ethyl]-4-hydroxy-2H-pyran-2-on;
3-(α-Cyclopropyl-meta-(3-pyridylacetylamino)benzyl)-6-(α-ethylphenethyl)-4-hydroxy-2H-pyran-2-on;
3-(α-Cyclopropyl-meta-(4-pyridylacetylamino)benzyl)-6-(α-ethylphenethyl)-4-hydroxy-2H-pyran-2-on;
3-(α-Cyclopropyl-meta-(4-chlorphenylsulfonylamino)benzyl)-6-(α-ethylphenethyl)-4-hydroxy-2H-pyran-2-on;
3-(α-Cyclopropyl-meta-(8-chinolinsulfonylamino)benzyl)-6-(α-ethylphenethyl)-4-hydroxy-2H-pyran-2-on;
3-(α-Cyclopropyl-meta-(ethylsulfonylamino)benzyl-6-[1-cyclopropylmethyl-2-(tetrahydropyran-3-yl)ethyl]-4-hydroxy-2H-pyran-2-on;
3-(α-Cyclopropyl-meta-(4-methylphenylsulfonylamino)benzyl-6-(α-ethylphenethyl)-4-hydroxy-2H-pyran-2-on;
3-(α-Cyclopropyl-meta-(4-methylphenylsulfonylamino)benzyl-6-[1-cyclopropylmethyl-2-(tetrahydropyran-3-yl)ethyl]-4-hydroxy-2H-pyran-2-on;
3-(α-Cyclopropyl-meta-(ethylsulfonylamino)benzyl-6-(α-ethyl-phenethyl)-4-hydroxy-2H-pyran-2-on;
(3S,6R)-3-(α-Ethylbenzyl)-6-(α-ethylphenethyl)-4-hydroxy-2H-pyran-2-on;
(3S,6S)-3-(α-Ethylbenzyl)-6-(α-ethylphenethyl)-4-hydroxy-2H-pyran-2-on;
Natrium-(3S,6R)-3-(α-ethylbenzyl)-6-(α-ethylphenethyl)-2H-pyran-2-on-4-oxid;
(3R,6R)-3-(α-Ethylbenzyl)-6-(α-ethylphenethyl)-4-hydroxy-2H-pyran-2-on und
(3R,6S)-3-(α-Ethylbenzyl)-6-(α-ethylphenethyl)-4-hydroxy-2H-pyran-2-on.

5. Verwendung nach Anspruch 4, wobei die Verbindung ausgewählt ist aus der Gruppe von:
3-(α-Ethylbenzyl)-6-(α-ethylphenethyl)-4-hydroxy-2H-pyran-2-on;
6-(1-Benzyl-propyl)-3-(cyclopropyl-phenyl-methyl)-4-hydroxy-2H-pyran-2-on;
3-(α-Ethylbenzyl)-6-(α-ethylphenmethyl)-4-hydroxy-2H-pyran-2-on;
3-(α-Ethylbenzyl)-6-phenethyl-4-hydroxy-2H-pyran-2-on und
4-Hydroxy-3-(1-phenylpropyl)-6-[(tetrahydro-2H-pyran-3-yl)methyl]propyl]-2H-pyran-2-on.

6. Verwendung nach Anspruch 5, wobei die Verbindung in einer Menge von 100 bis 400 mg/kg/Tag zu verabreichen ist.

## Revendications

1. Utilisation d'un composé de formule I 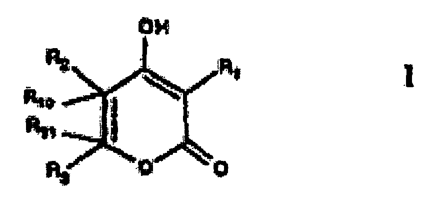 pour préparer un médicament destiné à la prévention ou au traitement de l'hypertrophie ou de l'hyperplasie prostatique bénigne, du cancer de la prostate, de l'alopécie, de l'hirsutisme, de l'acné vulgaire et de la séborrhée chez un mammifère nécessitant une telle prévention ou un tel traitement ;
formule dans laquelle R₁ représente un groupe
a) -(CH₂)ₙ-CH(R₅)-(CH₂)ₘ-R₄,
b) -CH(aryl)-CH [C(O)-O-(alkyle en C₁ à C₆)]₂,
c) -C(cycloalkyle en C₃ à C₅)-(CH₂)ₙ-R₄,
d) -C(aryle)=CH-aryle,
e) -CH(R₅)-S-(CH₂)ₘ-R₄, ou
f ) -(CH₂)ₚ-aryle ;
dans laquelle R₂ représente
a) un atome d'hydrogène,
b) un groupe halogéno,
c) un groupe (alkyle en C₁ à C₆)-[O-(CH₂)₂]_{q}-(CH₂)ₙ-,
d) un groupe alkyle en C₁ à C₆, ou
e) un groupe -(CH₂)ₙ-CH(R₅)-(CH₂)ₘ-R₄ ;
dans laquelle R₃ représente un groupe
a) alkyle en C₁ à C₁₀ facultativement substitué avec zéro (0) à cinq (5) substituants halogéno,
b) alcényle en C₂ à C₁₀,
C) R₄-(CH₂)ₘ-CH(R₆)-(CH₂)ₙ-,
d) R₄-(CH₂)ₚ-,
e) R₄-CH=CH-,
f) CH₂=CH-(CH₂)ₚ-,
g) R₄(CH₂)ₘX₁C(O)(CH₂)ₙ-,
h) R₄(CH₂)ₘC(O)X₁(CH₂)ₙ-,
i) aryle,
j) het,
k) cycloalkyle en C₃ à C₇,
l) (alkyle en C₁ à C₆)-O-C(O)-(CH₂)ₙ-,
m) (alkyle en C₁ à C₆)-[O-(CH₂)₂]_{q}-(CH₂)ₙ-,
n) R₄-CH(R₆)-CH(R₆)-,
p) R₁₂-(CH₂)ₘ-X₂-(CH₂)ₙ-(R₇)HC-, ou
q) R₁₅-(CH₂)ₘ-X₂-(CH₂)ₙ-(R₇)HC-;
dans laquelle R₄ représente un groupe
a) aryle,
b) het,
c) cycloalkyle en C₃ à C₇,
d) alcényle en C₂ à C₁₀,
e) (alkyle en C₁ à C₆)-[O-(CH₂)₂]_{q}-(CH₂)ₙ-,
f) halogéno,
g) het-O-,
h) het-C(O)-,
i) aryl-(CH₂)ₙ-O-C(O)-, ou
j) trifluorométhyle ;
dans laquelle R₅ représente un groupe
a) alkyle en C₁ à C₁₀,
b) alcényle en C₂ à C₁₀,
c) cycloalkyle en C₃ à C₇,
d) -(CH₂)ₙ-aryle,
e) -(CH₂)ₚ-het, ou
f) -(CH₂)ₙ-CH=CH-aryle ;
dans laquelle R₆ représente un groupe
a) alkyle en C₁ à C₁₀,
b) R₄-(alkyle en C₁ à C₅),
c) -(CH₂)ₙ-(cycloalkyle en C₃ à C₇),
d) -(CH₂)ₚ-CH=CH₂,
e) -(CH₂)ₚ-aryle,
f) -(CH₂)ₚ-het, ou
g) hydroxy- ;
dans laquelle X₁ représente un groupe -NR₇- ; dans laquelle R₇ représente
a) un atome d'hydrogène, ou
b) un groupe alkyle en C₁ à C₅;
où le groupe aryle est un groupe
a) phényle substitué avec zéro (0) à trois (3) substituants R₈,
b) naphtyle substitué avec zéro (0) à trois (3) substituants R₈,
e) biphényle substitué avec zéro (0) à trois (3) substituants R₈, ou
d) perhalogénophényle ;
où le groupe het est un noyau penta- ou hexagonal, saturé ou insaturé, contenant un (1) à quatre (4) hétéroatomes choisis dans le groupe consistant en azote, oxygène et soufre ; y compris n'importe quel groupe bicyclique dans lequel n'importe lequel des noyaux hétérocycliques précités est condensé avec un noyau benzénique, un groupe cycloalkyle en C₃ à C₈ ou un autre hétérocycle ; et, si cela est chimiquement possible, les atomes d'azote et de soufre peuvent être sous des formes oxydées ; et substituées avec zéro (0) à trois (3) substituants R₉ ;
les substituants R₈ et R₉ représentant indépendamment un groupe
a) alkyle en C₁ à C₈ substitué avec zéro (0) à trois (3) substituants halogéno,
b) alcényle en C₂ à C₈,
c) hydroxy,
d) hydroxyalkyle en C₁ à C₅,
e) -(CH₂)ₙ-O-(alkyle en C₁ à C₅) substitué avec zéro (0) à trois (3) substituants hydroxy,
f) -(CH₂)ₙ-O-(alcényle en C₂ à C₇) substitué avec zéro (0) à trois (3) substituants hydroxy,
g) halogéno,
h) amino,
i) amino-(alkyle en C₁ à C₅),
j) mono- ou di-(alkyle en C₁ à C₅)-amino,
k) -C (O) -alkyle en C₁ à C₅,
l) -CHO,
m) -COOH,
n) -COO- (alkyle en C₁ à C₅),
o) -CON(R₇)₂,
p) cycloalkyle en C₃ à C₇,
q) nitro,
r) -CN,
s) -SO₃H,
t ) -SO₂NH₂,
u) -O[(CH₂)₂-O]_{q}-CH₃,
v) -[CH₂-O]_{q}-(alkyle en C₁ à C₃),
w) -(CH₂)ₙ-NHC(O)-O-(CH₂)ₚ-R₁₂,
x) -(CH₂)ₙ-NHC(O)-O-(CH₂)ₚ-R₁₅,
y) -(CH₂)ₙ-R₁₂,
z) -SO₂-R₁₂,
a1) -(CH₂)ₙ-X₂-(CH₂)ₙ-R₁₂,
b1) -(CH₂)ₙ-X₂-(CH₂)ₙ-R₁₅,
c1) -(CH₂)ₙ-X₂-CH=CH-R₁₂,
d1) - (CH₂)ₙ-X₂-CH=CH-R₁₅,
e1) - (CH₂)ₙ-X₂-(alkyle en C₁ à C₁₀) substitué avec zéro (0) à trois (3) substituants halogéno,
f1) - (CH₂)ₙ-X₂- (alcényle en C₂ à C₅),
g1) -X₂-(CH₂)ₚ-CH(NH₂)(COOH),
h1) -NHCONH-SO₂-R₁₂,
i1) -X₂-(CH₂)ₚ-NH-C(O)-O-(alkyle en C₁ à C₆),
j1) -X₂-CH(X₃)-NH-C(O)-O-(alkyle en C₁ à C₆),
k1) -X₂-(CH₂)ₚ-CH[NH-C(O)-O-(CH₂)ₚ-R₁₂]-C(O)-O(CH₂)ₚ-R₁₂,
l1) -(CH₂)ₙ-X₄-N(alkyle en C₁ à C₃)₂,
m1) -(CH₂)ₙ-X₄-NHR₁₂,
n1) -NH-AA-P₁,
o1) -(CH₂)ₚ-N₃,
p1) -(CH₂)ₚ-R₁₂.
q1) -(CH₂)ₚ-R₁₅,
r1) -(CH₂)ₙ-NHC(SCH₃)=CHNO₂,
s1) -(CH₂)ₙ*-*NHC(NHR₇)=CHNO₂,
t1) -(CH₂)ₙ-NHO(SCH₃)=NCN, ou
u1) -(CH₂)ₙ-NHC(NHR₇)=NCN ;
où X₂ représente un groupe
a) -NH-C(O)-,
b) -NH-SO₂-,
c) -NH-C(O)-NH-, ou
d) -SO₂-NH- ;
où X₃ représente un groupe
a) alkyle en C₁ à C₆, ou
b) -(CH₂)ₚ-R₁₅ ;
où X₄ représente un groupe
a) -NH-C(O)-, ou
b) -NH-SO₂- ;
où R₁₀ représente un atome d'hydrogène ;
où R₁₁ représente
a) un atome d'hydrogène,
b) un groupe alkyle en C₁ à C₆,
c) un groupe -(CH₂)ₙ-aryle,
d) un groupe -(CH₂)ₙ-(cycloalkyle en C₃ à C₇), ou
e) un groupe -(CH₂)ₙ-het ;
ou bien dans laquelle R₁₀ et R₁₁, pris conjointement, forment une double liaison ;
ou bien dans laquelle R₃ et R₁₁, pris conjointement, forment
a) un groupe cycloalkyle en C₃ à C₈ substitué avec zéro (0) à trois (3) substituants hydroxy, =N-OH, =O(oxo), ou une de ses formes protégées, ou substitué en position α avec R₁₄ ; ou
b) un noyau penta- ou hexagonal saturé contenant un (1) ou deux (2) atomes d'oxygène ;
dans laquelle R₁₂ représente un groupe
a) phényle substitué avec zéro (0) à trois (3) substituant R₁₃, ou
b) naphtyle substitué avec zéro (0) à trois (3) substituants R₁₃ ;
dans laquelle R₁₃ représente un groupe
a) alkyle en C₁ à C₁₀ substitué avec zéro (0) à trois (3) substituants halogéno,
b) hydroxy,
c) hydroxy-(alkyle en C₁ à C₅),
d) -(CH₂)ₙ-O-(alkyle en C₁ à C₅) substitué avec zéro (0) à trois (3) substituants hydroxy ou halogéno,
e) -(CH₂)ₙ-O-(alcényle en C₂ à C₇) substitué avec zéro (0) à trois (3) substituants hydroxy ou halogène,
f) halogène,
g) amino,
h) amino-(alkyle en C₁ à C₅),
i) mono- ou di-(alkyle en C₁ à C₅)-amino,
j) -C(O) -alkyle en C₁ à C₅,
k) -CHO,
l) -COOH,
m) -CON(R₇)₂,
n) -NHCO-(alkyle en C₁ à C₃),
o) -NHOH,
p) nitro,
q) -CN,
r) -(CH₂)ₙ-phényle,
s) -COO-(alkyle en C₁ à C₅), ou
t) -SO₂-phényle substitué avec zéro (0) à trois (3) substituants alkyle en C₁ à C₅,
u) -(CH₂)ₙ-X₄-phényle, ou
v) -(CH₂)ₙ-N=N-phényle substitué avec zéro (0) ou un (1) substituant -N(alkyle en C₁ à C₃)₂ ;
dans laquelle R₁₄ représente un groupe
a) -(CH₂)ₙ-aryle,
b) -alkyle en C₁ à C₆, ou
c) -(CH₂)ₙ-cycloalkyle en C₄ à C₇;
dans laquelle R₁₅ représente un noyau penta- ou hexagonal, saturé ou insaturé, contenant un (1) à quatre (4) hétéroatomes choisis dans le groupe consistant en azote, oxygène et soufre ; y compris n'importe quel groupe bicyclique dans lequel n'importe lequel des noyaux hétérocycliques précités est condensé avec un noyau benzénique, un groupe cycloalkyle en C₃ à C₈ ou un autre hétérocycle ; et substitué avec zéro (0) à trois (3) substituants R₁₃ ;
dans laquelle AA représente un résidu aminoacide ;
dans laquelle P₁ représente un atome d'hydrogène ou un groupe protecteur de l'atome d'azote ;
dans laquelle m et n ont indépendamment une valeur de zéro (0) à cinq (5) inclus ;
dans laquelle p a une valeur de un (1) à cinq (5) inclus ;
dans laquelle q a une valeur de un (1) à cinq (5) inclus ; et
de ses sels pharmaceutiquement acceptables.

2. Utilisation suivant la revendication 1, dans laquelle le volume de la prostate chez un mammifère mâle est réduit ou maintenu.

3. Utilisation suivant la revendication 2,
dans laquelle R₁ représente un groupe -(CH₂)ₙ-CH(R₅)-(CH₂)ₘ-R₄ ;
dans laquelle R₂ représente un atome d'hydrogène ;
dans laquelle R₃ représente un groupe
a) R₄-(CH₂)ₘ-CH(R₆)-(CH₂)ₙ-, ou
b) R₄-(CH₂)ₚ-;
dans laquelle R₄ représente un groupe
a) phényle, ou
b) tétrahydropyrannyle ;
dans laquelle R₅ représente un groupe
a) propyle, ou
b) cyclopropyle ;
dans laquelle R₆ représente un groupe éthyle ;
dans laquelle m est égal à zéro (0) ou un (1) ;
dans laquelle n est égal à zéro (0) :
dans laquelle p est égal à deux (2) ;
dans laquelle R₁₀ et R₁₁, pris conjointement, forment une double liaison.

4. Utilisation suivant la revendication 1 ou la revendication 2, dans laquelle le composé est choisi dans le groupe consistant en :
3-(.alpha.-éthylbenzyl)-4-hydroxy-6-phényl-2H-pyranne-2-one;
6-benzyl-3-(.alpha.-éthylbenzyl)-4-hydroxy-2H-pyranne-2-one ;
3-(.alpha.-éthylbenzyl)-6-phénéthyl-4-hydroxy-2H-pyranne-2-one ;
4-hydroxy-6-phénéthyl-3-(.alpha.-propyl-p-bromobenzyl)-2H-pyranne-2-one ;
6-(p-bromophénéthyl)-3-(.alpha.-éthylbenzyl)-4-hydroxy-2H-pyranne-2-one ;
3-(.alpha.-éthylbenzyl)-6-(o-fluorophénéthyl)-4-hydroxy-2H-pyranne-2-one ;
3-(.alpha.-éthylbenzyl)-4-hydroxy-6-(3-phénylpropyl)-2H-pyranne-2-one ;
3-(.alpha.-éthylbenzyl)-4-hydroxy-6-propyl-2H-pyranne-2-one ;
3-(.alpha.-éthylbenzyl)-4-hydroxy-6-(3-butényl)-2H-pyranne-2-one ;
3-(.alpha.-éthylbenzyl)-4-hydroxy-6-[[(phénylamino)carbonyl]-méthyl]-2H-pyranne-2-one ;
4-hydroxy-6-phénéthyl-3-(.alpha.-vinylbenzyl)-2H-pyranne-2-one ;
3-(.alpha.-éthylbenzyl)-6-(.alpha.éthylphénéthyl)-4-hydroxy-2H-pyranne-2-one ;
3-(.alpha.-éthylbenzyl)-1-éthylpropyl-4-hydroxy-2H-pyranne-2-one ;
3-(.alpha.-éthylbenzyl)-4-hydroxy-6-(p-méthoxystyryl)-2H-pyranne-2-one ;
3-(.alpha.-éthylbenzyl)-4-hydroxy-6-(2-napht-2-yléthyl)-2H-pyranne-2-one ;
3-(.alpha.-éthylbenzyl)-4-hydroxy-6-(1-éthyl-2-napht-2-yléthyl)-2H-pyranne-2-one ;
3-(.alpha.-éthylbenzyl)-4-hydroxy-6-(2-napht-1-yléthyl)-2H-pyranne-2-one ;
3-(.alpha.-éthylbenzyl)-4-hydroxy-6-(1-éthyl-2-napht-1-yléthyl)-2H-pyranne-2-one ;
3-(.alpha.-cyclopropylbenzyl)-4-hydroxy-6-(3-phénylpropyl)-2H-pyranne-2-one ;
3-(.alpha.-cyclopropylbenzyl)-6-(1-éthylpropyl)-4-hydroxy-2H-pyranne-2-one ;
3-(.alpha.-cyclopropylbenzyl)-6-(.alpha.-benzylphénéthyl)-4-hydroxy-2H-pyranne-2-one ;
3-(.alpha.-cyclopropylbenzyl)-4-hydroxy-6-phénéthyl-2H-pyranne-2-one ;
3-(.alpha.-cyclopropylbenzyl)-6-(1-propylbutyl)-4-hydroxy-2H-pyranne-2-one ;
3-(.alpha.-cyclopropylbenzyl)-4-hydroxy-6-méthyl-2H-pyranne-2-one ;
4-hydroxy-6-méthyl-3-(3-phénylprop-2-ényl-2H-pyranne-2-one ;
3-[(4-hydroxy-2-oxo-6-phényl-2H-1-pyranne-3-yl) (4-nitrophényl)-1,3-propanedioate de diméthyle ;
3-[(4-hydroxy-2-oxo-6-phényl-2H-1-pyranne-3-yl)(3-nitrophényl)-1,3-propanedioate de diméthyle ;
6-éthyl-3-(α-éthylbenzyl)-6-phényltétrahydropyranne-2,4-dione ;
5,6-dihydro-4-hydroxy-6-cyclohexyl-6-phényl-3-[1-(3-hydroxyphényl)propyl]-2H-pyranne-2-one ;
sel de sodium de 4-hydroxy-1-oxa-3-(1-phénylpropyl)spiro-[5,5]-undéc-3-ène-2-one ;
6,6-diéthyl-3-(3-phénylpropyl)-tétrahydropyranne-2,4-dione;
dihydro-6-méthyl-6-phényl-3-(1-phényl-2-propényl)-2H-pyranne-2,4(3H)-dione ;
dihydro-3-(1-(3-hydroxyphényl)propyl-6-phényl-6-propyl-2H-pyranne-2,4(3H)-dione ;
5,6-dihydro-4-hydroxy-6-phényl-6-propyl-3-(1-phénylpropyl)-2H-pyranne-2-one ;
5,6-dihydro-4-hydroxy-6-phényl-6-propyl-3-[1-(3-hydroxyphényl)-propyl]-2H-pyranne-2-one ;
12-hydroxy-11-(1-phénylallyl)-1,4,9-trioxadispiro([4.2.5.2]pentadéc-11-ène-10-one ;
12-hydroxy-11-(1-phénylpropyl)-1,4,9-trioxadispiro[4.2.5.2]pentadéc-11-ène-10-one ;
4-hydroxy-3-(1-phénylpropyl)-1-oxaspiro[5.5]undéc-3-ène-2,9-dione;
6,6-dibenzyl-4-hydroxy-3-(1-phénylpropyl)-5,6-dihydropyranne-2-one;
4-hydroxy-3-(1-phénylallyl)-1,9-dioxaspiro[5.5]undéc-3-ène-2-one ;
4,9-dihydroxy-3-(1-phénylpropyl)-1-oxaspiro[5,5]undéc-3-ène-2-one ;
N-(3-cyclopropyl-[6-(1-éthylphénéthyl)-4-hydroxy-2-oxo-5,6-dihydro-2H-pyranne-3-yl]-méthyl)-phényl)-3-(tertiobutyloxycarbonyl-amino)-propionamide ;
N-(3-{cyclopropyl-[6-(2-cyclopropyl-1-cyclopropylméthyléthyl)-4-hydxoxy-2-oxo-5,6-dihydro-2H-pyranne-3-yl}-méthyl)-phényl)-3-indole-1-yl-propionamide ;
3-(cyclopropylphénylméthyl)-4-hydroxy-6-(1-(tétrahydrofuranne-3-ylméthyl)-propyl)-pyranne-2-one ;
6-(1-(5-chlorothiophène-2-ylméthyl)-propyl)-3-(cyclopropylphénylméthyl)-4-hydroxypyranne-2-one ;
3-(cyclopropylphénylméthyl)-6-(1-(3,5-diméthylisoxazole-4-ylméthyl)-propyl)-4-hydroxypyranne-2-one ;
3-(cyclopropylphénylméthyl)-4-hydroxy-6-(1-(tétrahydrofuranne-2-ylméthyl)-propyl)-pyranne-2-one;
3-(cyclopropylphénylméthyl)-4-hydroxy-6-(1-thiophène-2-ylméthylpropyl)-pyranne-2-one ;
3-(cyclopropylphénylméthyl)-4-hydroxy-6-(1-tétrahydropyranne-4-ylméthyl)-propyl)-pyranne-2-one ;
3-(cyclopropylphénylméthyl)-6-(1-furanne-2-ylméthylpropyl)-4-hydroxypyranne-2-one ;
3-(cyclopropylphénylméthyl)-6-(1-(1,3)-dioxolanne-2-ylméthyl-propyl)-4-hydroxypyranne-2-one ;
3-(cyclopropylphénylméthyl)-4-hydroxy-6-(1-(tétrahydropyranne-3-ylméthyl)-propyl)-pyranne-2-one ;
3-(cyclopropylphénylméthyl)-4-hydroxy-6-(1-(tétrahydropyranne-2-ylméthyl)-propyl)-pyranne-2-one;
3-(cyclopropylphénylméthyl)-4-hydroxy-6-(1-pyridine-2-ylméthylpropyl)-pyranne-2-one ;
6-(4-chloro-1-éthylbutyl)-3-(cyclopropylphénylméthyl)-4-hydroxypyranne-2-one ;
6-(3-chloro-1-éthylpropyl)-3-(cyclopropylphénylméthyl)-4-hydroxypyranne-2-one ;
3-(cyclopropylphénylméthyl)-6-[éthyl-3-(tétrahydropyranne-2-yloxy)-propyl]-4-hydroxypyranne-2-one;
3-(cyclopropylphénylméthy)-4-hydroxy-6-(1-pyridine-3-ylméthylpropyl)-pyranne-2-one ;
3-(cyclopropylphénylméthyl)-6-(1-éthyl-3-thiophène-3-ylpropyl)-4-hydroxypyranne-2-one ;
3-(cyclopropylphénylméthyl)-4-hydroxy-6-[1-(tétrahydropyranne-3-ylméthyl)-butyl]-pyranne-2-one ;
5-bromo-6-(2-cyclopropylcyclopropylméthyléthyl)-4-hydroxy-3-(1-phénylpropyl)-pyranne-2-one ;
3-(1-benzyl-2-phényléthyl)-6-(2-cyclopropyl-1-cyclopropyl-méthyléthyl)-4-hydroxypyranne-2-one ;
6-(2-cyclopropylméthyléthyl)-3-(cyclopropylphénylméthyl)-4-hydroxypyranne-2-one ;
6-(1-allylbut-3-ényl)-3-(cyclopropylphénylméthyl)-4-hydroxy-pyranne-2-one ;
3-(cyclopropylphénylméthyl)-6-(1-éthyl-3-(2-méthoxy-éthoxy)-propyl)-4-hydroxypyranne-2-one ;
6-(1-benzyl-3-(2-méthoxy-éthoxy)-propyl)-3-(cyclopropylphénylméthyl)-4-hydroxypyranne-2-one ;
3-(α-cyclopropyl-*méta*-(benzyloxycarbonylamino)benzyl)-6-(α-éthylphénéthyl)-4-hydroxy-2H-pyranne-2-one ;
3-(α-cyclopropyl-*méta*-(*tertio*-butyloxycarbonylamino)benzyl)-6-(α-cyclopropylméthylcyclopropyléthyl)-4-hydroxy-2H-pyranne-2-one ;
4-hydroxy-3-(1-phénylpropyl)-6-(1-propylbutyl)-pyranne-2-one ;
4-hydroxy-3-(1-phénylallyl)-6-(1-propylbutyl)-pyranne-2-one ;
ester tertiobutylique d'acide 3-(5-(cyclopropylphénylméthyl)-4-hydroxy-6-oxo-6H-pyranne-2-yl)-propionique ;
3-(cyclopropylphénylméthyl)-6-(2-(3,5-diméthyl-isoxazole-4-yl)-éthyl)-4-hydroxypyranne-2-one ;
6-(2-(5-tertiobutyl-(1,2,4-oxadiazole-3-yl)éthyl)-3-(cyclopropyl-phénylméthyl)-4-hydroxypyranne-2-one ;
3-(cyclopropylphénylméthyl)-4-hydroxy-6-(2-phényl-1-pyridine-2-ylméthyléthyl)-pyranne-2-one ;
3-(cyclopropylphénylméthyl)-6-(2-(1,3)dioxolanne-2-yléthyl)-4-hydroxypyranne-2-one ;
3-(cyclopropylphénylméthyl)-4-hydroxy-6-(4-morpholine-4-ylbutyl)-pyranne-2-one ;
3-(cyclopropylphénylméthyl)-4-hydroxy-6-(2-pyridine-2-yléthyl)-pyranne-2-one ;
3-(cyclopropylphénylméthyl)-4-hydroxy-6-(2-(2-méthyl-thiazole-4-yl)-éthyl)-pyranne-2-one ;
3-(cyclopropylphénylméthyl)-4-hydroxy-6-(2-quinoléine-2-yléthyl)-pyranne-2-one;
6-(3-chloropropyl)3-(cyclopropylphénylméthyl)-4-hydroxypyranne-2-one ;
6-(1-(2-(4-chlorophényl)-thiazole-4-ylméthyl)-propyl)-3-(cyclopropylphénylméthyl)-4-hydroxypyranne-2-one ;
3-(cyclopropylphénylméthyl)-6-(3-(1,3-dioxanne-2-yl-1-éthylpropyl)-4-hydroxypyranne-2-one ;
3-(cyclopropylphénylméthyl)-4-hydroxy-6-(1-pyridine-4-ylméthylpropyl)-pyranne-2-one;
3-(cyclopropylphénylméthyl)-6-(1-éthyl-3-morpholine-4-yl-3-oxopropyl)-4-hydroxypyranne-2-one ;
3-(cyclopropylphénylméthyl)-6-(1-éthyl-4-morpholine-4-ylbutyl)-4-hydroxypyranne-2-one ;
3 -(cyclopropylphénylméthyl)-6-[1-(2,3-dihydrobenzo[1,4 ]dioxine-2-ylméthyl)-propyl]-4-hydroxypyranne-2-one ;
3-(cyclopropylphénylméthyl)-4-hydroxy-6-isobutyl-pyranne-2-one ;
(cyclopropylphénylméthyl)-6-[1-(5,6-dihydro-2H-pyranne-3-éthyl)-propyl-4-hydroxypyranne-2-one ;
3-(cyclopropylphénylméthyl)-4-hydroxy-5-(2-(2-méthoxy-éthoxy)-éthyl)-6-propylpyranne-2-one ;
3-(cyclopropylphénylméthyl)-4-hydroxy-6-(2-isobutyl-3-méthylbutyl)-pyranne-2-one ;
3-dicyclopropylméthyl-4-hydroxy-6-phénéthylpyranne-2-one ;
6-(1-cyclopropyléthyl)-3-dicyclopropylméthyl-4-hydroxypyranne-2-one ;
6-(1-cyclopropyl-1-cyclopropylméthyléthyl)-3-dicyclopropylméthyl-4-hydroxypyranne-2-one ;
6-(1-cyclohexylméthylpropyl)-3-(cyclopropylphénylméthyl)-4-hydroxypyranne-2-one ;
6-(1-benzylpropyl)-3-(cyclopropylphénylméthyl)-4-hydroxypyranne-2-one ;
6-(2-cyclopropyl-1-cyclopropylméthyléthyl)-4-hydroxy-3(1-phénylpropyl)-pyranne-2-one ;
6-(1-benzyl-2-cyclopropyléthyl)-3-(cyclopropylphénylméthyl)-4-hydroxypyranne-2-one ;
6-(2-cyclopropyléthyl)-3-(cyclopropylphénylméthyl)-4-hydroxypyranne-2-one ;
6-(1-cyclopropylméthylpropyl)-3-(cyclopropylphénylméthyl)-4-hydroxypyranne-2-one ;
3-(cyclopropylphénylméthyl)-4-hydroxy-6-(4-phénylbutyl)-pyranne-2-one ;
3-(cyclohexylcyclopropylméthyl)-6-(2-cyclopropyl-1-cyclopropylméthyléthyl)-4-hydroxypyranne-2-one ;
3-cyclopropylphénylméthyl)-6-(2-éthyl-4-phénylbutyl)-4-hydroxypyranne-2-one ;
6-(3-cyclohexylpropyl)-3-(cyclopropylphénylméthyl)-4-hydroxypyranne-2-one ;
6-(3-cyclohexyl-1-éthylpropyl)-3-(cyclopropylphénylméthyl)-4-hydroxypyranne-2-one ;
6-(2-cyclopropyléthyl)-4-hydroxy-3-(1-phénylpropyl)-pyranne-2-one ;
6-but-3-ényl-3-(cyclopropylphénylméthyl)-4-hydroxypyranne-2-one ;
3-(cyclopropylphénylméthyl)-6-(1-éthyl-3-phénylpropyl)-4-hydroxypyranne-2-one ;
5-bromo-6-(2-cyclopropyléthyl)-4-hydroxy-3-(1-phénylpropyl)-pyranne-2-one ;
6-(1-benzyl-2-phényléthyl)-4-hydroxy-3-(1-phénylpropyl)-pyranne-2-one ;
3-(cyclopropylphénylméthyl)-4-hydroxy-6-(3-(2-méthoxy-éthoxy)-propyl)-pyranne-2-one ;
3-(cyclopropylphénylméthyl)-4-hydroxy-5-(2-(2-méthoxy-éthoxy)-éthyl)-6-(3-(2-méthoxy-éthoxy)-pyranne-2-one ;
3-(cyclopropylphénylméthyl)-4-hydroxy-6-propylpyranne-2-one ;
5-bromo-4-hydroxy-6-phényléthyl-3(1-phénylpropyl)-pyranne-2-one;
3-(cyclopropylphénylméthyl)-4-hydroxy-6-(3-(2-méthoxy-éthoxy)-éthoxy)-propyl)-pyranne-2-one ;
5-bromo-4-hydroxy-3-(1-phénylpropyl)-6-propylpyranne-2-one ;
3-(cyclopropylphénylméthyl)-6-(1-éthylpropyl)-4-hydroxy-5-(2-(2-méthoxy-éthoxy)éthyl)-pyranne-2-one ;
6-(1-benzylpropyl)-5-bromo-4-hydroxy-3-(1-phénylpropyl)-pyranne-2-one ;
6-(2-cyclopropyl-1-cyclopropylméthyléthyl)-3-(cyclopropylphénylméthyl)-4-hydroxy-5-(2-(2-méthoxy-éthoxy)-éthoxy)-éthyl)-pyranne-2-one ;
3-(cyclopropylphénylméthyl)-6-(2-furanne-2-yl-2-hydroxyéthyl)-4-hydroxypyranne-2-one ;
3-(cyclopropylphénylméthyl)-4-hydroxy-6-(4,4,4-trifluorobutyl)-pyranne-2-one ;
6-[2-(1-cyclohexyl-1H-tétrazole-5-yl)-éthyl]-3-(cyclopropylphénylméthyl)-4-hydroxy-2-one ;
monooxime de 4-hydroxy-3-(2-phénylpropyl)-1-oxaspiro[5.5]undéc-3-éne-2,9-dione ;
5,6-dihydro-4-hydroxy-6-phényl-6-(phénylméthyl)-3-(1-phényl-2-propényl)-2H-pyranne-2-one ;
5,6-dihydro-4-hydroxy-6-phényl-6-(phénylméthyl)-3-(1-phénylpropyl)-2H-pyranne-2-one ;
3-(1,3-diphényl-2-propényl)-5,6-dihydro-4-hydroxy-6-(2-phényléthyl)-6-propyl-, (E)-2H-pyranne-2-one ;
3-(1,3-diphényl-2-propyl)-5,6-dihydro-4-hydroxy-6-(2-phényléthyl)-6-propyl-, (E)-2H-pyranne-2-one ;
3-(1,3-diphényl-2-propényl)-5,6-dihydro-4-hydroxy-6-phényl-6-(phénylméthyl)-2H-pyranne-2-one;
3-(1,2-diphényléthényl)-5,6-dihydro-4-hydroxy-6-(2-phényléthyl)-6-propyl-, (E)-2H-pyranne-2-one ;
5,6-dihydro-4-hydroxy-6-(2-phényléthyl)-3-(1-phényl-2-propényl)-6-propyl-2H-pyranne-2-one ;
5,6-dihydro-4-hydroxy-6-(2-phényléthyl)-3-(1-phénylpropyl)-6-propyl-2H-pyranne-2-one ;
3-(1,3-diphényl-2-propényl)-5,6-dihydro-4-hydroxy-6-(phénylméthyl)-6-propyl-2H-pyranne-2-one ;
3-(1,3-diphénylpropyl)-5,6-dihydro-4-hydroxy-6-(phénylméthyl)-6-propyl-2H-pyranne-2-one ;
4-hydroxy-3-(1-phénylallyl)-1-oxaspiro[5.6]-dodéc-3-ène-2-one ;
4-hydroxy-3-(1-phénylallyl)-1-oxaspiro[5.4]-déc-3-ène-2-one ;
7-benzyl-4-hydroxy-3-(1-phénylallyl)-1-oxaspiro[5.5]undéc-3-ène-2-one ;
4-hydroxy-3-(1-phénylpropyl)-1-oxaspiro[5.4]-déc-3-ène-2-one ;
4-hydroxy-3-(1-phényl-2-propényl)-1-oxaspiro[5.7]tridéc-3-ène-2-one ;
4-hydroxy-3-(1-phénylpropyl)-1-oxaspiro[5.7]tridéc-3-ène-2-one ;
3-(α-cyclopropylbenzyl)-4-hydroxy-6-(α-éthyl-β-hydroxyphénéthyl)-2H-pyranne-2-one;
3-(α-cyclopropylbenzyl)-4-hydroxy-6-(β-hydroxy-p-méthylphénéthyl)-2H-pyranne-2-one ;
3-(α-cyclopropylbenzyl)-4-hydroxy-6-(β-hydroxy-p-fluorophénéthyl)-2H-pyranne-2-one ;
3-(α-cyclopropylbenzyl)-4-hydroxy-6-(β-hydroxy-p-chlorophénétyl)-2H-pyranne-2-one ;
3-(α-cyclopropylbenzyl)-4-hydroxy-6-(β-hydroxy-m-chlorophénéthyl)-2H-pyranne-2-one ;
3-(α-cyclopropylbenzyl)-4-hydroxy-6-(β-hydroxy-o-chlorophénéthyl)-2H-pyranne-2-one ;
3-(α-cyclopropylbenzyl)-4-hydroxy-6-(2-(furanne-3-yl)-2-hydroxyéthyl)-2H-pyranne-2-one ;
3-(α-cyclopropylbenzyl)-4-hydroxy-6-(2-(thiophène-3-yl)-2-hydroxyéthyl)-2H-pyranne-2-one ;
3-(α-cyclopropylbenzyl)-4-hydroxy-6-(α-éthyl-p-fluorophénéthyl)-2H-pyranne-2-one ;
3-(α-cyclopropylbenzyl)-4-hydroxy-6-(α-éthyl-p-chlorophénéthyl)-2H-pyranne-2-one ;
3-(α-cyclopropylbenzyl)-4-hydroxy-6-(α-éthyl-m-chlorophénéthyl)-2H-pyranne-2-one ;
3-(α-cyclopropylbenzyl)-4-hydroxy-6-(α-éthyl-o-chlorophénéthyl)-2H-pyranne-2-one ;
3-(α-cyclopropylbenzyl)-4-hydroxy-6-(α-éthyl-p-bromophénéthyl)-2H-pyranne-2-one ;
3-(α-cyclopropylbenzyl)-4-hydroxy-6-(α-éthyl-m-bromophénéthyl)-2H-pyranne-2-one ;
3-(α-cyclopropylbenzyl)-4-hydroxy-6-(α-éthyl-p-trifluorométhylphénéthyl)-2H-pyranne-2-one ;
3-(α-cyclopropylbenzyl)-4-hydroxy-6-(α-éthyl-m-trifluorométhylphénéthyl)-2H-pyranne-2-one ;
3-(α-cyclopropylbenzyl)-4-hydroxy-6-(α-éthyl-o-trifluorométhylphénéthyl)-2H-pyranne-2-one ;
3-(α-cyclopropylbenzyl)-4-hydroxy-6-(α-éthyl-p-méthoxyphénéthyl)-2H-pyranne-2-one ;
3-(α-cyclopropylbenzyl)-4-hydroxy-6-(α-éthyl-m-méthoxyphénéthyl)-2H-pyranne-2-one ;
3-(α-cyclopropylbenzyl)-4-hydroxy-6-(p-fluorophénéthyl)-2H-pyranne-2-one ;
3-(α-cyclopropylbenzyl)-4-hydroxy-6-(p-chlorophénéthyl)-2H-pyranne-2-one ;
3- (α-cyclopropylbenzyl)-4-hydroxy-6-(p-bromophénéthyl)-2H-pyranne-2-one ;
3-(α-cyclopropylphénylméthyl)-6-[1-éthyl-3-(4-morpholinyl)-propyl]-4-hydroxy-2H-pyranne-2-one ;
acide 3-(cyclopropylphénylméthyl)-β-éthyl-4-hydroxy-2-oxo-, ester phénylméthylique, acide 2H-pyranne-6-propanoique ;
3-(cyclopropylphénylméthyl)-4-hydroxy-6-[2-méthyl-1-(phénylméthyl)propyl]-2H-pyranne-2-one ;
3-(cyclopropylphénylméthyl)-4-hydroxy-6-[2-méthyl-1-[(tétrahydro-2H-pyranne-3-yl)méthyl]propyl]-2H-pyranne-2-one ;
4-hydroxy-3-(1-phénylpropyl)-6-[1-[(tétrahydro-2H-pyranne-3-yl)méthyl]propyl]-2H-pyranne-2-one ;
3-(cyclopropylphénylméthyl)-6-(1-éthyl-4,4,4-trifluorobutyl)-4-hydroxy-2H-pyranne-2-one ;
3-[2-[3-(cyclopropylphénylméthyl)-4-hydroxy-2-oxo-2H-pyranne-6-yl]butyl]-1-[(4-méthylphényl)sulfonyl]-pipéridine;
2-[2-[3-(cyclopropylphénylméthyl)-4-hydroxy-2-oxo-2H-pyranne-6-yl]butyl]-1-[(4-méthylphényl)sulfonyl]-pyrrolidine ;
3-(cyclopropylphénylméthyl)-4-hydroxy-6-(3,3,3-trifluoropropyl)-2H-pyranne-2-one ;
2-[2-[3-(cyclopropylphénylméthyl)-4-hydroxy-2-oxo-2H-pyranne-6-yl]butyl]-1-[(4-méthylphényl)sulfonyl]-pipéridine ;
4-[2-[3-(cyclopropylphénylméthyl)-4-hydroxy-2-oxo-2H-pyranne-6-yl]butyl]-1-[(4-méthylphényl)sulfonyl]-pipéridine;
4-[2-[3-(cyclopropylphénylméthyl)-4-hydroxy-2-oxo-2H-pyranne-6-yl]butyl]-1-(phénylméthyl)-2-pyrrolidinone ;
6-(cyclopentylméthyl)-3-(cyclopropylphénylméthyl)-4-hydroxy-2H-pyranne-2-one ;
3-(cyclopropylphénylméthyl)-4-hydroxy-6-[(tétrahydro-2H-pyranne-4-yl)méthyl]-2H-pyranne-2-one ;
3-(cyclopropylphénylméthyl)-6-(3-fluoropropyl)-4-hydroxy-2H-pyranne-2-one ;
4-hydroxy-3-(1-phénylcyclobutyl)-6-[1-(phénylméthyl)-propyl]-2H-pyranne-2-one ;
3-(α-éthylbenzyl)-6-(α-éthylbenzyl)-4-hydroxy-2H-pyranne-2-one ;
3-(α-cyclopropyl-*méta*-(phénylsulfonylamino)benzyl)-6-(α-éthylphénéthyl)-4-hydroxy-2H-pyranne-2-one ;
3-(α-cyclopropyl-*méta*-(propylsulfonylamino)benzyl)-6-(α-éthylphénéthyl)-4-hydroxy-2H-pyranne-2-one;
3-(α-cyclopropyl-*méta*-((E)-2-phényléthénylsulfonylamino)benzyl)-6-(α-éthylphénéthyl)-4-hydroxy-2H-pyranne-2-one;
3-(α-cyclopropyl-*méta*-(4-bromophénylsulfonylamino)benzyl)-6-(α-éthylphénéthyl)-4-hydroxy-2H-pyranne-2-one;
3-(α-cyclopropyl-*méta*-(2,5-dichlorophénylsulfonylamino)benzyl)-6-(α-éthylphénéthyl)-4-hydroxy-2H-pyranne-2-one ;
3-(α-cyclopropyl-*méta*-(4-tertiobutylphénylsulfonylamino)benzyl)-6-(α-éthylphénéthyl)-4-hydroxy-2H-pyranne-2-one ;
3-(α-cyclopropyl-*méta*-(4-cyanophénylsulfonylamino)benzyl)-6-(α-éthylphénéthyl)-4-hydroxy-2H-pyranne-2-one ;
3-(α-cyclopropyl-*méta*-(4-méthoxyphénylsulfonylamino)benzyl)-6-(α-éthylphénéthyl)-4-hydroxy-2H-pyranne-2-one ;
6-butyl-5,6-dihydro-3-(1,3-diphényl-2-propényl)-4-hydroxy-6-phénylméthyl-2H-pyranne-2-one ;
6-butyl-5,6-dihydro-3-(1,3-diphénylpropyl)-4-hydroxy-6-phénylméthyl-2H-pyranne-2-one ;
6-butyl-5,6-dihydro-4-hydroxy-6-phénylméthyl-3-(1-phényl-2-propényl)-2H-pyranne-2-one ;
6-butyl-5,6-dihydro-4-hydroxy-6-phénylméthyl-3-(1-phénylpropyl)-2H-pyranne-2-one ;
3-(α-cyclopropyl-[5-(méthoxyméthoxyméthyl)-thiophène-2-yl]-méthyl)-5,6-dihydro-4-hydroxy-6-(2-méthylpropyl)-6-(2-phényléthyl)-2H-pyranne-2-one ;
5,6-dihydro-4-hydroxy-6-(2-méthylpropyl)-6-(2-phényléthyl)-3-(1-phényl-2-propényl)-2H-pyranne-2-one ;
5,6-dihydro-4-hydroxy-6-(2-méthylpropyl)-6-(2-phényléthyl)-3-(1-phénylpropyl)-2H-pyranne-2-one ;
5,6-dihydro-3-diphénylméthyl-4-hydroxy-6,6-di-(2-phényléthyl)-2H-pyranne-2-one ;
5,6-dihydro-4-hydroxy-6,6-di-(2-phényléthyl)-3-(1,3-diphényl-2-propényl)-2H-pyranne-2-one ;
5,6-dihydro-4-hydroxy-6,6-di-(2-phényléthyl)-3-(1,3-diphénylpropyl)-2H-pyranne-2-one ;
5,6-dihydro-4-hydroxy-3-(1,3-diphényl-2-propényl)-6-(3-phénylpropyl)-6-propyl-2H-pyranne-2-one ;
5,6-dihydro-4-hydroxy-3-(1,3-diphénylpropyl)-6-(3-phénylpropyl)-6-propyl-2H-pyranne-2-one ;
3-(α-cyclopropyl-[5-méthoxyméthoxyméthyl)-thiophène-2-yl]-méthyl-5,6-dihydro-4-hydroxy-6-(2-phényléthyl)-6-propyl-2H-pyranne-2-one ;
3-(α-cyclopropyl-[5-(méthoxyméthoxyméthyl)-thiophène-2-yl]-méthyl)-5,6-dihydro-4-hydroxy-6-(3-phénylpropyl)-6-propyl-2H-pyranne-2-one ;
5,6-dihydro-3-diphénylméthyl-4-hydroxy-6-(3-phénylpropyl)-6-propyl-2H-pyranne-2-one ;
3-diphénylméthyl-5,6-dihydro-4-hydroxy-6-(2-méthylpropyl)-6-(2-phényléthyl)-2H-pyranne-2-one ;
3-(1,3-diphényl-2-propényl)-5,6-dihydro-4-hydroxy-6-(2-méthylpropyl)-6-(2-phényléthyl)-2H-pyranne-2-one ;
3-(1,3-diphényl-2-propyl)-5,6-dihydro-4-hydroxy-6-(2-méthylpropyl)-6-(2-phényléthyl)-2H-pyranne-2-one ;
3-(α-cyclopropyl-*méta*-[(phénylaminocarbonyl)amino]-benzyl)-6-(α-éthylphénéthyl)-4-hydroxy-2H-pyranne-2-one ;
3-(α-cyclopropyl-*méta*-[(4-méthoxyphénylaminocarbonyl)-amino]benzyl)-6-(α-éthylphénéthyl)-4-hydroxy-2H-pyranne-2-one ;
3-(α-cyclopropyl-*méta*-[(benzylaminocarbonyl)amino]-benzyl)-6-(α-éthylphénéthyl)-4-hydroxy-2H-pyranne-2-one ;
3-(α-cyclopropyl-*méta*-[(4-bromophénylaminocarbonyl)-amino]benzyl)-6-(α-éthylphénéthyl)-4-hydroxy-2H-pyranne-2-one ;
3-(α-cyclopropyl-*méta*-[(phénylsulfonylaminocarbonyl)-amino]benzyl)-6-(α-éthylphénéthyl)-4-hydroxy-2H-pyranne-2-one ;
3-(α-cyclopropyl-*méta*-(N-α-*tertio*-butyloxycarbonyl-N-im-p-toluènesulfonyl-L-histidylamino)benzyl)-6-(α-éthylphénéthyl)-4-hydroxy-2H-pyranne-2-one ;
3-(α-cyclopropyl-*méta*-(*tertio*-butyloxycarbonyl-L-alanylamino)benzyl)-6-(α-éthylphénéthyl)-4-hydroxy-2H-pyranne-2-one ;
3-(α-cyclopropyl-*méta*-(4-bromophénylbenzoylamino)benzyl)-6-(α-éthylphénéthyl)-4-hydroxy-2H-pyranne-2-one ;
3-(α-cyclopropyl-*méta*-(N-α-*tertio*-butyloxycarbonyl-L-histidylamino)benzyl)-6-(α-éthylphénéthyl)-4-hydroxy-2H-pyranne-2-one ;
3-(α-cyclopropyl-*méta*-(N-benzyloxycarbonyl-O-α-benzyl-L-glutamylamino)benzyl)-6-(α-éthylphénéthyl)-4-hydroxy-2H-pyranne-2-one ;
3-(α-cyclopropyl-*méta*-(4-cyanophénylbenzoylamino)benzyl)-6-(α-éthylphénéthyl)-4-hydroxy-2H-pyranne-2-one ;
3-(α-cyclopropyl-*méta*-(L-glutamylamino)benzyl-6-(α-éthylphénéthyl)-4-hydroxy-2H-pyranne-2-one ;
3-(α-cyclopropyl-*méta*-(3-(1-indolyl)propanoylamino)benzyl)-6-(α-éthylphénéthyl)-4-hydroxy-2H-pyranne-2-one ;
3-(α-cyclopropyl-*méta*-(2-pyridylacétylamino)benzyl)-6-(α-éthylphénéthyl)-4-hydroxy-2H-pyranne-2-one ;
3-(α-cyclopropylbenzyl)-6-[1-cyclopropylméthyl-2-(tétrahydropyranne-3-yl)éthyl]-4-hydroxy-2H-pyranne-2-one ;
3-(α-cyclopropyl-*méta*-(3-pyridylacétylamino)benzyl)-6-(α-éthylphénéthyl)-4-hydroxy-2H-pyranne-2-one ;
3-(α-cyclopropyl-*méta*-(4-pyridylacétylamino)benzyl)-6-(α-éthylphénéthyl)-4-hydroxy-2H-pyranne-2-one ;
3-(α-cyclopropyl-*méta-*(4-chlorophénylsulfonylamino)benzyl)-6-(α-éthylphénéthyl)-4-hydroxy-2H-pyranne-2-one ;
3-(α-cyclopropyl-*méta*-(8-quinoléinesulfonylamino)benzyl)-6-(α-éthylphénéthyl)-4-hydroxy-2H-pyranne-2-one ;
3-(α-cyclopropyl-*méta*-(éthylsulfonylamino)benzyl)-6-[1-cyclopropylméthyl-2-(tétrahydropyranne-3-yl)éthyl]-4-hydroxy-2H-pyranne-2-one ;
3-(α-cyclopropyl-*méta*-(4-méthylphénylsulfonylamino)benzyl)-6-(α-éthylphénéthyl)-4-hydroxy-2H-pyranne-2-one ;
3-(α-cyclopropyl-*méta*-(4-méthylphénylsulfonylamino)benzyl)-6-[1-cyclopropylméthyl-2-(tétrahydropyranne-3-yl)éthyl]-4-hydroxy-2H-pyranne-2-one ;
3-(α-cyclopropyl-*méta*-(éthylsulfonylamino)benzyl)-6-(α-éthylphénéthyl)-4-hydroxy-2H-pyranne-2-one ;
(3S,6R) 3-(α-éthylbenzyl)-6-(α-éthylphénéthyl)-4-hydroxy-2H-pyranne-2-one ;
(3S,6S) 3-(α-éthylbenzyl)-6-(α-éthylphénéthyl)-4-hydroxy-2H-pyranne-2-one ;
sel de sodium de 4-oxyde de (3S,6R) 3-(α-éthylbenzyl)-6-(α-éthylphénéthyl)-2H-pyranne-2-one;
(3R,6R) 3-(α-éthylbenzyl)-6-(α-éthylphénéthyl)-4-hydroxy-2H-pyranne-2-one ; et
(3R,6S) 3-(α-éthylbenzyl)-6-(α-éthylphénéthyl)-4-hydroxy-2H-pyranne-2-one;

5. Utilisation suivant la revendication 4, dans laquelle le composé est choisi dans le groupe consistant en :
3-(α-éthylbenzyl)-6-(α-éthylphénéthyl)-4-hydroxy-2H-pyranne-2-one ;
6-(1-benzylpropyl)-3-(cyclopropylphénylméthyl)-4-hydroxypyranne-2-one ;
3-(α-éthylbenzyl)-6-(α-éthylphenméthyl)-4-hydroxy-2H-pyranne-2-one ;
3-(α-éthylbenzyl)-6-phénéthyl-4-hydroxy-2H-pyranne-2-one ; et
4-hydroxy-3-(1-phénylpropyl)-6-[(tétrahydro-2H-pyranne-3-yl)méthyl]propyl]-2H-pyranne-2-one.

6. Utilisation suivant la revendication 5, dans laquelle le composé est destiné à être administré en une quantité de 100 à 400 mg/kg/jour.
